# EUROPÄISCHE PATENTSCHRIFT

(11) **EP 2 582 695 B1**
(45) Veröffentlichungstag und Bekanntmachung des Hinweises auf die Patenterteilung: **30.09.2015**
(21) Anmeldenummer: 11726115.6
(22) Anmeldetag: 10.06.2011
(51) Int. Cl.: C07D 403/06, C07D 405/14, C07D 409/14, C07D 417/14, A01N 43/78, A01N 43/713, A01N 43/653, A01N 43/56, A01N 43/707, C07D 401/14, C07D 403/14

(54) **ANTHRANILSÄUREDIAMID-DERIVATE MIT ZYKLISCHEN SEITENKETTEN**
ANTHRANILIC ACID DIAMIDE DERIVATIVES WITH CYCLICAL SIDE CHAINS
DÉRIVÉS D'ACIDE ANTHRANILIQUE DOTÉS DE CHAÎNES LATÉRALES CYCLIQUES

(30) Priorität: 15.06.2010 US 354899 P; 15.06.2010 EP 10166063
(43) Veröffentlichungstag der Anmeldung: 24.04.2013
(73) Patentinhaber: Bayer Intellectual Property GmbH, 40789 Monheim (DE)
(72) Erfinder: FISCHER, Rüdiger, 50259 Pulheim (DE); GRONDAL, Christoph, 50937 Köln (DE); HEIL, Markus, 42799 Leichlingen (DE); WROBLOWSKY, Heinz-Juergen, 40764 Langenfeld (DE); GESING, Ernst, Rudolf, 40699 Erkrath (DE); VOERSTE, Arnd, 50674 Köln (DE); GÖRGENS, Ulrich, 40882 Ratingen (DE)
(86) Internationale Anmeldenummer: PCT/EP2011/059692
(87) Internationale Veröffentlichungsnummer: WO 2011/157651

(56) Entgegenhaltungen:
- WO-A1-2007/144100
- WO-A1-2010/003350
- WO-A2-2004/046129
- WO-A2-2010/069502
- DE-A1-102006 032 168

## Beschreibung

Die vorliegende Erfindung betrifft neue Anthranilsäurediamid-Derivate, deren Anwendung als Insektizide und Akarizide zur Bekämpfung tierischer Schädlinge, auch in Kombination mit weiteren Mitteln zur Wirkungssteigerung, und mehrere Verfahren zu ihrer Herstellung.

Anthranilsäurederivate mit insektiziden Eigenschaften sind in der Literatur bereits beschrieben, z.B. in WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877, WO 2007/144100, WO2007/043677, WO2008/126889, WO2008/126890, WO2008/126933

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue Anthranilsäurediamid-Derivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Anthranilsäurediamid-Derivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Anthranilsäurediamid-Derivate der Formel (I) 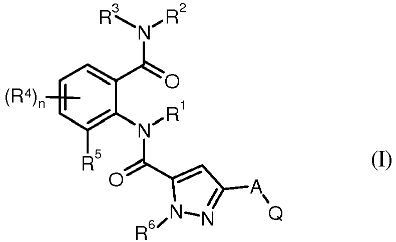 in welcher
- n: für 0 bis 3 steht,
- R¹: für Wasserstoff steht,
- R²: steht für Wasserstoff.
- R³: für einfach oder mehrfach, gleich oder verschieden substituiertes Methyl, Ethyl, Propyl, iso-Propyl, Cyclopropyl, Butyl, sec-Butyl und iso-Butyl steht, wobei die Substituenten unabhängig voneinander ausgewählt sind aus Aziridin, Oxiran, Thiiran, Azetidin, Oxetan, Thietan, Pyrrolidin, Pyrrolin, Pyrazolidin, Pyrazolin, Imidazolidin, Imidazolin, Tetrahydrofuran, Tetrahydrothiophen, Thiazolidin, Isothiazolidin, Piperidin, Piperazin, Hexahydropyridazin, Hexahydropyrimidin, Tetrahydropyran, Dioxan, Tetrahydrothiopyran, Morpholin, Thiomorpholin, Azepan, Diazepan, Oxepan, Dioxepan, Thiepan, Dithiepan, Pyrrolidon, Pyrrolidinon, Imidazolidon, Imidazolidinon, Triazolinon, Triazolidinon, Tetrazolinon, Tetrazolidinon, Thiazolon, Thiazolidinon, Oxazolon, Oxazolidinon Phenyl oder Naphthalin, welche einfach oder mehrfach, gleich oder verschieden substituiert sind mit Methyl, Ethyl, Propyl, iso-Propyl, Cyclopropyl, Fluor, Chlor, Brom, Cyano, Methoxy, Trifluormethyl, SF₅, Trifluormethoxy, Methylthio, Methylsulfonyl, Methoxycarbonyl, Methylcarbonyl oder Benzyl,
- R³: weiterhin für einfach oder mehrfach, gleich oder verschieden substituiertes Methyl, Ethyl, Propyl, iso-Propyl, Butyl, sec-Butyl und iso-Butyl steht, wobei die Substituenten unabhängig voneinander ausgewählt sind aus Furan, Thiophen, Pyrazol, Triazol, Imidazol, Thiazol, Oxazol, Isoxazol, Isothiazol, Thiadiazol, Oxadiazol, Pyrrol, Pyridin, Pyrimidin, Pyridazin oder Pyrazin, welche einfach oder mehrfach, gleich oder verschieden substituiert sind mit Methyl, Ethyl, Propyl, iso-Propyl, Cyclopropyl, Fluor, Chlor, Brom, Cyano, Methoxy, Trifluormethyl, Trifluormethoxy, SF₅, Methylthio, Methylsulfonyl, Methoxycarbonyl, Methylcarbonyl oder Benzyl,
- R³: weiterhin für substituiertes Phenyl, Furan, Thiophen, Pyrazol, Triazol, Imidazol, Thiazol, Oxazol, Isoxazol, Isothiazol, Thiadiazol, Oxadiazol, Pyrrol, Pyridin, Pyrimidin, Pyridazin oder Pyrazin steht, wobei die Substituenten unabhängig voneinander ausgewählt sind aus Methyl, Ethyl, Propyl, iso.Propyl, Cyclopropyl, Fluor, Chlor, Brom, Cyano, Methoxy, Trifluormethyl, SF₅, Trifluormethoxy, Methylthio, Methylsulfonyl, Methoxycarbonyl oder Methylcarbonyl,
- R³: weiterhin für substituiertes Aziridin, Oxiran, Thiiran, Azetidin, Oxetan, Thietan, Pyrrolidin, Pyrrolin, Pyrazolidin, Pyrazolin, Imidazolidin, Imidazolidinon, Imidazolin, Tetrahydrofuran, Tetrahydrothiophen, Tetrahydrothiophendioxid, Thiazolin, Thiazolidin, Isothiazolidin, Piperidin, Piperazin, Hexahydropyridazin, Hexahydropyrimidin, Tetrahydropyran, Dihydrofuranon, Dioxan, Tetrahydrothiopyran, Morpholin, Thiomorpholin, Thiomorpholindioxid, Azepan, Diazepan, Oxepan, Dioxepan, Thiazolin, Thiepan, Dithiepan, Dihydrothiophenon, Cyclobuten, Cyclopenten, Cyclopentadien, Cyclohexen, Cyclohexadien steht, wobei die Substituenten unabhängig voneinander ausgewählt sind aus Methyl, Ethyl, Propyl, iso-Propyl, Cyclopropyl, Fluor, Chlor, Brom, Cyano, Methoxy, Trifluormethyl, Trifluormethoxy, Methylthio, Methylsulfonyl, Methoxycarbonyl, Methylcarbonyl oder Benzyl,
- R⁴: steht für Wasserstoff, Methyl, Trifluormethyl, Cyano, Fluor, Chlor, Brom, Iod oder Trifluormethoxy. Ganz besonders bevorzugt stehen ausserdem zwei benachbarte Reste R⁴ für -(CH₂)₄-, oder -(CH=CH-)₂-.
- R⁴: steht insbesondere bevorzugt für Chlor oder Brom,
- R⁴: steht weiterhin insbesondere bevorzugt für Iod oder Cyano.

zwei benachbarte Reste R⁴ stehen insbesondere bevorzugt für -(CH=CH-)₂
- R⁵: für Methyl, Fluor, Chlor, Brom oder Iod steht,
- R⁵: insbesondere bevorzugt für Methyl oder Chlor steht,
- A: für -CH₂-, -CH(CH₃), C(CH₃)₂, -CH₂CH₂-, -CH(CN)-, -CH₂O- oder -C(=O)-CH₂-steht,
- A: insbesonders bevorzugt für CH₂, CH(CH₃), -CH₂O- oder -C(=O)-CH₂- steht,
- R⁶: für Methyl steht oder für den Rest 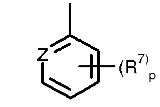 steht,
R⁷ für Fluor, Chlor oder Brom steht
R⁷ insbesondere bevorzugt für Chlor oder Brom steht,
p für 1 steht,
Z für N, CCl oder CH steht,
Q für einen gegebenenfalls einfach oder mehrfach substituierten 5- oder 6-gliedrigen heteroaromatischen Ring der Reihe Q-36 bis Q-40 ,Q43, Q-58 bis Q-59, Q62, Q63, einen aromatisches 9-gliedriges annelliertes heterobicyclisches Ringsystem Q-54 bis Q-56 sowie für ein 5-gliedrigen heterocyclischen Ring Q-60 bis Q-61 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,
- Q: insbesonders bevorzugt für einen gegebenenfalls einfach oder mehrfach substituierten heteroaromatischen Ring der Reihe Q-37, Q-38, Q-39, Q-40, Q43, Q-58, Q-59, Q62 und Q63, sowie für ein 5-gliedrigen heterocyclischen Ring Q-60 steht, wobei die Substituenten unabhängig voneinander ausgewählt sein können aus Chlor, Fluor, Iod, Brom, Cyano, Trifluormethyl und Pentafluorethyl.,
oder wobei die Substituenten unabhängig voneinander ausgewählt sein können aus Phenyl oder einem 5-oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können, 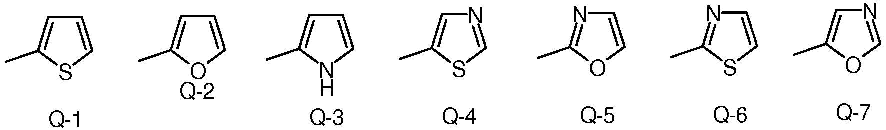 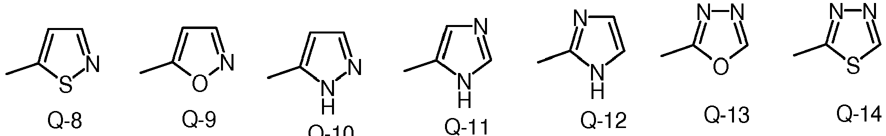 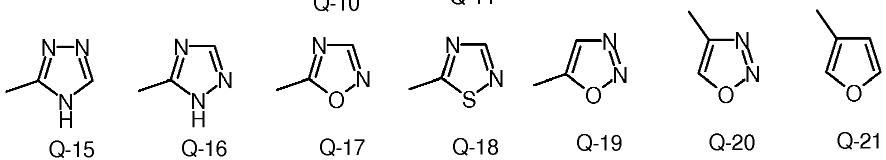 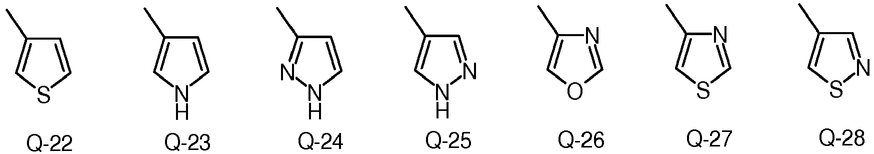 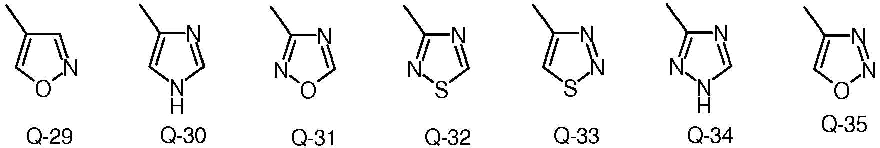 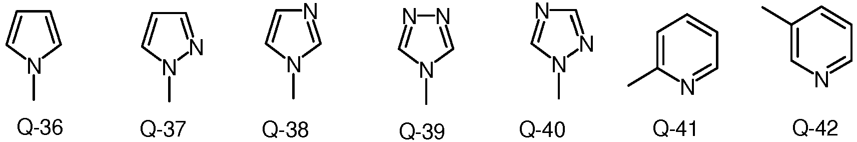 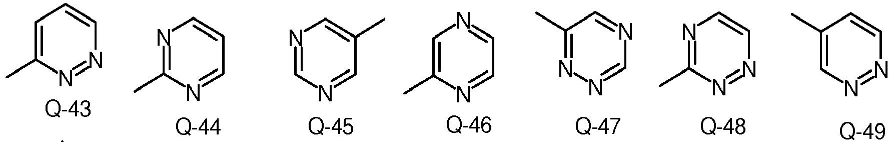 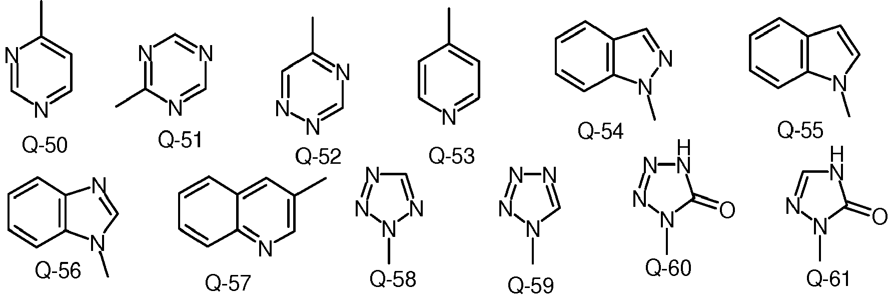 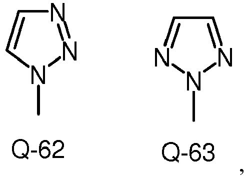 die Verbindungen der allgemeinen Formel (I) außerdem N-Oxide und Salze umfassen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die erfindungsgemäßen Verbindungen sind durch die Formel (I) allgemein definiert. Insbesonders bevorzugt sind Verbindungen der Formel (I), welche als insbesonders bevorzugt gekennzeichnete Restedefinitionen aufweisen.

Die oben aufgeführten Ringe oder Ringsysteme können gegebenenfalls unabhängig voneinander zusätzlich durch Oxo, Thioxo, (=O)=NH, (=O)=N-CN, (=O)₂ substituiert sein. Beispielhaft seien genannt Tetrahydrothiophendioxid, Imidazolidon.

Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und -S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere können die Verbindungen der Formeln (I) in Form verschiedener Regioisomere vorliegen. Beispielsweise in Form von Mischungen aus Verbindungen mit der Definition Q62 bzw. Q63 oder in Form von Mischungen aus Q58 und 59. Erfindungsgemäß umfasst sind daher auch Mischungen aus Verbindungen der Formeln (I), wobei Q die Bedeutungen Q62 und Q63, sowie Q58 und Q59 hat und die Verbindungen in verschiedenen Mischungsverhältnissen vorliegen können. Bevorzugt sind dabei Mischungsverhältnisse aus Verbindungen der Formel (I), worin der Rest Q für Q62 oder für Q58 steht zu Verbindungen der Formel (I) worin der Rest Q für Q63 oder für Q59 steht, von 60:40 bis 99:1, besonders bevorzugt von 70:30 bis 98:2, ganz besonders bevorzugt von 80:20 bis 97:3. Insbesonders bevorzugt sind die folgenden Mischungsverhältnisse einer Verbindung der Formel (I), wobei Q die Bedeutung Q62 oder Q58 hat zur Verbindung der Formel (I), wobei Q die Bedeutung Q63 oder Q59 hat: 80:20; 81:19; 82:18; 83:17; 84:16; 85:15, 86:14; 87:13; 88:12; 89:11; 90:10, 91:9; 92:8; 93:7; 94:6; 95;5; 96:4; 97:3.

### Herstellverfahren

### Anthranilamide der Formel (I)

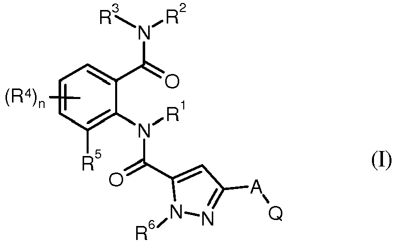

in welcher A, R¹, R², R³, R⁴, R⁵, R⁶, Q und n die oben angegebenen Bedeutungen haben, werden erhalten, indem man

Benzoxazinone der Formel (II) 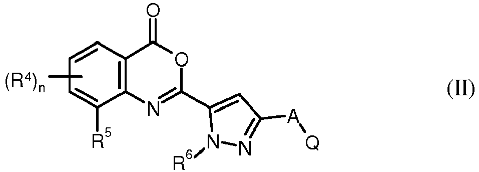 in welcher R⁴, R⁵, R⁶, A, Q und n die oben angegebenen Bedeutungen haben,
mit einem Amin der Formel (III) 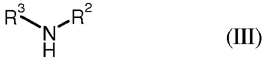 in welcher R² und R³ die oben angegebenen Bedeutungen haben,
in Gegenwart eines Verdünnungsmittels umsetzt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Emteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Prenmotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Linomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuellebomi, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (XI) definiert 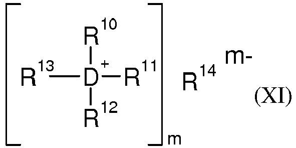 in welcher
- D: für Stickstoff oder Phosphor steht,
- D: bevorzugt für Stickstoff steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R¹⁰, R¹¹, R¹², and R¹³ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R¹⁰, R¹¹, R¹², and R¹³ besonders bevorzugt unabhängig voneinander für Wasserstoff, Methyl, Ethyl, n-Propyl, i-Propyl, n-Butyl, i-Butyl, s-Butyl oder t-Butyl stehen,
R¹⁰, R¹¹, R¹², and R¹³ ganz besonders bevorzugt für Wasserstoff stehen,
- m: für 1, 2, 3 oder 4 steht,
- m: bevorzugt für 1 oder 2 steht,
- R¹⁴: für ein anorganisches oder organisches Anion steht,
- R¹⁴: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R¹⁴: besonders bevorzugt für Laktat, Sulfat, Monohydrogenphosphat, Dihydrogenphosphat, Nitrat, Thiosulfat, Thiocyanat, Citrat, Oxalat oder Formiat steht.
- R¹⁴: ganz besonders bevorzugt für Sulfat steht.

Die Ammonium- und Phosphoniumsalze der Formel (XI) können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern ein Penetrationsförderer zugegeben. Selbst in diesen Fällen ist eine Wirkungssteigerung zu beobachten. Gegenstand der vorliegenden Erfindung ist also ebenfalls die Verwendung eines Penetrationsförders, sowie die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten ausgenommen zur therapeutischen Behandung des tierischen oder menschlichen Körpers.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)_{V}-R' (XII)

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (XII-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XII-b)

in welcher
- R: die oben angegebene Bedeutung hat,
- R': die oben angegebene Bedeutung hat,
- EO: für -CH₂-CH₂-O- steht,
- PO: für 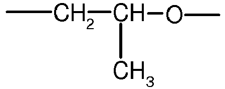 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XII-c)

in welcher
- R: die oben angegebene Bedeutung hat,
- R': die oben angegebene Bedeutung hat,
- EO: für -CH₂-CH₂-O- steht,
- PO: für 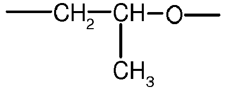 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XII-d)

in welcher
R und R' die oben angegebenen Bedeutungen haben,
- EO: für CH₂-CH₂-O- steht,
- BO: für 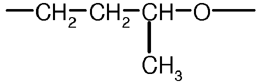 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XII-e)

in welcher
R und R' die oben angegebenen Bedeutungen haben,
- BO: für 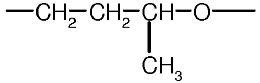 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XII-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XII-c) sei 2-Ethyl-hexyl-alkoxylat der Formel 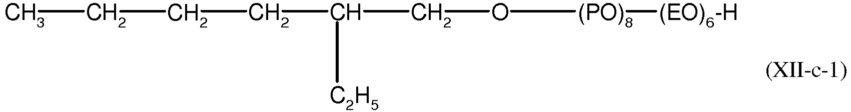 in welcher
- EO: für -CH₂-CH₂-O- steht,
- PO: für 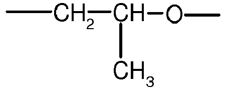 steht und

die Zahlen 8 und 6 Durchschnittswerte darstellen, genannt.

Als Beispiel für ein Alkanol-Alkoxylat der Formel (XII-d) sei die Formel

CH₃-(CH₂)₁₀-O-(-EO-)₆-(-BO-)₂-CH₃ (XII-d-1)

in welcher
- EO: für CH₂-CH₂-O- steht,
- BO: für 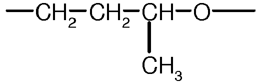 steht und
die Zahlen 10, 6 und 2 Durchschnittswerte darstellen, genannt.

Besonders bevorzugte Alkanol-Alkoxylate der Formel (XII-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (XII-f-1)

CH₃-(CH₂)ₜ-CH₂-O-(-CH₂-CH₂-O-)ᵤ-R' (XII-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer sind in folgender Tabelle aufgeführt."Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt:

| # | Wirkstoff | Salz | Penetrationförderer |
|---|---|---|---|
| 1 | I | Ammoniumsulfat | Gemäß Test |
| 2 | I | Ammoniumlaktat | Gemäß Test |
| 3 | I | Ammoniumnitrat | Gemäß Test |
| 4 | I | Ammoniumthiosulfat | Gemäß Test |
| 5 | I | Ammoniumthiocyanat | Gemäß Test |
| 6 | I | Ammoniumcitrat | Gemäß Test |
| 7 | I | Ammoniumoxalat | Gemäß Test |
| 8 | I | Ammoniumformiat | Gemäß Test |
| 9 | I | Ammoniumhydrogenphosphat | Gemäß Test |
| 10 | I | Ammioniumdihydrogenphosphat | Gemäß Test |
| 11 | I | Ammoniumcarbonat | Gemäß Test |
| 12 | I | Ammoniumbenzoat | Gemäß Test |
| 13 | I | Ammoniumsulfit | Gemäß Test |
| 14 | I | Ammoniumbenzoat | Gemäß Test |
| 15 | I | Ammoniumhydrogenoxalat | Gemäß Test |
| 16 | I | Ammoniumhydrogencitrat | Gemäß Test |
| 17 | I | Ammoniumacetat | Gemäß Test |
| 18 | I | Tetramethylammoniumsulfat | Gemäß Test |
| 19 | I | Tetramethylammoniumlaktat | Gemäß Test |
| 20 | I | Tetramethylammoniumnitrat | Gemäß Test |
| 21 | I | Tetramethylammoniumthiosulfat | Gemäß Test |
| 22 | I | Tetramethylammoniumthiocyanat | Gemäß Test |
| 23 | I | Tetramethylammoniumcitrat | Gemäß Test |
| 24 | I | Tetramethylammoniumoxalat | Gemäß Test |
| 25 | I | Tetramethylammoniumformiat | Gemäß Test |
| 26 | I | Tetramethylammoniumhydrogenphosphat | Gemäß Test |
| 27 | I | Tetramethylammoniumdihydrogenphosphat | Gemäß Test |
| 28 | I | Tetraethylammoniumsulfat | Gemäß Test |
| 29 | I | Tetraethylammoniumlaktat | Gemäß Test |
| 30 | I | Tetraethylammoniumnitrat | Gemäß Test |
| 31 | I | Tetraethylammioniumthiosulfat | Gemäß Test |
| 32 | I | Tetraethylammoniumthiocyanat | Gemäß Test |
| 33 | I | Tetraethylammioniumcitrat | Gemäß Test |
| 34 | I | Tetraethylammoniumoxalat | Gemäß Test |
| 35 | I | Tetraethylammoniumformiat | Gemäß Test |
| 36 | I | Tetraethylammioniumhydrogenphosphat | Gemäß Test |
| 37 | I | Tetraethylammoniumdihydrogenphosphat | Gemäß Test |

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky-oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Emteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Emteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitierten - seien die folgenden Insekten genannt:
Käfer wie Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Omithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggem, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Herstellverfahren und Zwischenprodukte

Benzoxazinone der Formel (II) sind bekannt (z.B. WO2007/144100) oder können nach bekannten Methoden hergestellt werden.

Aniline der Formel (III) sind bekannt (z.B. WO2007/144100) oder können nach bekannten Methoden hergestellt werden.

### Herstellungsbeispiele

Gemäß den oben beschriebenen Herstellverfahren lassen sich die Verbindungen der Formel (I) erhalten, beispielsweise die folgende Verbindungen der Formel (I):

### Beispiel Nr. 29

### Synthese von 1-(3-Chlorpyridin-2-yl)-N-{4-cyano-2-[(1,1-dioxidotetrahydrothiophen-3-yl)carbamoyl]-6-methylphenyl}-3-{[5-(trifluormethyl)-2H-tetrazol-2-yl]methyl}-1H-pyrazol-5-carboxamide

100 mg (0.17 mmol) 2-[1-(3-Chlorpyridin-2-yl)-3-{[5-(trifluormethyl)-2H-tetrazol-2-yl]methyl}-1H-pyrazol-5-yl]-8-methyl-4-oxo-4H-3,1-benzoxazin-6-carbonitrile wurden in 5 ml Tetrahydrofuran gelöst und mit 69.5 mg (0.51 mmol) Tetrahydrothiophen-3-amine 1,1-dioxid versetzt. Es wurde zunächst 3h bei Raumtemperatur und anschließend 3,5h bei 70°C gerührt.

Nach dem Abkühlen wurde das Reaktionsgemisch im Vakuum vom Lösungsmittel befreit. Durch chromatographische Reinigung des Rückstands wurde das gewünschte Produkt isoliert (logP (HCOOH): 2,47; MH⁺: 649; ¹H-NMR (400 MHz, DMSO, δ, ppm): 1,97-2.17 (m, 1H), 2,26 (s, 3H), 2,26-2,29 (m, 1H), 2,81-2,85 (m, 1H), 3,10-3,13 (m, 1H), 3,25-3,35 (m, 2H), 4,44-4,48 (m, 1H), 6,33 (s, 2H), 7,46 (s, 1H), 7,60-7,62 (m, 1H), 7,78 (s, 1H), 7,90 (s, 1H), 8.18 (d, 1H), 8,48 (m, 1H), 8,78 (br. s, 1H), 10.49 (s, 1H).

Die folgenden Beispiele können auf analoge Art und Weise erhalten werden, wobei die Verbindungen der Formel (I) telis als Regioisomere vorliegen können. In der folgenden Tabelle sind in Bezug auf die NMR-Daten jeweils die chemischen Verschiebungen und die dazugehörigen Signalintensitäten angegeben, beispielsweise steht für Verbindung 1:
Signal 1 : 10.308;0.58; für 10.308 ppm (chemische Verschiebung), 0.58 (Signalintensität);
Signal 2: 8.893;0.63; für 8.893 ppm (chemische Verschiebung), 0.63 (Signalintensität)

| NR | Structure | Log p | MH+ | |
|---|---|---|---|---|
| 1 | 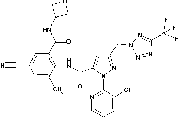 | 2,49 | 587 | (10.308;0.58),(8.893;0.63),(8.460;2.08),(8.457;2.04),(8.449;2.20),(8.445;1.99),(8.265;0.58),(8.261; 0.50),(8.114;2.13),(8.111;1.98),(8.094;2.36),(8.090;2.06),(7.908;0.34),(7.826;2.36),(7.795;2.67),(7. 791;2.07),(7.592;0.33),(7.583;2.34),(7.572;2.29),(7.563;1.99),(7.551;1.92),(7.337;4.24),(7.292;0.7 9),(6.270;10.29),(4.838;0.48),(4.820;0.99),(4.804;1.08),(4.786;0.72),(4.690;2.55),(4.674;3.75),(4.6 56;2.11),(4.620;0.54),(4.601;0.33),(4.560;0.49),(4.458;2.54),(4.442;4.54),(4.426;2.16),(3.118;317. 32),(2.658;0.35),(2.527;1.32),(2.511;0.80),(2.498;19.95),(2.493;38.44),(2.489;50.73),(2.484;34.58) ,(2.479;16.00),(2.315;0.35),(2.305;0.64),(2.295;0.39),(2.221;12.49),(2.137;0.58),(1.939;1.57),(-0.000;1.10) |
| 2 | 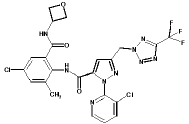 | 2,8 | 596 | (8.791;0.72),(8.776;0.75),(8.456;2.82),(8.452;3.00),(8.444;3.00),(8.440;2.97),(8.107;2.87),(8.103;2 .93),(8.087;3.25),(8.083;3.02),(7.577;3.09),(7.565;2.97),(7.557;2.85),(7.545;2.81),(7.439;2.19),(7.4 37;2.28),(7.432;2.78),(7.431;2.62),(7.382;2.74),(7.376;2.30),(7.306;2.44),(6.262;12.15),(6.246;0.3 9),(4.835;0.48),(4.817;1.00),(4.801;1.11),(4.783;0.74),(4.678;2.92),(4.662;4.14),(4.644;2.46),(4.62 4;0.40),(4.608;0.44),(4.606;0.45),(4.590;0.41),(4.453;3.07),(4.436;5.60),(4.421;2.59),(4.249;0.33),( 4.234;0.70),(4.217;0.37),(3.116;38.03),(2.526;0.91),(2.510;0.42),(2.505;0.59),(2.497;9.68),(2.493; 20.03),(2.488;28.14),(2.483;19.55),(2.478;9.25),(2.166;16.00),(2.049;0.36),(1.365;1.60),(-0.000;1.73) |
| 3 | 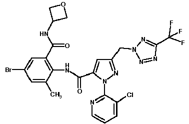 | 2,87 | 638/6 40 | (10.042;0.37),(8.788;0.74),(8.773;0.77),(8.457;2.91),(8.454;3.00),(8.446;3.03),(8.442;2.95),(8.106; 2.91),(8.102;2.93),(8.086;3.24),(8.082;2.97),(7.581;3.52),(7.576;2.56),(7.575;2.68),(7.569;5.51),(7. 560;3.00),(7.549;2.91),(7.505;2.73),(7.500;2.38),(7.301;2.58),(6.876;0.56),(6.254;12.19),(6.242;0. 43),(4.830;0.46),(4.813;0.89),(4.795;1.03),(4.779;0.70),(4.679;2.94),(4.662;4.17),(4.644;2.45),(4.4 51;3.14),(4.434;5.67),(4.419;2.62),(3.439;0.39),(3.396;0.56),(3.378;0.58),(3.362;0.55),(3.339;0.64) ,(3.198;1945.58),(3.090;0.62),(3.064;0.48),(3.026;0.36),(2.670;0.40),(2.665;0.46),(2.661;0.36),(2.5 34;1.61),(2.518;1.15),(2.513;1.56),(2.506;27.95),(2.501;58.65),(2.496;83.31),(2.491;58.27),(2.487; 27.74),(2.328;0.37),(2.323;0.61),(2.320;0.37),(2.318;0.40),(2.183;1.03),(2.164;16.00),(2.049;0.34), (2.038;0.39),(1.364;10.14),(-0.000;2.32) |
| 4 | 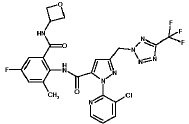 | 2,53 | 580 | (9.983;0.36),(8.721;0.81),(8.704;0.84),(8.456;2.78),(8.452;2.98),(8.444;2.97),(8.440;2.93),(8.106;2 .90),(8.102;2.95),(8.085;3.23),(8.082;3.09),(7.576;3.01),(7.564;2.90),(7.556;2.78),(7.544;2.74),(7.3 06;2.41),(7.227;1.18),(7.219;1.31),(7.202;1.09),(7.196;1.29),(7.164;1.33),(7.157;1.10),(7.142;1.35) ,(7.135;1.05),(6.260;10.73),(6.244;0.48),(4.840;0.48),(4.823;0.99),(4.806;1.09),(4.788;0.71),(4.677 ;2.88),(4.661;4.09),(4.643;2.45),(4.448;3.01),(4.431;5.54),(4.416;2.58),(4.236;0.36),(3.128;87.86),( 3.029;0.35),(3.022;0.32),(2.527;1.13),(2.511;0.46),(2.507;0.66),(2.498;12.04),(2.494;25.11),(2.489 ;35.64),(2.484;24.94),(2.480;11.95),(2.171;16.00),(2.064;0.44),(1.364;1.80),(-0.000;1.34) |
| 5 | 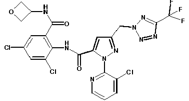 | 2,79 | 614 | (8.933;0.66),(8.457;3.26),(8.453;3.49),(8.446;3.55),(8.442;3.50),(8.101;3.17),(8.098;3.31),(8.081;3 .59),(8.078;3.49),(7.758;4.59),(7.752;4.94),(7.577;3.49),(7.565;3.44),(7.557;3.34),(7.545;3.39),(7.5 37;5.83),(7.531;5.61),(7.526;0.60),(7.519;0.36),(7.367;5.92),(6.265;16.00),(4.815;0.47),(4.797;1.0 1),(4.781;1.13),(4.763;0.77),(4.676;4.01),(4.660;5.58),(4.642;3.20),(4.629;0.50),(4.612;0.60),(4.61 1;0.58),(4.595;0.53),(4.498;0.36),(4.430;4.02),(4.413;7.36),(4.398;3.50),(4.261;0.42),(4.245;0.85),( 4.229;0.48),(4.000;0.33),(3.402;0.52),(3.385;0.56),(3.118;11.61),(3.053;0.54),(3.046;0.46),(2.526; 0.56),(2.510;0.38),(2.505;0.59),(2.497;7.76),(2.493;16.05),(2.488;22.62),(2.483;15.75),(2.478;7.54 ),(1.365;0.34),(1.109;0.44),(1.091;0.89),(1.074;0.42) |
| 6 | 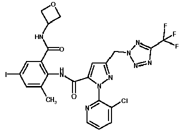 | 3 | 688 | (8.772;0.95),(8.755;0.97),(8.454;2.79),(8.450;3.01),(8.442;3.00),(8.438;2.97),(8.105;2.86),(8.101;2 .91),(8.085;3.19),(8.081;3.04),(7.737;2.48),(7.735;2.66),(7.732;2.99),(7.730;2.69),(7.655;2.82),(7.6 50;2.60),(7.576;3.08),(7.564;2.97),(7.556;2.87),(7.544;2.82),(7.299;2.91),(6.259;12.38),(6.245;0.4 6),(4.826;0.54),(4.808;1.08),(4.791;1.20),(4.773;0.85),(4.674;2.92),(4.658;4.19),(4.640;2.47),(4.45 1;3.08),(4.435;5.67),(4.419;2.64),(3.116;47.01),(2.526;0.63),(2.510;0.39),(2.506;0.57),(2.497;9.29) ,(2.493;19.35),(2.488;27.41),(2.483;19.18),(2.478;9.19),(2.296;0.46),(2.131;16.00),(2.025;0.38),(1. 365;0.86),(-0.000;1.47) |
| 7 | 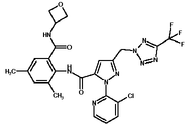 | 2,67 | 576 | (9.936;0.43),(8.592;1.07),(8.576;1.05),(8.453;2.72),(8.449;2.47),(8.441;2.78),(8.438;2.33),(8.101;2 .64),(8.097;2.32),(8.080;2.89),(8.077;2.43),(7.571;2.52),(7.560;2.48),(7.551;2.30),(7.539;2.21),(7.2 95;2.26),(7.155;5.23),(6.256;10.68),(4.852;0.58),(4.835;1.16),(4.818;1.26),(4.800;0.76),(4.677;2.7 7),(4.661;4.24),(4.643;2.41),(4.618;1.37),(4.602;1.92),(4.584;1.37),(4.453;3.07),(4.437;5.31),(4.42 1;2.53),(4.236;1.39),(4.220;2.59),(4.204;1.27),(3.989;0.66),(3.971;0.99),(3.955;0.62),(3.118;48.27) ,(2.526;0.66),(2.497;16.38),(2.493;29.74),(2.488;37.38),(2.483;25.01),(2.479;11.24),(2.315;0.42),( 2.282;14.12),(2.118;16.00),(1.364;0.61),(-0.000;0.50) |
| 8 | 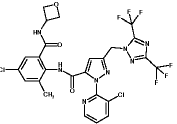 | 3,24 | 663 | (8.448;1.07),(8.444;1.15),(8.436;1.15),(8.432;1.11),(8.094;1.07),(8.090;1.11),(8.074;1.22),(8.070;1 .14),(7.567;1.16),(7.555;1.12),(7.546;1.08),(7.535;1.05),(7.434;0.84),(7.429;0.99),(7.378;1.04),(7.3 73;0.87),(7.223;0.75),(6.874;0.86),(6.873;0.85),(5.864;3.12),(4.811;0.35),(4.794;0.38),(4.674;1.12) ,(4.657;1.60),(4.639;0.94),(4.450;1.17),(4.433;2.14),(4.418;0.99),(3.116;28.29),(2.526;0.33),(2.506 ;0.32),(2.497;5.17),(2.493;10.75),(2.488;15.22),(2.483;10.61),(2.479;5.06),(2.183;1.57),(2.161;6.0 0),(1.364;16.00),(-0.000;0.67) |
| 9 | 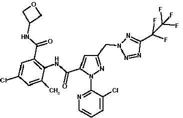 | 3,21 | 646 | (8.807;0.54),(8.454;2.63),(8.450;2.77),(8.443;2.82),(8.439;2.78),(8.104;2.66),(8.101;2.75),(8.084;3 .02),(8.080;2.88),(7.575;2.85),(7.563;2.77),(7.555;2.65),(7.543;2.60),(7.435;2.25),(7.430;2.77),(7.3 84;2.86),(7.378;2.38),(7.305;2.39),(6.286;11.83),(6.272;0.38),(4.834;0.40),(4.817;0.84),(4.800;0.9 2),(4.783;0.60),(4.676;2.97),(4.660;4.28),(4.641;2.55),(4.618;2.16),(4.602;2.81),(4.600;2.88),(4.58 4;2.56),(4.452;3.11),(4.436;5.67),(4.420;2.67),(4.236;2.21),(4.220;4.48),(4.204;2.33),(4.006;0.49),( 3.988;1.13),(3.972;1.74),(3.955;0.92),(3.938;0.32),(3.302;0.39),(3.283;0.35),(3.114;26.60),(2.944; 0.34),(2.658;0.37),(2.653;0.35),(2.510;0.76),(2.506;1.12),(2.498;14.60),(2.493;29.64),(2.488;41.39 ),(2.484;28.84),(2.479;13.73),(2.315;0.35),(2.163;16.00),(1.364;1.54),(-0.000;1.19) |
| 10 | 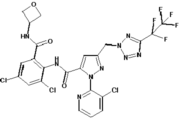 | 3,18 | 666 | (8.998;0.53),(8.454;3.33),(8.450;3.47),(8.442;3.55),(8.439;3.43),(8.096;3.11),(8.092;3.21),(8.075;3 .49),(8.072;3.33),(7.746;4.00),(7.740;4.26),(7.714;0.38),(7.708;0.39),(7.613;0.36),(7.607;0.34),(7.5 73;3.45),(7.561;3.46),(7.553;3.39),(7.541;8.86),(7.535;5.61),(7.521;0.40),(7.515;0.32),(7.352;5.41) ,(6.874;0.37),(6.300;0.35),(6.284;16.00),(4.813;0.46),(4.796;0.94),(4.780;1.05),(4.763;0.66),(4.694 ;0.42),(4.673;4.22),(4.656;5.94),(4.638;3.42),(4.622;3.06),(4.606;3.98),(4.604;4.06),(4.590;2.05),(4 .588;3.53),(4.507;0.32),(4.490;0.52),(4.425;4.17),(4.408;7.67),(4.393;3.67),(4.245;2.97),(4.229;6.1 7),(4.213;3.19),(4.016;0.68),(3.999;1.42),(3.982;2.32),(3.966;1.13),(3.948;0.42),(3.602;0.41),(3.34 9;0.33),(3.333;0.34),(3.272;0.38),(3.124;78.61),(3.008;0.71),(3.000;0.68),(2.977;0.55),(2.970;0.52) ,(2.793;0.33),(2.663;0.40),(2.658;0.48),(2.653;0.39),(2.527;0.37),(2.511;0.92),(2.506;1.41),(2.498; 23.65),(2.493;48.80),(2.489;68.76),(2.484;48.28),(2.479;23.26),(2.437;0.32),(2.320;0.34),(2.316;0. 43),(2.183;0.64),(1.762;0.43),(1.364;6.14),(-0.000;2.28) |
| 11 | 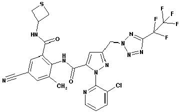 | 3,43 | 653 | (8.792;0.54),(8.460;3.22),(8.456;3.39),(8.448;3.46),(8.444;3.42),(8.113;3.11),(8.109;3.17),(8.093;3 .55),(8.089;3.38),(7.819;2.78),(7.816;3.03),(7.750;3.17),(7.746;2.93),(7.582;3.61),(7.570;3.51),(7.5 62;3.39),(7.550;3.36),(7.349;5.70),(6.300;13.88),(6.147;0.36),(5.033;0.73),(5.012;1.30),(4.992;1.2 8),(4.972;0.79),(3.381;2.28),(3.358;4.15),(3.336;2.75),(3.204;0.41),(3.180;0.61),(3.170;0.94),(3.16 1;3.44),(3.157;2.62),(3.152;1.65),(3.140;6.08),(3.137;6.47),(3.116;89.90),(2.657;0.40),(2.632;0.34) ,(2.527;0.51),(2.510;0.81),(2.506;1.20),(2.498;18.19),(2.493;37.33),(2.488;52.40),(2.484;36.48),(2. 479;17.32),(2.315;0.36),(2.303;1.07),(2.236;0.40),(2.214;16.00),(2.137;1.01),(1.477;0.52),(1.364;2 .18),(1.283:1.89),(1.254:0.68),(-0.000:2.45) |
| 12 | 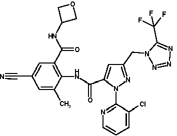 | 2,17 | 587 | (10.301;2.56),(8.891;1.09),(8.874;1.14),(8.447;3.33),(8.443;3.41),(8.435;3.44),(8.431;3.32),(8.097; 2.94),(8.093;2.92),(8.077;3.28),(8.073;3.12),(7.974;0.41),(7.827;2.88),(7.793;3.56),(7.570;3.11),(7. 558;3.03),(7.550;2.90),(7.538;2.85),(7.280;4.33),(6.059;11.96),(4.835;0.69),(4.818;1.43),(4.801;1. 53),(4.784;1.15),(4.767;0.38),(4.693;3.85),(4.677;5.54),(4.659;3.19),(4.458;3.56),(4.442;6.39),(4.4 26;3.02),(3.169;0.84),(3.117;913.61),(3.072;0.55),(2.667;0.35),(2.662;0.72),(2.657;1.06),(2.653;0. 74),(2.648;0.41),(2.527;3.22),(2.510;2.63),(2.506;3.94),(2.498;57.14),(2.493;117.07),(2.488;164.24 ),(2.484;114.81),(2.479;55.05),(2.448;0.36),(2.325;0.34),(2.320;0.73),(2.315;0.97),(2.310;0.71),(2. 306;0.40),(2.214;16.00),(2.040;1.56),(1.296;0.61),(1.245;0.54),(-0.000;7.18) |
| 13 | 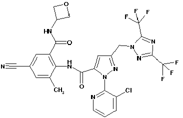 | 2,95 | 654 | (10.296;0.57),(8.887;0.72),(8.452;2.95),(8.448;3.18),(8.440;3.15),(8.436;3.16),(8.102;2.96),(8.099; 3.00),(8.082;3.34),(8.078;3.13),(7.825;2.75),(7.792;3.31),(7.789;2.73),(7.573;3.13),(7.562;3.00),(7. 553;2.93),(7.541;2.85),(7.254;4.18),(5.872;9.17),(4.832;0.58),(4.814;1.30),(4.798;1.41),(4.780;0.9 5),(4.686;3.39),(4.669;4.90),(4.651;2.78),(4.455;3.38),(4.439;6.10),(4.423;2.86),(3.116;405.33),(2. 662;0.43),(2.657;0.62),(2.652;0.52),(2.632;0.76),(2.526;2.01),(2.510;1.69),(2.506;2.45),(2.497;34.88),(2.493;71.60),(2.488;100.47),(2.483;70.23),(2.479;33.53),(2.320;0.44),(2.315;0.63),(2.310;0.45 ),(2.215;16.00),(2.041;0.65),(1.296;0.36),(-0.000;3.30) |
| 14 | 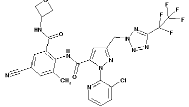 | 2,87 | 637 | (10.325;2.88),(8.896;1.25),(8.881;1.24),(8.459;2.84),(8.456;3.01),(8.448;2.99),(8.444;2.95),(8.114; 3.00),(8.111;3.00),(8.094;3.34),(8.090;3.18),(7.833;2.67),(7.830;3.17),(7.795;3.19),(7.792;2.62),(7. 583;3.15),(7.571;3.04),(7.563;2.92),(7.551;2.86),(7.343;6.43),(6.298;13.07),(4.837;0.57),(4.819;1. 21),(4.803;1.34),(4.785;0.95),(4.688;3.24),(4.672;4.66),(4.654;2.72),(4.459;3.39),(4.443;6.12),(4.4 27;2.85),(3.119;303.77),(2.662;0.42),(2.657;0.53),(2.653;0.42),(2.526;0.63),(2.510;1.40),(2.506;2. 13),(2.497;30.21),(2.493;61.93),(2.488;86.93),(2.483;60.85),(2.479;29.10),(2.320;0.35),(2.315;0.52 ),(2.310;0.38),(2.220;16.00),(2.041;0.50),(-0.000;2.84) |
| 15 | 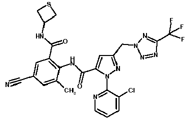 | 3,06 | 603 | (12.829;0.71),(10.307;2.90),(8.775;1.61),(8.759;1.67),(8.460;4.89),(8.457;5.36),(8.449;5.21),(8.44 5;5.04),(8.116;3.17),(8.112;3.32),(8.096;3.34),(8.092;3.04),(7.975;2.42),(7.822;3.68),(7.746;4.12),( 7.584;3.58),(7.572;3.32),(7.564;3.31),(7.552;3.26),(7.527;0.63),(7.350;4.80),(6.274;16.00),(5.049; 0.74),(5.029;1.43),(5.010;2.22),(4.990;2.41),(4.970;1.18),(3.768;0.82),(3.580;0.75),(3.500;0.62),(3. 431;0.69),(3.401;0.74),(3.377;2.98),(3.355;5.82),(3.332;3.65),(3.303;1.38),(3.255;1.41),(3.192;3.0 8),(3.186;3.28),(3.160;9.90),(3.120;3764.07),(3.081;3.22),(2.666;1.70),(2.662;3.09),(2.658;4.26),(2 .653;3.13),(2.648;1.68),(2.634;0.63),(2.575;0.81),(2.572;0.76),(2.561;1.01),(2.527;14.46),(2.511;1 1.93),(2.506;17.64),(2.498;229.75),(2.493;468.11),(2.488;656.13),(2.484;461.08),(2.479;223.03),(2 .324;1.39),(2.320;2.86),(2.315;4.02),(2.310;2.82),(2.306;1.70),(2.216;15.64),(2.185;0.68),(2.040;0. 77),(1.901:1.47),(1.404:4.01),(1.298:1.40),(1.296:1.47),(1.245:2.46),(1.082:0.78),(1.056:0.62),(0.8 95;1.57),(-0.000;2.92),(-0.676;0.62),(-3.155;0.61) |
| 16 | 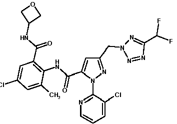 | 2,37 | 578 | (8.799;0.66),(8.786;0.66),(8.453;2.78),(8.449;2.96),(8.441;2.96),(8.437;2.92),(8.104;2.85),(8.100;2 .91),(8.083;3.24),(8.080;2.99),(7.590;2.45),(7.573;3.05),(7.562;2.95),(7.553;2.82),(7.548;0.36),(7.5 41;2.89),(7.459;5.17),(7.437;2.22),(7.435;2.32),(7.431;2.92),(7.429;2.71),(7.379;2.79),(7.374;2.37) ,(7.328;2.66),(7.282;2.69),(6.185;11.95),(6.173;0.43),(4.835;0.47),(4.817;0.95),(4.800;1.05),(4.782 ;0.69),(4.681;3.18),(4.665;4.29),(4.647;2.49),(4.620;0.66),(4.604;0.79),(4.602;0.82),(4.600;0.39),(4 .588;0.41),(4.586;0.76),(4.453;3.09),(4.437;5.60),(4.422;2.65),(4.241;0.64),(4.225;1.29),(4.209;0.6 6),(3.977;0.49),(3.121;50.04),(2.526;0.38),(2.510;0.57),(2.506;0.81),(2.497;12.59),(2.493;26.09),(2 .488;36.81),(2.483;25.73),(2.478;12.29),(2.240;0.67),(2.163;16.00),(2.112;0.70),(1.364;1.34),(-0.000;1.80) |
| 17 | 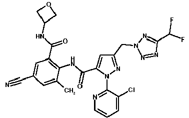 | 2,05 | 569 | (10.305;2.87),(8.891;1.22),(8.875;1.27),(8.457;2.89),(8.453;3.18),(8.446;2.97),(8.442;3.04),(8.112; 2.89),(8.108;3.08),(8.092;3.45),(8.088;3.17),(7.830;2.77),(7.827;3.11),(7.791;3.16),(7.787;2.67),(7. 593;2.57),(7.580;3.18),(7.568;3.17),(7.560;3.06),(7.548;2.80),(7.461;5.40),(7.330;2.82),(7.325;0.3 8),(7.314;6.85),(6.195;13.16),(4.836;0.60),(4.819;1.19),(4.802;1.32),(4.784;0.92),(4.768;0.33),(4.6 99;0.38),(4.692;3.29),(4.676;4.67),(4.658;2.65),(4.458;3.27),(4.442;6.07),(4.426;2.84),(3.231;0.34),(3.214;0.36),(3.180;3.21),(3.167;1.02),(3.156;1.53),(3.117;742.68),(3.059;0.49),(3.055;0.48),(3.04 2;0.35),(2.666;0.55),(2.662;1.04),(2.657;1.54),(2.652;1.14),(2.648;0.62),(2.632;1.32),(2.547;0.44),( 2.537;0.51),(2.526;2.05),(2.510;3.82),(2.505;5.76),(2.497;84.62),(2.493;175.16),(2.488;247.02),(2. 483;172.78),(2.478;82.61),(2.319;1.04),(2.315;1.50),(2.310;1.13),(2.305;0.70),(2.219;16.00),(2.041 ;2.47),(1.901;0.60),(1.404;0.34),(1.245;0.64),(0.895;0.74),(0.008;1.47),(-0.000;41.40),(-0.008;1.37) |
| 18 | 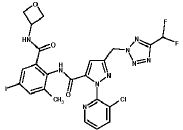 | 2,59 | 670 | (10.027;1.60),(8.769;0.79),(8.755;0.78),(8.450;2.97),(8.447;3.16),(8.439;3.17),(8.435;3.09),(8.102; 2.88),(8.098;2.88),(8.082;3.20),(8.078;2.98),(7.733;2.58),(7.729;2.77),(7.652;2.87),(7.648;2.60),(7. 589;2.62),(7.572;3.09),(7.561;3.00),(7.552;2.88),(7.540;2.79),(7.457;5.42),(7.326;2.82),(7.273;2.7 9),(6.182;12.85),(4.825;0.52),(4.807;1.06),(4.790;1.17),(4.772;0.79),(4.677;3.25),(4.661;4.63),(4.6 43;2.67),(4.451;3.10),(4.435;5.52),(4.419;2.57),(3.116;176.95),(3.092;14.41),(2.657;0.36),(2.526;0 .42),(2.510;0.87),(2.505;1.26),(2.497;18.40),(2.492;37.87),(2.488;53.32),(2.483;37.35),(2.478;17.9 1),(2.315;0.33),(2.199;0.40),(2.127;16.00),(2.078;0.42),(2.040;1.35),(1.364;0.62),(0.008;0.33),(-.0.000;10.54),(-0.008;0.38) |
| 19 | 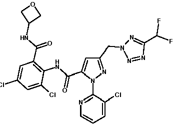 | 2,37 | 596 | (10.269;0.78),(8.875;0.95),(8.859;0.92),(8.455;3.34),(8.451;3.54),(8.443;3.55),(8.439;3.50),(8.102; 3.22),(8.098;3.30),(8.082;3.68),(8.078;3.45),(7.827;0.37),(7.821;0.34),(7.768;3.81),(7.762;4.11),(7. 703;0.44),(7.697;0.40),(7.610;0.50),(7.604;0.45),(7.592;3.10),(7.577;3.59),(7.565;3.50),(7.557;3.3 6),(7.545;3.32),(7.529;5.81),(7.523;5.62),(7.460;6.44),(7.352;5.80),(7.329;3.36),(6.371;0.33),(6.19 .0;16.00),(5.689;1.82),(4.811;0.74),(4.793;1.51),(4.776;1.63),(4.759;1.25),(4.743;0.41),(4.712;0.36) ,(4.695;0.58),(4.678;4.38),(4.662;5.82),(4.644;3.27),(4.516;0.37),(4.500;0.63),(4.484;0.34),(4.432; 4.12),(4.416;7.51),(4.400;3.52),(3.114;405.61),(2.661;0.52),(2.657;0.71),(2.652;0.50),(2.526;0.82), (2.510;1.70),(2.505;2.46),(2.497;36.09),(2.492;74.46),(2.488;105.04),(2.483;73.72),(2.478;35.43),( 2.319;0.50),(2.315;0.64),(2.310;0.42),(2.040;2.18),(0.008;0.67),(-0.000;20.14),(-0.008;0.66) |
| 20 | 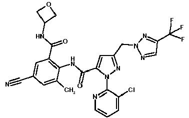 | 2,51 | 586 | (8.914;0.35),(8.457;3.22),(8.453;3.41),(8.445;3.54),(8.441;3.50),(8.432;4.33),(8.431;4.31),(8.421;0 .51),(8.413;0.33),(8.259;0.46),(8.105;3.04),(8.101;3.16),(8.085;3.39),(8.081;3.25),(7.812;2.70),(7.7 90;3.55),(7.786;2.79),(7.583;0.37),(7.573;3.34),(7.561;3.27),(7.553;3.08),(7.541;3.02),(7.222;3.62) ,(7.188;0.76),(5.931;1.06),(5.911;13.05),(4.836;0.58),(4.819;1.16),(4.802;1.29),(4.785;0.87),(4.769 ;0.32),(4.686;3.51),(4.670;5.00),(4.652;2.94),(4.606;0.46),(4.538;0.46),(4.512;0.46),(4.452;3.37),(4 .436;6.19),(4.420;2.91),(3.176;0.39),(3.158;0.73),(3.115;50.05),(3.057;0.57),(3.033;0.38),(3.026;0. 37),(2.657;0.35),(2.526;0.48),(2.510;0.78),(2.505;1.11),(2.497;19.23),(2.493;40.02),(2.488;56.60),( 2.483;39.83),(2.478;19.26),(2.315;0.40),(2.310;0.36),(2.303;1.23),(2.213;16.00),(2.191;1.01),(2.13 6:1.20),(1.939:1.32),(1.403:0.66),(1.364:0.32),(1.007:0.53),(-0.000:3.93) |
| 21 | 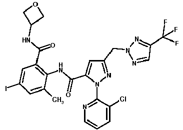 | 2,98 | 687 | (8.757;0.56),(8.450;3.00),(8.447;3.22),(8.439;3.30),(8.435;3.30),(8.427;3.87),(8.425;3.81),(8.097;2 .99),(8.093;3.06),(8.077;3.35),(8.073;3.19),(7.727;2.53),(7.723;2.80),(7.647;2.85),(7.643;2.64),(7.5 68;3.20),(7.556;3.15),(7.548;3.00),(7.536;2.96),(7.187;2.63),(5.898;11.54),(4.822;0.43),(4.804;0.9 1),(4.788;0.97),(4.771;0.62),(4.670;3.05),(4.654;4.33),(4.636;2.55),(4.602;0.37),(4.601;0.39),(4.58 5;0.34),(4.446;3.19),(4.430;5.80),(4.414;2.71),(4.237;0.32),(4.221;0.60),(3.304;0.34),(3.132;278.1 5),(2.663;0.34),(2.658;0.49),(2.654;0.39),(2.528;0.38),(2.512;1.02),(2.507;1.44),(2.499;21.08),(2.4 94;43.56),(2.489;61.51),(2.485;43.02),(2.480;20.59),(2.321;0.34),(2.316;0.40),(2.124;16.00),(2.106 ;0.92),(2.004;0.47),(1.403;2.57),(1.364;1.49),(0.989;0.52),(-0.000;2.68) |
| 22 | 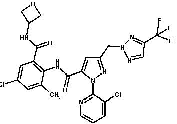 | 2,78 | 595 | (10.016;2.19),(8.768;1.31),(8.753;1.28),(8.453;2.13),(8.449;2.50),(8.447;1.80),(8.441;2.49),(8.437; 2.83),(8.435;2.35),(8.429;4.22),(8.101;2.10),(8.097;2.50),(8.095;1.59),(8.080;2.45),(8.077;2.61),(8. 075;1.66),(7.570;2.25),(7.568;1.68),(7.558;2.27),(7.556;1.69),(7.550;2.14),(7.548;1.60),(7.538;2.1 .0),(7.536;1.50),(7.432;2.71),(7.426;3.05),(7.371;3.05),(7.365;2.60),(7.197;2.82),(5.902;10.94),(4.8 30;0.63),(4.813;1.30),(4.796;1.42),(4.779;0.94),(4.763;0.35),(4.673;2.93),(4.656;4.52),(4.639;2.63) ,(4.447;3.02),(4.432;5.65),(4.416;2.63),(3.118;348.14),(3.116;281.21),(3.058;0.39),(2.662;0.49),(2. 658;0.56),(2.526;0.67),(2.497;30.78),(2.493;62.61),(2.488;88.12),(2.486;66.64),(2.484;65.18),(2.48 1;45.27),(2.479;33.34),(2.315;0.54),(2.160;16.00),(2.040;0.68),(2.038;0.58),(1.404;0.75),(-.0.000;2.90),(-0.002;2.02) |
| 23 | 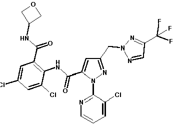 | 2,76 | 615 | (19.231;1.54),(12.550;2.00),(12.316;1.27),(10.243;3.58),(8.857;2.23),(8.843;2.31),(8.451;4.26),(8. 442;3.91),(8.431;6.04),(8.095;3.58),(8.074;3.30),(7.832;3.79),(7.759;4.40),(7.573;2.91),(7.561;3.3 7),(7.552;2.78),(7.541;2.73),(7.522;4.69),(7.271;5.26),(5.906;15.38),(4.806;1.48),(4.792;2.27),(4.7 73;2.40),(4.758;2.00),(4.670;4.51),(4.655;7.21),(4.637;3.60),(4.427;4.37),(4.411;7.94),(4.397;3.75) ,(3.924;1.52),(3.342;1.24),(3.333;1.41),(3.301;1.49),(3.292;1.53),(3.264;1.49),(3.199;3.01),(3.115; 2693.21),(3.112;1998.39),(3.092;44.09),(3.041;1.76),(3.008;1.25),(2.657;5.52),(2.628;1.30),(2.582; 1.72),(2.497;278.03),(2.493;573.05),(2.488;811.99),(2.485;606.22),(2.484;623.59),(2.481;399.74),( 2.315:4.36),(2.040:5.19),(1.404:16.00),(1.401:10.14),(1.244:2.28),(-0.000:18.10),(-0.003:11.91),(-0.740;1.27) |
| 24 | 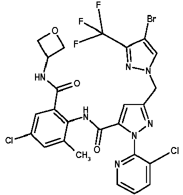 | 3,2 | 674 | (8.468;1.21),(8.465;1.33),(8.457;1.32),(8.454;1.33),(8.427;2.38),(8.139;1.13),(8.137;1.21),(8.119;1 .25),(8.117;1.25),(7.591;1.12),(7.579;1.13),(7.570;1.10),(7.559;1.04),(7.465;1.48),(7.459;1.65),(7.3 78;1.84),(7.372;1.67),(7.164;2.46),(5.578;5.01),(4.830;0.34),(4.813;0.67),(4.796;0.76),(4.779;0.52) ,(4.676;1.92),(4.660;2.86),(4.642;1.81),(4.608;7.98),(4.591;12.58),(4.575;9.07),(4.436;1.85),(4.420 ;3.39),(4.405;1.79),(4.240;8.39),(4.225;15.00),(4.209;8.47),(4.169;0.30),(3.992;1.59),(3.974;4.68),( 3.957;6.19),(3.940;3.94),(3.923;1.12),(3.664;0.33),(3.651;0.33),(3.633;0.35),(3.596;0.41),(3.548;0. 59),(3.301;17.35),(3.114;1.38),(3.033;0.80),(2.949;0.56),(2.915;0.50),(2.890;0.50),(2.863;0.47),(2. 857;0.44),(2.838;0.42),(2.788;0.39),(2.755;0.38),(2.732;0.38),(2.693;0.42),(2.669;0.67),(2.654;0.4 1),(2.633;0.50),(2.622;0.51),(2.590;0.50),(2.540;2.52),(2.504;35.43),(2.500;43.58),(2.328;0.50),(2.159;7.92),(2.069;0.55),(1.261;0.32),(1.249;0.41),(-0.000;2.84) |
| 25 | 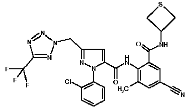 | 3,46 | 602 | (10.335;3.71),(8.944;1.84),(8.925;1.87),(7.869;3.56),(7.759;4.07),(7.570;2.66),(7.557;1.38),(7.551; 3.45),(7.546;3.09),(7.499;1.41),(7.494;1.85),(7.490;2.10),(7.484;2.88),(7.477;4.08),(7.470;5.41),(7. 466;4.74),(7.461;3.88),(7.450;3.82),(7.446;3.75),(7.438;1.06),(7.433;1.89),(7.429;2.24),(7.413;1.0 4),(7.409;0.85),(7.351;4.83),(6.289;13.35),(5.007;1.08),(4.986;1.97),(4.966;2.00),(4.946;1.10),(3.3 50;2.53),(3.328;5.38),(3.294;698.76),(3.270;2.23),(3.254;0.65),(3.232;0.33),(3.177;0.33),(3.128;3. 68),(3.107;5.86),(3.105;5.49),(3.084;2.95),(2.673;0.57),(2.668;0.79),(2.664;0.56),(2.659;0.30),(2.5 38;6.04),(2.522;2.52),(2.517;4.07),(2.508;44.75),(2.504;90.25),(2.499;122.10),(2.495;86.78),(2.490 ;40.68),(2.336;0.32),(2.331;0.60),(2.326;0.79),(2.321;0.61),(2.221;15.00),(2.067;0.67),(2.049;0.35) ,(1.399;0.34),(1.237;0.32),(1.075;0.39),(1.057;0.69),(1.047;0.91),(1.040;0.41),(1.031;0.90),(0.008; 0.74),(-0.000;18.55),(-0.008;0.58) |
| 26 | 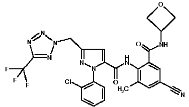 | 2,85 / 2,56 | 586 | (10.340;3.39),(9.063;2.01),(9.047;2.00),(7.875;3.70),(7.811;4.21),(7.807;3.85),(7.572;1.75),(7.568; 1.47),(7.564;0.99),(7.551;4.04),(7.547;2.01),(7.535;0.49),(7.532;0.53),(7.502;1.08),(7.496;1.32),(7. 484;2.15),(7.479;3.04),(7.475;2.09),(7.461;5.93),(7.454;3.51),(7.450;3.17),(7.438;2.58),(7.426;0.9 2),(7.418;0.61),(7.414;0.58),(7.411;0.47),(7.407;0.86),(7.402;0.54),(7.338;5.05),(7.287;0.77),(6.28 6;11.68),(6.069;1.70),(4.820;0.70),(4.802;1.55),(4.785;1.73),(4.768;1.14),(4.751;0.33),(4.676;3.78) ,(4.660;5.68),(4.641;3.09),(4.431;3.53),(4.415;6.56),(4.400;3.08),(3.295;421.16),(3.271;1.39),(3.25 8;0.37),(3.252;0.30),(2.673;0.36),(2.668;0.49),(2.664;0.36),(2.538;3.59),(2.522;1.62),(2.508;27.23) ,(2.504;54.19),(2.499;72.87),(2.495;51.46),(2.490;23.91),(2.331;0.34),(2.326;0.48),(2.321;0.32),(2. 224:15.00),(2.067:0.87),(1.399:0.42),(1.057:0.37),(1.047:0.56),(1.031:0.54),(-0.000:6.58) |
| 27 | 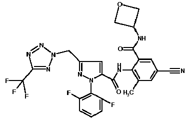 | 2,79 / 2,48 | 588 | (10.453;3.74),(9.070;2.37),(9.054;2.39),(7.894;4.22),(7.811;4.52),(7.807;4.31),(7.619;0.44),(7.603; 1.02),(7.598;0.94),(7.582;2.01),(7.565;1.19),(7.560;1.24),(7.544;0.66),(7.411;5.14),(7.366;0.85),(7. 297;3.12),(7.276;5.43),(7.255;3.16),(7.232;0.65),(6.871;0.55),(6.313;12.79),(6.097;1.83),(4.785;0. 76),(4.767;1.58),(4.749;1.82),(4.732;1.29),(4.716;0.46),(4.679;0.33),(4.664;5.27),(4.648;7.19),(4.6 29;3.83),(4.423;3.85),(4.407;7.21),(4.392;3.38),(3.360;0.54),(3.308;1135.75),(3.285;5.83),(3.268;0 .70),(2.674;0.83),(2.670;1.17),(2.665;0.84),(2.660;0.41),(2.539;1.58),(2.523;3.31),(2.518;4.99),(2.5 .09;64.75),(2.505;127.41),(2.500;172.01),(2.496;118.59),(2.491;55.89),(2.336;0.42),(2.332;0.82),(2. 327;1.21),(2.323;0.86),(2.316;1.03),(2.304;0.53),(2.233;16.00),(2.183;0.91),(2.126;0.51),(2.069;1. 33),(1.357;7.22),(1.330;0.41),(1.237;0.72),(0.008;0.42),(-0.000;12.30),(-0.008;0.44) |
| 28 | 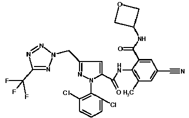 | 3,05 / 2,71 | 621 | (10.420;0.34),(10.409;0.32),(9.060;0.72),(7.988;0.65),(7.983;0.65),(7.927;0.60),(7.924;0.65),(7.87 4;3.70),(7.802;4.29),(7.799;3.99),(7.610;5.34),(7.607;5.81),(7.589;11.02),(7.570;0.64),(7.524;4.65) ,(7.506;4.05),(7.502;3.31),(7.492;0.33),(7.484;2.14),(7.412;4.13),(6.957;0.58),(6.324;13.35),(6.115 ;0.59),(5.747;0.85),(4.843;0.32),(4.815;0.78),(4.797;1.63),(4.780;1.81),(4.762;1.20),(4.746;0.37),(4 .712;0.68),(4.696;1.05),(4.677;0.87),(4.669;4.47),(4.653;6.45),(4.635;3.83),(4.532;0.67),(4.516;1.24),(4.500;0.59),(4.419;3.75),(4.403;6.96),(4.388;3.26),(3.303;246.25),(3.280;3.25),(2.674;0.45),(2. 669;0.60),(2.664;0.44),(2.539;0.80),(2.522;1.96),(2.518;3.06),(2.509;31.74),(2.505;60.41),(2.500;7 9.82),(2.496;54.66),(2.491;25.50),(2.331;0.39),(2.327;0.57),(2.322;0.46),(2.304;2.88),(2.216;16.00 ),(2.201;1.46),(2.142;0.88),(2.126;2.88),(2.070;0.96),(1.357;0.87),(-0.000;5.63) |
| 29 | 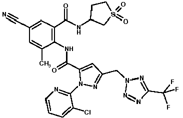 | 2,47 / 2,17 | 649 | (10.489;1.36),(8.783;1.42),(8.771;1.45),(8.483;3.87),(8.481;4.06),(8.475;4.15),(8.473;4.12),(8.160; 2.97),(8.157;3.07),(8.146;3.21),(8.144;3.20),(7.910;3.00),(7.782;3.85),(7.615;2.88),(7.608;2.95),(7. 602;2.94),(7.594;2.87),(7.458;3.21),(6.333;11.00),(6.321;0.79),(6.111;0.27),(4.482;0.90),(4.469;1. 67),(4.458;1.67),(4.445;0.94),(4.433;0.29),(3.349;250.70),(3.321;1.55),(3.307;1.57),(3.298;1.71),(3 .285;1.74),(3.267;1.59),(3.256;1.33),(3.246;0.92),(3.149;1.07),(3.136;1.48),(3.135;1.46),(3.127;0.9 9),(3.122;1.27),(3.115;1.09),(3.112;1.12),(3.100;0.83),(2.922;0.40),(2.848;1.32),(2.836;1.36),(2.82 6;1.27),(2.814;1.21),(2.618;0.31),(2.615;0.43),(2.612;0.32),(2.543;1.99),(2.524;0.84),(2.521;1.09),( 2.518;1.17),(2.509;23.98),(2.506;51.80),(2.503;71.70),(2.500;53.69),(2.497;25.70),(2.390;0.34),(2. 388;0.47),(2.385;0.34),(2.298;0.50),(2.287;1.09),(2.276;1.71),(2.256;13.50),(2.177;0.57),(2.077;0. 61),(2.037;0.55),(2.023;1.24),(2.014;0.69),(2.009;1.29),(2.001;1.15),(1.995;0.72),(1.987;1.05),(1.9 73;0.47),(1.910;0.37),(1.755;0.71),(-0.000;2.69) |
| 30 | 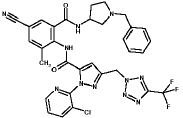 | 1,90 / 1,79 | 690 | (8.463;1.08),(8.425;5.57),(8.423;5.92),(8.417;6.08),(8.415;6.07),(8.090;4.06),(8.087;4.07),(8.076;4 .46),(8.074;4.32),(7.859;2.02),(7.728;2.80),(7.561;3.98),(7.553;3.88),(7.547;3.92),(7.539;4.00),(7.3 97:1.77),(7.332:1.95),(7.329:1.14),(7.321:3.56),(7.319:7.00),(7.307:13.50),(7.303:10.72),(7.292:2. 97),(7.260;1.60),(7.257;2.18),(7.253;1.74),(7.249;2.24),(7.246;3.30),(7.241;1.37),(7.238;1.37),(7.2 34;1.52),(7.231;0.83),(6.312;6.80),(5.762;4.86),(4.213;0.74),(4.211;0.78),(4.202;1.34),(4.199;1.28) ,(4.194;1.21),(4.190;1.25),(4.187;1.32),(4.178;0.78),(3.568;1.24),(3.546;2.90),(3.519;2.78),(3.497; 1.16),(3.356;60.70),(2.721;1.17),(2.707;1.76),(2.694;1.27),(2.543;3.90),(2.524;1.29),(2.521;1.65),( 2.518;1.58),(2.509;35.02),(2.506;76.96),(2.503;107.79),(2.500;77.86),(2.497;35.78),(2.469;1.28),(2 .456;2.23),(2.443;2.22),(2.431;1.26),(2.287;1.91),(2.278;1.33),(2.269;1.44),(2.263;1.44),(2.254;1.1 9),(2.204;9.29),(2.124;1.94),(2.036;0.97),(2.026;1.14),(2.021;1.07),(2.015;1.13),(2.010;1.07),(2.00 1;0.94),(1.599;0.78),(1.587;1.06),(1.578;1.03),(-0.000;12.56) |
| 31 | 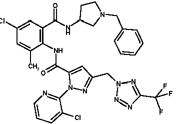 | 2,03 / 1,94 | 699 | (10.172;1.20),(8.425;2.37),(8.422;2.45),(8.413;2.62),(8.410;2.45),(8.306;1.25),(8.290;1.31),(8.090; 2.18),(8.087;2.09),(8.070;2.45),(8.066;2.26),(7.565;2.13),(7.553;2.21),(7.545;2.05),(7.533;1.99),(7. 444;2.68),(7.440;2.73),(7.349;3.93),(7.329;1.44),(7.310;4.82),(7.294;15.00),(7.280;2.11),(7.277;2. 18),(7.255;1.53),(7.251;1.55),(7.242;1.72),(7.234;1.86),(7.225;1.07),(7.219;0.87),(7.213;0.56),(6.2 91;8.64),(6.260;0.33),(6.070;0.57),(4.204;0.98),(4.187;0.95),(3.563;1.03),(3.530;4.42),(3.517;4.19) ,(3.485;0.93),(3.460;0.33),(3.301;148.74),(2.735;1.31),(2.716;1.74),(2.711;1.77),(2.693;1.36),(2.66 8;0.55),(2.539;2.31),(2.504;47.73),(2.500;56.78),(2.496;39.85),(2.468;2.02),(2.451;2.79),(2.444;2. 53),(2.426;1.60),(2.403;0.38),(2.326;0.49),(2.278;1.45),(2.265;1.54),(2.255;1.45),(2.241;1.29),(2.2 19;0.75),(2.149;12.55),(2.103;0.79),(2.069;0.46),(2.057;0.43),(2.039;0.75),(2.017;0.85),(2.007;0.89),(1.986:0.80),(1.967:0.31),(1.622:0.41),(1.609:0.76),(1.590:1.01),(1.576:0.96),(1.561:0.62),(1.54 4;0.31),(-0.000;9.74) |
| 32 | 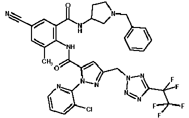 | 2,14 / 2,06 | 740 | (8.426;2.90),(8.422;3.01),(8.414;3.19),(8.410;2.96),(8.085;2.30),(8.081;2.26),(8.065;2.74),(8.061;2 .55),(8.046;0.35),(8.042;0.32),(7.833;1.80),(7.734;2.87),(7.560;2.24),(7.548;2.46),(7.540;2.23),(7.5 28;2.28),(7.516;0.37),(7.373;1.54),(7.335;1.29),(7.316;4.89),(7.300;15.00),(7.285;2.04),(7.260;1.5 0),(7.255;1.66),(7.246;1.73),(7.239;1.81),(7.230;1.08),(7.223;0.86),(7.218;0.53),(6.321;6.88),(6.10 7;0.71),(4.215;0.98),(4.210;0.97),(4.203;0.96),(4.198;0.98),(4.193;0.98),(3.578;1.07),(3.545;4.13),( 3.529;3.96),(3.496;1.08),(3.310;129.10),(2.736;1.25),(2.717;1.78),(2.713;1.72),(2.694;1.36),(2.674 ;0.44),(2.670;0.53),(2.665;0.42),(2.539;1.61),(2.505;45.48),(2.500;55.68),(2.496;39.43),(2.468;2.8 .0),(2.448;2.55),(2.428;1.50),(2.406;0.59),(2.332;0.49),(2.327;0.57),(2.323;0.49),(2.302;1.46),(2.28 8;1.72),(2.279;1.51),(2.265;1.37),(2.198;9.82),(2.125;0.57),(2.069;0.80),(2.049;0.81),(2.034;0.96),( 2.028;0.90),(2.016;0.96),(1.996;0.85),(1.987;0.52),(1.977;0.40),(1.626;0.41),(1.611;0.75),(1.594;1. 00),(1.579:0.98),(1.566:0.64),(1.548:0.33),(-0.000:9.86) |
| 33 | 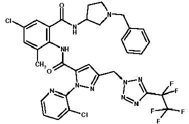 | 2,30 / 2,21 | 749 | (10.179;0.50),(8.424;2.35),(8.421;2.48),(8.413;2.72),(8.409;2.48),(8.292;0.91),(8.088;2.12),(8.084; 2.09),(8.067;2.51),(8.064;2.31),(7.563;2.16),(7.551;2.40),(7.543;2.15),(7.531;2.21),(7.438;2.70),(7. 351;3.54),(7.329;1.41),(7.310;5.03),(7.308;5.10),(7.294;15.00),(7.281;2.30),(7.278;2.28),(7.255;1. 58),(7.251;1.62),(7.241;1.93),(7.234;1.98),(7.225;1.21),(7.219;0.99),(7.213;0.66),(6.315;8.15),(6.1 .01;0.84),(4.220;0.60),(4.207;0.96),(4.202;0.95),(4.196;0.93),(4.189;0.95),(4.185;0.94),(4.172;0.57) ,(3.564;0.94),(3.531;4.68),(3.520;4.50),(3.502;0.60),(3.487;0.91),(3.307;201.11),(2.735;1.33),(2.71 7;1.80),(2.712;1.78),(2.694;1.40),(2.669;0.63),(2.539;2.02),(2.504;48.35),(2.500;59.69),(2.496;42. 18),(2.470;2.18),(2.453;2.84),(2.438;2.52),(2.423;1.63),(2.401;0.51),(2.327;0.53),(2.282;1.50),(2.2 69;1.62),(2.259;1.56),(2.245;1.40),(2.219;1.14),(2.177;0.51),(2.147;12.45),(2.104;1.22),(2.069;0.6 1),(2.038;0.78),(2.023;0.86),(2.016;0.86),(2.006;0.92),(1.987;0.89),(1.608;0.74),(1.590;1.00),(1.57 5;0.98),(1.561;0.63),(1.559;0.62),(-0.000;11.26),(-0.008;0.65) |
| 34 | 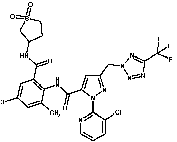 | 2,78 | 658 | (10.222;4.29),(8.692;0.36),(8.668;2.23),(8.650;2.25),(8.476;2.86),(8.473;2.94),(8.465;2.99),(8.461; 2.76),(8.148;2.66),(8.144;2.64),(8.127;3.01),(8.123;2.66),(7.608;2.68),(7.596;2.62),(7.588;2.60),(7. 576;2.47),(7.491;3.25),(7.486;3.39),(7.454;0.34),(7.406;7.14),(7.361;0.44),(7.349;3.67),(7.343;3.4 3),(6.311;11.14),(4.491;0.74),(4.472;1.20),(4.464;0.62),(4.454;1.30),(4.438;0.84),(3.566;0.34),(3.5 47;0.36),(3.481;0.58),(3.451;0.77),(3.422;1.06),(3.303;1291.83),(3.249;3.34),(3.229;2.27),(3.147;1 .28),(3.127;1.63),(3.113;1.10),(3.106;1.28),(3.093;1.10),(3.073;0.87),(3.057;0.39),(2.878;1.39),(2.8 60;1.33),(2.845;1.24),(2.826;1.19),(2.669;2.14),(2.664;1.67),(2.624;0.61),(2.539;4.48),(2.504;220. 98),(2.500;272.64),(2.496;189.51),(2.331;1.72),(2.326;2.05),(2.322;1.82),(2.301;1.12),(2.287;1.42) ,(2.271;1.42),(2.254;1.19),(2.239;0.69),(2.192;15.00),(2.145;0.52),(2.132;0.69),(2.107;0.37),(2.069 ;2.39),(2.061;0.79),(2.039;1.17),(2.020;1.24),(2.008;1.08),(1.986;0.99),(1.966;0.43),(1.908;0.41),(1 .399;0.72),(1.237;0.45),(0.901;0.91),(0.891;0.57),(-0.000;5.23) |
| 35 | 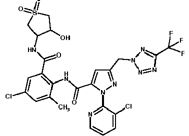 | 2,58 | 674 | (10.278;3.69),(8.479;3.18),(8.471;3.28),(8.446;2.20),(8.435;2.20),(8.159;2.50),(8.146;2.66),(7.618; 1.82),(7.610;2.07),(7.605;2.07),(7.596;1.83),(7.504;8.63),(7.405;5.32),(6.318;9.66),(5.759;1.91),(5. 658;2.61),(4.492;1.24),(4.479;1.19),(4.430;2.97),(4.425;2.84),(3.551;0.30),(3.485;2.17),(3.477;2.3 8),(3.462;2.92),(3.454;2.80),(3.371;6205.03),(3.347;39.57),(3.283;0.73),(3.242;4.22),(3.219;3.65),( 3.177;1.04),(3.157;2.38),(3.136;1.70),(3.106;1.53),(3.094;1.68),(3.073;0.84),(2.619;2.89),(2.616;3. 94),(2.613;2.86),(2.543;0.57),(2.525;6.79),(2.522;8.52),(2.519;8.95),(2.507;458.01),(2.504;615.25) ,(2.501;447.99),(2.391;2.77),(2.388;3.80),(2.385;2.71),(2.189;13.50),(2.076;3.51),(1.897;0.53),(1.2 34;0.77),(0.889;0.58),(0.096;0.42),(0.005;3.00),(-0.000;84.77),(-0.005;2.79),(-0.100;0.40) |
| 36 | 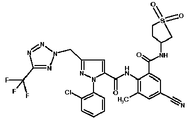 | 2,82 / 2,54 | 648 | (10.369;0.36),(8.745;0.40),(8.727;0.40),(7.888;0.78),(7.774;0.83),(7.574;0.43),(7.555;0.62),(7.550; 0.51),(7.506;0.33),(7.500;0.35),(7.495;0.39),(7.489;0.55),(7.483;0.73),(7.476;0.89),(7.472;0.83),(7. 467;0.74),(7.456;0.63),(7.452;0.61),(7.440;0.33),(7.435;0.40),(7.419;0.31),(7.387;0.91),(6.871;1.2 9),(6.611;0.74),(6.295;2.06),(6.078;0.36),(4.460;0.31),(4.444;0.30),(3.601;0.62),(3.392;0.36),(3.30 2;170.37),(3.263;1.12),(3.246;0.69),(3.231;0.60),(3.215;0.43),(3.138;0.30),(3.118;0.37),(2.881;0.3 4),(2.863;0.33),(2.847;0.31),(2.829;0.34),(2.669;0.38),(2.664;0.32),(2.539;1.57),(2.504;32.87),(2.5 .00;40.39),(2.496;28.62),(2.326;0.39),(2.322;0.34),(2.310;0.63),(2.275;0.41),(2.250;2.85),(2.228;0. 49),(2.183;2.18),(2.168;0.31),(2.133;0.56),(1.769;0.31),(1.760;0.69),(1.356;15.00),(-0.000;8.84),(-0.008;0.52) |
| 37 | 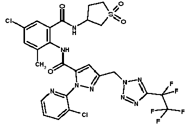 | 3,13 / 2,84 | 708 | (10.227;4.15),(8.659;2.19),(8.641;2.24),(8.475;2.88),(8.471;3.08),(8.463;3.18),(8.460;3.07),(8.143; 2.81),(8.139;2.82),(8.122;3.22),(8.119;2.95),(7.605;2.99),(7.593;2.95),(7.585;2.82),(7.573;2.83),(7. 490;2.97),(7.488;3.02),(7.484;3.32),(7.409;7.48),(7.351;3.60),(7.345;3.30),(6.335;10.88),(4.495;0. 63),(4.476;1.13),(4.459;1.15),(4.440;0.67),(3.329;1.40),(3.293;226.04),(3.276;2.86),(3.261;1.53),(3 .246;1.13),(3.228;0.82),(3.144;0.83),(3.122;1.19),(3.110;0.68),(3.102;0.99),(3.089;0.80),(3.069;0.6 1),(2.886;1.19),(2.867;1.18),(2.852;1.06),(2.833;1.02),(2.668;0.32),(2.53 8;0.68),(2.522;1.05),(2.51 7;1.71),(2.508;18.44),(2.504;37.02),(2.499;49.77),(2.495;34.91),(2.490;16.03),(2.326;0.51),(2.322; .0.50),(2.308;0.68),(2.290;1.02),(2.275;1.13),(2.257;0.83),(2.243;0.40),(2.192;15.00),(2.067;0.62),( 2.046;1.00),(2.033;0.54),(2.025;1.02),(2.012;0.84),(2.004;0.52),(1.991;0.75),(1.970;0.33),(-.0.000;2.24) |
| 38 | 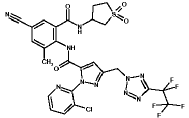 | 2,82 / 2,55 | 699 | (10.458;4.10),(8.736;2.21),(8.718;2.27),(8.481;2.82),(8.477;3.09),(8.469;3.13),(8.465;3.05),(8.149; 2.83),(8.146;2.86),(8.129;3.22),(8.126;3.01),(7.895;3.33),(7.892;3.59),(7.777;3.63),(7.773;3.56),(7. 612;2.95),(7.600;2.89),(7.592;2.78),(7.580;2.79),(7.445;4.94),(6.346;11.25),(5.742;2.20),(4.497;0. 64),(4.478;1.17),(4.461;1.18),(4.442;0.66),(3.329;1.62),(3.295;300.21),(3.276;2.44),(3.263;1.41),(3 .245;1.08),(3.231;0.80),(3.151;0.83),(3.131;1.16),(3.129;1.16),(3.118;0.65),(3.109;0.95),(3.096;0.7 9),(3.077;0.60),(2.875;1.27),(2.856;1.27),(2.841;1.12),(2.823;1.06),(2.669;0.35),(2.539;0.75),(2.52 2;1.07),(2.517;1.67),(2.509;19.02),(2.504;38.64),(2.500;52.38),(2.495;37.18),(2.491;17.27),(2.331; .0.32),(2.326;0.55),(2.308;0.73),(2.291;1.12),(2.276;1.34),(2.254;15.00),(2.067;0.71),(2.047;1.01),(2.033;0.49),(2.025;1.00),(2.013;0.82),(2.005;0.53),(1.991;0.75),(1.971;0.33),(-0.000;4.13) |
| 39 | 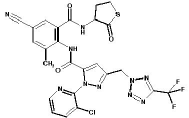 | 2,89 / 2,57 | 631 | (10.682;1.05),(8.778;0.64),(8.765;0.67),(8.573;1.58),(8.571;1.66),(8.565;1.72),(8.563;1.68),(8.232; 1.41),(8.229;1.43),(8.219;1.62),(8.216;1.55),(8.027;5.85),(7.929;1.60),(7.877;1.89),(7.874;1.83),(7. 683;1.52),(7.675;1.51),(7.670;1.52),(7.662;1.52),(7.575;1.62),(6.436;7.23),(6.246;0.43),(4.853;0.5 5),(4.841;0.70),(4.840;0.67),(4.832;0.68),(4.828;0.63),(4.820;0.69),(4.819;0.72),(4.807;0.56),(3.55 4;0.47),(3.545;0.59),(3.535;0.96),(3.526;0.95),(3.516;0.91),(3.504;13.50),(3.396;0.60),(3.394;0.65) ,(3.385;0.73),(3.382;0.73),(3.378;0.58),(3.375;0.56),(3.366;0.54),(3.364;0.47),(2.953;9.01),(2.923; 3.76),(2.920;7.99),(2.916;11.32),(2.913;7.76),(2.910;3.75),(2.784;8.91),(2.751;4.44),(2.748;9.16),( 2.745;13.39),(2.741;9.22),(2.738;4.47),(2.522;0.28),(2.520;0.33),(2.513;0.38),(2.511;0.57),(2.508; 0.41),(2.502;0.71),(2.499;0.74),(2.493;0.43),(2.490;0.65),(2.488;0.47),(2.481;0.35),(2.479;0.33),(2. 316;9.14),(2.274;0.30),(2.263;0.35),(2.254;0.71),(2.243;0.76),(2.234;0.71),(2.222;0.68),(2.213;0.2 9),(1.379:0.30),(1.296:0.59),(1.280:0.97),(-0.000:8.65) |
| 40 | 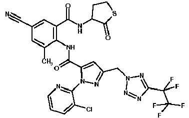 | 3,25 / 2,95 | 681 | (10.457;4.09),(8.690;2.22),(8.669;2.29),(8.490;2.82),(8.486;3.02),(8.478;3.16),(8.474;3.08),(8.158; 2.79),(8.155;2.83),(8.138;3.25),(8.134;3.04),(7.906;3.39),(7.903;3.60),(7.712;3.69),(7.708;3.62),(7. 612;2.95),(7.600;2.93),(7.592;2.77),(7.580;2.87),(7.496;0.33),(7.404;4.86),(6.340;11.20),(6.161;0. 37),(6.123;0.53),(4.698;0.72),(4.678;1.00),(4.667;0.97),(4.660;0.92),(4.648;1.06),(4.629;0.70),(3.4 47;0.56),(3.433;0.74),(3.418;1.42),(3.405;1.41),(3.390;1.18),(3.376;0.93),(3.293;348.00),(3.266;1. 37),(3.171;0.31),(2.695;0.36),(2.673;0.42),(2.668;0.62),(2.664;0.43),(2.538;1.20),(2.522;1.77),(2.5 17;2.86),(2.508;32.10),(2.504;65.13),(2.499;88.45),(2.494;62.52),(2.490;28.93),(2.330;0.45),(2.326 ;0.62),(2.321;0.46),(2.310;0.48),(2.296;0.79),(2.283;1.05),(2.279;1.10),(2.265;1.00),(2.248;0.67),(2 .227;15.00),(2.080;0.37),(2.067;0.50),(2.063;0.54),(2.050;1.00),(2.032;1.12),(2.020;0.96),(2.002;0. 93),(1.989;0.38),(1.972;0.31),(1.726;0.60),(-0.000;4.91) |
| 41 | 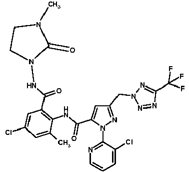 | 2,64 | 638 | (10.188;2.96),(10.107;3.73),(8.470;1.96),(8.466;1.94),(8.458;1.99),(8.454;1.88),(8.155;1.88),(8.15 2;1.77),(8.135;2.07),(8.132;1.86),(7.606;1.82),(7.594;1.83),(7.586;1.76),(7.574;1.69),(7.535;2.29),( 7.530;2.35),(7.395;4.68),(7.377;0.33),(7.359;0.39),(7.325;2.67),(7.319;2.41),(6.307;7.73),(5.746;3. 38),(3.304;404.49),(3.272;4.54),(3.247;3.05),(3.231;2.32),(2.686;15.00),(2.668;1.74),(2.539;3.51),( 2.504;67.13),(2.500;81.84),(2.496;57.36),(2.331;0.48),(2.326;0.59),(2.322;0.46),(2.197;10.35),(2.1 11;0.73),(2.069;0.55),(-0.000;19.08),(-0.008; 1.09) |
| 42 | 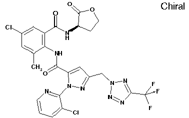 | 2,84 / 2,53 | 624 | (10.202;3.87),(8.769;2.27),(8.749;2.28),(8.474;2.70),(8.471;2.86),(8.463;3.10),(8.459;3.04),(8.449; .0.64),(8.445;0.57),(8.147;2.62),(8.143;2.58),(8.127;3.35),(8.123;3.10),(8.106;0.55),(8.102;0.51),(7. 600;2.79),(7.588;2.84),(7.580;2.63),(7.573;0.78),(7.568;2.68),(7.553;0.51),(7.508;3.32),(7.503;3.4 6),(7.365;6.44),(7.332;3.57),(7.327;3.53),(7.312;1.20),(6.302;10.51),(6.083;1.64),(4.642;0.64),(4.6 20;1.15),(4.616;1.13),(4.596;1.21),(4.573;0.68),(4.343;0.75),(4.338;0.82),(4.321;2.11),(4.316;2.03) ,(4.299;1.26),(4.294;1.08),(4.252;0.87),(4.235;1.30),(4.229;1.10),(4.226;1.23),(4.210;1.26),(4.204;0.93),(4.188;0.62),(3.289;270.39),(2.668;0.43),(2.538;1.86),(2.521;1.60),(2.508;24.88),(2.503;49.0 3),(2.499;65.43),(2.494;46.24),(2.490;21.70),(2.326;0.53),(2.320;0.52),(2.318;0.52),(2.313;0.48),(2 .302;0.52),(2.296;0.75),(2.288;0.87),(2.282;0.85),(2.272;0.89),(2.265;0.99),(2.260;0.75),(2.248;0.5 9),(2.242;0.65),(2.176;15.00),(2.157;0.84),(2.153;0.80),(2.127;1.12),(2.104;1.28),(2.101;1.23),(2.0 97;1.08),(2.074;0.83),(2.067;0.56),(2.049;0.52),(-0.000;3.30) |
| 43 | 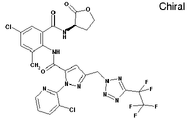 | 3,21 / 2,92 | 674 | (10.217;2.71),(8.770;1.97),(8.750;2.00),(8.473;2.72),(8.469;2.97),(8.461;3.07),(8.457;2.90),(8.144; 2.66),(8.141;2.63),(8.124;3.12),(8.120;2.85),(7.998;3.23),(7.598;2.77),(7.586;2.97),(7.578;2.63),(7. 566;2.76),(7.508;3.07),(7.504;3.33),(7.373;6.01),(7.332;3.49),(7.326;3.77),(7.318;1.16),(6.328;10. 17),(6.114;0.97),(4.618;1.17),(4.594;1.25),(4.558;1.98),(4.545;3.78),(4.532;1.98),(4.319;1.99),(4.3 14;2.04),(4.297;1.21),(4.292;1.25),(4.249;1.07),(4.233;1.31),(4.228;1.21),(4.224;1.32),(4.208;1.39) ,(4.202;1.05),(3.892;1.36),(3.887;1.45),(3.875;2.59),(3.870;1.65),(3.862;1.43),(3.85 8;1.32),(3.552; 1.85),(3.537;4.48),(3.523;4.22),(3.509;1.98),(3.285;80.99),(2.538;3.65),(2.521;3.18),(2.508;37.57), (2.503;74.46),(2.499;99.73),(2.494;71.34),(2.490;33.90),(2.290;1.30),(2.206;1.28),(2.175;15.00),(2 .156;1.25),(2.127;1.20),(2.104;1.45),(1.986;1.81),(1.966;1.34),(1.954;1.57),(1.949;1.64),(1.937;1.6 .0),(1.932;1.99),(1.920;1.91),(1.915;1.09),(1.907;1.13),(1.903;1.03),(1.754;1.15),(1.738;1.85),(1.71 9;2.36),(1.703;2.09),(1.685;1.36),(1.357;7.44),(-0.000;19.04) |
| 44 | 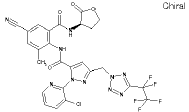 | 2,90 / 2,62 | 665 | (10.463;4.53),(8.873;2.32),(8.853;2.33),(8.477;2.89),(8.474;3.05),(8.466;3.20),(8.462;3.02),(8.151; 2.52),(8.147;2.49),(8.130;2.91),(8.127;2.75),(7.911;4.07),(7.726;4.26),(7.603;2.30),(7.591;2.47),(7. 583;2.32),(7.571;2.22),(7.407;4.71),(6.338;10.95),(6.122;0.52),(4.644;0.99),(4.621;1.79),(4.598;1. 83),(4.575;1.00),(4.344;0.96),(4.340;1.05),(4.323;2.58),(4.318;2.57),(4.301;1.59),(4.296;1.43),(4.2 56;1.30),(4.240;1.81),(4.231;1.83),(4.214;1.89),(4.209;1.33),(4.193;0.92),(3.293;604.98),(3.249;0. 56),(2.695;1.13),(2.673;0.61),(2.668;0.78),(2.664;0.56),(2.538;9.88),(2.522;3.14),(2.508;44.91),(2. 504;87.35),(2.499;115.61),(2.495;81.70),(2.490;38.22),(2.335;0.37),(2.330;0.66),(2.326;0.86),(2.32 1;0.72),(2.316;0.66),(2.309;0.67),(2.292;1.04),(2.284;1.35),(2.278;1.23),(2.268;1.34),(2.262;1.47),( 2.233;15.00),(2.197;0.38),(2.176;0.41),(2.142;0.71),(2.117;1.57),(2.093;1.73),(2.090;1.66),(2.086; 1.46),(2.067;1.59),(2.063;1.22),(2.049;0.64),(2.038;0.48),(0.008;0.35),(-0.000;7.08) |
| 45 | 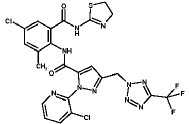 | 3,50 / 3,14 | 625 | (8.479;2.74),(8.475;2.89),(8.467;3.22),(8.463;3.15),(8.454;0.71),(8.450;0.65),(8.306;0.31),(8.153;2 .63),(8.149;2.65),(8.133;3.46),(8.129;3.21),(8.113;0.66),(8.109;0.58),(7.888;0.31),(7.759;2.33),(7.7 37;0.33),(7.605;2.90),(7.593;2.96),(7.585;2.75),(7.580;0.86),(7.573;2.79),(7.559;0.59),(7.469;0.86) ,(7.454;3.25),(7.449;3.32),(7.379;3.83),(7.329;0.77),(6.320;1.45),(6.300;11.12),(6.083;2.07),(5.742 ;13.10),(3.782;0.49),(3.697;0.70),(3.670;1.92),(3.651;3.83),(3.630;2.21),(3.342;0.51),(3.320;1.81),( 3.291;352.76),(3.271;4.71),(3.259;2.26),(3.250;5.37),(3.239;1.35),(3.231;2.64),(3.219;0.83),(3.200 ;0.37),(2.695;1.28),(2.673;0.48),(2.668;0.63),(2.664;0.47),(2.538;6.17),(2.522;2.90),(2.508;36.26),( 2.504;69.71),(2.499;91.59),(2.495;64.16),(2.490;29.71),(2.331;0.42),(2.326;0.59),(2.322;0.40),(2.2 01;0.46),(2.175;0.30),(2.155;0.33),(2.120;15.00),(2.112;4.47),(2.067;0.32),(2.049;0.50),(1.732;1.49),(1.721;0.38),(-0.000;3.37) |
| 46 | 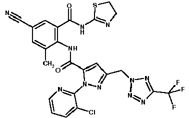 | 3,11 / 2,75 | 616 | (11.050;0.55),(8.486;2.78),(8.482;2.92),(8.474;3.02),(8.470;2.86),(8.164;2.66),(8.160;2.63),(8.144; 3.14),(8.140;3.01),(8.124;2.15),(7.850;3.36),(7.846;3.35),(7.614;2.81),(7.602;2.76),(7.594;2.64),(7. 582;2.58),(7.533;0.48),(7.410;6.97),(6.329;0.87),(6.310;12.03),(5.743;13.59),(3.690;1.69),(3.670;3 .38),(3.650;2.00),(3.358;0.37),(3.348;0.33),(3.335;0.47),(3.290;239.18),(3.258;2.84),(2.695;0.55),( 2.673;0.37),(2.668;0.50),(2.664;0.37),(2.539;3.29),(2.522;1.88),(2.509;26.72),(2.504;51.88),(2.499 ;68.89),(2.495;48.91),(2.490;23.16),(2.331;0.34),(2.326;0.45),(2.322;0.40),(2.163;15.00),(2.068;0. 55),(2.049;0.33),(1.737;0.91),(0.008;0.37),(-0.000;7.27) |
| 47 | 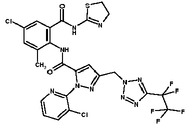 | 3,90 / 3,58 | 675 | (10.644;0.84),(8.477;2.79),(8.473;2.95),(8.465;3.14),(8.462;2.84),(8.457;0.51),(8.453;0.40),(8.151; 2.64),(8.147;2.66),(8.131;3.11),(8.127;2.74),(8.113;0.38),(8.109;0.34),(7.766;1.99),(7.604;2.91),(7. 592;3.04),(7.583;2.72),(7.572;2.84),(7.560;0.34),(7.474;0.65),(7.459;3.17),(7.454;3.28),(7.391;3.7 4),(7.335;0.42),(6.345;1.06),(6.328;10.87),(6.117;1.02),(5.743;10.91),(3.793;0.37),(3.692;0.67),(3. 668;1.80),(3.648;3.65),(3.628;2.14),(3.320;0.90),(3.288;248.48),(3.271;4.36),(3.250;5.24),(3.231;2 .58),(2.695;0.84),(2.673;0.41),(2.668;0.53),(2.664;0.42),(2.538;5.41),(2.521;2.37),(2.508;31.21),(2. 504;60.40),(2.499;79.76),(2.495;56.20),(2.490;26.22),(2.330;0.40),(2.326;0.51),(2.321;0.36),(2.202 ;0.43),(2.122;15.00),(2.067;0.58),(2.049;0.41),(1.730;1.02),(0.008;0.46),(-0.000;9.14),(-0.008;0.31) |
| 48 | 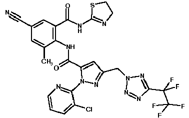 | 3,49 / 3,18 | 666 | (8.482;2.95),(8.478;3.12),(8.470;3.24),(8.466;3.06),(8.159;2.88),(8.155;2.89),(8.139;3.49),(8.135;3 .67),(8.129;2.48),(7.840;2.97),(7.836;2.96),(7.609;3.00),(7.598;2.98),(7.589;2.82),(7.578;2.80),(7.5 38;0.55),(7.421;7.19),(6.354;0.89),(6.331;11.60),(5.742;6.58),(3.787;0.40),(3.694;1.90),(3.674;3.7 6),(3.654;2.24),(3.358;0.41),(3.291;412.98),(3.270;6.82),(3.250;2.92),(3.239;0.56),(2.695;0.72),(2. 673;0.50),(2.669;0.64),(2.664;0.52),(2.539;4.97),(2.522;2.72),(2.509;39.08),(2.504;75.92),(2.499;1 .00.58),(2.495;70.95),(2.490;33.02),(2.331;0.47),(2.326;0.67),(2.322;0.47),(2.157;15.00),(2.049;0.4 9),(1.736;1.00),(-0.000;2.45) |
| 49 | 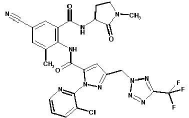 | 2,39 / 2,19 | 628 | (10.443;3.38),(8.630;1.71),(8.610;1.72),(8.488;0.34),(8.479;2.33),(8.476;2.38),(8.468;2.45),(8.464; 2.35),(8.155;2.39),(8.151;2.37),(8.135;2.54),(8.131;2.35),(7.883;2.79),(7.880;2.89),(7.724;3.12),(7. 720;2.90),(7.607;2.30),(7.602;0.51),(7.595;2.27),(7.587;2.17),(7.575;2.12),(7.401;4.44),(6.310;9.6 2),(6.089;0.32),(4.426;0.50),(4.404;1.34),(4.383;1.32),(4.360;0.47),(3.289;472.64),(3.269;5.05),(3. 247;2.86),(3.239;1.86),(3.223;0.71),(3.215;0.52),(2.797;0.30),(2.783;0.32),(2.747;15.00),(2.677;1. 15),(2.672;1.20),(2.668;1.66),(2.658;0.58),(2.538;2.01),(2.521;5.79),(2.508;80.42),(2.503;156.24),( 2.499;207.18),(2.494;146.48),(2.490;68.74),(2.334;0.59),(2.330;1.06),(2.326;1.38),(2.321;0.99),(2. 210;13.31),(2.188;1.01),(2.179;0.99),(2.171;1.04),(2.165;0.78),(2.158;0.61),(2.148;0.43),(2.141;0. 39),(2.067;2.91),(2.049;0.35),(1.770;0.32),(1.747;0.92),(1.739;0.41),(1.725;0.98),(1.717;0.86),(1.7 02:0.41),(1.694:0.79),(1.238:0.43),(0.008:0.95),(-0.000:19.77),(-0.008:0.68) |
| 50 | 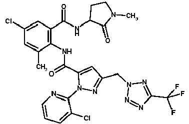 | 2,66 / 2,36 | 637 | (10.179;2.96),(8.507;1.63),(8.486;1.77),(8.474;2.25),(8.470;2.30),(8.462;2.33),(8.458;2.28),(8.435; 0.79),(8.432;0.81),(8.424;0.85),(8.420;0.80),(8.357;0.83),(8.353;0.84),(8.224;0.76),(8.221;0.76),(8. 204;0.82),(8.200;0.75),(8.148;2.09),(8.145;2.09),(8.128;2.37),(8.124;2.18),(7.953;0.99),(7.601;2.2 .0),(7.589;2.93),(7.581;2.21),(7.577;1.22),(7.569;2.67),(7.557;0.83),(7.505;2.83),(7.479;2.44),(7.47 4;2.59),(7.367;4.80),(7.337;2.92),(7.331;2.61),(6.300;8.31),(6.288;0.63),(4.425;0.51),(4.402;1.26),( 4.381;1.26),(3.720;6.44),(3.287;494.85),(3.260;3.39),(3.237;2.45),(3.230;1.66),(3.213;0.57),(2.968 ;0.55),(2.740;15.00),(2.672;1.12),(2.668;1.84),(2.663;1.06),(2.538;1.86),(2.521;5.05),(2.508;72.68) ,(2.503;142.17),(2.499;189.60),(2.494;135.28),(2.490;64.25),(2.330;0.95),(2.326;1.23),(2.321;0.94) ,(2.316;0.50),(2.227;0.82),(2.211;0.45),(2.203;0.46),(2.187;1.14),(2.180;0.99),(2.171;1.22),(2.156; 12.36),(2.133;0.58),(2.107;0.90),(2.067;4.23),(1.924;4.51),(1.749;0.84),(1.726;0.92),(1.718;0.83),( 1.695;0.75),(1.237;0.48),(0.008;0.80),(-0.000;17.46),(-0.008;0.63) |
| 51 | 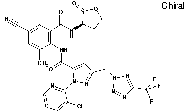 | 2,52 | 515 | (10.355;0.42),(8.060;0.46),(7.898;0.41),(7.885;0.42),(7.780;0.54),(7.621;0.63),(7.435;0.71),(7.416; 1.06),(7.263;71.87),(7.163;0.36),(7.087;0.40),(6.059;1.44),(4.550;0.32),(3.649;0.33),(3.641;0.31),( 3.498;0.59),(2.966;4.26),(2.897;1.00),(2.269;2.44),(2.014;1.34),(1.603;2.01),(1.479;0.40),(1.433;0. 85),(1.411:0.27),(1.333:0.51),(1.284:1.36),(1.253:13.50),(1.222:1.12),(1.105:0.43),(0.892:1.12),(0. 880;1.71),(0.868;1.02),(0.855;0.50),(0.840;0.68),(0.069;0.93),(-0.000;49.30),(-0.006;1.89) |
| 52 | 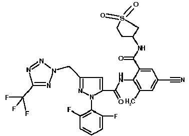 | 2,75 / 2,47 | 650 | (10.483;4.56),(8.768;2.43),(8.751;2.52),(7.910;3.46),(7.909;3.83),(7.905;4.14),(7.784;4.01),(7.780; 3.98),(7.610;0.93),(7.604;0.95),(7.588;1.83),(7.583;0.78),(7.572;1.20),(7.567;1.11),(7.551;0.58),(7. 461;6.22),(7.411;1.06),(7.306;0.73),(7.285;1.66),(7.264;1.64),(7.243;0.74),(6.321;12.30),(6.312;1. 26),(6.107;1.87),(4.452;0.70),(4.434;1.30),(4.417;1.28),(4.398;0.73),(3.342;0.47),(3.308;603.37),(3 .274;2.45),(3.259;3.10),(3.240;2.57),(3.226;1.00),(3.147;0.99),(3.127;1.36),(3.125;1.35),(3.114;0.7 7),(3.105;1.13),(3.092;0.93),(3.072;0.67),(2.869;1.32),(2.851;1.30),(2.835;1.16),(2.817;1.10),(2.67 4;0.46),(2.670;0.63),(2.665;0.46),(2.540;0.85),(2.523;1.48),(2.518;2.21),(2.510;33.07),(2.505;66.2 5),(2.500;91.10),(2.496;62.59),(2.491;29.67),(2.332;0.47),(2.327;0.64),(2.323;0.46),(2.277;0.95),(2 .258;16.00),(2.228;1.06),(2.174;1.03),(2.069;2.49),(2.053;0.49),(2.032;1.03),(2.019;0.49),(2.011;1. 10),(2.000;0.86),(1.991;0.58),(1.977;0.83),(-0.000;5.88) |
| 53 | 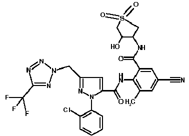 | 2,64 7 2,36 | 664 | (10.404;4.75),(10.394;1.69),(8.522;1.93),(8.513;1.05),(8.503;2.02),(7.924;3.44),(7.920;5.18),(7.89 4;5.04),(7.891;4.12),(7.587;1.65),(7.585;1.52),(7.583;1.28),(7.568;3.69),(7.564;2.37),(7.552;0.93),( 7.550;1.00),(7.516;1.23),(7.510;1.44),(7.500;1.90),(7.497;1.56),(7.494;3.26),(7.486;2.35),(7.481;1. 52),(7.475;5.21),(7.472;4.80),(7.467;2.69),(7.464;3.60),(7.460;3.59),(7.448;1.82),(7.444;1.66),(7.4 38;1.14),(7.435;1.28),(7.419;1.48),(7.414;1.01),(7.377;6.48),(7.319;1.79),(6.294;12.51),(6.078;3.2 2),(5.653;3.00),(5.644;3.03),(4.506;1.13),(4.496;1.00),(4.486;1.11),(4.478;0.95),(4.416;2.12),(4.40 7;1.99),(3.498;1.22),(3.487;1.24),(3.463;1.72),(3.452;1.49),(3.308;871.73),(3.250;2.99),(3.215;2.3 5),(3.175;0.89),(3.145;2.23),(3.115;1.63),(3.085;1.49),(3.066;1.60),(3.053;0.91),(2.669;0.87),(2.53 9;1.25),(2.523;2.34),(2.518;3.57),(2.509;46.83),(2.505;92.00),(2.500;124.44),(2.496;84.39),(2.491;38.94),(2.246;16.00),(2.240;6.71),(2.069;13.76),(-0.000;3.65) |
| 54 | 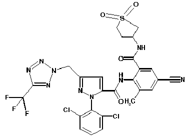 | 2,95 / 2,65 | 682 | (10.448;4.62),(10.435;1.05),(8.739;2.51),(8.721;2.54),(7.894;3.89),(7.891;4.12),(7.890;3.86),(7.77 2;4.01),(7.768;4.13),(7.624;2.44),(7.620;3.01),(7.604;3.66),(7.600;4.73),(7.594;2.42),(7.590;2.77),( 7.585;0.83),(7.581;1.03),(7.573;5.37),(7.569;3.59),(7.555;1.00),(7.529;4.68),(7.514;1.18),(7.509;5. 33),(7.494;1.08),(7.489;2.09),(7.451;8.15),(7.391;1.39),(6.331;12.83),(6.123;1.83),(4.478;0.75),(4. 460;1.34),(4.442;1.28),(3.308;1276.36),(3.283;4.22),(3.262;2.75),(3.248;1.84),(3.233;1.38),(3.215; 1.04),(3.138;0.96),(3.118;1.38),(3.103;0.80),(3.097;1.08),(3.085;0.89),(3.082;0.87),(2.862;1.37),(2. 843;1.34),(2.828;1.21),(2.809;1.18),(2.674;0.92),(2.669;1.28),(2.665;0.95),(2.539;1.70),(2.523;3.0 9),(2.518;4.57),(2.509;68.08),(2.505;136.19),(2.500;186.61),(2.496;127.85),(2.491;60.37),(2.332;0. 91),(2.327;1.31),(2.322;0.92),(2.284;0.87),(2.267;1.41),(2.246;16.00),(2.237;4.59),(2.069;1.06),(2. 032;1.14),(2.011;1.16),(1.999;0.98),(1.978;0.89),(-0.000;2.64) |
| 55 | 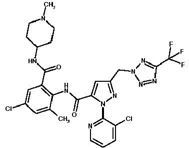 | 4,14 | 637 | (10.188;0.30),(8.462;1.75),(8.458;1.83),(8.450;1.88),(8.446;1.74),(8.136;1.68),(8.132;1.63),(8.116; 1.91),(8.112;1.70),(8.087;0.99),(8.068;0.98),(7.602;1.72),(7.590;1.69),(7.582;1.62),(7.570;1.54),(7. 447;2.00),(7.441;2.10),(7.371;4.20),(7.277;2.22),(7.272;2.05),(6.309;6.71),(6.287;0.36),(3.555;0.4 8),(3.546;0.56),(3.535;0.52),(3.527;0.55),(3.300;76.45),(2.897;1.91),(2.765;0.30),(2.709;1.28),(2.6 79;1.41),(2.539;1.84),(2.508;27.35),(2.504;47.02),(2.500;58.17),(2.496;40.05),(2.331;0.38),(2.326; .0.48),(2.322;0.38),(2.158;15.00),(2.139;2.00),(2.069;0.51),(2.040;0.45),(1.951;0.75),(1.927;1.23),( 1.899;0.83),(1.795;0.31),(1.624;1.03),(1.597;1.28),(1.429;0.57),(1.421;0.55),(1.395;1.30),(1.370;0. 99),(1.363;0.96),(1.342;0.38),(1.331;0.33),(-0.000;4.01) |
| 56 | 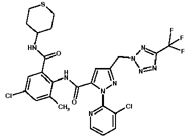 | 3,58 | 640 | (8.466;2.37),(8.462;2.47),(8.454;2.52),(8.451;2.34),(8.133;2.21),(8.129;2.14),(8.113;2.43),(8.109;2 .19),(7.594;2.21),(7.583;2.19),(7.574;2.08),(7.562;1.99),(7.421;2.52),(7.415;2.64),(7.341;3.13),(7.3 .05;2.93),(7.299;2.65),(6.298;9.26),(6.286;1.14),(3.667;0.72),(3.639;1.21),(3.630;1.16),(3.620;1.29) ,(3.613;1.18),(3.604;1.00),(3.594;0.97),(3.383;4.68),(3.367;4.55),(3.362;4.51),(3.349;4.42),(3.342; 4.41),(2.904;2.94),(2.669;0.91),(2.664;0.72),(2.641;2.32),(2.630;3.96),(2.617;6.30),(2.610;9.77),(2. 597;15.00),(2.589;13.38),(2.562;5.50),(2.556;5.78),(2.539;2.24),(2.528;2.84),(2.522;3.88),(2.504;4 3.37),(2.500;53.34),(2.496;37.17),(2.148;12.35),(2.123;0.69),(2.069;0.58),(2.038;1.12),(2.016;3.57 ),(2.007;3.61),(1.993;2.58),(1.984;4.35),(1.975;4.07),(1.943;1.71),(1.934;1.70),(1.911;1.82),(1.902; 1.66),(1.502;0.77),(1.490;1.01),(1.475;1.37),(1.464;1.61),(1.451;1.45),(1.445;1.57),(1.438;1.59),(1. 429;1.74),(1.422;1.14),(1.406;0.81),(1.393;1.55),(1.383;1.55),(1.367;2.90),(1.357;3.96),(1.334;3.0 3),(1.326;2.53),(1.308;1.28),(1.299;1.22),(-0.000;2.20) |
| 57 | 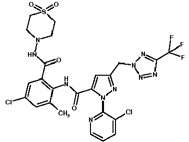 | 2,57 | 673 | (10.290;0.28),(10.270;3.82),(9.616;4.57),(8.487;2.50),(8.485;2.80),(8.479;2.74),(8.477;2.89),(8.18 2;2.46),(8.180;2.58),(8.169;2.74),(8.166;2.71),(7.619;2.58),(7.611;2.46),(7.605;2.51),(7.597;2.52),( 7.498;2.67),(7.495;2.86),(7.434;7.36),(7.412;0.77),(7.325;3.18),(7.321;3.13),(6.872;0.29),(6.324;1 0.44),(6.317;0.74),(6.312;0.89),(3.701;0.27),(3.536;0.30),(3.531;0.31),(3.366;17.57),(3.263;0.61),( 3.259;0.57),(3.248;0.61),(3.241;0.54),(3.178;3.63),(3.170;4.96),(3.151;7.59),(3.144;8.58),(3.136;7. 21),(3.097;0.52),(3.076;1.82),(3.069;2.12),(3.065;1.97),(3.059;3.14),(3.053;5.53),(3.047;5.02),(3.0 43;5.00),(3.035;3.89),(2.987;1.22),(2.982;2.45),(2.972;2.29),(2.964;1.48),(2.616;0.30),(2.525;0.55) ,(2.522;0.69),(2.519;0.69),(2.510;15.67),(2.507;33.88),(2.504;46.71),(2.501;34.40),(2.498;15.95),( 2.388;0.29),(2.301;0.46),(2.202;13.50),(2.184;0.53),(2.135;0.97),(2.125;0.54),(2.077;0.39),(1.355; 3.51),(-0.000;0.38) |
| 58 | 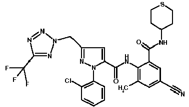 | 3,65 / 3,31 | 631 | (7.802;0.37),(7.766;3.88),(7.550;1.13),(7.532;2.20),(7.513;0.52),(7.485;0.81),(7.479;0.89),(7.468;1 .33),(7.462;1.90),(7.457;1.60),(7.444;4.51),(7.437;2.60),(7.425;1.56),(7.411;0.66),(7.405;0.49),(7.3 88;0.49),(7.275;1.42),(6.871;1.41),(6.611;0.46),(6.268;6.45),(6.051;1.11),(3.681;0.38),(3.655;0.76) ,(3.646;0.70),(3.636;0.79),(3.628;0.80),(3.618;1.37),(3.601;2.60),(3.585;1.12),(3.378;1.21),(3.301; 30.75),(2.904;0.31),(2.773;0.43),(2.766;0.40),(2.746;0.35),(2.673;0.60),(2.669;0.70),(2.631;2.43),( 2.622;3.09),(2.605;4.92),(2.598;4.64),(2.586;4.60),(2.576;3.74),(2.539;2.27),(2.504;43.67),(2.500; 53.55),(2.496;38.99),(2.405;0.39),(2.331;0.44),(2.326;0.49),(2.277;0.80),(2.175;7.88),(2.125;1.02), (2.095;0.35),(2.069;1.04),(2.054;0.52),(2.044;0.74),(2.035;0.75),(1.992;0.31),(1.944;1.46),(1.936;1 .42),(1.912;1.61),(1.903;1.40),(1.776;1.13),(1.768;1.37),(1.760;2.81),(1.751;1.33),(1.743;1.06),(1.4 93;0.60),(1.474;1.10),(1.444;1.93),(1.436;1.91),(1.429;2.31),(1.422;1.71),(1.415;1.60),(1.396;0.78) ,(1.382;0.56),(1.357;15.00),(1.237;0.38),(-0.000;12.98) |
| 59 | 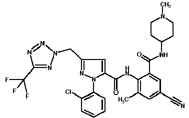 | 1,79 / 1,67 | 628 | (8.045;0.32),(8.040;0.33),(7.834;1.31),(7.829;1.13),(7.653;0.90),(7.641;0.37),(7.531;0.41),(7.527;0 .54),(7.524;0.37),(7.510;1.17),(7.506;0.83),(7.489;0.37),(7.462;0.35),(7.455;0.70),(7.447;0.81),(7.4 40;0.95),(7.436;0.56),(7.433;0.67),(7.429;0.56),(7.421;2.51),(7.416;1.45),(7.404;0.94),(7.394;0.39) ,(7.390;0.42),(7.364;0.38),(7.128;1.29),(7.057;0.37),(6.232;3.71),(6.016;1.00),(3.618;0.57),(3.607; .0.60),(3.601;0.96),(3.596;0.69),(3.591;0.65),(3.585;0.78),(3.568;0.71),(3.559;0.78),(3.550;0.78),(3. 542;0.84),(3.532;0.81),(3.516;0.79),(3.355;1.62),(3.171;0.63),(2.702;1.25),(2.673;1.63),(2.610;0.8 4),(2.581;0.76),(2.538;1.24),(2.522;0.47),(2.508;11.08),(2.504;22.70),(2.499;31.05),(2.495;22.53),( 2.490;11.00),(2.237;1.39),(2.125;15.00),(2.118;3.81),(2.106;5.58),(2.099;6.47),(2.083;1.95),(2.067 ;0.46),(1.924;0.64),(1.895;1.80),(1.866;2.03),(1.837;0.90),(1.832;0.88),(1.769;0.39),(1.760;0.72),(1 .751;0.32),(1.743;0.32),(1.715;0.96),(1.687;1.18),(1.657;0.77),(1.624;0.82),(1.373;0.66),(1.357;1.7 4),(1.345;1.40),(1.315;1.30),(1.286;0.51),(-0.000;1.76) |
| 60 | 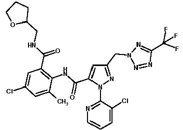 | 3,25 | 624 | (8.471;2.58),(8.467;2.70),(8.459;2.77),(8.456;2.62),(8.275;1.29),(8.148;2.43),(8.144;2.40),(8.128;2 .73),(8.124;2.50),(7.601;2.49),(7.589;2.48),(7.581;2.38),(7.569;2.30),(7.453;2.99),(7.448;3.22),(7.3 43;5.45),(7.316;3.58),(7.310;3.29),(6.306;10.00),(3.815;1.34),(3.799;2.06),(3.783;1.45),(3.776;0.7 1),(3.767;0.61),(3.756;0.73),(3.707;0.90),(3.689;1.44),(3.671;1.87),(3.654;1.01),(3.647;0.62),(3.55 9;0.98),(3.541;1.92),(3.524;1.46),(3.505;0.71),(3.376;0.68),(3.302;86.42),(3.235;1.01),(3.220;1.27) ,(3.202;1.52),(3.187;2.20),(3.172;1.32),(3.162;1.29),(3.147;2.10),(3.132;1.37),(3.114;0.98),(3.098; 0.60),(2.889;2.29),(2.695;0.58),(2.675;0.73),(2.669;0.57),(2.664;0.55),(2.539;1.74),(2.504;48.89),( 2.500;60.23),(2.496;41.97),(2.144;15.00),(1.837;0.59),(1.826;0.66),(1.822;0.69),(1.816;0.63),(1.80 9;0.67),(1.804;0.74),(1.790;0.81),(1.783;0.86),(1.768;1.30),(1.760;1.01),(1.752;1.89),(1.742;1.52),( 1.734;2.09),(1.717;2.16),(1.699;1.90),(1.682;1.10),(1.503;0.59),(1.486;0.76),(1.474;0.98),(1.465;0. 80),(1.457;1.21),(1.450;0.68),(1.440;0.80),(-0.000;1.85) |
| 61 | 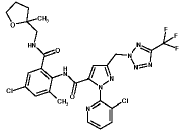 | 3,48 | 638 | (10.215;2.78),(8.472;2.30),(8.469;2.36),(8.461;2.43),(8.457;2.25),(8.178;0.95),(8.163;1.86),(8.151; 2.81),(8.147;2.93),(8.130;2.44),(8.127;2.20),(7.601;2.18),(7.589;2.16),(7.581;2.08),(7.569;1.98),(7. 457;2.70),(7.451;2.85),(7.323;6.14),(7.312;3.35),(7.306;2.94),(6.306;9.12),(3.778;0.43),(3.761;0.3 2),(3.656;2.14),(3.650;1.31),(3.640;3.89),(3.624;2.10),(3.303;99.02),(3.168;3.35),(3.152;3.19),(3.1 32;0.33),(2.884;0.68),(2.846;1.12),(2.539;0.79),(2.504;22.61),(2.500;28.03),(2.496;19.72),(2.143;1 3.01),(1.876;0.34),(1.863;0.35),(1.847;0.34),(1.790;0.39),(1.768;1.18),(1.754;2.51),(1.751;2.40),(1. 744;2.97),(1.736;3.13),(1.721;1.38),(1.705;0.59),(1.697;0.42),(1.418;0.85),(1.403;1.02),(1.396;1.1 3),(1.387;0.96),(1.188;2.41),(1.117;0.64),(1.024;15.00),(-0.000;0.78) |
| 62 | 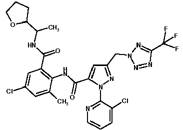 | 3,45 | 638 | (10.179;2.65),(10.171;2.04),(8.467;2.42),(8.463;2.64),(8.455;2.57),(8.451;2.42),(8.141;2.20),(8.12 2;2.18),(8.048;0.91),(8.027;0.88),(7.904;1.19),(7.884;1.11),(7.600;1.76),(7.589;1.66),(7.580;1.74),( 7.569;1.61),(7.453;3.11),(7.448;3.31),(7.351;3.04),(7.341;4.12),(7.283;2.08),(7.277;2.04),(7.255;1. 53),(7.249;1.44),(6.309;10.27),(3.921;0.86),(3.903;1.01),(3.884;0.86),(3.786;1.40),(3.772;0.86),(3. 754;0.89),(3.703;0.96),(3.686;1.81),(3.671;2.06),(3.654;1.61),(3.635;1.48),(3.616;1.62),(3.596;1.4 6),(3.580;2.04),(3.561;2.12),(3.536;1.47),(3.519;2.03),(3.500;2.03),(3.483;1.83),(3.305;2961.58),(3 .203;2.27),(3.176;1.61),(3.083;0.75),(3.068;0.77),(2.693;1.01),(2.673;3.60),(2.669;4.63),(2.664;3.5 5),(2.618;1.62),(2.539;14.76),(2.504;445.98),(2.500;547.78),(2.496;387.21),(2.349;0.90),(2.331;3.4 6),(2.327;4.01),(2.322;3.16),(2.149;15.00),(2.069;2.22),(1.746;0.90),(1.730;1.20),(1.714;1.14),(1.6 98;1.77),(1.685;2.29),(1.669;2.38),(1.651;1.82),(1.510;0.78),(1.489;0.93),(1.462;0.78),(1.237;1.78) ,(1.003;3.62),(0.987;3.64),(0.965;5.08),(0.948;4.95),(-0.000;29.23) |
| 63 | 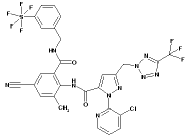 | 4,02 | 747 | (8.452;1.07),(8.448;1.17),(8.440;1.17),(8.436;1.14),(8.086;0.97),(8.083;0.99),(8.066;1.07),(8.062;1 .03),(7.885;0.50),(7.799;2.80),(7.789;1.35),(7.721;0.56),(7.715;0.66),(7.701;0.65),(7.697;0.63),(7.6 14;0.41),(7.561;1.34),(7.550;1.75),(7.541;1.34),(7.530;1.71),(7.472;0.56),(7.452;0.73),(7.251;0.77) ,(6.245;4.50),(4.457;1.44),(4.443;1.44),(3.886;0.85),(3.120;185.81),(2.632;0.92),(2.527;1.30),(2.51 1;0.69),(2.506;1.01),(2.498;16.38),(2.493;33.95),(2.489;48.12),(2.484;33.58),(2.479;16.00),(2.198; 5.37),(1.364;1.47),(-0.000;3.05) |
| 64 | 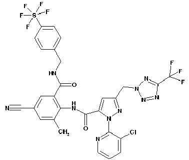 | 4,01 | 747 | (8.444;2.39),(8.440;2.56),(8.432;2.58),(8.429;2.54),(8.091;2.28),(8.087;2.31),(8.071;2.56),(8.067;2 .37),(7.845;0.38),(7.838;2.31),(7.833;3.16),(7.825;1.92),(7.820;0.80),(7.808;2.68),(7.803;3.23),(7.7 90;0.41),(7.767;0.34),(7.755;0.54),(7.748;4.44),(7.743;1.47),(7.731;1.60),(7.726;5.26),(7.720;0.68) ,(7.637;0.35),(7.614;0.38),(7.580;1.11),(7.566;2.99),(7.555;3.17),(7.546;2.61),(7.535;2.54),(7.479; 2.67),(7.457;2.42),(7.295;3.09),(7.011;0.43),(6.249;0.96),(6.239;10.07),(4.437;2.52),(4.423;2.51),( 3.866;1.11),(3.117;39.09),(2.632;1.54),(2.630;1.09),(2.527;1.28),(2.511;0.71),(2.506;1.01),(2.498; 16.59),(2.493;34.25),(2.489;48.31),(2.484;33.65),(2.479;16.00),(2.293;0.65),(2.208;12.39),(2.142;0 .60),(2.021;0.73),(-0.000;5.57) |
| 65 | 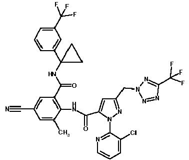 | 3,97 | 715 | (8.945;1.29),(8.475;0.34),(8.472;2.36),(8.468;2.36),(8.464;0.49),(8.460;2.59),(8.456;2.32),(8.120;0 .33),(8.116;2.14),(8.112;2.15),(8.104;0.32),(8.100;0.37),(8.096;2.44),(8.092;2.25),(8.017;0.44),(8.0 14;0.34),(7.833;1.97),(7.830;2.43),(7.805;2.55),(7.801;1.97),(7.617;0.34),(7.588;2.56),(7.576;2.49) ,(7.568;2.46),(7.556;3.35),(7.536;1.49),(7.532;1.53),(7.522;0.34),(7.455;0.82),(7.434;1.51),(7.405; 3.00),(7.384;1.55),(7.365;0.57),(7.283;4.74),(6.259;9.56),(3.125;94.82),(2.658;0.32),(2.634;0.98),( 2.632;1.38),(2.631;0.97),(2.527;1.72),(2.511;0.79),(2.507;1.10),(2.498;16.41),(2.494;34.06),(2.489 ;48.11),(2.484;33.58),(2.480;16.00),(2.294;0.44),(2.214;10.95),(2.144;0.45),(2.117;1.03),(1.364;0. 66),(1.202;13.55),(1.125;0.39),(1.120;0.48),(1.112;0.38),(1.075;0.39),(1.067;0.47),(1.061;0.38),(-0.000;0.52) |
| 66 | 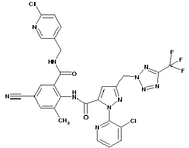 | 3,11 | 656 | (8.969;0.00),(8.956;0.00),(8.942;0.00),(8.495;0.00),(8.492;0.00),(8.483;0.00),(8.480;0.00),(8.333;0 .00),(8.327;0.00),(8.149;0.00),(8.145;0.00),(8.129;0.00),(8.125;0.00),(8.003;0.00),(7.889;0.00),(7.8 33;0.00),(7.709;0.00),(7.703;0.00),(7.688;0.00),(7.682;0.00),(7.608;0.00),(7.596;0.00),(7.588;0.00) ,(7.576;0.00),(7.346;0.01),(7.327;0.01),(6.307;0.01),(4.344;0.01),(4.330;0.01),(3.660;0.00),(3.577; .0.00),(3.561;0.02),(3.547;0.00),(3.533;0.00),(3.509;0.05),(3.503;0.02),(3.500;0.02),(3.461;16.00),( 3.416;0.02),(3.408;0.02),(3.385;0.01),(3.372;0.01),(3.367;0.01),(3.362;0.01),(3.361;0.01),(3.350;0. .00),(3.339;0.00),(3.334;0.00),(3.317;0.00),(3.311;0.00),(3.287;0.00),(3.275;0.00),(3.256;0.00),(2.6 85;0.00),(2.680;0.00),(2.676;0.00),(2.559;0.00),(2.554;0.00),(2.550;0.00),(2.533;0.01),(2.528;0.01) ,(2.520;0.10),(2.515;0.21),(2.511;0.29),(2.506;0.21),(2.502;0.09),(2.342;0.00),(2.338;0.00),(2.333; 0.00),(2.220;0.01),(0.008;0.00),(-0.000;0.09) |
| 67 | 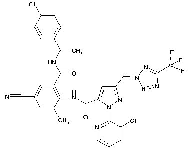 | 3,91 | 669 | (10.307;1.20),(8.568;0.93),(8.553;0.93),(8.464;3.35),(8.460;3.46),(8.452;3.70),(8.448;3.46),(8.102; 2.99),(8.098;2.91),(8.081;3.32),(8.078;3.08),(7.974;1.24),(7.812;2.67),(7.760;3.55),(7.581;3.34),(7. 569;3.30),(7.561;3.24),(7.549;3.09),(7.335;3.49),(7.314;8.56),(7.285;12.27),(7.280;5.88),(7.269;2. 25),(7.264;4.22),(6.259;14.30),(4.999;0.34),(4.981;1.41),(4.963;2.15),(4.945;1.43),(4.927;0.39),(3. 108;297.63),(2.662;0.42),(2.657;0.63),(2.652;0.48),(2.526;0.43),(2.520;0.35),(2.510;1.41),(2.497;3 4.94),(2.492;70.47),(2.488;97.51),(2.483;67.22),(2.478;31.58),(2.319;0.41),(2.315;0.54),(2.309;0.4 4),(2.207;16.00),(1.330;10.94),(1.312;10.82),(1.247;0.40),(0.008;0.36),(-0.000;9.41) |
| 68 | 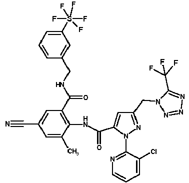 | 3,69 | 747 | (9.031;0.38),(8.441;3.08),(8.437;3.26),(8.429;3.31),(8.426;3.20),(8.070;2.89),(8.066;2.92),(8.050;3 .19),(8.046;3.03),(7.940;0.40),(7.891;1.23),(7.840;0.34),(7.802;10.64),(7.734;1.89),(7.728;1.34),(7. 713;2.07),(7.708;1.50),(7.636;0.60),(7.616;0.99),(7.569;0.76),(7.549;4.59),(7.537;5.57),(7.529;3.8 1),(7.517;3.27),(7.489;1.43),(7.469;1.89),(7.449;0.88),(7.193;3.65),(6.991;0.52),(6.034;10.79),(6.0 21;0.91),(5.569;0.49),(4.460;3.68),(4.446;3.64),(3.894;3.28),(3.116;19.59),(2.633;0.41),(2.527;0.9 8),(2.511;0.69),(2.506;0.94),(2.498;15.40),(2.493;32.07),(2.488;45.48),(2.484;31.68),(2.479;15.12) ,(2.290;1.08),(2.193;16.00),(2.138;1.07),(2.004;1.01),(-0.000;2.28) |
| 69 | 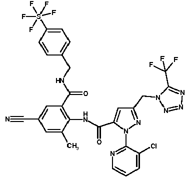 | 3,68 | 747 | (9.847;0.64),(8.430;3.76),(8.426;4.15),(8.418;4.21),(8.414;4.19),(7.948;3.82),(7.944;4.05),(7.928;4 .25),(7.924;4.26),(7.765;0.82),(7.759;7.15),(7.754;2.73),(7.742;2.74),(7.737;11.99),(7.732;5.93),(7. 731;6.36),(7.643;4.30),(7.641;5.26),(7.639;5.60),(7.637;5.85),(7.621;1.16),(7.477;4.67),(7.465;8.6 4),(7.457;5.43),(7.445;7.88),(7.163;8.88),(5.911;16.00),(4.497;5.59),(4.483;5.66),(2.158;1.86),(2.1 31;22.76),(1.951;3.70),(1.945;7.31),(1.939;10.66),(1.932;7.45),(1.926;3.86),(-0.000;3.42) |
| 70 | 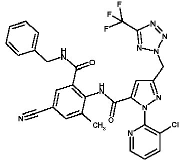 | 3,38 | 621 | (10.511;1.12),(10.501;2.62),(8.911;0.53),(8.870;2.18),(8.855;1.34),(8.490;2.85),(8.479;3.13),(8.47 6;2.96),(8.148;2.60),(8.130;2.86),(8.116;0.53),(7.876;3.87),(7.807;4.30),(7.628;0.56),(7.609;2.48),( 7.597;2.60),(7.588;2.46),(7.577;2.08),(7.341;4.09),(7.260;2.30),(7.242;11.89),(7.226;7.84),(7.208; 2.95),(7.199;2.25),(7.194;2.66),(7.187;1.88),(7.178;2.22),(7.163;0.81),(6.308;10.64),(4.345;6.12),( 4.331;5.99),(3.671;0.51),(3.637;0.55),(3.568;2.86),(3.551;0.55),(3.531;0.63),(3.503;0.73),(3.499;0. 65),(3.480;0.98),(3.448;1.18),(3.440;1.14),(3.433;1.09),(3.418;1.42),(3.305;3787.74),(3.282;62.98) ,(3.206;1.18),(3.188;1.24),(3.172;0.79),(3.138;0.73),(3.127;0.60),(3.092;0.52),(3.079;0.55),(2.890; 0.53),(2.729;0.61),(2.692;0.58),(2.674;3.99),(2.669;5.50),(2.664;3.90),(2.624;0.86),(2.615;0.92),(2. 606;0.98),(2.522;23.56),(2.509;301.75),(2.504;558.02),(2.500;723.26),(2.496;498.87),(2.491;240.4 6),(2.382;0.76),(2.354;0.74),(2.331;3.69),(2.327;5.07),(2.322;3.71),(2.249;0.66),(2.209;15.00),(2.0 84;1.57),(2.069;1.72),(1.398;1.16),(1.270;0.52),(1.236;0.70),(-0.000;19.73) |
| 71 | 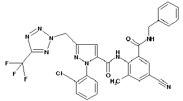 | 3,77 / 3,47 | 620 | (10.423;4.44),(8.875;1.13),(8.861;2.04),(8.847;1.04),(7.866;3.94),(7.803;4.25),(7.565;1.80),(7.562; 2.06),(7.545;2.82),(7.543;3.10),(7.528;0.39),(7.495;1.00),(7.490;1.19),(7.477;2.14),(7.472;2.39),(7. 458;1.95),(7.452;2.74),(7.436;2.78),(7.433;2.86),(7.417;4.53),(7.398;1.12),(7.392;0.74),(7.369;0.3 5),(7.347;0.46),(7.342;0.38),(7.334;0.99),(7.316;5.30),(7.254;3.86),(7.245;4.46),(7.240;4.45),(7.22 6;1.21),(7.223;1.29),(7.210;2.90),(7.208;2.90),(7.203;6.65),(7.200;5.97),(7.192;7.94),(7.186;6.57),( 7.177;0.82),(6.288;10.98),(6.068;0.90),(4.774;0.36),(4.334;4.74),(4.320;4.75),(3.344;0.48),(3.291; 664.83),(3.267;12.25),(2.677;0.40),(2.672;0.76),(2.668;1.02),(2.663;0.74),(2.551;0.41),(2.538;1.48 ),(2.521;4.04),(2.508;57.74),(2.503;113.47),(2.499;151.84),(2.494;108.21),(2.490;51.41),(2.330;0.7 4),(2.326;0.98),(2.321;0.72),(2.208;15.00),(2.067;0.67),(1.399;0.55),(0.008;1.22),(-0.000;27.10),(-0.008;0.97) |
| 72 | 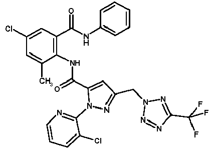 | 3,81 | 616 | (10.29;4.70), (10.23;4.18), (8.33;2.77), (8.33;2.91), (8.32;2.96), (8.32;2.80), (8.01;2.75), (8.01;2.72), (7.99;3.08), (7.99;2.83), (7.61;3.77), (7.59;4.26), (7.52;3.98), (7.52;3.50), (7.51;6.07), (7.50;3.14), (7.49;2.92), (7.47;3.46), (7.47;2.82), (7.34;8.66), (7.32;2.98), (7.31;4.70), (7.28;2.99), (7.10;1.76), (7.08;2.87), (7.06;1.27), (6.28;12.11), (3.32;314.33), (2.67;0.38), (2.54;0.63), (2.51;21.63), (2.51;39.04), (2.50;50.07), (2.50;34.51), (2.49;16.39), (2.33;0.34), (2.20;16.00), (2.07;0.33), (0.00;0.36) |
| 73 | 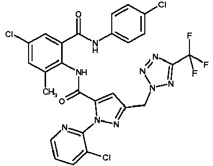 | 4,17 | 650 | (10.38;4.23), (10.30;4.86), (8.34;2.90), (8.33;3.06), (8.33;3.14), (8.32;2.98), (8.01;2.86), (8.01;2.85), (7.99;3.22), (7.99;2.99), (7.64;4.88), (7.62;5.73), (7.53;6.18), (7.52;4.22), (7.51;3.52), (7.51;3.06), (7.49;3.00), (7.48;3.64), (7.47;3.02), (7.37;6.84), (7.37;2.58), (7.35;6.50), (7.35;9.05), (6.29;12.71), (4.04;0.38), (4.02;0.39), (3.30;328.22), (3.28;4.08), (2.67;0.42), (2.67;0.55), (2.66;0.41), (2.54;0.80), (2.52;2.68), (2.51;32.72), (2.50;60.10), (2.50;77.92), (2.50;54.30), (2.49;26.25), (2.34;0.33), (2.33;0.46), (2.33;0.57), (2.32;0.42), (2.20;16.00), (1.99;0.38), (1.99;1.75), (1.19;0.50), (1.17;0.97), (1.16;0.48), (0.00;1.27) |
| 74 | 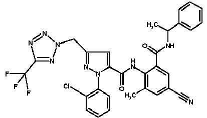 | 3,68 | 635 | (10.40;0.38), (10.37;2.06), (8.74;1.82), (8.73;1.84), (7.97;0.34), (7.95;0.38), (7.86;3.97), (7.78;5.60), (7.56;2.90), (7.54;5.45), (7.53;1.94), (7.52;1.84), (7.51;0.68), (7.50;1.65), (7.49;2.07), (7.48;2.69), (7.48;3.47), (7.47;2.72), (7.46;3.11), (7.46;3.69), (7.44;7.13), (7.43;6.18), (7.38;1.29), (7.36;1.02), (7.32;5.62), (7.30;8.31), (7.26;1.99), (7.25;6.46), (7.23;8.79), (7.21;5.36), (7.19;4.52), (7.19;3.88), (7.18;2.05), (7.17;4.20), (7.17;1.21), (7.15;1.17), (6.28;13.60), (6.06;4.04), (4.99;0.67), (4.97;2.61), (4.95;3.82), (4.93;2.58), (4.92;0.67), (3.43;0.35), (3.31;1636.11), (3.28;17.72), (3.20;0.41), (2.68;0.96), (2.67;1.84), (2.67;2.41), (2.66;1.82), (2.66;0.93), (2.58;2.74), (2.57;0.69), (2.54;2.66), (2.52;10.78), (2.51;140.80), (2.50;259.93), (2.50;337.18), (2.50;234.85), (2.49;112.88), (2.42;0.39), (2.34;0.98), (2.33;1.79), (2.33;2.34), (2.32;1.77), (2.30;0.40), (2.20;16.00), (2.16;1.04), (2.07;3.20), (2.03;0.61), (1.91;0.34), (1.46;0.62), (1.44;0.57), (1.40;0.94), (1.39;0.32), (1.30;13.67), (1.29;13.50), (1.24;0.69), (0.89;0.73), (0.01;0.59), (0.00;12.28), (-0.01;0.51) |
| 75 | 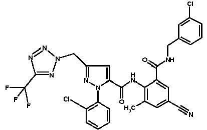 | 3,76 | 654 | (10.46;1.40), (10.11;0.37), (9.02;0.35), (8.95;1.91), (7.98;0.84), (7.98;0.87), (7.94;0.74), (7.89;3.86), (7.88;1.53), (7.83;5.47), (7.56;3.66), (7.54;5.24), (7.53;0.61), (7.53;0.62), (7.51;0.84), (7.48;1.37), (7.47;2.43), (7.46;3.22), (7.46;2.69), (7.45;3.47), (7.44;8.85), (7.43;5.49), (7.38;0.53), (7.37;0.51), (7.35;0.96), (7.32;8.49), (7.28;0.59), (7.27;2.43), (7.27;2.09), (7.26;3.42), (7.26;3.17), (7.26;3.97), (7.25;3.56), (7.25;2.09), (7.24;1.73), (7.23;2.07), (7.23;10.75), (7.22;5.94), (7.22;8.82), (7.21;1.14), (7.03;0.51), (6.29;11.58), (6.07;1.56), (4.38;0.99), (4.37;1.03), (4.32;4.49), (4.31;4.64), (3.34;476.81), (3.32;7.09), (2.62;0.52), (2.62;1.12), (2.61;1.56), (2.61;1.14), (2.61;0.51), (2.54;0.73), (2.52;3.22), (2.52;4.06), (2.52;4.12), (2.51;83.62), (2.51;179.56), (2.50;244.91), (2.50;179.64), (2.50;83.09), (2.39;0.50), (2.39;1.12), (2.39;1.52), (2.38;1.08), (2.29;3.46), (2.22;16.00), (2.18;0.33), (2.13;3.80), (2.10;0.33), (2.08;0.60), (1.35;1.13), (0.01;0.41), (0.00;13.75), (-0.01;0.43) |
| 76 | 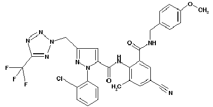 | 3,77 | 651 | (7.84;1.13), (7.78;1.67), (7.56;0.89), (7.56;0.96), (7.54;1.38), (7.54;1.41), (7.49;0.55), (7.48;0.60), (7.47;1.08), (7.47;1.13), (7.45;0.80), (7.45;1.03), (7.44;0.99), (7.44;0.87), (7.42;1.15), (7.42;1.16), (7.40;0.72), (7.40;0.69), (7.39;1.15), (7.38;1.06), (7.37;0.57), (7.35;0.32), (7.29;0.69), (7.25;0.44), (7.23;0.56), (7.19;0.55), (7.18;2.69), (7.15;2.91), (6.90;0.51), (6.88;0.48), (6.87;0.45), (6.79;0.44), (6.77;0.86), (6.76;3.34), (6.76;1.17), (6.75;1.19), (6.74;2.96), (6.29;4.50), (6.06;0.49), (4.26;1.80), (4.25;1.77), (3.80;0.79), (3.75;1.08), (3.73;1.63), (3.73;3.07), (3.72;2.80), (3.71;16.00), (3.68;0.86), (3.62;1.18), (3.62;0.86), (3.61;0.85), (3.61;1.13), (3.60;2.71), (3.60;1.08), (3.59;0.79), (3.59;0.85), (3.58;1.14), (3.30;148.98), (3.28;2.44), (2.54;0.47), (2.51;19.24), (2.50;34.49), (2.50;44.10), (2.50;30.49), (2.49;14.65), (2.33;0.34), (2.27;0.40), (2.19;5.90), (2.14;0.46), (2.10;1.09), (1.78;1.21), (1.77;1.24), (1.76;1.01), (1.76;3.22), (1.75;0.95), (1.75;1.16), (1.74;1.09), (1.40;0.34), (1.36;4.59), (0.00;1.08) |
| 77 | 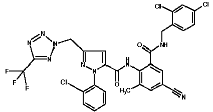 | 4,42 | 689 | (10.41;1.96), (8.94;0.65), (8.92;1.03), (8.91;0.53), (7.97;0.49), (7.90;1.93), (7.86;2.03), (7.62;0.35), (7.58;0.54), (7.57;2.11), (7.57;2.48), (7.56;1.20), (7.55;1.77), (7.50;0.71), (7.50;0.77), (7.49;0.96), (7.48;1.11), (7.47;1.02), (7.46;1.20), (7.45;1.94), (7.45;2.42), (7.44;2.20), (7.42;0.68), (7.41;1.59), (7.38;1.86), (7.31;2.84), (7.25;0.57), (7.21;1.19), (7.21;1.28), (7.19;1.04), (7.19;0.99), (6.28;5.29), (6.06;0.60), (4.47;0.46), (4.46;0.43), (4.39;0.44), (4.35;1.93), (4.33;1.93), (4.28;0.36), (4.09;0.32), (3.80;0.45), (3.76;0.34), (3.71;0.41), (3.67;0.50), (3.65;0.45), (3.63;0.49), (3.61;0.50), (3.60;0.66), (3.59;0.62), (3.54;0.69), (3.53;0.68), (3.53;0.72), (3.52;0.76), (3.47;1.22), (3.39;3.50), (3.31;4350.54), (3.29;51.72), (3.23;1.36), (3.18;0.65), (3.17;0.57), (3.13;0.43), (3.08;0.37), (3.03;0.37), (2.94;0.34), (2.85;0.32), (2.79;0.37), (2.77;0.33), (2.75;0.41), (2.73;0.36), (2.69;0.72), (2.67;2.95), (2.67;3.86), (2.66;2.89), (2.65;0.59), (2.64;0.59), (2.63;0.65), (2.54;6.43), (2.52;19.18), (2.51;224.62), (2.51;413.16), (2.50;538.46), (2.50;378.81), (2.49;185.60), (2.36;0.37), (2.33;2.70), (2.33;3.60), (2.32;2.64), (2.29;0.85), (2.23;6.97), (2.18;0.35), (2.14;0.87), (2.11;1.00), (2.07;0.36), (1.99;0.44), (1.91;0.32), (1.47;16.00), (1.36;1.66), (1.29;0.42), (1.25;0.39), (1.24;0.54), (1.18;0.32), (1.11;0.68), (1.09;0.88), (1.07;0.39), (0.89;0.33), (0.00;6.30) |
| 78 | 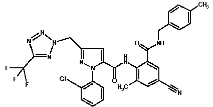 | 3,74 | 634 | (8.87;0.37), (7.99;0.78), (7.99;0.84), (7.92;0.56), (7.91;0.33), (7.87;1.01), (7.84;0.75), (7.79;2.02), (7.56;1.65), (7.56;1.69), (7.55;2.28), (7.55;2.36), (7.52;0.64), (7.49;0.82), (7.48;0.87), (7.47;1.72), (7.47;1.71), (7.46;1.24), (7.46;1.27), (7.44;0.91), (7.43;1.62), (7.41;0.90), (7.37;1.19), (7.36;0.99), (7.34;0.73), (7.21;0.41), (7.19;0.59), (7.14;1.73), (7.14;4.23), (7.12;4.79), (7.09;0.39), (7.02;0.65), (7.01;0.59), (7.00;4.91), (6.99;4.13), (6.30;4.82), (6.08;0.60), (4.32;0.87), (4.31;0.90), (4.27;2.01), (3.57;0.50), (3.34;318.31), (3.32;5.36), (2.62;0.69), (2.61;0.96), (2.61;0.68), (2.54;0.50), (2.52;2.08), (2.52;2.65), (2.52;2.78), (2.51;51.18), (2.51;110.00), (2.50;149.56), (2.50;109.30), (2.50;49.83), (2.39;0.67), (2.39;0.92), (2.38;0.68), (2.38;0.32), (2.34;0.38), (2.27;4.07), (2.26;2.21), (2.24;16.00), (2.22;3.73), (2.20;6.57), (2.14;3.41), (2.10;1.13), (2.08;0.58), (1.35;1.22), (0.01;0.39), (0.00;12.57), (-0.01;0.41) |
| 79 | 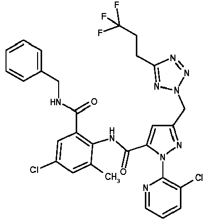 | 3,56 | 658 | (8.78;0.57), (8.48;2.27), (8.48;2.38), (8.47;2.43), (8.47;2.32), (8.14;2.16), (8.13;2.14), (8.12;2.39), (8.11;2.24), (7.60;2.22), (7.58;2.19), (7.58;2.12), (7.56;2.06), (7.47;2.18), (7.46;2.26), (7.37;2.84), (7.36;2.62), (7.35;0.44), (7.34;0.51), (7.23;7.47), (7.22;16.00), (7.20;2.34), (7.20;2.04), (7.19;0.94), (7.18;1.24), (7.18;0.63), (7.17;0.62), (6.05;8.42), (4.33;4.41), (4.32;4.37), (3.79;0.49), (3.31;471.81), (3.29;6.20), (3.15;2.81), (3.14;3.99), (3.12;3.39), (2.83;0.50), (2.81;0.66), (2.80;1.50), (2.79;1.05), (2.78;1.71), (2.77;1.66), (2.76;1.35), (2.76;1.15), (2.75;1.69), (2.75;0.73), (2.74;1.23), (2.73;0.62), (2.71;0.43), (2.67;0.47), (2.67;0.61), (2.66;0.44), (2.54;0.85), (2.52;2.84), (2.51;34.47), (2.50;63.14), (2.50;81.66), (2.50;57.41), (2.49;28.04), (2.33;0.46), (2.33;0.59), (2.32;0.45), (2.15;12.59), (2.07;0.35), (0.00;0.46) |
| 80 | 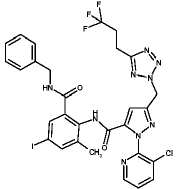 | 3,78 | 750 | (8.76;0.65), (8.48;1.99), (8.48;2.05), (8.47;2.12), (8.46;1.97), (8.14;1.91), (8.13;1.85), (8.12;2.12), (8.11;1.91), (7.75;2.22), (7.75;2.28), (7.63;2.52), (7.63;2.35), (7.60;1.97), (7.58;1.94), (7.58;1.83), (7.56;1.80), (7.25;0.45), (7.23;5.11), (7.22;16.00), (7.20;1.78), (7.20;1.27), (7.19;1.03), (7.18;0.93), (7.17;0.44), (6.05;6.99), (4.32;3.45), (4.31;3.37), (3.86;0.33), (3.33;514.60), (3.31;5.11), (3.15;2.20), (3.13;3.12), (3.11;2.61), (2.83;0.38), (2.81;0.53), (2.80;1.18), (2.78;0.87), (2.78;1.37), (2.77;1.30), (2.76;1.08), (2.76;0.92), (2.75;1.33), (2.74;0.56), (2.73;0.96), (2.72;0.48), (2.71;0.33), (2.67;0.35), (2.54;0.58), (2.51;20.69), (2.51;36.58), (2.50;46.27), (2.50;31.82), (2.49;15.17), (2.33;0.33), (2.11;10.48) |
| 81 | 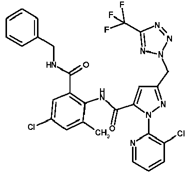 | 3,77 | 630 | (8.85;0.35), (8.48;2.96), (8.48;3.09), (8.47;3.20), (8.46;3.06), (8.14;2.80), (8.14;2.77), (8.12;3.17), (8.12;2.94), (7.60;2.89), (7.59;2.88), (7.58;2.77), (7.57;2.70), (7.46;2.71), (7.45;2.86), (7.38;3.67), (7.38;3.27), (7.35;0.66), (7.33;2.25), (7.33;3.18), (7.31;1.85), (7.30;0.79), (7.29;1.03), (7.27;2.33), (7.24;2.11), (7.22;12.93), (7.22;7.91), (7.21;7.32), (7.20;1.96), (7.19;1.82), (7.19;2.14), (7.19;2.13), (7.18;1.84), (7.17;2.08), (7.17;1.63), (7.16;1.42), (7.16;1.63), (7.15;0.77), (7.14;0.58), (7.14;0.35), (6.29;10.73), (4.34;5.39), (4.33;s.33), (3.75;4.03), (3.31;326.s6), (2.68;0.35), (2.67;0.61), (2.67;0.83), (2.66;0.59), (2.54;1.11), (2.52;3.85), (2.51;46.69), (2.50;85.19), (2.50;109.85), (2.50;76.36), (2.49;36.84), (2.43;0.41), (2.41;0.34), (2.34;0.38), (2.33;0.63), (2.33;0.79), (2.32;0.61), (2.32;0.37), (2.15;16.00), (2.07;1.20), (2.05;1.41), (1.36;0.42), (1.05;0.62), (1.03;0.60),(0.00;2.34) |
| 82 | 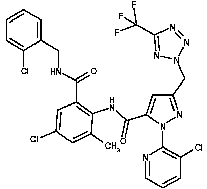 | 4,06 | 664 | (10.26;4.44), (8.82;1.14), (8.81;2.28), (8.79;1.14), (8.49;3.01), (8.48;3.25), (8.48;3.33), (8.47;3.22), (8.15;2.87), (8.14;2.89), (8.13;3.31), (8.12;3.09), (7.61;3.09), (7.60;3.02), (7.59;2.92), (7.58;2.90), (7.50;3.20), (7.49;3.74), (7.43;4.02), (7.43;3.51), (7.41;2.45), (7.41;2.50), (7.39;2.95), (7.39;2.86), (7.30;9.47), (7.28;2.38), (7.28;2.44), (7.25;0.44), (7.24;1.07), (7.24;1.16), (7.22;2.25), (7.22;2.03), (7.20;1.47), (7.20;1.25), (7.17;1.93), (7.16;1.92), (7.15;2.45), (7.14;2.36), (7.13;0.95), (7.13;0.88), (6.29;11.60), (4.38;4.53), (4.37;4.54), (3.40;0.41), (3.31;1011.13), (3.18;0.35), (2.68;0.54), (2.67;0.97), (2.67;1.28), (2.66;0.96), (2.66;0.51), (2.54;2.12), (2.52;6.21), (2.51;75.25), (2.50;138.48), (2.50;179.75), (2.50;125.77), (2.49;61.31), (2.33;1.07), (2.33;1.37), (2.32;1.02), (2.17;16.00), (2.07;2.32), (1.40;0.86), (1.36;0.48), (1.24;0.38), (0.01;0.70), (0.00;13.05), (-0.01:0.57) |
| 83 | 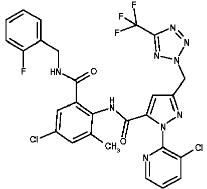 | 3,77 | 648 | (10.24;1.67), (8.78;1.94), (8.48;3.10), (8.48;3.23), (8.47;3.24), (8.47;3.15), (8.45;0.38), (8.14;2.78), (8.14;2.76), (8.12;3.05), (8.12;2.91), (7.60;2.65), (7.59;2.72), (7.58;2.65), (7.57;2.51), (7.48;3.33), (7.38;3.83), (7.32;0.32), (7.28;5.12), (7.27;3.69), (7.26;2.37), (7.24;2.11), (7.22;2.22), (7.21;1.24), (7.17;0.64), (7.15;0.66), (7.14;2.24), (7.12;2.53), (7.09;1.58), (7.03;2.35), (7.01;3.65), (6.99;1.71), (6.29;10.67), (4.37;5.64), (4.35;5.53), (3.78;1.41), (3.57;0.33), (3.54;0.49), (3.52;0.62), (3.51;0.68), (3.46;1.38), (3.32;8249.28), (3.29;113.19), (3.14;1.66), (3.05;1.04), (3.04;0.95), (3.03;0.98), (2.99;0.80), (2.97;0.74), (2.95;0.78), (2.94;0.70), (2.93;0.68), (2.91;0.69), (2.89;0.65), (2.89;0.62), (2.86;0.58), (2.83;0.55), (2.78;0.51), (2.77;0.53), (2.73;0.51), (2.69;0.79), (2.67;5.00), (2.67;6.48), (2.67;4.65), (2.66;2.50), (2.63;0.81), (2.61;0.93), (2.54;9.60), (2.52;33.65), (2.51;380.66), (2.51;682.15), (2.50;871.22), (2.50;599.38), (2.49;286.03), (2.37;1.06), (2.34;2.98), (2.33;5.11), (2.33;6.52), (2.32;4.78), (2.32;2.73), (2.28;0.70), (2.26;0.64), (2.23;0.70), (2.16;16.00), (2.07;7.45), (2.03;0.38), (2.01;0.42), (2.00;0.43), (1.98;0.36), (1.97;0.46), (1.96;0.41), (1.94;0.36), (1.91;0.72), (1.89;0.39), (1.83;0.35), (1.76;0.35), (1.73;0.33), (1.36;0.43), (1.28;0.33), (1.26;0.37), (1.24;0.88), (1.05;0.40), (0.89;0.67), (0.85;0.34), (0.00;5.09) |
| 84 | 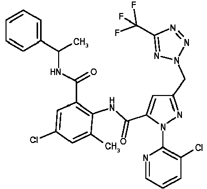 | 3,92 | 644 | (8.63;0.51), (8.48;3.37), (8.48;3.50), (8.47;3.61), (8.46;3.48), (8.14;2.92), (8.13;2.91), (8.12;3.32), (8.11;3.09), (7.60;3.05), (7.59;3.04), (7.58;2.94), (7.57;2.81), (7.45;2.65), (7.39;0.69), (7.37;1.12), (7.33;3.60), (7.32;1.64), (7.30;3.69), (7.29;4.29), (7.27;8.54), (7.24;4.38), (7.22;6.66), (7.21;3.29), (7.16;2.47), (7.14;2.98), (7.12;1.03), (6.29;11.64), (4.99;0.38), (4.97;1.51), (4.96;2.22), (4.94;1.51), (4.92;0.38), (4.07;0.39), (4.06;0.39), (3.31;763.97), (3.29;14.60), (2.68;0.51), (2.67;0.88), (2.67;1.22), (2.66;0.87), (2.66;0.49), (2.54;1.66), (2.52;5.57), (2.51;67.81), (2.50;124.47), (2.50;161.20), (2.50;113.70), (2.49;55.90), (2.34;0.50), (2.33;0.89), (2.33;1.14), (2.32;0.86), (2.15;16.00), (2.07;1.41), (1.30;10.83), (1.28;11.42), (0.00;1.92) |
| 85 | 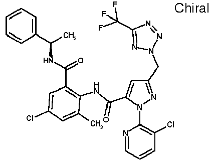 | 3,92 | 644 | (10.19;0.95), (8.62;1.03), (8.61;1.01), (8.48;3.55), (8.48;3.67), (8.47;3.77), (8.47;3.56), (8.14;3.00), (8.14;2.93), (8.12;3.42), (8.12;3.10), (7.60;3.16), (7.59;3.14), (7.58;3.02), (7.57;2.89), (7.46;2.79), (7.41;0.38), (7.39;0.66), (7.36;0.45), (7.34;0.80), (7.33;3.70), (7.32;3.48), (7.29;4.32), (7.28;9.27), (7.24;4.13), (7.23;6.42), (7.21;3.14), (7.16;2.43), (7.14;2.91), (7.12;1.00), (6.29;11.65), (4.99;0.39), (4.97;1.46), (4.95;2.14), (4.94;1.48), (4.92;0.37), (3.45;0.34), (3.42;0.59), (3.31;1825.81), (3.29;26.91), (3.18;0.54), (3.14;0.35), (2.68;0.84), (2.67;1.59), (2.67;2.05), (2.66;1.48), (2.66;0.75), (2.58;0.56), (2.54;3.07), (2.51;120.55), (2.50;215.79), (2.50;275.75), (2.50;190.13), (2.49;91.01), (2.33;1.55), (2.33;2.01), (2.32;1.50), (2.32;0.84), (2.15;16.00), (2.07;3.27), (1.91;0.34), (1.36;0.38), (1.34;0.89), (1.32;1.08), (1.30;9.64), (1.28;9.44), (1.24;0.35), (0.00;4.01) |
| 86 | 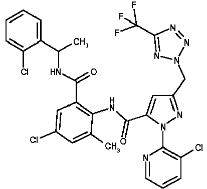 | 4,26 | 678 | (10.18;4.07), (8.80;2.39), (8.78;2.43), (8.49;3.78), (8.48;4.00), (8.48;4.14), (8.47;3.85), (8.14;2.93), (8.12;3.20), (7.61;2.54), (7.60;2.78), (7.59;2.68), (7.58;2.36), (7.47;4.22), (7.42;2.52), (7.40;2.90), (7.36;8.51), (7.35;7.59), (7.34;4.70), (7.33;5.13), (7.26;5.64), (7.21;1.57), (7.19;3.10), (7.17;4.93), (7.17;3.78), (7.15;3.61), (7.15;3.32), (7.13;1.31), (6.28;10.82), (6.06;0.33), (5.27;0.61), (5.25;2.14), (5.23;3.17), (5.21;2.05), (5.20;0.55), (3.57;1.91), (3.56;0.37), (3.53;0.37), (3.52;0.42), (3.51;0.42), (3.49;0.49), (3.43;0.94), (3.31;2018.09), (3.21;0.69), (3.17;0.51), (2.69;0.46), (2.68;1.31), (2.67;2.23), (2.67;2.92), (2.66;2.22), (2.66;1.23), (2.65;0.45), (2.64;0.44), (2.58;1.32), (2.54;5.28), (2.52;14.84), (2.51;165.65), (2.50;303.01), (2.50;391.64), (2.50;274.76), (2.49;133.39), (2.33;2.08), (2.33;2.71), (2.32;2.01), (2.32;1.09), (2.21;0.33), (2.16;16.00), (2.10;0.54), (2.07;5.22), (2.03;0.76), (1.90;0.33), (1.60;4.20), (1.40;0.71), (1.26;9.66), (1.24;9.74), (0.89;0.61), (0.01;1.71), (0.00;30.49), (-0.01;1.27) |
| 87 | 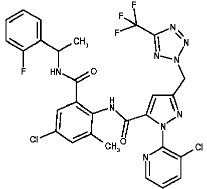 | 3,98 | 662 | (10.18;2.34), (8.73;1.65), (8.71;1.68), (8.48;3.19), (8.48;3.34), (8.47;3.47), (8.47;3.27), (8.14;2.90), (8.14;2.91), (8.12;3.31), (8.12;3.07), (7.61;3.16), (7.59;3.04), (7.59;3.07), (7.57;2.94), (7.47;3.10), (7.47;3.29), (7.38;1.01), (7.37;1.08), (7.36;2.25), (7.36;2.30), (7.35;4.17), (7.34;4.48), (7.26;5.66), (7.23;0.38), (7.22;0.68), (7.20;1.39), (7.18;1.60), (7.18;1.11), (7.17;0.93), (7.16;0.83), (7.11;1.68), (7.08;1.93), (7.06;2.52), (7.05;2.58), (7.03;1.16), (6.29;11.37), (5.21;0.32), (5.19;1.28), (5.17;1.96), (5.16;1.24), (5.14;0.32), (3.43;0.54), (3.31;1654.19), (3.28;20.23), (2.67;1.66), (2.67;2.12), (2.66;1.58), (2.54;3.15), (2.52;10.37), (2.51;124.37), (2.50;227.87), (2.50;294.73), (2.50;204.32), (2.49;98.07), (2.34;0.90), (2.33;1.62), (2.33;2.12), (2.32;1.58), (2.32;0.85), (2.16;16.00), (2.07;1.28), (2.04;0.45), (1.37;0.59), (1.36;1.26), (1.28;8.35), (1.27;8.27), (1.24;0.44), (0.89;0.54), (0.00;2.14) |
| 88 | 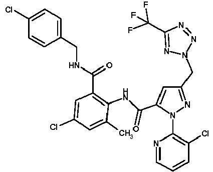 | 4,04 | 664 | (8.48;1.52), (8.48;1.60), (8.47;1.68), (8.47;1.58), (8.14;1.46), (8.14;1.46), (8.12;1.68), (8.12;1.56), (7.60;1.54), (7.59;1.52), (7.58;1.47), (7.57;1.44), (7.52;0.58), (7.46;1.54), (7.46;1.66), (7.39;0.39), (7.38;2.28), (7.37;2.16), (7.36;16.00), (7.29;1.90), (7.28;1.02), (7.27;0.77), (7.25;9.17), (7.25;7.79), (7.23;0.61), (7.23;0.72), (6.29;5.87), (4.31;2.55), (4.30;2.54), (3.75;0.61), (3.72;7.24), (3.60;0.67), (3.31;44.26), (2.67;0.46), (2.54;0.63), (2.52;1.86), (2.51;22.14), (2.50;40.42), (2.50;52.22), (2.50;36.70), (2.49;17.91), (2.33;0.40), (2.15;8.49), (1.76;0.78), (0.00;2.31) |
| 89 | 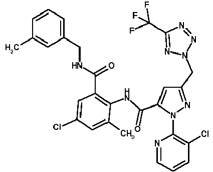 | 4,08 | 644 | (10.25;2.63), (8.76;0.89), (8.75;1.68), (8.73;0.88), (8.47;2.58), (8.47;2.75), (8.46;2.79), (8.46;2.71), (8.14;2.42), (8.14;2.40), (8.12;2.74), (8.12;2.52), (7.60;2.59), (7.59;2.54), (7.58;2.43), (7.57;2.41), (7.47;2.66), (7.47;2.90), (7.37;3.22), (7.36;2.92), (7.29;5.32), (7.12;1.15), (7.10;2.92), (7.08;2.32), (7.05;3.24), (7.03;2.35), (7.01;1.60), (7.00;2.09), (6.98;1.40), (6.29;9.53), (4.30;4.02), (4.29;4.00), (3.31;1341.01), (3.29;17.80), (2.68;0.60), (2.67;1.05), (2.67;1.40), (2.66;1.04), (2.66;0.54), (2.54;2.32), (2.52;6.82), (2.51;81.53), (2.50;149.23), (2.50;193.02), (2.50;133.98), (2.49;64.62), (2.36;0.35), (2.33;1.13), (2.33;1.45), (2.32;1.10), (2.32;0.63), (2.22;0.87), (2.20;16.00), (2.15;13.87), (2.07;0.79), (1.24;0.43), (0.89;0.52), (0.01;0.49), (0.00;9.71), (-0.01;0.40) |
| 90 | 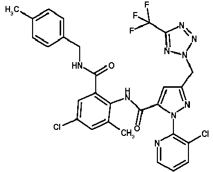 | 4,08 | 644 | (10.24;2.24), (8.74;0.98), (8.73;1.92), (8.71;0.98), (8.48;2.92), (8.48;3.07), (8.47;3.15), (8.47;3.06), (8.14;2.77), (8.14;2.73), (8.12;3.16), (8.12;2.88), (7.60;2.91), (7.59;2.86), (7.58;2.75), (7.57;2.72), (7.47;3.08), (7.46;3.34), (7.35;3.73), (7.34;3.43), (7.32;6.05), (7.12;4.06), (7.10;6.16), (7.04;5.72), (7.02;3.70), (6.30;10.90), (4.29;4.32), (4.27;4.26), (3.42;0.69), (3.32;1675.47), (3.30;19.18), (2.67;0.87), (2.67;1.11), (2.67;0.85), (2.54;1.90), (2.52;5.94), (2.51;64.94), (2.51;116.60), (2.50;148.87), (2.50;103.41), (2.49;50.09), (2.40;0.33), (2.34;0.56), (2.33;0.90), (2.33;1.15), (2.32;0.86), (2.31;0.33), (2.25;16.00), (2.15;15.75), (2.07;0.66), (0.01;0.40), (0.00;7.06) |
| 91 | 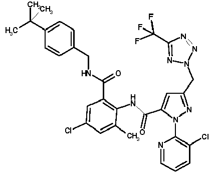 | 4,85 | 686 | (8.47;0.58), (8.47;0.60), (8.46;0.63), (8.46;0.60), (8.14;0.55), (8.14;0.53), (8.12;0.63), (8.12;0.57), (7.60;0.57), (7.59;0.56), (7.58;0.54), (7.57;0.53), (7.46;0.61), (7.46;0.66), (7.37;0.88), (7.37;0.91), (7.36;1.00), (7.27;1.00), (7.26;0.46), (7.25;1.45), (7.16;1.25), (7.14;0.84), (6.30;2.07), (4.31;0.85), (4.29;0.85), (3.31;12.80), (2.51;2.58), (2.50;4.66), (2.50;5.95), (2.50;4.12), (2.49;1.97), (2.15;3.09), (1.27;1.29), (1.25;1.32), (1.24;16.00) |
| 92 | 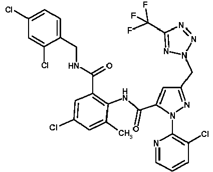 | 4,41 | 699 | (10.26;0.95), (8.85;0.85), (8.84;1.49), (8.83;0.87), (8.49;2.97), (8.48;3.17), (8.47;3.25), (8.47;3.13), (8.15;2.82), (8.14;2.81), (8.13;3.20), (8.12;2.96), (7.61;2.95), (7.60;3.07), (7.59;2.82), (7.58;2.96), (7.55;4.58), (7.55;4.45), (7.50;3.08), (7.49;3.42), (7.43;4.06), (7.42;3.53), (7.31;6.24), (7.29;4.67), (7.24;3.26), (7.24;3.07), (7.22;1.83), (7.22;1.81), (6.29;11.47), (4.35;4.62), (4.33;4.62), (3.80;0.54), (3.31;521.82), (3.28;9.54), (2.67;0.52), (2.67;0.70), (2.66;0.52), (2.54;1.16), (2.52;3.46), (2.51;41.42), (2.50;75.74), (2.50;98.04), (2.50;68.94), (2.49;33.67), (2.33;0.65), (2.33;0.76), (2.32;0.55), (2.17;16.00), (2.07;0.36), (0.00;4.51) |
| 93 | 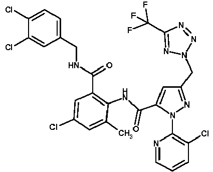 | 4,37 | 699 | (10.25;3.24), (8.84;1.12), (8.83;2.14), (8.82;1.07), (8.47;3.09), (8.47;3.21), (8.46;3.36), (8.45;3.12), (8.13;2.87), (8.13;2.82), (8.11;3.28), (8.11;2.96), (7.59;3.06), (7.58;3.03), (7.57;2.86), (7.56;2.84), (7.51;4.14), (7.50;4.08), (7.49;3.39), (7.48;3.64), (7.46;4.34), (7.44;4.96), (7.39;3.90), (7.38;3.52), (7.31;6.76), (7.23;2.50), (7.22;2.31), (7.21;2.07), (7.20;1.91), (6.29;11.64), (4.32;4.76), (4.30;4.63), (3.45;0.41), (3.31;963.27), (3.28;16.37), (3.24;0.39), (2.67;1.06), (2.67;1.38), (2.66;1.04), (2.66;0.59), (2.54;2.73), (2.51;80.85), (2.50;143.08), (2.50;180.83), (2.50;124.44), (2.49;58.99), (2.34;0.48), (2.33;0.92), (2.33;1.24), (2.32;0.91), (2.16;16.00), (2.07;0.51), (0.89;0.48), (0.01;0.50), (0.00;8.68), (-0.01;0.34) |
| 94 | 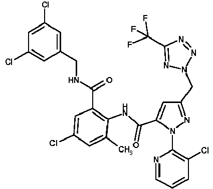 | 4,49 | 699 | (10.26;1.70), (9.27;0.52), (8.85;0.97), (8.83;1.85), (8.82;0.98), (8.46;2.87), (8.45;3.04), (8.45;3.15), (8.44;3.05), (8.12;2.69), (8.11;2.72), (8.10;3.05), (8.09;2.84), (7.91;3.50), (7.91;3.83), (7.83;0.90), (7.83;1.42), (7.82;0.72), (7.57;2.86), (7.56;2.84), (7.55;2.72), (7.54;2.70), (7.50;0.91), (7.50;1.85), (7.49;3.75), (7.49;3.65), (7.42;2.40), (7.42;4.08), (7.41;2.66), (7.40;4.08), (7.40;3.73), (7.39;2.99), (7.38;2.63), (7.31;9.07), (7.31;8.57), (7.30;7.37), (6.28;11.39), (4.48;1.64), (4.47;1.65), (4.34;4.47), (4.32;4.49), (3.88;0.33), (3.31;635.58), (3.28;12.50), (2.67;0.71), (2.67;0.93), (2.66;0.70), (2.54;1.55), (2.52;4.56), (2.51;55.37), (2.50;101.95), (2.50;132.37), (2.50;93.98), (2.49;46.45), (2.34;0.45), (2.33;0.78), (2.33;1.05), (2.32;0.77), (2.17;16.00), (2.07;0.46), (0.89;0.34), (0.00;5.67) |
| 95 | 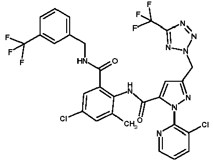 | 4,23 | 698 | (10.25;2.27), (8.89;1.01), (8.88;1.90), (8.86;0.98), (8.48;2.97), (8.47;3.18), (8.47;3.30), (8.46;3.16), (8.13;2.79), (8.13;2.82), (8.11;3.19), (8.11;3.00), (7.62;3.58), (7.59;3.05), (7.58;3.11), (7.57;3.01), (7.56;3.65), (7.54;3.73), (7.52;2.49), (7.49;3.17), (7.48;3.40), (7.44;2.02), (7.42;2.63), (7.40;1.18), (7.38;3.83), (7.38;3.49), (7.30;5.94), (6.29;11.29), (4.42;4.51), (4.40;4.49), (3.31;822.04), (3.28;13.76), (2.68;0.42), (2.67;0.77), (2.67;1.02), (2.66;0.75), (2.66;0.40), (2.54;1.70), (2.52;5.04), (2.51;59.54), (2.50;109.32), (2.50;142.11), (2.50;100.03), (2.49;48.87), (2.34;0.47), (2.33;0.82), (2.33;1.07), (2.32;0.89), (2.16;16.00), (2.07;0.47), (0.89;0.33), (0.01;0.38), (0.00;7.23) |
| 96 | 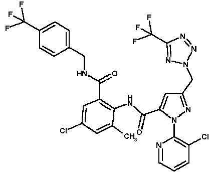 | 4,21 | 698 | (10.27;4.03), (8.91;1.12), (8.90;2.32), (8.88;1.13), (8.47;2.89), (8.47;3.11), (8.46;3.17), (8.46;3.09), (8.14;2.79), (8.14;2.77), (8.12;3.16), (8.12;2.92), (7.60;3.12), (7.59;3.28), (7.58;6.37), (7.57;3.69), (7.56;5.48), (7.49;3.33), (7.49;3.75), (7.45;5.01), (7.43;3.79), (7.40;3.96), (7.40;3.57), (7.34;7.62), (6.29;11.53), (4.41;3.84), (4.40;3.87), (3.32;334.88), (3.29;5.00), (2.67;0.37), (2.54;0.61), (2.51;21.97), (2.51;40.66), (2.50;52.98), (2.50;37.95), (2.49;19.00), (2.33;0.35), (2.33;0.46), (2.32;0.33), (2.17;16.00), (0.00;2.38) |
| 97 | 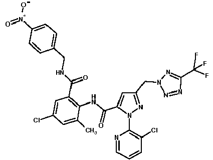 | 3,65 | 675 | (10.26;0.70), (10.18;0.73), (8.94;1.53), (8.75;0.34), (8.48;3.19), (8.48;3.18), (8.47;3.59), (8.47;3.22), (8.34;0.85), (8.32;1.03), (8.31;0.52), (8.29;0.53), (8.27;0.35), (8.25;0.96), (8.23;2.62), (8.21;3.47), (8.20;2.36), (8.19;2.04), (8.18;2.38), (8.16;1.17), (8.15;3.16), (8.14;3.05), (8.12;3.54), (8.12;3.29), (8.11;1.30), (8.10;1.24), (8.09;1.96), (8.08;6.45), (8.06;6.50), (8.03;0.38), (7.89;0.41), (7.82;0.74), (7.80;0.76), (7.69;0.85), (7.67;0.95), (7.66;2.23), (7.65;1.53), (7.64;2.14), (7.63;1.41), (7.60;3.03), (7.59;3.10), (7.58;2.95), (7.57;3.29), (7.55;0.79), (7.52;0.56), (7.50;8.42), (7.47;5.24), (7.45;0.67), (7.42;3.86), (7.41;3.44), (7.34;0.35), (7.31;6.95), (7.28;0.78), (7.05;0.56), (6.92;0.59), (6.90;0.66), (6.87;0.39), (6.48;0.72), (6.46;0.67), (6.31;1.56), (6.28;11.12), (6.16;0.97), (6.05;0.36), (4.57;0.49), (4.55;0.48), (4.45;4.16), (4.43;4.15), (4.16;0.46), (4.15;0.47), (3.97;3.71), (3.83;2.22), (3.71;0.44), (3.55;0.46), (3.51;0.33), (3.48;0.34), (3.31;1996.67), (3.28;49.99), (3.20;1.70), (3.11;0.74), (3.06;0.50), (3.00;0.36), (2.97;0.38), (2.93;0.34), (2.69;0.57), (2.67;3.26), (2.67;5.76), (2.54;6.09), (2.52;17.94), (2.51;222.84), (2.50;416.16), (2.50;545.58), (2.50;390.29), (2.49;196.18), (2.37;0.71), (2.33;3.24), (2.33;4.10), (2.32;3.17), (2.29;0.49), (2.28;0.46), (2.26;0.46), (2.22;0.42), (2.17;16.00), (2.14;2.19), (2.11;0.35), (2.09;0.32), (2.07;2.01), (2.05;0.38), (2.01;0.33), (1.98;0.48), (1.91;0.77), (1.67;0.75), (1.36;4.47), (1.24;1.59), (1.18;0.45), (0.89;1.43), (0.01;1.20), (0.00;22.23) |
| 98 | 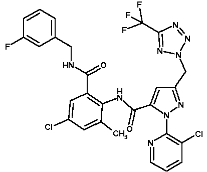 | 3,79 | 648 | (10.24;3.88), (8.82;1.11), (8.81;2.18), (8.79;1.11), (8.48;3.00), (8.47;3.16), (8.46;3.24), (8.46;3.13), (8.13;2.80), (8.13;2.82), (8.11;3.16), (8.11;2.98), (7.59;3.02), (7.58;2.98), (7.57;2.84), (7.56;2.74), (7.48;3.22), (7.48;3.60), (7.39;3.87), (7.38;3.44), (7.30;7.09), (7.28;0.91), (7.26;1.90), (7.24;1.91), (7.22;1.22), (7.08;4.44), (7.06;3.64), (7.01;0.88), (7.01;1.01), (7.00;0.77), (6.99;1.64), (6.98;1.39), (6.97;0.77), (6.29;11.39), (4.34;4.73), (4.33;4.72), (3.52;0.33), (3.48;0.39), (3.46;0.46), (3.30;1385.27), (3.28;24.20), (2.70;0.34), (2.67;1.59), (2.67;2.12), (2.66;1.56), (2.66;0.87), (2.59;0.65), (2.54;3.95), (2.52;10.70), (2.51;116.38), (2.50;213.03), (2.50;275.83), (2.50;192.88), (2.49;93.22), (2.34;0.70), (2.33;1.34), (2.33;1.87), (2.32;1.33), (2.32;0.74), (2.16;16.00), (2.07;0.84), (1.91;0.32), (1.24:0.50), (0.89:0.71), (0.01:0.69), (0.00;13.24), (-0.01;0.55) |
| 99 | 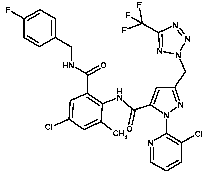 | 3,81 | 648 | (10.24;1.37), (8.78;1.55), (8.49;2.95), (8.48;3.14), (8.47;3.24), (8.47;3.11), (8.15;2.75), (8.14;2.77), (8.13;3.10), (8.12;2.94), (7.60;2.91), (7.59;2.85), (7.58;2.78), (7.57;2.72), (7.47;2.94), (7.47;3.06), (7.36;3.79), (7.36;3.61), (7.33;0.42), (7.32;0.63), (7.31;4.53), (7.27;2.87), (7.25;3.53), (7.25;3.85), (7.23;3.15), (7.14;0.48), (7.02;3.73), (7.02;1.43), (7.00;6.26), (6.98;1.27), (6.98;3.02), (6.30;11.23), (4.45;0.42), (4.44;0.42), (4.31;4.75), (4.29;4.71), (3.85;0.41), (3.77;0.45), (3.70;0.38), (3.67;0.41), (3.66;0.39), (3.64;0.44), (3.62;0.44), (3.62;0.47), (3.61;0.49), (3.59;0.55), (3.55;0.63), (3.54;0.65), (3.49;0.85), (3.31;2723.53), (3.29;35.91), (2.70;0.43), (2.67;2.01), (2.67;2.61), (2.67;1.99), (2.66;1.11), (2.54;4.94), (2.52;13.28), (2.51;144.84), (2.51;265.48), (2.50;344.29), (2.50;242.76), (2.49;118.84), (2.44;0.50), (2.33;1.69), (2.33;2.21), (2.32;1.62), (2.32;0.80), (2.16;16.00), (2.07;0.94), (1.91;0.35), (1.36;0.43), (1.24;0.57), (0.89;0.77), (0.00;14.28), (-0.01;0.64) |
| 100 | 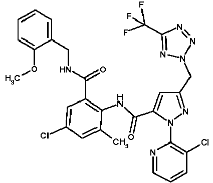 | 3,94 | 660 | (8.59;0.51), (8.48;1.79), (8.48;1.88), (8.47;1.92), (8.46;1.85), (8.14;1.68), (8.14;1.66), (8.12;1.90), (8.12;1.75), (7.60;1.76), (7.59;1.72), (7.58;1.65), (7.57;1.62), (7.47;1.80), (7.47;2.01), (7.41;2.42), (7.40;2.07), (7.28;2.62), (7.19;0.66), (7.18;0.72), (7.16;1.34), (7.15;0.87), (7.14;1.11), (7.14;1.40), (7.12;1.49), (6.94;2.01), (6.93;1.76), (6.77;1.02), (6.75;1.74), (6.73;0.82), (6.29;6.67), (4.30;2.86), (4.29;2.81), (3.81;0.70), (3.80;0.35), (3.77;16.00), (3.76;1.85), (3.31;168.43), (3.29;2.29), (2.54;0.41), (2.51;12.03), (2.50;21.67), (2.50;27.73), (2.50;19.31), (2.49;9.25), (2.16;9.73), (0.00;1.10) |
| 101 | 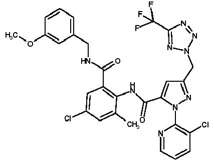 | 3,81 | 660 | (10.25;0.34), (8.78;0.61), (8.47;1.49), (8.47;1.61), (8.46;1.72), (8.46;1.63), (8.13;1.40), (8.13;1.44), (8.11;1.59), (8.11;1.49), (7.59;1.47), (7.58;1.46), (7.57;1.42), (7.56;1.36), (7.47;1.61), (7.47;1.74), (7.37;2.07), (7.37;1.87), (7.29;2.30), (7.15;0.92), (7.13;1.74), (7.11;0.99), (6.82;3.57), (6.81;1.30), (6.78;1.01), (6.77;1.08), (6.77;0.80), (6.76;0.95), (6.75;0.83), (6.29;5.83), (4.32;2.51), (4.30;2.52), (3.79;0.43), (3.79;0.59), (3.75;0.64), (3.74;0.36), (3.73;0.38), (3.70;1.00), (3.69;16.00), (3.31;174.99), (3.28;3.48), (2.54;0.53), (2.51;17.09), (2.50;31.29), (2.50;40.55), (2.50;29.08), (2.49;14.63), (2.16;8.62), (0.00;1.64) |
| 102 | 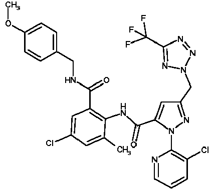 | 3,76 | 660 | (8.47;1.27), (8.47;1.36), (8.46;1.40), (8.46;1.35), (8.14;1.18), (8.13;1.18), (8.12;1.34), (8.11;1.24), (7.59;1.21), (7.58;1.22), (7.57;1.17), (7.56;1.13), (7.43;1.18), (7.36;1.48), (7.36;1.32), (7.25;2.26), (7.23;2.25), (7.15;2.74), (7.13;3.00), (6.89;0.45), (6.88;2.62), (6.87;0.97), (6.86;0.91), (6.86;2.30), (6.79;3.51), (6.77;3.03), (6.28;4.66), (4.27;2.27), (4.25;2.26), (3.73;12.12), (3.71;16.00), (3.67;4.49), (3.31;181.39), (2.67;0.55), (2.67;0.81), (2.66;0.53), (2.54;1.11), (2.51;39.22), (2.50;71.85), (2.50;93.05), (2.50;65.89), (2.49;32.42), (2.33;0.54), (2.33;0.68), (2.32;0.51), (2.14;6.85), (0.00;4.40) |
| 103 | 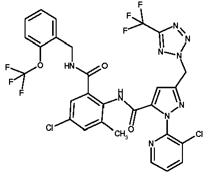 | 4,34 | 714 | (10.26;1.72), (8.83;0.97), (8.81;1.87), (8.80;0.96), (8.48;2.95), (8.48;3.11), (8.47;3.20), (8.46;3.04), (8.13;2.73), (8.13;2.73), (8.11;3.12), (8.11;2.89), (7.60;2.94), (7.59;2.87), (7.58;2.79), (7.57;2.72), (7.49;3.18), (7.49;3.59), (7.41;3.93), (7.40;3.56), (7.37;2.10), (7.35;3.12), (7.33;2.03), (7.32;2.03), (7.31;5.34), (7.30;7.54), (7.22;1.59), (7.21;1.45), (7.20;1.85), (7.18;0.88), (7.18;0.90), (6.29;11.47), (4.39;4.51), (4.37;4.51), (3.31;343.99), (3.28;6.64), (2.67;0.39), (2.67;0.52), (2.66;0.38), (2.54;0.87), (2.51;30.58), (2.50;56.12), (2.50;72.69), (2.50;51.26), (2.49;25.19), (2.33;0.45), (2.33;0.59), (2.32;0.40), (2.17;16.00), (1.36;0.74), (0.00;3.18) |
| 104 | 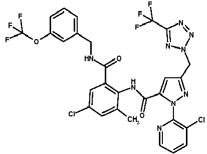 | 4,36 | 714 | (10.25;2.76), (8.88;1.07), (8.87;2.16), (8.85;1.08), (8.48;2.83), (8.48;3.03), (8.47;3.09), (8.46;2.99), (8.14;2.64), (8.13;2.67), (8.12;2.99), (8.11;2.83), (7.60;2.80), (7.58;2.81), (7.58;2.67), (7.56;2.60), (7.49;3.27), (7.48;3.64), (7.38;3.98), (7.38;3.67), (7.36;1.37), (7.34;3.29), (7.32;2.62), (7.30;7.33), (7.27;3.02), (7.25;5.00), (7.18;1.78), (7.16;1.42), (6.29;11.43), (4.38;4.45), (4.36;4.46), (3.32;547.59), (3.30;7.16), (2.67;0.33), (2.67;0.44), (2.67;0.34), (2.54;0.69), (2.51;25.42), (2.51;46.99), (2.50;61.20), (2.50;44.16), (2.33;0.38), (2.33;0.48), (2.32;0.42), (2.16;16.00), (1.36;0.43), (0.00;2.69) |
| 105 | 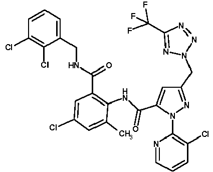 | 4,37 | 699 | (10.27;0.58), (8.89;0.95), (8.88;1.81), (8.87;0.94), (8.49;3.01), (8.49;3.20), (8.48;3.37), (8.48;3.23), (8.15;2.94), (8.15;2.92), (8.13;3.27), (8.13;3.04), (7.61;2.99), (7.60;2.98), (7.59;2.90), (7.58;2.89), (7.57;0.59), (7.55;0.59), (7.55;0.49), (7.54;0.49), (7.50;3.30), (7.50;3.99), (7.49;2.62), (7.48;2.55), (7.47;2.49), (7.46;2.41), (7.44;4.03), (7.44;3.44), (7.42;0.44), (7.42;0.37), (7.41;0.50), (7.39;0.68), (7.37;0.36), (7.30;7.48), (7.29;0.86), (7.28;1.82), (7.27;1.82), (7.26;2.81), (7.25;2.77), (7.24;0.41), (7.19;2.81), (7.17;4.00), (7.15;1.68), (6.29;11.58), (4.40;4.39), (4.39;4.63), (3.92;1.62), (3.31;287.88), (3.29;6.87), (2.67;0.41), (2.67;0.55), (2.66;0.41), (2.54;0.87), (2.52;2.65), (2.51;32.88), (2.50;60.36), (2.50;77.99), (2.50;54.49), (2.49;26.47), (2.33;0.51), (2.33;0.60), (2.32;0.45), (2.18;16.00), (2.04;0.66), (1.36;0.39), (0.00;3.43) |
| 106 | 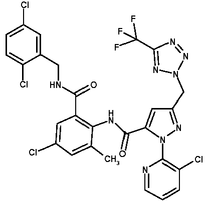 | 4,39 | 699 | (10.26;0.86), (8.82;1.49), (8.46;2.80), (8.45;2.98), (8.44;3.09), (8.44;2.99), (8.11;2.68), (8.11;2.63), (8.09;2.96), (8.09;2.79), (7.64;0.39), (7.64;0.39), (7.58;2.83), (7.57;2.81), (7.56;2.73), (7.55;2.61), (7.50;3.05), (7.50;3.44), (7.47;3.99), (7.45;5.59), (7.43;4.16), (7.43;3.96), (7.37;3.30), (7.36;4.32), (7.35;0.52), (7.34;3.20), (7.33;2.27), (7.32;2.36), (7.31;1.94), (7.29;5.50), (6.28;11.23), (4.38;4.68), (4.37;4.70), (3.80;1.25), (3.31;571.74), (3.29;10.06), (2.67;0.47), (2.67;0.66), (2.67;0.49), (2.54;1.01), (2.51;37.44), (2.51;68.83), (2.50;89.42), (2.50;64.02), (2.49;32.30), (2.34;0.39), (2.33;0.55), (2.33;0.67), (2.32;0.50), (2.18;16.00), (1.36;0.61), (0.00;3.36) |
| 107 | 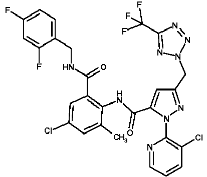 | 3,9 | 666 | (10.24;4.32), (8.80;1.13), (8.78;2.28), (8.77;1.10), (8.48;2.97), (8.48;3.17), (8.47;3.24), (8.47;3.12), (8.15;2.84), (8.14;2.83), (8.13;3.24), (8.12;2.99), (7.60;2.99), (7.59;2.96), (7.58;2.83), (7.57;2.78), (7.48;3.27), (7.48;3.55), (7.37;3.88), (7.36;3.51), (7.33;0.84), (7.31;1.95), (7.29;8.24), (7.27;0.87), (7.16;1.08), (7.15;1.13), (7.13;1.41), (7.13;1.54), (7.13;1.55), (7.11;1.05), (7.10;1.02), (6.88;0.89), (6.88;0.88), (6.86;1.63), (6.86;1.53), (6.84;0.80), (6.84;0.73), (6.30;11.52), (4.32;4.03), (4.30;3.98), (3.31;781.02), (3.29;9.52), (2.67;0.59), (2.67;0.77), (2.67;0.59), (2.54;1.45), (2.52;4.13), (2.51;44.05), (2.51;79.67), (2.50;102.46), (2.50;71.55), (2.49;34.46), (2.33;0.53), (2.33;0.69), (2.32;0.52), (2.16;16.00), (1.36;0.37), (0.00;4.29) |
| 108 | 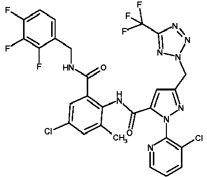 | 4,01 | 684 | (10.23;1.49), (8.85;1.06), (8.84;2.07), (8.82;1.02), (8.49;2.76), (8.48;2.88), (8.47;3.02), (8.47;2.85), (8.15;2.63), (8.15;2.59), (8.13;3.01), (8.13;2.76), (7.61;2.72), (7.59;2.73), (7.59;2.60), (7.57;2.57), (7.49;3.30), (7.48;3.54), (7.37;3.93), (7.37;3.53), (7.29;7.26), (7.13;0.33), (7.10;1.12), (7.09;3.41), (7.07;2.18), (7.05;1.12), (6.29;11.33), (4.34;3.85), (4.33;3.79), (3.96;0.38), (3.31;433.69), (3.29;6.72), (2.67;0.43), (2.67;0.53), (2.67;0.40), (2.54;1.07), (2.51;31.57), (2.51;56.42), (2.50;71.83), (2.50;50.48), (2.33;0.35), (2.33;0.46), (2.32;0.37), (2.16;16.00), (1.36;0.45), (0.00;2.69) |
| 109 | 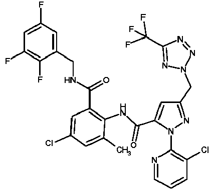 | 3,98 | 684 | (10.26;1.43), (8.87;1.14), (8.86;2.28), (8.84;1.11), (8.46;2.99), (8.46;3.26), (8.45;3.20), (8.44;3.03), (8.12;2.83), (8.12;2.75), (8.10;3.19), (8.10;2.87), (7.59;3.06), (7.57;3.01), (7.57;2.84), (7.55;2.79), (7.52;0.35), (7.49;3.32), (7.49;3.81), (7.42;4.03), (7.42;3.52), (7.38;0.68), (7.37;1.03), (7.36;1.02), (7.36;1.19), (7.35;1.19), (7.34;1.21), (7.33;1.28), (7.33;1.43), (7.32;1.22), (7.31;0.99), (7.30;7.99), (7.28;0.36), (7.25;0.52), (7.21;0.36), (7.03;0.93), (7.02;1.15), (7.01;1.16), (7.01;1.14), (7.00;1.09), (6.28;11.50), (4.39;3.93), (4.37;3.87), (4.11;0.91), (3.32;546.05), (3.29;5.90), (2.67;0.49), (2.67;0.62), (2.67;0.47), (2.54;1.18), (2.52;3.32), (2.51;35.64), (2.51;64.51), (2.50;82.90), (2.50;57.69), (2.49;27.61), (2.33;0.44), (2.33;0.61), (2.32;0.47), (2.17;16.00), (0.00;3.78) |
| 110 | 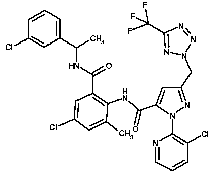 | 4,31 | 678 | (10.20;2.45), (8.67;1.82), (8.65;1.84), (8.47;2.95), (8.47;3.09), (8.46;3.14), (8.46;3.04), (8.13;2.71), (8.13;2.70), (8.11;3.04), (8.11;2.88), (7.60;0.41), (7.60;3.03), (7.59;2.97), (7.58;2.79), (7.56;2.70), (7.55;0.35), (7.47;3.35), (7.47;3.61), (7.42;0.40), (7.37;3.90), (7.33;3.84), (7.33;3.60), (7.29;6.97), (7.27;3.41), (7.26;2.70), (7.25;7.57), (7.25;2.59), (7.24;2.10), (7.24;2.99), (7.23;2.61), (7.23;1.73), (7.21;0.65), (7.21;0.62), (6.29;11.54), (4.98;0.34), (4.96;1.27), (4.94;1.93), (4.92;1.29), (4.91;0.32), (3.31;1119.16), (3.29;17.60), (2.67;0.98), (2.67;1.26), (2.66;0.95), (2.66;0.53), (2.60;0.47), (2.54;2.30), (2.52;6.44), (2.51;72.94), (2.50;133.75), (2.50;173.15), (2.50;122.51), (2.49;60.22), (2.44;0.32), (2.34;0.51), (2.33;0.90), (2.33;1.18), (2.32;0.88), (2.16;16.00), (2.07;0.53), (2.05;0.79), (1.44;0.50), (1.42;0.45), (1.30;8.20), (1.28;8.08), (1.24;0.35), (0.89;0.40), (0.00;6.83) |
| 111 | 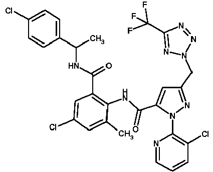 | 4,3 | 678 | (10.19;2.71), (8.67;1.73), (8.65;1.79), (8.48;2.84), (8.48;3.05), (8.47;3.17), (8.47;2.96), (8.14;2.71), (8.14;2.65), (8.12;3.09), (8.12;2.77), (7.60;3.02), (7.59;2.84), (7.58;2.87), (7.57;2.61), (7.47;3.37), (7.46;3.24), (7.35;0.34), (7.33;3.83), (7.32;4.04), (7.31;1.67), (7.29;16.00), (7.28;11.21), (7.27;1.26), (7.26;2.06), (7.25;0.65), (7.24;0.32), (6.29;11.10), (4.95;1.18), (4.93;1.82), (4.91;1.22), (3.47;0.37), (3.47;0.37), (3.45;0.43), (3.31;2319.03), (3.28;38.34), (3.19;0.81), (3.13;0.48), (3.13;0.46), (3.08;0.32), (2.70;0.41), (2.68;1.25), (2.67;2.24), (2.67;2.89), (2.66;2.12), (2.62;0.42), (2.58;1.06), (2.54;4.91), (2.52;14.32), (2.51;169.79), (2.50;310.83), (2.50;402.11), (2.50;280.58), (2.49;135.60), (2.40;0.57), (2.40;0.53), (2.35;0.44), (2.34;1.29), (2.33;2.27), (2.33;2.98), (2.32;2.17), (2.32;1.22), (2.30;0.34), (2.27;0.50), (2.20;0.38), (2.16;15.07), (2.07;1.43), (2.04;0.76), (1.91;0.52), (1.42;0.32), (1.36;0.37), (1.29;7.61), (1.27;7.50), (1.24;0.89), (0.89;1.07), (0.01;1.07), (0.00;18.58), (-0.01;0.77) |
| 112 | 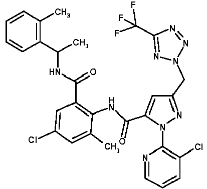 | 4,2 | 658 | (10.16;1.34), (8.62;1.16), (8.60;1.17), (8.48;2.65), (8.48;2.82), (8.47;2.88), (8.47;2.77), (8.14;2.40), (8.14;2.36), (8.12;2.74), (8.12;2.51), (7.61;2.59), (7.59;2.52), (7.59;2.45), (7.57;2.39), (7.45;2.48), (7.45;2.55), (7.32;1.65), (7.30;1.77), (7.30;1.61), (7.29;3.04), (7.28;2.87), (7.26;4.05), (7.09;0.75), (7.09;1.13), (7.08;1.61), (7.07;3.67), (7.06;4.66), (7.05;3.18), (7.04;0.94), (7.04;1.02), (7.02;0.43), (7.02;0.33), (6.29;9.07), (5.14;1.10), (5.12;1.64), (5.10;1.11), (3.31;829.82), (3.29;11.86), (2.67;0.68), (2.67;0.88), (2.66;0.66), (2.54;1.61), (2.52;4.52), (2.51;51.02), (2.50;92.59), (2.50;119.14), (2.50;82.17), (2.49;39.22), (2.45;0.38), (2.33;1.13), (2.33;0.99), (2.32;0.74), (2.32;0.49), (2.29;16.00), (2.15;12.96), (2.08;0.77), (2.07;0.50), (1.36;0.39), (1.25;7.24), (1.23;7.25), (0.89;0.32), (0.00;5.38) |
| 113 | 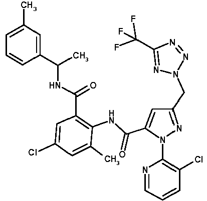 | 4,28 | 658 | (10.19;2.23), (8.58;1.57), (8.56;1.55), (8.47;2.59), (8.47;2.72), (8.46;2.80), (8.46;2.66), (8.14;2.40), (8.13;2.39), (8.12;2.71), (8.11;2.52), (7.60;2.54), (7.59;2.50), (7.58;2.40), (7.57;2.37), (7.46;2.75), (7.46;2.90), (7.32;3.23), (7.32;2.97), (7.27;5.19), (7.14;0.85), (7.12;2.58), (7.10;6.53), (7.07;0.89), (6.98;1.98), (6.97;1.40), (6.29;9.79), (4.94;1.10), (4.92;1.60), (4.90;1.10), (3.31;707.55), (3.29;10.65), (2.67;0.67), (2.67;0.87), (2.66;0.64), (2.66;0.37), (2.56;0.58), (2.54;1.60), (2.52;4.50), (2.51;48.82), (2.50;88.96), (2.50;114.58), (2.50;80.35), (2.49;38.97), (2.33;0.63), (2.33;0.90), (2.32;0.65), (2.23;16.00), (2.16;13.85), (2.07;0.39), (1.29;7.29), (1.27;7.18), (0.00;5.47) |
| 114 | 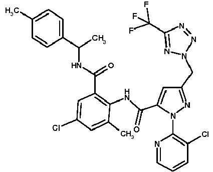 | 4,24 | 658 | (10.19;2.08), (8.57;1.88), (8.55;1.88), (8.48;3.07), (8.47;3.27), (8.47;3.35), (8.46;3.25), (8.14;2.91), (8.13;2.91), (8.12;3.33), (8.11;3.09), (7.60;3.12), (7.59;3.03), (7.58;2.97), (7.57;2.92), (7.46;3.30), (7.46;3.33), (7.45;3.53), (7.35;0.46), (7.33;0.70), (7.31;8.60), (7.30;3.79), (7.27;0.37), (7.26;0.37), (7.25;0.53), (7.23;0.40), (7.18;4.58), (7.16;6.10), (7.10;0.32), (7.05;5.33), (7.03;3.95), (6.30;11.32), (4.94;1.15), (4.92;1.68), (4.91;1.12), (3.31;1332.50), (3.28;21.12), (3.18;0.37), (2.68;0.72), (2.67;1.27), (2.67;1.64), (2.66;1.25), (2.66;0.65), (2.54;2.91), (2.52;8.23), (2.51;97.23), (2.50;177.51), (2.50;229.31), (2.50;158.66), (2.49;76.14), (2.40;0.37), (2.38;0.37), (2.34;0.77), (2.33;1.38), (2.33;1.74), (2.32;1.35), (2.32;0.83), (2.31;1.69), (2.29;0.41), (2.27;0.56), (2.24;15.98), (2.15;16.00), (2.07;1.02), (1.47;0.86), (1.45;0.82), (1.28;8.02), (1.26;7.90), (1.24;0.62), (0.89;0.57), (0.01;0.60), (0.00;11.45), (-0.01;0.47) |
| 115 | 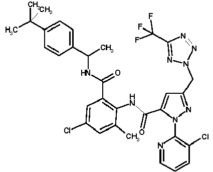 | 5,06 | 700 | (10.22;0.78), (8.59;0.42), (8.57;0.43), (8.47;0.58), (8.47;0.60), (8.46;0.62), (8.46;0.58), (8.13;0.54), (8.13;0.51), (8.11;0.60), (8.11;0.56), (7.60;0.57), (7.59;0.57), (7.58;0.53), (7.57;0.52), (7.46;0.65), (7.46;0.70), (7.36;1.37), (7.32;0.76), (7.32;0.71), (7.29;0.88), (7.27;1.57), (7.22;1.45), (7.19;0.84), (6.30;2.24), (4.93;0.35), (3.31;406.27), (3.28;6.67), (2.67;0.44), (2.67;0.56), (2.66;0.42), (2.54;1.09), (2.52;3.04), (2.51;32.15), (2.50;57.97), (2.50;74.25), (2.50;51.65), (2.49;24.73), (2.33;0.36), (2.33;0.47), (2.32;0.33), (2.16;3.02), (1.82;0.34), (1.30;0.37), (1.29;1.61), (1.27;1.66), (1.26;1.52), (1.25;1.32), (1.24;16.00), (0.00;3.35) |
| 116 | 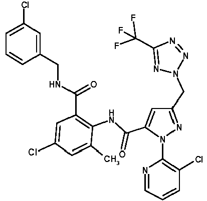 | 4,1 | 664 | (10.25;3.12), (8.83;1.07), (8.81;2.11), (8.80;1.07), (8.48;3.14), (8.47;3.24), (8.46;3.31), (8.46;3.14), (8.13;2.85), (8.13;2.89), (8.11;3.25), (8.11;3.00), (7.59;3.12), (7.58;3.06), (7.57;2.91), (7.56;2.86), (7.48;3.17), (7.48;3.42), (7.38;3.86), (7.37;3.48), (7.32;3.75), (7.31;3.77), (7.30;7.11), (7.25;4.83), (7.24;3.85), (7.24;3.76), (7.23;6.86), (7.21;1.23), (7.21;1.24), (7.20;2.00), (7.20;1.88), (7.19;1.83), (7.18;0.80), (6.29;11.50), (4.33;4.79), (4.32;4.73), (3.77;0.34), (3.75;0.32), (3.74;0.34), (3.68;0.39), (3.67;0.40), (3.64;0.45), (3.61;0.52), (3.60;0.64), (3.58;0.56), (3.57;0.60), (3.55;0.65), (3.51;0.79), (3.31;2921.83), (3.29;38.05), (2.69;0.55), (2.67;2.25), (2.67;2.95), (2.66;2.18), (2.66;1.25), (2.63;0.57), (2.54;5.82), (2.52;15.66), (2.51;168.67), (2.50;305.81), (2.50;393.32), (2.50;270.94), (2.49;128.46), (2.44;0.53), (2.34;0.98), (2.33;1.96), (2.33;2.58), (2.32;1.88), (2.16;16.00), (2.07;1.49), (1.91;0.47), (1.24;0.68), (0.89;1.05), (0.01;0.95), (0.00;18.11), (-0.01;0.69) |
| 117 | 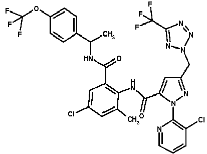 | 4,52 | 728 | (10.21;4.31), (8.70;2.19), (8.68;2.30), (8.62;0.45), (8.61;0.46), (8.61;0.48), (8.60;0.43), (8.48;3.12), (8.47;3.29), (8.47;3.39), (8.46;3.25), (8.33;0.39), (8.33;0.43), (8.31;0.46), (8.31;0.44), (8.14;2.96), (8.14;2.96), (8.12;3.27), (8.12;3.05), (7.89;0.44), (7.89;0.45), (7.77;0.48), (7.77;0.48), (7.76;0.50), (7.75;0.46), (7.74;0.40), (7.73;0.39), (7.60;3.20), (7.59;3.13), (7.58;3.07), (7.57;2.92), (7.47;4.35), (7.47;3.80), (7.41;5.00), (7.39;6.27), (7.35;3.86), (7.34;3.65), (7.32;7.67), (7.23;4.36), (7.21;3.63), (6.32;1.84), (6.29;11.65), (5.01;0.34), (4.99;1.17), (4.97;1.79), (4.95;1.20), (4.93;0.33), (3.31;2596.58), (3.29;31.92), (3.21;1.18), (3.19;0.82), (3.11;0.48), (3.11;0.47), (3.08;0.40), (3.06;0.35), (2.72;0.41), (2.69;0.39), (2.68;1.05), (2.67;1.92), (2.67;2.53), (2.66;1.85), (2.66;0.98), (2.61;0.39), (2.54;4.22), (2.52;12.39), (2.51;145.69), (2.50;266.09), (2.50;343.85), (2.50;238.09), (2.49;114.75), (2.38;0.48), (2.33;1.99), (2.33;2.56), (2.32;1.88), (2.29;0.38), (2.16;16.00), (2.07;1.70), (1.91;0.41), (1.73;2.39), (1.50;0.36), (1.48;0.34), (1.36;0.36), (1.31;7.92), (1.29;7.85), (1.24;0.77), (0.89;0.87), (0.01;0.88), (0.00;17.09), (-0.01;0.70) |
| 118 | 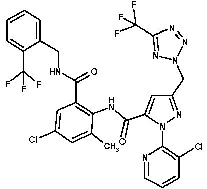 | 4,21 | 698 | (10.27;1.28), (8.88;0.97), (8.87;1.87), (8.85;0.96), (8.48;2.90), (8.48;3.08), (8.47;3.16), (8.47;3.02), (8.14;2.70), (8.13;2.71), (8.12;3.06), (8.11;2.83), (7.71;0.35), (7.68;2.32), (7.66;2.52), (7.61;2.91), (7.60;2.89), (7.59;2.77), (7.58;2.70), (7.50;3.25), (7.50;3.76), (7.46;0.90), (7.45;7.78), (7.44;6.05), (7.42;0.93), (7.40;1.28), (7.38;1.44), (7.37;0.84), (7.30;7.32), (6.29;11.66), (4.50;3.44), (4.49;3.50), (4.01;0.51), (3.31;453.95), (3.28;9.21), (2.67;0.53), (2.67;0.70), (2.66;0.50), (2.54;1.13), (2.52;3.42), (2.51;41.55), (2.50;76.26), (2.50;98.86), (2.50;69.93), (2.49;34.41), (2.34;0.37), (2.33;0.58), (2.33;0.73), (2.32;0.54), (2.18;16.00), (2.15;0.41), (2.07;0.36), (2.05;0.39), (1.36;0.39), (0.00;4.31) |
| 119 | 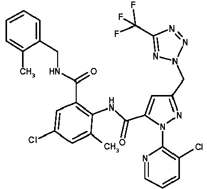 | 4,03 | 644 | (10.24;0.43), (8.67;0.84), (8.48;2.36), (8.48;2.54), (8.47;2.62), (8.47;2.53), (8.15;2.21), (8.14;2.24), (8.13;2.49), (8.12;2.37), (7.60;2.36), (7.59;2.33), (7.58;2.26), (7.57;2.21), (7.47;2.40), (7.46;2.62), (7.37;3.01), (7.37;2.82), (7.28;3.28), (7.17;1.82), (7.16;2.28), (7.12;0.73), (7.11;4.38), (7.10;2.97), (7.09;2.39), (7.09;1.99), (7.07;0.64), (7.07;0.56), (7.04;1.14), (7.03;0.90), (7.02;1.22), (7.00;0.73), (7.00;0.55), (6.29;8.91), (4.31;3.89), (4.29;3.92), (3.31;677.12), (3.28;11.99), (2.67;0.65), (2.67;0.86), (2.66;0.63), (2.54;1.36), (2.52;3.99), (2.51;48.98), (2.50;90.92), (2.50;118.81), (2.50;84.33), (2.49;41.65), (2.34;0.40), (2.33;0.70), (2.33;0.89), (2.32;0.67), (2.30;0.61), (2.23;16.00), (2.20;0.36), (2.15;13.11), (2.07;0.45), (1.28;2.06), (1.24;1.06), (0.00;5.79) |
| 120 | 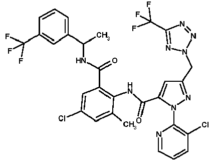 | 4,42 | 712 | (10.21;3.83), (8.76;2.18), (8.74;2.22), (8.48;2.97), (8.47;3.10), (8.47;3.21), (8.46;3.05), (8.13;2.74), (8.13;2.72), (8.11;3.08), (8.11;2.85), (7.89;0.34), (7.75;0.33), (7.67;3.64), (7.61;1.90), (7.60;4.67), (7.59;3.29), (7.58;2.89), (7.57;2.80), (7.55;1.61), (7.53;2.46), (7.48;4.07), (7.47;6.07), (7.45;2.59), (7.44;1.00), (7.34;3.85), (7.33;3.59), (7.30;7.43), (6.32;1.01), (6.29;11.47), (5.05;1.21), (5.03;1.82), (5.02;1.21), (3.31;212.58), (3.29;3.79), (2.67;0.33), (2.54;0.58), (2.51;20.11), (2.51;36.67), (2.50;47.32), (2.50;33.46), (2.49;16.47), (2.33;0.35), (2.32;0.32), (2.16;16.00), (1.73;1.13), (1.52;0.45), (1.50;0.46), (1.33;8.05), (1.31;7.96), (0.00;1.95) |
| 121 | 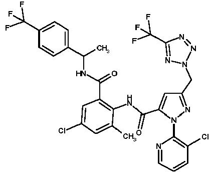 | 4,32 | 712 | (10.21;3.60), (8.77;2.13), (8.75;2.15), (8.62;1.04), (8.61;1.09), (8.61;1.11), (8.60;1.04), (8.49;0.51), (8.49;0.60), (8.48;3.20), (8.47;3.28), (8.47;3.28), (8.46;3.12), (8.34;1.01), (8.33;1.01), (8.31;1.13), (8.31;1.04), (8.17;0.40), (8.16;0.41), (8.15;0.62), (8.14;2.87), (8.14;2.87), (8.12;3.17), (8.12;2.91), (7.89;1.05), (7.89;1.16), (7.77;1.23), (7.77;1.29), (7.77;1.25), (7.76;1.20), (7.76;1.34), (7.75;1.11), (7.74;1.04), (7.73;1.09), (7.71;0.50), (7.64;0.51), (7.64;0.52), (7.61;0.87), (7.60;6.90), (7.59;4.00), (7.58;8.29), (7.57;3.17), (7.51;5.33), (7.49;3.60), (7.48;3.61), (7.47;6.39), (7.36;3.83), (7.35;3.51), (7.33;7.42), (7.28;0.61), (6.32;4.68), (6.29;1.90), (6.28;11.67), (5.04;0.33), (5.02;1.13), (5.01;1.68), (4.99;1.12), (3.44;0.34), (3.38;0.83), (3.31;607.07), (3.28;11.60), (2.75;0.36), (2.67;0.84), (2.67;1.09), (2.66;0.81), (2.54;2.08), (2.52;5.73), (2.51;62.21), (2.50;113.17), (2.50;145.82), (2.50;101.94), (2.49;49.25), (2.35;0.40), (2.34;0.39), (2.33;0.74), (2.33;1.00), (2.32;0.77), (2.19;0.67), (2.16;16.00), (2.08;1.22), (2.07;0.94), (1.73;6.01), (1.50;0.47), (1.48;0.48), (1.36;0.35), (1.33;8.04), (1.31;7.90), (1.28;0.34), (0.89;0.39), (0.01;0.35), (0.00;6.54) |
| 122 | 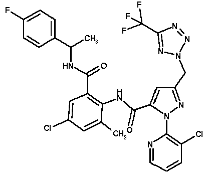 | 4 | 662 | (10.19;2.69), (8.64;1.86), (8.62;1.89), (8.49;2.96), (8.48;3.13), (8.47;3.22), (8.47;3.05), (8.14;2.79), (8.14;2.80), (8.12;3.14), (8.12;2.92), (7.61;2.97), (7.59;2.93), (7.59;2.80), (7.57;2.75), (7.46;3.25), (7.46;3.49), (7.32;3.44), (7.32;5.25), (7.31;6.19), (7.30;4.42), (7.30;8.36), (7.29;3.67), (7.04;3.29), (7.04;1.28), (7.02;5.69), (7.00;1.18), (7.00;2.76), (6.99;0.40), (6.29;11.46), (4.96;1.17), (4.95;1.74), (4.93;1.14), (3.31;638.52), (3.29;7.57), (2.68;0.33), (2.67;0.59), (2.67;0.78), (2.66;0.59), (2.66;0.35), (2.57;0.35), (2.54;1.37), (2.52;3.96), (2.51;44.54), (2.50;80.98), (2.50;104.29), (2.50;72.90), (2.49;35.23), (2.35;0.37), (2.33;0.61), (2.33;0.73), (2.32;0.54), (2.16;16.00), (2.07;0.33), (1.29;8.19), (1.27;8.01), (0.00;4.90) |
| 123 | 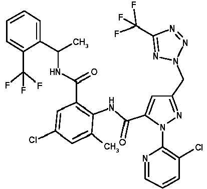 | 4,35 | 712 | (10.15;4.25), (8.85;2.14), (8.83;2.16), (8.62;1.09), (8.62;1.17), (8.61;1.21), (8.60;1.14), (8.49;3.20), (8.48;3.34), (8.48;3.47), (8.47;3.27), (8.33;1.09), (8.33;1.09), (8.31;1.24), (8.31;1.13), (8.14;3.01), (8.14;2.96), (8.12;3.36), (8.12;3.08), (7.90;1.15), (7.89;1.16), (7.89;1.30), (7.77;1.31), (7.77;1.34), (7.77;1.33), (7.76;1.36), (7.76;1.56), (7.75;1.22), (7.74;1.15), (7.73;1.13), (7.66;1.97), (7.64;2.66), (7.61;3.41), (7.60;5.40), (7.59;3.43), (7.58;5.51), (7.54;1.35), (7.52;2.24), (7.50;1.17), (7.47;3.61), (7.46;3.48), (7.46;3.69), (7.32;1.42), (7.31;5.83), (7.30;4.09), (7.29;1.08), (7.19;6.54), (6.32;4.89), (6.30;0.42), (6.28;8.19), (6.28;8.09), (6.24;0.36), (5.28;1.05), (5.26;1.56), (5.24;1.03), (3.44;0.33), (3.32;664.49), (3.30;7.60), (2.68;0.46), (2.67;0.61), (2.67;0.46), (2.62;0.40), (2.58;0.34), (2.54;1.12), (2.52;3.17), (2.51;35.37), (2.51;63.93), (2.50;81.87), (2.50;56.34), (2.49;26.77), (2.33;0.44), (2.33;0.58), (2.32;0.42), (2.30;0.41), (2.15;16.00), (1.73;6.27), (1.51;0.37), (1.50;0.37), (1.36;0.81), (1.28;7.85), (1.26;7.72), (0.00;4.29) |
| 124 | 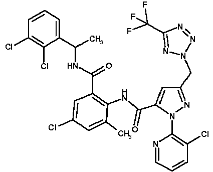 | 4,57 | 712 | (10.18;3.36), (8.88;2.06), (8.86;2.09), (8.49;2.93), (8.49;3.18), (8.48;3.26), (8.48;3.20), (8.15;2.86), (8.15;2.87), (8.13;3.22), (8.13;3.02), (7.62;0.34), (7.61;3.00), (7.60;3.08), (7.59;2.87), (7.58;2.80), (7.48;3.27), (7.47;3.72), (7.45;0.39), (7.43;2.22), (7.43;2.36), (7.41;2.56), (7.41;2.82), (7.39;2.12), (7.39;2.03), (7.37;6.05), (7.36;4.12), (7.29;0.34), (7.27;6.61), (7.22;2.18), (7.20;3.38), (7.18;1.48), (6.29;10.46), (5.24;1.33), (5.22;2.06), (5.21;1.33), (3.31;982.55), (3.28;18.01), (3.18;0.32), (2.68:0.57), (2.67:1.03), (2.67;1.36), (2.66;1.01), (2.54:2.18), (2.52:6.53), (2.51;78.11), (2.50;144.16), (2.50;187.71), (2.50;132.61), (2.49;65.20), (2.42;0.41), (2.34;0.63), (2.33;1.12), (2.33;1.39), (2.32;1.10), (2.29;0.41), (2.18;0.83), (2.16;16.00), (2.07;0.75), (1.38;0.55), (1.37;0.61), (1.36;2.84), (1.27;8.50), (1.26;8.40), (1.24;0.59), (0.89;0.52), (0.01;0.43), (0.00;8.00), (-0.01;0.37) |
| 125 | 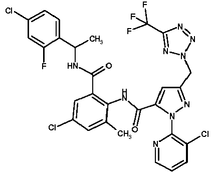 | 4,42 | 696 | (10.18;3.28), (8.77;2.05), (8.75;2.11), (8.62;0.77), (8.61;0.81), (8.61;0.80), (8.60;0.74), (8.49;3.22), (8.48;3.42), (8.47;3.50), (8.47;3.41), (8.33;0.79), (8.33;0.74), (8.31;0.85), (8.31;0.73), (8.14;3.02), (8.14;2.98), (8.12;3.40), (8.12;3.14), (7.89;0.75), (7.89;0.80), (7.77;0.89), (7.77;0.90), (7.77;0.90), (7.76;0.82), (7.76;0.92), (7.75;0.77), (7.74;0.78), (7.73;0.75), (7.61;3.20), (7.60;3.17), (7.59;3.03), (7.58;3.02), (7.47;3.55), (7.47;5.55), (7.38;1.53), (7.36;3.24), (7.35;4.15), (7.34;4.22), (7.32;2.33), (7.31;2.38), (7.29;2.44), (7.29;2.46), (7.27;6.61), (7.16;2.21), (7.15;2.06), (7.14;1.80), (7.13;1.69), (6.32;3.43), (6.28;11.89), (5.15;0.36), (5.13;1.31), (5.11;1.99), (5.10;1.29), (3.62;0.35), (3.62;0.34), (3.60;0.37), (3.59;0.37), (3.57;0.39), (3.31;1849.29), (3.28;27.31), (2.70;0.43), (2.67;1.77), (2.67;2.30), (2.66;1.74), (2.66;0.96), (2.60;0.64), (2.54;4.68), (2.52;12.30), (2.51;130.97), (2.50;235.88), (2.50;302.02), (2.50;207.03), (2.49;97.59), (2.34;0.72), (2.33;1.48), (2.33;1.97), (2.32;1.38), (2.16;16.00), (2.07;1.15), (1.91;0.36), (1.73;4.31), (1.36;1.53), (1.27;8.05), (1.26;7.88), (1.24;0.72), (0.89;0.82), (0.01;0.76), (0.00;14.64), (-0.01;0.54) |
| 126 | 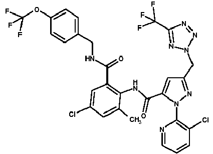 | 4,46 | 714 | (10.26;2.13), (8.85;1.00), (8.84;2.00), (8.82;1.02), (8.47;2.87), (8.47;3.09), (8.46;3.12), (8.46;3.04), (8.14;2.74), (8.14;2.77), (8.12;3.06), (8.12;2.92), (7.60;2.90), (7.59;2.85), (7.58;2.88), (7.57;2.73), (7.48;3.21), (7.48;3.57), (7.39;3.87), (7.38;3.53), (7.35;4.76), (7.34;9.88), (7.21;4.54), (7.19;3.48), (6.29;11.56), (4.35;4.30), (4.34;4.35), (3.31;459.07), (3.28;8.86), (2.67;0.50), (2.67;0.67), (2.66;0.52), (2.54;1.09), (2.52;3.16), (2.51;38.25), (2.50;71.03), (2.50;92.80), (2.50;66.37), (2.49;33.32), (2.33;0.53), (2.33;0.71), (2.32;0.58), (2.16;16.00), (1.36;0.42), (0.00;4.17) |
| 127 | 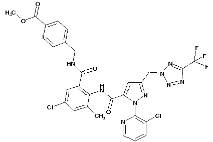 | 3,68 | 688 | (10.25;2.81), (8.88;0.71), (8.87;1.49), (8.85;0.72), (8.49;2.20), (8.48;2.20), (8.47;2.40), (8.47;2.14), (8.16;0.56), (8.16;0.58), (8.14;2.11), (8.14;2.04), (8.12;2.05), (8.12;1.92), (7.84;3.92), (7.82;4.24), (7.63;0.65), (7.63;0.69), (7.61;0.69), (7.60;2.01), (7.60;0.86), (7.59;2.28), (7.58;1.90), (7.58;0.83), (7.57;1.80), (7.49;2.10), (7.48;2.40), (7.40;2.61), (7.40;2.41), (7.38;3.73), (7.36;3.36), (7.31;5.30), (6.30;2.28), (6.28;7.49), (4.41;2.74), (4.39;2.73), (3.84;16.00), (3.60;0.37), (3.32;1131.89), (2.89;0.36), (2.67;1.00), (2.67;1.40), (2.67;1.00), (2.54;2.10), (2.52;6.03), (2.51;72.77), (2.51;134.40), (2.50;175.08), (2.50;124.06), (2.49;61.52), (2.34;0.58), (2.33;1.01), (2.33;1.29), (2.32;1.03), (2.18;0.76), (2.16;10.40), (2.12;1.84), (2.07;0.62), (1.76;0.37), (1.36;2.93), (1.24;0.42), (1.18;0.54), (0.89;0.44), (0.00;6.82), (-0.01;0.33) |
| 128 | 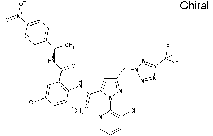 | 3,85 | 689 | (10.19;4.42), (8.82;2.23), (8.80;2.27), (8.49;3.49), (8.48;3.55), (8.47;3.80), (8.47;3.41), (8.16;0.80), (8.16;0.81), (8.15;3.20), (8.14;3.34), (8.12;3.40), (8.12;3.17), (8.10;6.06), (8.08;6.40), (7.63;0.93), (7.63;0.99), (7.61;1.11), (7.61;3.18), (7.60;1.25), (7.59;3.42), (7.59;3.15), (7.58;1.38), (7.57;3.58), (7.56;5.96), (7.54;5.41), (7.48;3.45), (7.48;3.85), (7.38;3.85), (7.37;3.45), (7.32;1.07), (7.28;7.17), (6.31;2.89), (6.27;11.55), (5.07;0.35), (5.05;1.24), (5.03;1.90), (5.02;1.21), (3.42;0.52), (3.31;1018.26), (3.28;18.73), (3.16;0.35), (2.67;1.52), (2.67;2.03), (2.66;1.44), (2.66;0.78), (2.54;3.51), (2.52;10.98), (2.51;116.18), (2.50;207.94), (2.50;265.52), (2.50;184.54), (2.49;89.02), (2.42;0.47), (2.42;0.46), (2.33;1.47), (2.33;1.94), (2.32;1.50), (2.18;0.97), (2.16;16.00), (2.13;2.62), (2.07;0.93), (1.91;0.41), (1.36;3.28), (1.34;8.20), (1.32;8.03), (1.24;0.58), (1.09;0.47), (1.04;0.35), (0.89;0.75), (0.01;0.71), (0.00;12.67), (-0.01;0.55) |
| 129 | 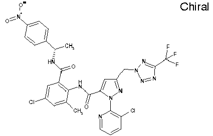 | 3,86 | 689 | (10.19;2.91), (8.82;2.04), (8.81;2.07), (8.49;0.95), (8.49;3.21), (8.48;3.43), (8.47;3.44), (8.47;3.20), (8.17;0.67), (8.17;0.68), (8.15;1.13), (8.15;3.35), (8.14;2.96), (8.12;3.33), (8.12;3.15), (8.10;6.04), (8.08;6.44), (7.64;0.75), (7.64;0.77), (7.62;0.84), (7.61;3.28), (7.59;3.34), (7.59;3.23), (7.57;3.43), (7.56;5.89), (7.54;5.37), (7.48;3.34), (7.48;3.64), (7.38;3.79), (7.37;3.44), (7.28;7.30), (7.27;1.15), (7.26;0.38), (7.09;0.73), (7.06;0.79), (6.87;0.44), (6.57;0.87), (6.55;0.81), (6.29;2.93), (6.27;11.48), (5.07;0.35), (5.05;1.24), (5.03;1.87), (5.02;1.20), (5.00;0.34), (3.81;1.23), (3.51;0.45), (3.31;1751.94), (3.29;25.02), (2.67;1.44), (2.67;2.18), (2.67;1.37), (2.66;0.75), (2.54;3.39), (2.52;9.49), (2.51;105.21), (2.51;191.44), (2.50;246.77), (2.50;171.56), (2.49;82.31), (2.33;1.26), (2.33;1.69), (2.32;1.29), (2.18;1.19), (2.16;16.00), (2.07;1.76), (2.06;1.92), (1.36;6.26), (1.34;8.19), (1.32;7.91), (1.24;0.54), (1.09;0.80), (1.04;0.51), (0.89;0.65), (0.00;9.74) |
| 130 | 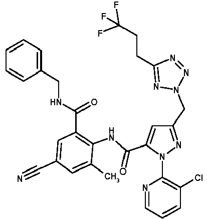 | 3,21 | 649 | (10.47;4.04), (8.88;1.19), (8.87;2.27), (8.85;1.20), (8.49;2.89), (8.49;3.08), (8.48;3.15), (8.47;3.04), (8.15;2.78), (8.14;2.82), (8.13;3.08), (8.12;2.95), (7.87;3.88), (7.80;4.30), (7.80;4.00), (7.60;2.78), (7.59;2.80), (7.58;2.72), (7.57;2.57), (7.27;5.49), (7.26;2.13), (7.25;10.49), (7.24;10.13), (7.24;10.92), (7.23;1.93), (7.22;3.53), (7.21;2.36), (7.20;1.48), (7.20;1.77), (7.19;0.77), (7.19;0.78), (7.18;0.52), (7.18;0.39), (6.07;11.62), (4.34;6.07), (4.33;6.03), (3.31;1019.56), (3.29;10.57), (3.16;4.12), (3.14;5.82), (3.12;4.91), (2.83;0.74), (2.81;0.95), (2.80;2.19), (2.79;1.51), (2.78;2.48), (2.78;2.43), (2.77;1.97), (2.76;1.66), (2.76;2.48), (2.75;1.10), (2.74;1.80), (2.73;0.93), (2.71;0.65), (2.67;0.79), (2.67;1.04), (2.66;0.78), (2.54;2.08), (2.52;4.75), (2.51;57.45), (2.50;107.39), (2.50;141.29), (2.50;100.25), (2.49;49.76), (2.34;0.49), (2.33;0.82), (2.33;1.10), (2.32;0.82), (2.21;16.00), (2.07;1.08), (1.24;0.49), (0.01;0.68), (0.00;14.07), (-0.01;0.63) |
| 131 | 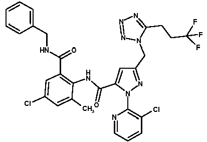 | 3,2 | 658 | (10.21;3.93), (8.78;1.02), (8.77;2.08), (8.75;1.02), (8.47;2.63), (8.47;2.85), (8.46;2.90), (8.46;2.81), (8.14;2.55), (8.13;2.60), (8.12;2.86), (8.11;2.68), (7.59;2.71), (7.58;2.67), (7.57;2.57), (7.56;2.53), (7.47;2.86), (7.47;3.18), (7.37;3.38), (7.36;3.08), (7.24;8.51), (7.23;16.00), (7.21;1.25), (7.21;0.99), (7.20;2.03), (7.19;7.86), (7.18;1.66), (7.17;0.73), (7.17;0.62), (7.16;0.33), (5.89;9.49), (4.34;4.66), (4.32;4.62), (3.31;583.58), (3.28;4.30), (3.26;1.19), (3.25;3.10), (3.23;3.58), (3.21;3.21), (2.88:0.46), (2.85;1.45), (2.84;1.00), (2.83;1.47), (2.82;1.77), (2.81;1.55), (2.80;1.47), (2.80:0.97), (2.78;1.24), (2.78;0.56), (2.76;0.40), (2.67;0.57), (2.67;0.75), (2.66;0.58), (2.66;0.32), (2.54;1.19), (2.52;3.63), (2.51;42.34), (2.50;78.70), (2.50;103.20), (2.50;73.65), (2.49;36.73), (2.33;0.59), (2.33;0.77), (2.32;0.57), (2.15;14.26), (2.07;0.68), (0.01;0.51), (0.00;10.02), (-0.01;0.46) |
| 132 | 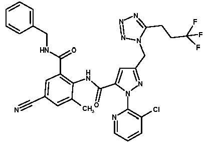 | 2,84 | 649 | (10.47;4.53), (8.88;1.26), (8.87;2.45), (8.85;1.16), (8.48;2.86), (8.47;3.02), (8.47;3.02), (8.46;2.84), (8.14;2.76), (8.14;2.73), (8.12;3.08), (8.12;2.87), (7.87;3.93), (7.81;4.22), (7.80;3.87), (7.60;2.75), (7.58;2.67), (7.58;2.61), (7.56;2.54), (7.26;10.58), (7.25;11.01), (7.23;6.59), (7.22;2.18), (7.21;1.95), (7.20;1.38), (7.20;1.64), (7.19;0.85), (7.19;0.87), (5.90;11.36), (4.34;5.66), (4.33;5.49), (4.04;0.35), (3.82;0.38), (3.80;0.36), (3.79;0.38), (3.78;0.38), (3.74;0.40), (3.73;0.39), (3.69;0.43), (3.65;0.55), (3.61;0.65), (3.59;0.63), (3.56;0.70), (3.48;1.12), (3.47;1.15), (3.47;1.24), (3.46;1.31), (3.31;3350.86), (3.28;19.04), (3.27;3.57), (3.25;4.82), (3.23;4.71), (3.21;4.03), (2.88;0.68), (2.85;1.84), (2.83;1.86), (2.83;2.21), (2.81;1.98), (2.80;1.84), (2.79;1.61), (2.78;0.87), (2.76;0.65), (2.75;0.37), (2.73;0.38), (2.71;0.42), (2.69;0.67), (2.67;3.00), (2.67;3.89), (2.66;3.04), (2.66;1.71), (2.64;0.72), (2.63;0.75), (2.54;7.54), (2.52;20.69), (2.51;216.36), (2.50;396.90), (2.50;515.80), (2.50;362.54), (2.49;176.13), (2.33;2.50), (2.33;3.29), (2.32;2.31), (2.20;16.00), (2.07;3.18), (1.99;0.46), (1.76;0.43), (1.75;0.42), (1.43;1.39), (1.24;0.91), (0.89;0.38), (0.01;2.37), (0.00;46.60), (-0.01;2.02) |
| 133 | 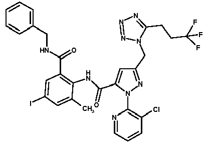 | 3,45 | 750 | (10.20;3.34), (8.76;0.89), (8.75;1.82), (8.73;0.88), (8.47;2.29), (8.47;2.44), (8.46;2.48), (8.45;2.37), (8.14;2.21), (8.13;2.25), (8.12;2.47), (8.11;2.32), (7.75;2.68), (7.75;2.87), (7.64;3.01), (7.63;2.81), (7.59;2.31), (7.58;2.27), (7.57;2.16), (7.56;2.14), (7.26;0.50), (7.24;3.75), (7.24;4.82), (7.23;16.00), (7.22;1.78), (7.21;1.54), (7.20;1.48), (7.19;6.00), (7.17;0.59), (5.88;7.99), (4.33;3.93), (4.31;3.88), (3.31;394.48), (3.29;2.34), (3.27;0.48), (3.26;0.47), (3.25;2.24), (3.23;2.78), (3.21;2.53), (2.88;0.38), (2.85;1.25), (2.84;0.79), (2.83;0.85), (2.83;1.25), (2.82;1.50), (2.81;1.31), (2.80;1.23), (2.79;0.80), (2.78;1.04), (2.77;0.44), (2.75;0.34), (2.67;0.32), (2.67;0.43), (2.66;0.33), (2.54;0.83), (2.51;24.09), (2.50;44.07), (2.50;57.15), (2.50;40.26), (2.49;19.59), (2.33;0.36), (2.11;12.05), (2.07;0.35), (0.00;5.34) |
| 134 | 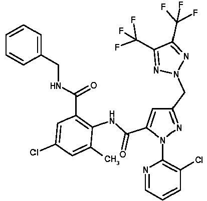 | 4,52 | 697 | (10.24;4.40), (8.79;1.14), (8.77;2.27), (8.76;1.12), (8.49;3.01), (8.48;3.20), (8.48;3.26), (8.47;3.15), (8.15;2.84), (8.14;2.83), (8.13;3.18), (8.12;2.91), (7.60;3.09), (7.59;2.98), (7.58;2.88), (7.57;2.83), (7.47;3.19), (7.47;3.39), (7.37;3.84), (7.36;3.48), (7.28;7.64), (7.24;1.75), (7.22;9.59), (7.20;5.86), (7.19;1.54), (7.18;1.99), (7.17;1.44), (7.17;1.84), (7.16;1.32), (7.15;1.56), (7.14;0.51), (7.14;0.51), (6.08;10.17), (4.34;5.05), (4.32;4.97), (3.59;0.37), (3.57;0.41), (3.52;0.49), (3.51;0.52), (3.46;0.76), (3.43;0.98), (3.31;1983.81), (3.28;11.60), (2.69;0.39), (2.68;1.09), (2.67;1.92), (2.67;2.46), (2.66;1.88), (2.66;1.06), (2.64;0.42), (2.60;0.71), (2.54;4.58), (2.52;12.83), (2.51;137.24), (2.50;250.88), (2.50;324.77), (2.50;226.64), (2.49;109.31), (2.33;1.60), (2.33;2.13), (2.32;1.55), (2.32;0.78), (2.16;16.00), (2.07;2.01), (1.24;0.55), (0.01;1.73), (0.00;32.99), (-0.01;1.37) |
| 135 | 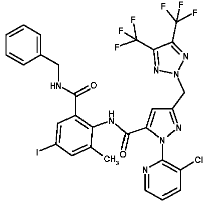 | 4,75 | 789 | (10.23;4.41), (8.77;1.16), (8.75;2.37), (8.74;1.17), (8.48;2.93), (8.48;3.13), (8.47;3.20), (8.47;3.10), (8.15;2.82), (8.14;2.82), (8.12;3.17), (8.12;2.94), (7.76;3.45), (7.75;3.72), (7.63;3.95), (7.63;3.76), (7.60;3.01), (7.59;2.95), (7.58;2.84), (7.57;2.79), (7.28;7.32), (7.22;11.04), (7.21;7.47), (7.20;7.27), (7.19;1.21), (7.19;1.66), (7.18;1.62), (7.17;1.53), (7.17;1.64), (7.16;1.30), (7.16;1.20), (7.15;1.44), (7.14;0.74), (7.13;0.33), (6.08;10.04), (4.33;5.11), (4.31;5.05), (3.31;981.64), (3.28;7.49), (3.18;0.37), (2.68;0.48), (2.67;0.92), (2.67;1.20), (2.66;0.93), (2.54;1.83), (2.52;5.75), (2.51;69.88), (2.50;129.84), (2.50;169.81), (2.50;120.41), (2.49;59.54), (2.34;0.60), (2.33;0.98), (2.33;1.26), (2.32;0.98), (2.12;16.00), (2.09;0.34), (2.07;0.90), (1.24;0.35), (0.01;0.79), (0.00;15.53), (-0.01:0.69) |
| 136 | 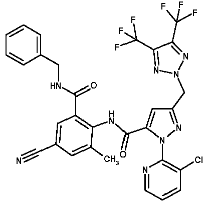 | 4,15 | 688 | (10.54;2.41), (8.93;0.75), (8.92;1.53), (8.91;0.77), (8.50;1.68), (8.50;1.78), (8.49;1.85), (8.49;1.78), (8.16;1.71), (8.16;1.72), (8.15;1.88), (8.15;1.77), (7.89;2.37), (7.81;2.49), (7.81;2.39), (7.70;4.12), (7.61;1.63), (7.61;1.58), (7.60;1.60), (7.59;1.59), (7.34;3.33), (7.25;1.51), (7.24;3.72), (7.23;2.46), (7.22;3.42), (7.21;1.51), (7.18;1.17), (7.17;1.40), (7.16;0.53), (6.10;6.18), (4.33;2.91), (4.33;2.91), (4.03;0.33), (4.02;0.35), (3.63;0.37), (3.62;0.34), (3.61;0.33), (3.61;0.33), (3.60;0.38), (3.58;0.36), (3.57;0.40), (3.56;0.47), (3.56;0.55), (3.55;0.41), (3.55;0.55), (3.54;0.56), (3.53;0.61), (3.52;0.77), (3.52;0.81), (3.51;0.70), (3.51;0.78), (3.50;0.95), (3.49;1.30), (3.48;1.50), (3.48;1.31), (3.48;1.31), (3.47;1.51), (3.47;1.69), (3.46;2.28), (3.46;1.96), (3.45;2.36), (3.45;2.88), (3.44;3.64), (3.44;3.73), (3.43;4.28), (3.43;4.97), (3.42;7.19), (3.41;16.00), (3.36;50.41), (3.35;5.77), (3.35;5.21), (3.34;3.46), (3.34;3.50), (3.34;2.52), (3.33;2.18), (3.32;1.04), (3.32;1.07), (3.32;1.00), (3.31;0.91), (3.31;0.93), (3.31;1.03), (3.31;1.12), (3.30;0.39), (3.29;0.42), (3.29;0.36), (3.26;0.36), (3.26;0.33), (2.62;2.71), (2.62;6.14), (2.62;8.51), (2.61;6.07), (2.61;2.81), (2.56;0.41), (2.56;0.45), (2.54;3.97), (2.54;1.27), (2.53;16.52), (2.52;20.68), (2.52;20.31), (2.51;471.65), (2.51;1037.69), (2.51;1434.82), (2.50;1038.39), (2.50;471.88), (2.40;2.63), (2.39;5.93), (2.39;8.22), (2.39;5.91), (2.38;2.65), (2.21;9.96), (2.08;4.94), (1.99;1.66), (1.40;1.24), (1.23;1.80), (1.19;0.45), (1.17;0.96), (1.16;0.48), (1.11;0.37), (0.85;0.36), (0.10;0.43), (0.01;3.53), (0.00;115.55), (-0.01;3.61), (-0.10;0.45) |
| 137 | 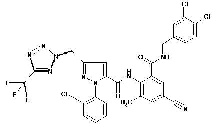 | 3,99 | 689 | (10.42;4.24), (8.94;1.17), (8.93;2.36), (8.91;1.15), (7.89;3.85), (7.88;4.18), (7.83;4.51), (7.82;3.78), (7.55;1.64), (7.54;2.25), (7.53;2.04), (7.53;3.68), (7.52;2.87), (7.51;4.40), (7.51;4.36), (7.48;1.02), (7.47;1.41), (7.46;2.15), (7.45;2.93), (7.45;2.07), (7.44;5.34), (7.43;3.72), (7.43;5.19), (7.43;6.71), (7.42;7.61), (7.42;4.46), (7.41;3.12), (7.41;0.85), (7.38;0.38), (7.32;6.47), (7.27;2.67), (7.27;2.44), (7.25;2.61), (7.25;2.00), (6.29;12.20), (6.06;1.04), (4.31;4.10), (4.30;4.08), (3.30;189.92), (2.67;0.40), (2.54;0.86), (2.51;23.60), (2.50;42.82), (2.50;55.02), (2.50;38.32), (2.49;18.62), (2.33;0.39), (2.22;16.00), (2.07;5.06), (0.00;5.78) |
| 138 | 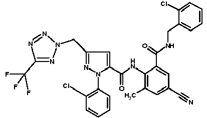 | 3,71 | 654 | (10.43;4.49), (8.92;1.20), (8.90;2.34), (8.89;1.17), (7.89;4.54), (7.87;4.90), (7.57;1.67), (7.57;2.04), (7.55;2.22), (7.55;3.78), (7.54;0.53), (7.53;0.54), (7.50;1.01), (7.49;1.41), (7.49;1.83), (7.48;2.52), (7.47;2.06), (7.46;2.69), (7.45;5.22), (7.45;7.39), (7.44;4.54), (7.44;3.77), (7.42;2.72), (7.42;2.82), (7.41;0.86), (7.41;0.85), (7.40;3.28), (7.40;3.16), (7.39;0.60), (7.38;2.40), (7.36;2.67), (7.31;6.10), (7.28;1.33), (7.27;1.38), (7.26;2.85), (7.24;1.44), (7.23;1.24), (7.16;1.89), (7.16;2.05), (7.14;2.74), (7.14;2.69), (7.12;1.18), (7.12;1.08), (6.28;11.98), (6.06;1.27), (4.39;4.32), (4.37;4.33), (3.31;439.67), (2.67;0.40), (2.67;0.53), (2.67;0.40), (2.54;1.02), (2.52;2.55), (2.51;31.76), (2.50;57.88), (2.50;74.27), (2.50;51.78), (2.49;25.23), (2.33;0.44), (2.33;0.56), (2.32;0.44), (2.23;16.00), (0.01;0.57), (0.00;10.76), (-0.01;0.46) |
| 139 | 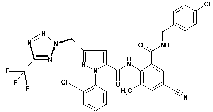 | 3,73 | 654 | (10.42;4.27), (8.92;1.17), (8.91;2.25), (8.89;1.11), (7.88;3.94), (7.88;4.21), (7.81;4.53), (7.81;3.86), (7.57;1.83), (7.56;2.14), (7.55;2.62), (7.55;2.63), (7.54;3.05), (7.54;0.53), (7.53;0.49), (7.50;1.09), (7.49;1.28), (7.48;2.32), (7.47;2.51), (7.46;2.66), (7.45;2.83), (7.44;3.05), (7.44;3.27), (7.42;5.26), (7.42;3.20), (7.40;1.33), (7.40;0.80), (7.38;0.50), (7.38;0.43), (7.36;0.36), (7.36;0.35), (7.33;6.30), (7.30;3.58), (7.29;1.94), (7.27;8.68), (7.25;9.51), (7.24;2.55), (7.23;1.76), (7.22;3.67), (6.30;12.00), (6.08;1.24), (4.31;4.13), (4.29;4.10), (3.31;94.21), (2.54;0.41), (2.51;10.87), (2.50;19.54), (2.50;24.84), (2.50;17.35), (2.22;16.00), (2.07;1.97), (0.00;3.46) |
| 140 | 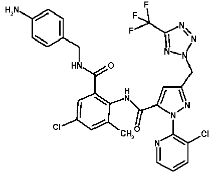 | 2,55 | 645 | (10.24;1.20), (10.23;4.54), (10.11;0.52), (8.73;0.55), (8.73;0.34), (8.57;1.21), (8.56;2.32), (8.55;1.19), (8.49;3.19), (8.48;3.45), (8.48;3.21), (8.47;3.16), (8.25;0.66), (8.25;0.64), (8.15;2.96), (8.14;3.28), (8.13;3.32), (8.12;3.34), (7.61;3.26), (7.59;3.23), (7.59;3.10), (7.57;2.97), (7.49;0.69), (7.47;1.60), (7.45;3.33), (7.45;3.50), (7.37;0.64), (7.35;0.51), (7.32;4.36), (7.32;4.13), (7.30;0.44), (7.28;7.31), (7.20;0.83), (7.18;0.78), (7.17;0.38), (7.07;0.35), (6.92;5.27), (6.89;5.74), (6.48;6.81), (6.46;6.36), (6.31;11.49), (4.91;4.12), (4.29;0.63), (4.27;0.86), (4.18;0.35), (4.16;4.74), (4.15;4.68), (3.73;0.33), (3.69;0.32), (3.67;0.33), (3.65;0.36), (3.64;0.34), (3.62;0.43), (3.60;0.48), (3.56;0.50), (3.52;0.63), (3.51;0.72), (3.50;0.76), (3.48;0.91), (3.46;1.06), 645(3.43;1.34), (3.31;3082.91), (3.29;25.28), (3.19;0.71), (3.18;0.56), (3.12;0.39), (3.11;0.38), (3.00;0.34), (2.89;0.35), (2.73;0.38), (2.71;0.42), (2.70;0.74), (2.68;1.57), (2.67;2.69), (2.67;3.54), (2.66;2.59), (2.63;0.65), (2.61;0.86), (2.58;1.14), (2.54;9.43), (2.51;204.09), (2.50;367.77), (2.50;469.66), (2.50;325.18), (2.49;156.58), (2.34;1.48), (2.33;2.47), (2.33;3.16), (2.32;2.34), (2.15;3.69), (2.14;16.00), (2.07;2.34), (1.40;1.22), (1.32;0.33), (1.24;1.55), (0.01;2.18), (0.00;40.88), (-0.01;1.65) |
| 141 | 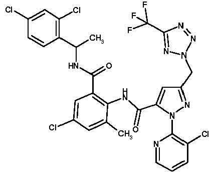 | 4,67 | 713 | (10.17;4.62), (8.83;2.34), (8.82;2.38), (8.49;3.02), (8.49;3.25), (8.48;3.31), (8.48;3.18), (8.15;2.91), (8.14;2.87), (8.13;3.31), (8.12;3.04), (7.61;3.10), (7.60;3.05), (7.59;2.97), (7.58;2.90), (7.49;4.63), (7.49;4.91), (7.48;3.44), (7.47;3.93), (7.41;2.74), (7.39;3.86), (7.36;3.82), (7.35;3.48), (7.29;2.75), (7.29;2.68), (7.27;8.00), (6.32;0.55), (6.28;11.80), (5.21;0.35), (5.19;1.38), (5.17;2.11), (5.16;1.37), (3.50;0.33), (3.31;1344.62), (2.69;0.33), (2.67;1.15), (2.67;1.55), (2.66;1.18), (2.66;0.66), (2.54;3.37), (2.51;89.45), (2.50;162.71), (2.50;209.79), (2.50;145.62), (2.49;70.52), (2.33;1.05), (2.33;1.45), (2.32;1.08), (2.16;16.00), (2.07;2.44), (1.73;0.52), (1.25;8.50), (1.23;8.75), (0.01;0.38), (0.00;6.80) |
| 142 | 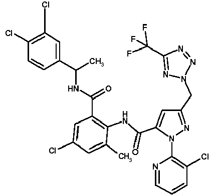 | 4,58 | 713 | (10.19;4.58), (8.70;2.30), (8.68;2.34), (8.47;2.99), (8.47;3.22), (8.46;3.29), (8.46;3.18), (8.13;2.80), (8.12;2.79), (8.11;3.12), (8.10;2.89), (7.60;3.01), (7.58;3.02), (7.58;3.04), (7.56;6.99), (7.56;4.53), (7.48;4.39), (7.48;3.65), (7.47;3.94), (7.46;5.33), (7.34;3.86), (7.33;3.59), (7.30;7.58), (7.29;2.64), (7.28;2.45), (7.27;2.05), (7.26;1.93), (6.29;11.71), (4.97;0.34), (4.95;1.28), (4.93;1.89), (4.92;1.24), (3.44;0.51), (3.42;0.51), (3.42;0.53), (3.32;1273.31), (3.29;11.86), (3.23;0.70), (2.68;0.48), (2.67;0.81), (2.67;1.04), (2.67;0.83), (2.66;0.46), (2.54;2.20), (2.52;5.26), (2.51;62.90), (2.51;114.98), (2.50;148.61), (2.50;103.66), (2.49;50.63), (2.33;0.80), (2.33;1.09), (2.32;0.84), (2.16;16.00), (2.07;1.18), (1.30;7.96), (1.28;7.86), (1.24;0.38), (0.00;4.51) |
| 143 | 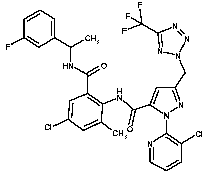 | 4 | 662 | (10.19;4.49), (8.68;0.33), (8.66;2.27), (8.64;2.39), (8.47;3.07), (8.47;3.13), (8.46;3.32), (8.46;3.00), (8.13;2.60), (8.13;2.75), (8.11;3.08), (8.11;2.71), (7.60;2.88), (7.59;2.87), (7.58;2.86), (7.57;2.69), (7.47;3.33), (7.47;3.54), (7.34;3.79), (7.33;3.49), (7.29;7.50), (7.28;2.12), (7.26;1.93), (7.24;1.08), (7.14;3.65), (7.12;3.68), (7.00;1.02), (6.99;0.96), (6.98;1.74), (6.97;1.61), (6.96;0.90), (6.95;0.96), (6.49;0.43), (6.29;11.60), (5.75;0.69), (5.00;0.49), (4.98;1.42), (4.96;2.06), (4.94;1.36), (4.93;0.45), (4.92;0.40), (4.10;0.32), (4.00;0.38), (3.96;0.33), (3.91;0.33), (3.85;0.36), (3.84;0.35), (3.80;0.40), (3.78;0.34), (3.77;0.39), (3.74;0.42), (3.73;0.37), (3.71;0.42), (3.67;0.44), (3.63;0.53), (3.63;0.52), (3.59;0.68), (3.56;0.69), (3.55;0.72), (3.52;0.81), (3.51;0.87), (3.48;0.97), (3.46;1.24), (3.43;1.54), (3.31;3191.17), (3.28;32.44), (2.79;0.33), (2.79;0.32), (2.77;0.35), (2.75;0.37), (2.74;0.44), (2.74;0.42), (2.73;0.42), (2.73;0.45), (2.72;0.42), (2.70;0.94), (2.67;2.95), (2.67;3.79), (2.66;2.87), (2.66;1.67), (2.64;0.67), (2.62;0.83), (2.54;8.64), (2.52;19.17), (2.51;217.30), (2.50;394.59), (2.50;508.10), (2.50;350.97), (2.49;168.84), (2.44;0.63), (2.42;0.48), (2.34;1.29), (2.33;2.59), (2.33;3.36), (2.32;2.50), (2.17;1.12), (2.16;16.00), (2.07;5.60), (1.40;1.14), (1.33;0.42), (1.30;8.32), (1.28;8.19), (1.24;1.06), (0.01;1.34), (0.00;25.98), (-0.01;1.06) |
| 144 | 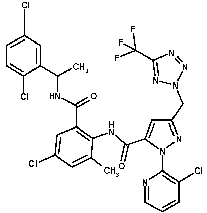 | 4,58 | 713 | (10.20;4.34), (8.77;2.18), (8.75;2.27), (8.46;3.07), (8.46;3.29), (8.45;3.36), (8.45;3.26), (8.12;2.77), (8.11;2.75), (8.10;3.04), (8.09;2.88), (7.60;2.98), (7.59;2.96), (7.58;2.90), (7.57;2.79), (7.53;4.08), (7.52;4.26), (7.49;3.32), (7.49;3.59), (7.45;4.63), (7.43;6.38), (7.37;3.80), (7.36;3.53), (7.33;3.37), (7.32;3.18), (7.31;2.57), (7.30;2.61), (7.29;6.70), (6.29;11.42), (5.27;0.34), (5.25;1.39), (5.23;2.11), (5.21;1.40), (5.20;0.35), (3.31;574.12), (3.28;6.77), (2.67;0.54), (2.67;0.75), (2.67;0.56), (2.54;1.56), (2.52;3.64), (2.51;43.20), (2.50;79.10), (2.50;102.35), (2.50;71.83), (2.49;35.54), (2.33;0.61), (2.33;0.75), (2.32;0.54), (2.17;16.00), (2.07;1.30), (1.26;8.27), (1.25;8.22), (0.00;5.59) |
| 145 | 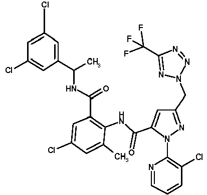 | 4,66 | 713 | (10.20;4.56), (8.68;2.30), (8.67;2.46), (8.62;0.62), (8.61;0.64), (8.61;0.57), (8.60;0.61), (8.47;0.37), (8.46;2.88), (8.46;2.99), (8.45;3.10), (8.45;2.98), (8.34;0.65), (8.33;0.61), (8.31;0.59), (8.31;0.67), (8.12;2.73), (8.11;2.68), (8.10;2.98), (8.09;2.81), (7.90;0.66), (7.89;0.68), (7.77;0.79), (7.76;0.73), (7.75;0.59), (7.74;0.52), (7.73;0.62), (7.59;2.93), (7.58;3.01), (7.57;2.76), (7.56;2.77), (7.49;3.37), (7.48;3.59), (7.47;2.19), (7.42;2.32), (7.42;4.53), (7.41;3.14), (7.38;10.19), (7.37;8.30), (7.35;3.77), (7.35;3.59), (7.30;7.54), (6.32;2.95), (6.29;11.77), (4.98;0.38), (4.96;1.22), (4.95;1.84), (4.93;1.25), (3.99;0.33), (3.87;0.35), (3.82;0.33), (3.80;0.40), (3.79;0.36), (3.74;0.38), (3.70;0.39), (3.69;0.41), (3.66;0.45), (3.64;0.44), (3.63;0.48), (3.63;0.51), (3.61;0.60), (3.59;0.64), (3.57;0.64), (3.54;0.64), (3.54;0.71), (3.53;0.68), (3.31;2823.37), (3.29;21.74), (3.23;0.98), (3.21;0.48), (3.19;0.40), (3.18;0.42), (3.15;0.32), (3.14;0.33), (2.76;0.36), (2.71;0.41), (2.70;0.71), (2.67;2.53), (2.67;3.20), (2.66;2.54), (2.66;1.36), (2.54;7.20), (2.52;17.69), (2.51;191.79), (2.50;343.83), (2.50;437.53), (2.50;302.73), (2.49;145.37), (2.33;2.30), (2.33;2.96), (2.32;2.14), (2.17;16.00), (2.07;2.39), (1.73;3.60), (1.40;1.43), (1.33;0.33), (1.30;8.02), (1.28;7.81), (1.24;1.30), (0.00;28.01) |
| 146 | 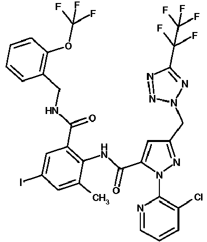 | 4,84 | 856 | (8.80;0.91), (8.48;3.15), (8.47;3.29), (8.46;3.35), (8.46;3.16), (8.13;2.97), (8.13;2.90), (8.11;3.28), (8.11;2.99), (7.77;3.30), (7.77;3.50), (7.67;3.91), (7.67;3.64), (7.65;0.49), (7.65;0.48), (7.64;0.43), (7.63;0.46), (7.63;0.52), (7.60;3.16), (7.59;3.06), (7.58;2.94), (7.57;2.96), (7.41;0.38), (7.39;0.77), (7.39;0.92), (7.38;0.74), (7.38;1.28), (7.37;0.90), (7.36;2.46), (7.35;1.16), (7.34;2.96), (7.33;2.12), (7.33;1.91), (7.31;4.63), (7.31;5.03), (7.29;5.92), (7.22;1.65), (7.22;1.50), (7.21;1.97), (7.20;1.63), (7.19;0.86), (7.18;0.88), (6.87;0.50), (6.31;10.82), (4.38;4.45), (4.37;4.38), (3.80;3.05), (3.31;100.35), (2.67;0.33), (2.54;0.49), (2.52;1.48), (2.51;18.57), (2.51;33.79), (2.50;43.33), (2.50;29.25), (2.49;13.46), (2.18;0.89), (2.13;16.00), (2.07;0.49), (2.00;0.34), (1.36;6.40), (1.09;0.34), (0.00;1.91) |
| 147 | 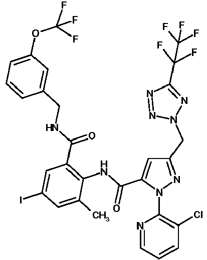 | 4,86 | 856 | (10.24;1.63), (8.86;0.95), (8.85;1.74), (8.83;0.91), (8.59;1.46), (8.47;3.10), (8.47;3.25), (8.46;3.30), (8.46;3.11), (8.13;2.97), (8.13;2.93), (8.11;3.26), (8.11;2.91), (7.83;0.80), (7.81;0.88), (7.77;3.43), (7.76;3.76), (7.64;4.12), (7.64;4.01), (7.62;1.27), (7.60;0.95), (7.59;3.17), (7.58;3.21), (7.57;2.94), (7.56;2.96), (7.52;0.58), (7.50;1.71), (7.48;1.43), (7.40;0.84), (7.38;0.66), (7.36;1.47), (7.34;4.20), (7.32;2.81), (7.30;5.95), (7.27;2.98), (7.25;4.73), (7.18;1.72), (7.16;1.37), (6.31;10.98), (4.85;2.37), (4.54;0.35), (4.53;0.36), (4.37;4.35), (4.35;4.31), (3.42;0.34), (3.31;1291.92), (3.29;9.60), (3.24;0.59), (2.67;1.11), (2.67;1.41), (2.66;1.05), (2.66;0.57), (2.54;1.85), (2.52;5.87), (2.51;85.55), (2.51;158.95), (2.50;207.03), (2.50;141.91), (2.49;67.07), (2.34;0.56), (2.33;1.08), (2.33;1.41), (2.32;1.04), (2.12;16.00), (2.09;0.37), (2.07;2.54), (1.36;0.73), (1.24;0.40), (0.00;9.19), (-0.01:0.35) |
| 148 | 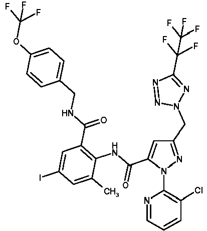 | 4,85 | 856 | (8.85;0.44), (8.84;0.47), (8.46;2.98), (8.46;3.28), (8.45;3.29), (8.45;3.26), (8.13;2.82), (8.13;2.91), (8.11;3.16), (8.11;3.08), (7.75;2.89), (7.66;3.55), (7.65;3.47), (7.59;2.98), (7.58;2.92), (7.57;2.87), (7.56;2.82), (7.47;1.78), (7.45;2.32), (7.34;5.33), (7.32;8.50), (7.30;2.20), (7.28;1.48), (7.21;5.13), (7.19;3.89), (6.31;10.96), (4.35;4.66), (4.33;4.65), (3.76;4.35), (3.31;559.37), (3.19;0.35), (2.68;0.44), (2.67;0.74), (2.67;1.04), (2.67;0.76), (2.54;1.31), (2.52;4.00), (2.51;56.52), (2.51;105.88), (2.50;138.81), (2.50;96.19), (2.49;46.09), (2.33;0.68), (2.33;0.95), (2.32;0.70), (2.18;0.48), (2.12;16.00), (2.09;0.44), (2.07;1.83), (1.36;2.33), (0.00;5.78) |
| 149 | 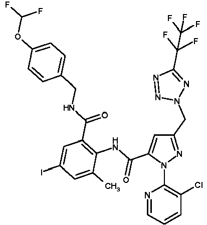 | 4,35 | 838 | (8.88;0.35), (8.50;0.45), (8.47;2.95), (8.46;3.16), (8.46;3.24), (8.45;3.15), (8.13;2.80), (8.13;2.90), (8.11;3.17), (8.11;3.03), (7.85;0.57), (7.83;0.63), (7.74;2.99), (7.65;3.54), (7.64;3.41), (7.59;2.98), (7.58;2.93), (7.57;2.83), (7.56;2.80), (7.39;3.95), (7.37;4.55), (7.35;2.38), (7.33;2.89), (7.31;0.90), (7.29;2.67), (7.28;6.11), (7.26;6.94), (7.24;0.85), (7.19;0.62), (7.17;4.40), (7.16;0.92), (7.15;6.00), (7.14;0.82), (7.13;1.17), (7.12;4.70), (7.10;3.98), (7.05;6.69), (7.03;5.69), (7.00;0.39), (6.98;2.19), (6.96;2.96), (6.31;11.13), (4.76;0.81), (4.31;4.53), (4.30;4.54), (4.04;0.35), (4.02;0.36), (3.72;9.50), (3.32;170.34), (2.67;0.49), (2.67;0.66), (2.67;0.51), (2.54;0.78), (2.52;2.43), (2.51;35.58), (2.51;66.34), (2.50;86.42), (2.50;59.83), (2.49;28.76), (2.33;0.46), (2.33;0.62), (2.32;0.47), (2.18;0.45), (2.11;16.00), (2.07;0.99), (1.99;1.58), (1.36;2.29), (1.19;0.44), (1.18;0.87), (1.16;0.42), (0.00;3.63) |
| 150 | 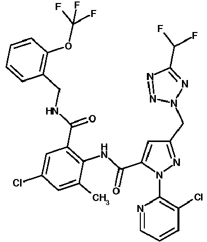 | 3,81 | 696 | (10.25;0.38), (8.81;1.09), (8.48;3.02), (8.47;3.24), (8.47;3.28), (8.46;3.16), (8.13;2.90), (8.12;2.89), (8.11;3.21), (8.10;3.00), (7.65;2.68), (7.64;0.50), (7.63;0.39), (7.63;0.39), (7.60;3.16), (7.59;3.06), (7.58;2.95), (7.57;2.99), (7.52;5.33), (7.49;3.03), (7.48;3.43), (7.45;0.35), (7.40;4.06), (7.39;4.05), (7.39;4.02), (7.38;1.34), (7.37;2.55), (7.36;1.01), (7.35;2.94), (7.34;1.96), (7.33;1.83), (7.32;4.37), (7.30;0.91), (7.30;1.07), (7.29;0.90), (7.28;5.14), (7.22;1.62), (7.22;1.53), (7.20;2.06), (7.19;0.98), (7.18;0.97), (6.87;0.48), (6.49;0.41), (6.21;11.40), (4.90;0.42), (4.39;4.45), (4.37;4.44), (3.81;2.12), (3.31;396.48), (2.67;0.46), (2.67;0.62), (2.66;0.45), (2.54;0.86), (2.52;2.80), (2.51;34.71), (2.50;64.72), (2.50;84.66), (2.50;58.28), (2.49;27.70), (2.33;0.44), (2.33;0.62), (2.32;0.44), (2.23;0.57), (2.21;0.33), (2.18;1.29), (2.17;16.00), (2.12;0.59), (2.07;1.06), (1.36;6.29), (0.00;3.72) |
| 151 | 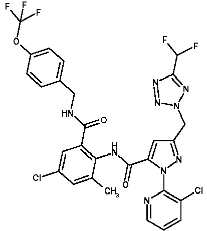 | 3,78 | 696 | (10.25;0.43), (10.24;0.42), (8.83;0.96), (8.47;2.94), (8.47;3.07), (8.46;3.22), (8.46;2.98), (8.14;2.79), (8.13;2.76), (8.12;3.13), (8.11;2.88), (7.64;2.39), (7.60;3.02), (7.59;2.93), (7.58;2.81), (7.57;2.77), (7.51;5.40), (7.48;2.86), (7.47;3.08), (7.42;0.44), (7.39;4.02), (7.38;5.86), (7.36;4.95), (7.34;6.70), (7.30;3.79), (7.23;4.84), (7.21;3.63), (7.19;0.42), (7.15;0.36), (7.13;0.45), (6.21;11.38), (4.35;4.59), (4.34;4.61), (3.80;0.70), (3.31;913.11), (3.28;7.92), (2.67;0.92), (2.67;1.19), (2.66;0.88), (2.54;1.55), (2.52;4.85), (2.51;69.91), (2.50;129.71), (2.50;168.27), (2.50;116.07), (2.49;55.42), (2.44;0.32), (2.34;0.50), (2.33;0.85), (2.33;1.14), (2.32;0.84), (2.22;0.63), (2.16;16.00), (2.13;0.49), (2.12;0.68), (2.07;1.74), (1.36;0.78), (0.00;7.05) |
| 152 | 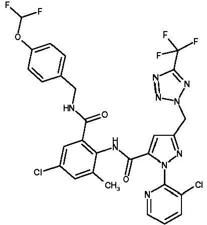 | 3,87 | 696 | (8.90;0.34), (8.50;0.38), (8.47;2.91), (8.47;3.13), (8.46;3.23), (8.46;3.15), (8.14;2.78), (8.13;2.81), (8.12;3.16), (8.11;3.01), (7.85;0.47), (7.83;0.50), (7.60;2.87), (7.58;2.87), (7.57;2.76), (7.56;2.73), (7.45;2.90), (7.38;5.41), (7.38;4.00), (7.37;2.69), (7.37;4.67), (7.35;2.40), (7.33;2.86), (7.31;0.74), (7.29;3.26), (7.28;6.58), (7.26;6.97), (7.24;0.67), (7.19;0.49), (7.17;4.30), (7.16;0.74), (7.16;0.57), (7.15;5.87), (7.13;1.06), (7.12;4.58), (7.10;3.88), (7.04;6.58), (7.02;5.55), (7.00;0.35), (6.98;2.15), (6.96;2.87), (6.29;11.27), (4.76;0.62), (4.32;4.57), (4.31;4.59), (3.72;9.56), (3.31;116.30), (3.13;0.33), (2.67;0.53), (2.67;0.71), (2.67;0.50), (2.54:0.83), (2.52;2.53), (2.51;37.34), (2.51;69.63), (2.50;90.75), (2.50;62.92), (2.49;30.27), (2.33;0.50), (2.33;0.65), (2.32;0.48), (2.15;16.00), (2.07;1.20), (0.00;3.77) |
| 153 | 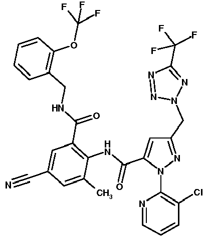 | 3,89 | 705 | (8.93;0.69), (8.87;0.51), (8.85;0.45), (8.57;0.44), (8.48;3.89), (8.47;4.06), (8.47;4.13), (8.46;4.02), (8.37;0.54), (8.36;0.52), (8.33;0.46), (8.33;0.49), (8.32;0.44), (8.32;0.48), (8.25;0.45), (8.25;0.49), (8.23;0.52), (8.23;0.44), (8.13;3.54), (8.13;3.57), (8.11;3.93), (8.11;3.74), (8.07;0.59), (8.00;0.82), (7.94;0.34), (7.88;2.65), (7.85;5.79), (7.66;0.47), (7.65;0.34), (7.64;0.60), (7.63;0.40), (7.60;3.49), (7.59;3.51), (7.58;3.41), (7.57;3.29), (7.56;0.72), (7.55;0.63), (7.54;0.59), (7.53;0.50), (7.50;0.36), (7.46;0.47), (7.44;0.87), (7.41;3.49), (7.39;3.69), (7.38;0.64), (7.36;1.57), (7.35;3.71), (7.34;3.58), (7.33;6.06), (7.33;6.93), (7.32;6.65), (7.32;5.42), (7.30;2.07), (7.28;0.50), (7.27;0.41), (7.26;0.66), (7.24;2.70), (7.24;2.59), (7.22;3.38), (7.21;1.98), (7.20;1.92), (7.19;0.49), (6.83;1.36), (6.49;1.47), (6.29;12.19), (6.25;2.07), (5.45;1.09), (4.44;0.48), (4.43;0.50), (4.40;6.52), (4.38;6.43), (3.90;1.08), (3.31;1791.93), (3.29;14.49), (2.67;1.44), (2.67;2.11), (2.67;1.52), (2.63;3.17), (2.54;2.62), (2.52;8.04), (2.51;117.02), (2.51;218.27), (2.50;284.96), (2.50;195.75), (2.49;92.75), (2.41;0.35), (2.33;1.50), (2.33;1.96), (2.32;1.45), (2.32;0.81), (2.29;1.36), (2.22;16.00), (2.18;0.88), (2.14;1.35), (2.07;3.81), (1.99;2.64), (1.36;1.09), (1.24;0.44), (0.00;12.13), (-0.01;0.44) |
| 154 | 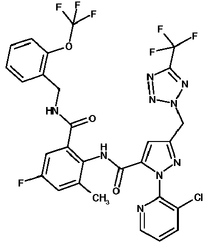 | 3,92 | 698 | (10.18;1.93), (8.77;1.02), (8.76;2.07), (8.74;0.98), (8.48;3.04), (8.48;3.20), (8.47;3.28), (8.46;3.10), (8.13;2.85), (8.13;2.82), (8.11;3.21), (8.11;2.95), (7.60;3.09), (7.59;2.96), (7.58;2.89), (7.57;2.84), (7.43;0.36), (7.42;0.33), (7.38;0.35), (7.36;2.11), (7.34;2.70), (7.32;2.00), (7.32;2.04), (7.30;5.19), (7.29;10.15), (7.28;2.26), (7.26;1.57), (7.26;1.61), (7.20;1.96), (7.20;3.01), (7.19;1.83), (7.18;2.95), (7.18;2.90), (7.16;0.83), (7.16;0.91), (6.49;0.52), (6.29;12.18), (4.39;4.48), (4.38;4.41), (3.91;0.74), (3.32;619.14), (2.67;0.44), (2.67;0.58), (2.67;0.44), (2.54;0.76), (2.52;2.43), (2.51;34.03), (2.51;63.22), (2.50;82.31), (2.50;56.33), (2.49;26.50), (2.33;0.49), (2.33;0.58), (2.32;0.40), (2.17;16.00), (2.07;1.07), (2.06;0.34), (1.99;0.36), (1.36;0.68), (0.00;3.49) |
| 155 | 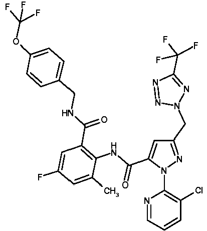 | 3,92 | 698 | (10.18;1.26), (8.79;0.86), (8.78;1.62), (8.76;0.89), (8.47;2.86), (8.47;2.99), (8.46;3.07), (8.46;2.97), (8.14;2.74), (8.14;2.71), (8.12;3.05), (8.12;2.85), (7.60;2.87), (7.59;2.81), (7.58;2.67), (7.57;2.65), (7.35;4.87), (7.33;10.32), (7.27;1.52), (7.27;1.68), (7.25;1.53), (7.24;1.74), (7.19;4.57), (7.19;2.91), (7.18;3.98), (7.17;2.39), (7.16;1.70), (6.29;12.09), (4.36;4.33), (4.34;4.24), (3.86;0.60), (3.44;0.34), (3.41;0.50), (3.31;1084.17), (3.29;8.58), (2.68;0.48), (2.67;0.94), (2.67;1.19), (2.66;0.88), (2.54;1.58), (2.52;4.90), (2.51;70.30), (2.50;130.73), (2.50;170.25), (2.50;117.51), (2.49;56.19), (2.34;0.48), (2.33;0.86), (2.33;1.22), (2.32;0.87), (2.17;16.00), (2.14;0.43), (2.07;1.81), (2.05;0.32), (0.01;0.35), (0.00;7.03) |
| 156 | 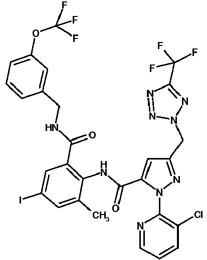 | 4,47 | 806 | (10.23;1.52), (8.86;1.06), (8.85;2.05), (8.83;0.97), (8.48;3.01), (8.47;3.25), (8.46;3.21), (8.46;3.16), (8.13;2.93), (8.13;2.94), (8.11;3.22), (8.11;2.93), (7.77;3.42), (7.76;3.75), (7.66;0.33), (7.64;4.07), (7.64;3.75), (7.61;0.35), (7.59;3.18), (7.58;3.28), (7.57;3.11), (7.56;2.99), (7.55;0.62), (7.53;0.37), (7.50;0.42), (7.48;1.07), (7.46;0.50), (7.37;0.44), (7.36;1.45), (7.34;3.56), (7.32;2.58), (7.30;7.51), (7.26;2.89), (7.25;4.96), (7.19;1.71), (7.16;1.35), (6.29;11.58), (4.85;0.39), (4.37;4.31), (4.35;4.23), (4.05;1.31), (4.01;0.35), (3.46;0.39), (3.46;0.38), (3.44;0.46), (3.31;1354.20), (3.29;10.65), (2.88;0.73), (2.68;0.74), (2.67;1.23), (2.67;1.71), (2.66;1.29), (2.64;0.34), (2.63;0.42), (2.54;2.22), (2.52;7.47), (2.51;100.28), (2.50;184.18), (2.50;237.56), (2.50;161.66), (2.49;75.18), (2.33;1.19), (2.33;1.56), (2.32;1.14), (2.32;0.56), (2.29;2.39), (2.13;16.00), (2.11;0.57), (2.09;0.55), (2.07;2.46), (1.99;0.55), (1.24;0.38), (1.17;0.36), (0.01;0.45), (0.00;9.80), (-0.01;0.39) |
| 157 | 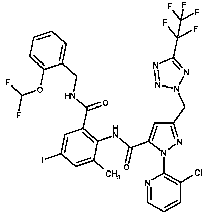 | 4,45 | 838 | (10.25;3.57), (8.73;1.19), (8.72;2.25), (8.70;1.08), (8.48;2.95), (8.47;3.12), (8.47;3.22), (8.46;3.00), (8.13;2.78), (8.13;2.79), (8.11;3.10), (8.11;2.85), (7.77;3.35), (7.77;3.61), (7.67;3.84), (7.67;3.58), (7.60;3.06), (7.59;3.04), (7.58;2.86), (7.57;2.98), (7.55;0.41), (7.53;0.37), (7.48;0.33), (7.46;0.40), (7.37;2.59), (7.35;0.42), (7.34;0.49), (7.33;0.46), (7.32;0.49), (7.30;7.46), (7.28;3.05), (7.26;4.07), (7.24;0.76), (7.24;1.94), (7.22;0.66), (7.20;0.49), (7.18;5.09), (7.15;2.68), (7.13;1.89), (7.08;1.58), (7.06;2.59), (7.05;1.13), (7.00;2.37), (6.87;1.26), (6.61;0.78), (6.31;10.70), (4.82;0.35), (4.49;0.40), (4.48;0.40), (4.34;4.21), (4.33;4.21), (4.04;0.75), (3.43;0.42), (3.31;1010.47), (3.29;7.16), (2.67;0.85), (2.67;1.12), (2.66;0.82), (2.54;1.49), (2.52;4.64), (2.51;64.14), (2.50;119.90), (2.50;156.70), (2.50;109.14), (2.49;52.71), (2.46;0.50), (2.33;0.77), (2.33;1.06), (2.32;0.78), (2.32;0.39), (2.18;2.11), (2.13;15.30), (2.09;0.37), (2.07;0.51), (1.99;0.47), (1.36;16.00), (1.24;0.43), (1.18;0.41), (1.18;0.33), (1.09;0.39), (0.00;5.52) |
| 158 | 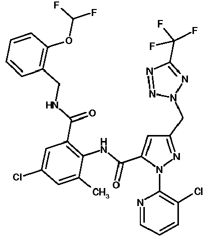 | 4 | 696 | (10.26;0.47), (8.76;0.89), (8.75;1.57), (8.73;0.89), (8.48;2.98), (8.48;3.13), (8.47;3.22), (8.47;3.05), (8.14;2.82), (8.14;2.82), (8.12;3.13), (8.12;2.88), (7.60;3.09), (7.59;3.14), (7.58;2.94), (7.57;3.03), (7.54;0.48), (7.52;0.36), (7.49;3.11), (7.48;3.63), (7.47;0.49), (7.46;0.51), (7.41;4.06), (7.40;3.50), (7.39;0.57), (7.39;0.49), (7.37;2.59), (7.35;0.55), (7.34;0.46), (7.33;0.48), (7.32;0.49), (7.30;7.35), (7.29;2.52), (7.27;3.57), (7.27;3.63), (7.25;2.84), (7.24;1.95), (7.23;1.45), (7.22;0.75), (7.21;0.91), (7.20;1.00), (7.18;5.44), (7.15;2.80), (7.13;1.98), (7.08;1.66), (7.07;1.58), (7.06;2.62), (7.06;2.43), (7.04;1.27), (7.00;2.48), (6.29;11.44), (4.83;0.61), (4.49;0.43), (4.48;0.44), (4.35;4.30), (4.34;4.30), (3.89;0.96), (3.31;274.91), (2.67;0.36), (2.67;0.47), (2.66;0.33), (2.54;0.54), (2.52;1.89), (2.51;28.39), (2.50;53.23), (2.50;69.61), (2.50;48.44), (2.49;23.41), (2.33;0.39), (2.33;0.55), (2.32;0.40), (2.29;0.35), (2.17;16.00), (2.14;0.42), (2.13;0.53), (2.04;0.49), (0.00;2.37) |
| 159 | 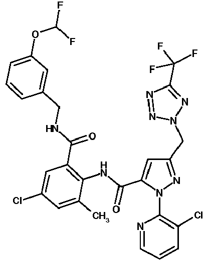 | 3,95 | 696 | (10.27;0.39), (10.27;0.41), (10.26;0.58), (8.83;1.09), (8.54;0.35), (8.48;2.97), (8.47;3.26), (8.46;3.25), (8.46;3.13), (8.14;2.89), (8.13;2.85), (8.12;3.20), (8.11;2.97), (7.59;3.13), (7.58;3.24), (7.57;2.86), (7.56;2.85), (7.53;0.40), (7.48;2.79), (7.48;3.02), (7.41;0.53), (7.40;0.43), (7.38;4.18), (7.38;3.63), (7.36;0.61), (7.35;2.98), (7.30;3.95), (7.29;2.24), (7.27;4.06), (7.25;2.86), (7.24;0.65), (7.22;0.65), (7.20;1.24), (7.17;5.99), (7.15;0.38), (7.12;2.67), (7.10;2.23), (7.08;3.45), (7.04;0.48), (7.02;1.97), (7.01;2.00), (7.00;1.67), (6.98;2.99), (6.29;11.15), (4.80;0.66), (4.35;4.60), (4.33;4.62), (3.82;1.33), (3.53;0.34), (3.52;0.34), (3.49;0.41), (3.49;0.45), (3.45;0.52), (3.45;0.53), (3.44;0.60), (3.42;0.78), (3.31;1679.69), (3.29;12.25), (3.19;0.33), (2.67;1.21), (2.67;1.63), (2.66;1.22), (2.66;0.68), (2.54;2.15), (2.52;6.44), (2.51;92.39), (2.51;173.85), (2.50;228.66), (2.50;158.75), (2.49;76.69), (2.34;0.58), (2.33;1.11), (2.33;1.56), (2.32;1.14), (2.29;0.36), (2.16;16.00), (2.13;0.56), (2.07;0.51), (1.36;0.94), (1.24;0.34), (0.00;7.92), (-0.01;0.37) |
| 160 | 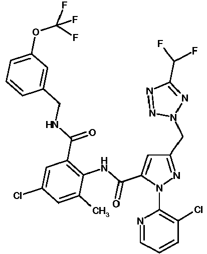 | 3,79 | 696 | (10.25;4.10), (8.88;1.13), (8.87;2.29), (8.85;1.07), (8.59;1.32), (8.48;3.01), (8.47;3.19), (8.47;3.21), (8.46;3.02), (8.21;0.35), (8.13;3.00), (8.13;2.96), (8.11;3.19), (8.11;2.93), (7.83;0.75), (7.81;0.78), (7.76;0.76), (7.67;0.33), (7.65;2.35), (7.64;0.55), (7.64;0.70), (7.62;1.21), (7.60;1.00), (7.59;3.18), (7.58;3.77), (7.57;3.05), (7.56;3.43), (7.52;5.22), (7.52;1.27), (7.51;1.57), (7.50;1.93), (7.49;3.75), (7.48;4.17), (7.46;0.44), (7.40;1.20), (7.39;3.01), (7.38;4.26), (7.37;4.39), (7.35;3.82), (7.33;2.75), (7.31;0.56), (7.28;10.17), (7.26;5.09), (7.20;1.75), (7.18;1.39), (6.87;0.34), (6.22;11.46), (4.85;2.22), (4.54;0.49), (4.53;0.47), (4.37;4.34), (4.36;4.32), (4.11;2.33), (3.32;810.69), (2.67;0.60), (2.67;0.81), (2.67;0.61), (2.54;1.31), (2.52;3.88), (2.51;47.68), (2.51;88.77), (2.50;115.60), (2.50;80.07), (2.49;38.25), (2.33;0.59), (2.33;0.79), (2.32;0.60), (2.21;0.42), (2.18;0.83), (2.16;16.00), (2.13;0.51), (2.07;1.15), (1.36;4.25), (1.24;0.44), (0.00;3.78) |
| 161 | 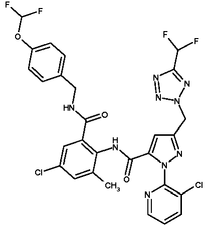 | 3,42 | 678 | (10.24;4.51), (9.98;1.10), (8.81;1.14), (8.79;2.32), (8.78;1.06), (8.50;0.41), (8.48;3.03), (8.47;3.18), (8.47;3.28), (8.46;3.10), (8.14;3.01), (8.14;2.95), (8.12;3.36), (8.12;3.04), (8.00;0.91), (8.00;0.33), (7.99;0.37), (7.98;0.97), (7.85;0.47), (7.83;0.48), (7.65;2.34), (7.61;0.49), (7.60;3.23), (7.59;3.07), (7.58;2.93), (7.57;2.89), (7.52;5.69), (7.50;0.91), (7.48;3.23), (7.47;3.56), (7.43;0.78), (7.39;1.27), (7.38;3.01), (7.37;4.24), (7.36;3.67), (7.35;2.67), (7.31;0.74), (7.29;11.68), (7.27;6.21), (7.25;1.18), (7.24;0.61), (7.24;0.61), (7.23;0.60), (7.19;0.49), (7.16;5.77), (7.14;0.49), (7.13;0.36), (7.07;1.34), (7.06;6.04), (7.04;4.95), (6.99;0.32), (6.97;2.66), (6.22;11.50), (4.76;0.65), (4.32;4.39), (4.30;4.39), (4.03;0.39), (3.53;0.35), (3.53;0.35), (3.46;0.60), (3.32;1551.08), (2.67;1.26), (2.67;1.64), (2.67;1.22), (2.66;0.64), (2.54;2.66), (2.52;7.01), (2.51;95.12), (2.51;177.47), (2.50;231.52), (2.50;159.78), (2.49;75.86), (2.34;0.55), (2.33;1.14), (2.33;1.56), (2.32;1.13), (2.32;0.58), (2.22;0.71), (2.16;16.00), (2.12;0.80), (2.07;2.72), (1.40;0.47), (1.36;1.25), (1.24;0.75), (0.01;0.38), (0.00;8.11), (-0.01;0.34) |
| 162 | 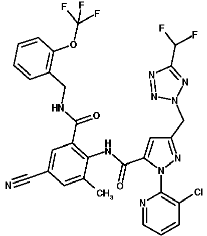 | 3,37 | 687 | (10.49;3.26), (10.11;0.33), (8.90;2.13), (8.47;3.66), (8.47;3.67), (8.13;3.32), (8.11;3.55), (8.00;0.47), (8.00;0.53), (7.94;0.58), (7.89;4.08), (7.84;5.17), (7.65;4.29), (7.60;2.76), (7.59;2.91), (7.58;2.74), (7.57;2.46), (7.52;9.43), (7.46;0.37), (7.42;2.95), (7.40;3.65), (7.39;5.32), (7.38;1.54), (7.36;3.87), (7.35;3.34), (7.34;5.28), (7.33;5.97), (7.33;7.01), (7.32;6.38), (7.32;5.14), (7.25;2.51), (7.24;2.43), (7.23;3.45), (7.21;1.55), (7.21;1.54), (7.06;0.34), (6.49;1.23), (6.23;10.68), (5.75;2.47), (4.44;0.62), (4.43;0.76), (4.39;6.49), (4.38;6.43), (4.04;0.32), (4.02;0.32), (3.54;0.32), (3.51;0.42), (3.50;0.41), (3.47;0.53), (3.46;0.60), (3.41;1.06), (3.32;2461.44), (3.29;20.13), (3.26;1.52), (3.22;0.55), (3.19;0.39), (3.08;0.42), (2.68;0.81), (2.67;1.49), (2.67;2.14), (2.67;1.45), (2.54;3.15), (2.52;8.04), (2.51;121.25), (2.51;229.06), (2.50;301.30), (2.50;209.60), (2.49;101.09), (2.33;1.62), (2.33;2.11), (2.32;1.54), (2.29;1.74), (2.22;14.54), (2.18;0.37), (2.16;0.37), (2.14;1.83), (2.07;16.00), (1.99;1.28), (1.24;0.73), (1.19;0.35), (1.17;0.68), (1.16;0.37), (1.11;1.33), (0.88;0.38), (0.86;0.98), (0.85;0.36), (0.84;0.43), (0.06;2.33), (0.00;2.96) |
| 163 | 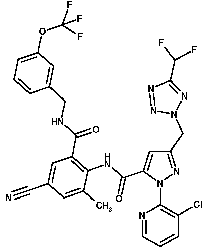 | 3,42 | 687 | (10.50;4.28), (8.96;1.05), (8.95;2.04), (8.93;1.05), (8.48;3.15), (8.48;3.39), (8.47;3.44), (8.47;3.33), (8.14;3.12), (8.13;3.12), (8.12;3.45), (8.11;3.26), (7.89;3.66), (7.82;4.33), (7.82;3.95), (7.66;2.80), (7.59;3.22), (7.58;3.19), (7.57;3.06), (7.56;2.99), (7.52;6.41), (7.39;3.30), (7.39;1.70), (7.37;4.32), (7.35;3.71), (7.32;5.46), (7.31;3.79), (7.29;3.50), (7.28;3.53), (7.21;2.00), (7.20;1.57), (7.19;1.61), (6.23;12.42), (4.43;0.37), (4.41;0.40), (4.38;4.95), (4.37;4.89), (3.32;252.14), (3.29;2.07), (2.54;0.43), (2.52;1.11), (2.51;15.75), (2.51;29.54), (2.50;38.72), (2.50;26.67), (2.49;12.65), (2.29;1.15), (2.22;16.00), (2.13;1.12), (2.07;0.81), (0.00;0.97) |
| 164 | 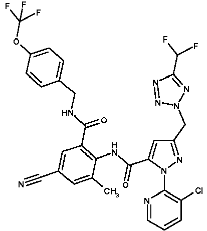 | 3,42 | 687 | (10.50;4.23), (8.92;2.34), (8.91;1.40), (8.48;3.49), (8.47;3.53), (8.14;3.07), (8.12;3.29), (8.01;0.35), (7.92;0.37), (7.88;4.37), (7.86;0.39), (7.83;5.01), (7.65;3.84), (7.60;2.76), (7.59;2.82), (7.58;2.71), (7.57;2.46), (7.52;8.46), (7.39;7.35), (7.38;7.43), (7.37;9.07), (7.34;4.77), (7.24;7.86), (7.22;5.92), (6.49;0.39), (6.23;12.16), (4.40;0.51), (4.36;6.51), (4.34;6.42), (3.55;0.34), (3.54;0.37), (3.52;0.37), (3.51;0.40), (3.50;0.40), (3.48;0.50), (3.47;0.50), (3.43;0.71), (3.40;1.29), (3.31;2676.37), (3.29;21.16), (3.25;1.27), (3.20;0.47), (3.17;0.35), (2.68;0.92), (2.67;1.72), (2.67;2.29), (2.67;1.74), (2.66;0.82), (2.58;0.44), (2.54;3.71), (2.52;9.33), (2.51;136.28), (2.51;255.32), (2.50;334.69), (2.50;230.23), (2.49;109.30), (2.38;0.34), (2.33;1.73), (2.33;2.38), (2.32;1.73), (2.32;0.93), (2.29;1.03), (2.22;16.00), (2.14;1.03), (2.07;7.47), (1.99;0.99), (1.91;0.35), (1.24;0.73), (1.19;0.32), (1.17;0.57), (0.00;4.00) |
| 165 | 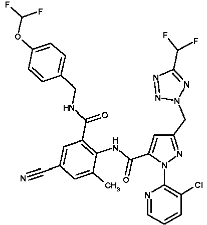 | 3,04 | 669 | (10.49;4.39), (8.90;1.24), (8.88;2.29), (8.87;1.19), (8.48;3.15), (8.48;3.31), (8.47;3.38), (8.47;3.16), (8.15;2.97), (8.14;2.97), (8.13;3.26), (8.12;3.04), (7.88;3.97), (7.80;4.51), (7.65;3.38), (7.60;2.90), (7.59;2.92), (7.58;2.81), (7.57;2.62), (7.52;7.59), (7.39;3.81), (7.35;4.01), (7.33;5.29), (7.32;7.13), (7.30;7.76), (7.17;8.13), (7.07;8.64), (7.05;7.28), (6.98;4.00), (6.23;12.16), (4.32;5.75), (4.31;5.71), (3.42;0.39), (3.31;1034.37), (3.29;8.90), (3.25;0.55), (2.68;0.40), (2.67;0.78), (2.67;1.03), (2.66;0.75), (2.54;2.37), (2.52;4.10), (2.51;59.27), (2.50;111.63), (2.50;146.90), (2.50;101.37), (2.49;48.31), (2.34;0.39), (2.33;0.82), (2.33;1.02), (2.32;0.72), (2.32;0.39), (2.21;16.00), (2.07;3.47), (1.24;0.36), (0.00;2.96) |
| 166 | 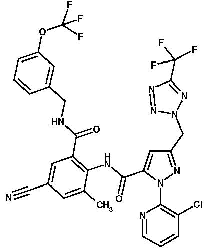 | 3,91 | 705 | (10.51;3.90), (8.97;1.09), (8.96;1.96), (8.94;1.07), (8.48;3.18), (8.48;3.38), (8.47;3.43), (8.47;3.27), (8.14;3.05), (8.14;3.09), (8.12;3.36), (8.12;3.17), (7.93;0.34), (7.89;3.67), (7.83;4.57), (7.82;4.24), (7.65;0.46), (7.60;3.12), (7.59;3.12), (7.58;3.17), (7.57;2.90), (7.48;0.46), (7.46;0.34), (7.37;2.08), (7.35;5.28), (7.34;6.15), (7.30;3.93), (7.28;4.46), (7.20;2.23), (7.18;1.76), (6.30;12.11), (4.54;0.56), (4.53;0.53), (4.39;5.51), (4.37;5.47), (3.41;0.33), (3.32;619.27), (3.29;4.81), (2.67;0.42), (2.67;0.58), (2.67;0.42), (2.54;0.95), (2.52;2.43), (2.51;33.07), (2.51;61.94), (2.50;81.14), (2.50;56.12), (2.49;26.67), (2.33;0.43), (2.33;0.55), (2.32;0.39), (2.22;16.00), (2.07;1.96), (0.00;1.44) |
| 167 | 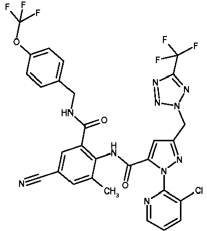 | 3,9 | 705 | (10.51;4.00), (8.93;2.41), (8.48;3.90), (8.47;3.90), (8.15;3.29), (8.13;3.41), (7.92;0.35), (7.89;4.33), (7.83;5.15), (7.63;0.33), (7.60;2.87), (7.59;2.93), (7.58;2.75), (7.57;2.46), (7.38;8.56), (7.36;10.13), (7.22;8.19), (7.20;6.42), (6.34;0.32), (6.30;12.07), (5.75;0.71), (4.39;0.54), (4.36;7.06), (4.35;6.77), (3.74;0.32), (3.70;0.37), (3.69;0.33), (3.67;0.32), (3.66;0.32), (3.63;0.37), (3.62;0.39), (3.59;0.42), (3.55;0.54), (3.54;0.56), (3.51;0.58), (3.50;0.72), (3.47;0.87), (3.47;0.86), (3.46;0.92), (3.42;1.44), (3.41;1.68), (3.31;3861.16), (3.29;33.65), (3.24;1.49), (3.20;0.74), (3.14;0.43), (3.08;0.34), (2.70;0.52), (2.67;2.58), (2.67;3.62), (2.66;2.61), (2.57;0.79), (2.54;5.52), (2.52;14.39), (2.51;211.97), (2.51;396.07), (2.50;517.00), (2.50;357.10), (2.49;170.03), (2.43;0.90), (2.38;0.50), (2.33;2.78), (2.33;3.55), (2.32;2.50), (2.29;0.45), (2.25;0.69), (2.25;0.70), (2.22;16.00), (2.13;0.41), (2.07;10.43), (1.99;0.33), (1.24;1.07), (1.11;0.55), (0.86;0.34), (0.06;0.83), (0.00;6.11) |
| 168 | 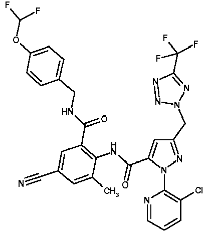 | 3,49 | 687 | (10.50;3.94), (8.89;2.17), (8.48;3.90), (8.47;3.93), (8.47;3.79), (8.15;3.35), (8.13;3.57), (7.88;3.94), (7.81;5.07), (7.61;2.98), (7.59;3.11), (7.58;3.05), (7.57;2.74), (7.36;4.00), (7.34;5.48), (7.31;8.18), (7.29;9.59), (7.16;10.19), (7.05;11.22), (7.03;9.61), (6.97;5.14), (6.30;12.37), (4.33;7.19), (4.31;7.17), (3.41;0.41), (3.36;1.99), (3.31;1297.13), (3.29;12.03), (3.12;0.64), (3.04;0.42), (2.67;1.07), (2.67;1.41), (2.66;1.09), (2.54;2.17), (2.52;5.57), (2.51;79.73), (2.50;150.11), (2.50;197.07), (2.50;136.76), (2.49;65.56), (2.38;0.78), (2.34;0.57), (2.33;1.04), (2.33;1.41), (2.32;1.00), (2.25;0.39), (2.21;16.00), (2.19;1.53), (2.07;5.85), (1.40;1.20), (1.24;0.38), (0.00;3.16) |
| 169 | 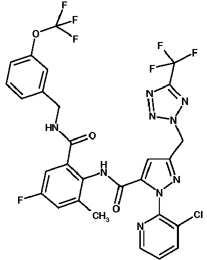 | 3,95 | 698 | (10.17;3.62), (10.05;0.38), (8.83;1.10), (8.81;2.22), (8.80;1.06), (8.59;1.84), (8.48;2.98), (8.48;3.12), (8.47;3.16), (8.46;2.96), (8.14;2.81), (8.13;2.78), (8.12;3.12), (8.11;2.84), (7.83;1.03), (7.81;1.09), (7.76;1.05), (7.64;0.64), (7.64;1.02), (7.62;1.58), (7.60;1.20), (7.59;3.04), (7.58;3.15), (7.57;2.91), (7.56;2.87), (7.52;0.75), (7.50;2.23), (7.48;1.92), (7.46;0.42), (7.40;1.03), (7.38;0.88), (7.37;0.33), (7.34;2.49), (7.32;3.65), (7.30;3.33), (7.29;8.73), (7.28;2.58), (7.27;4.27), (7.25;6.24), (7.24;0.56), (7.18;2.80), (7.17;3.25), (7.16;2.85), (7.15;2.64), (6.29;12.14), (4.85;3.12), (4.54;0.51), (4.53;0.49), (4.38;4.41), (4.37;4.37), (4.08;0.50), (3.32;1134.84), (3.29;13.89), (2.67;0.80), (2.67;1.06), (2.67;0.78), (2.66;0.41), (2.54;1.67), (2.52;4.49), (2.51;62.41), (2.51;115.90), (2.50;150.32), (2.50;103.91), (2.49;49.43), (2.33;0.77), (2.33;1.08), (2.32;0.80), (2.32;0.38), (2.17;16.00), (2.14;0.64), (2.07;1.59), (1.36;2.16), (1.24;0.75), (0.00;4.97) |
| 170 | 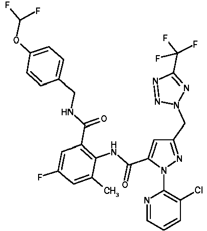 | 3,55 | 680 | (10.17;4.20), (8.75;1.10), (8.74;2.25), (8.72;1.07), (8.48;2.93), (8.47;3.03), (8.46;3.17), (8.46;3.02), (8.14;2.73), (8.14;2.73), (8.12;3.06), (8.12;2.83), (7.60;2.88), (7.59;2.80), (7.58;2.70), (7.57;2.65), (7.33;2.70), (7.31;8.03), (7.28;5.18), (7.26;7.35), (7.25;1.66), (7.24;1.59), (7.17;1.78), (7.16;1.58), (7.14;6.33), (7.03;6.05), (7.01;5.12), (6.96;2.65), (6.29;12.14), (4.32;4.49), (4.31;4.46), (3.31;1199.14), (3.29;10.84), (2.67;0.84), (2.67;1.15), (2.66;0.89), (2.54;1.74), (2.52;4.50), (2.51;66.46), (2.51;125.26), (2.50;164.44), (2.50;114.36), (2.49;54.91), (2.33;0.85), (2.33;1.17), (2.32;0.90), (2.32;0.48), (2.19;0.38), (2.16;16.00), (2.07;5.36), (1.40;0.36), (0.00;2.99) |
| 171 | 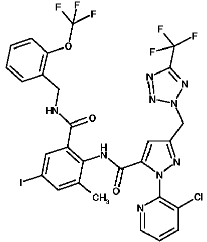 | 4,52 | 806 | (10.24;3.95), (8.80;1.14), (8.78;2.31), (8.77;1.09), (8.48;3.16), (8.47;3.33), (8.47;3.39), (8.46;3.19), (8.13;3.00), (8.13;2.94), (8.11;3.35), (8.11;3.06), (7.78;3.51), (7.77;3.75), (7.67;4.00), (7.66;3.71), (7.60;3.37), (7.59;3.21), (7.58;3.12), (7.57;3.04), (7.43;0.35), (7.42;0.34), (7.38;0.48), (7.37;0.36), (7.35;2.21), (7.33;3.11), (7.33;2.29), (7.32;1.98), (7.31;4.64), (7.31;4.88), (7.29;8.11), (7.22;1.61), (7.21;1.46), (7.20;1.89), (7.19;1.55), (7.19;1.38), (7.18;0.83), (7.18;0.87), (6.29;11.69), (4.38;4.51), (4.36;4.46), (3.41;0.60), (3.39;0.86), (3.32;1025.69), (3.29;10.90), (2.92;1.23), (2.68;0.34), (2.67;0.67), (2.67;0.87), (2.67;0.64), (2.66;0.34), (2.54;1.48), (2.52;3.82), (2.51;50.62), (2.51;93.68), (2.50;121.54), (2.50;83.10), (2.49;39.01), (2.33;0.61), (2.33;0.81), (2.32;0.58), (2.18;0.43), (2.13;16.00), (2.07;1.51), (1.36;2.50), (1.24;0.80), (0.00;3.85) |
| 172 | 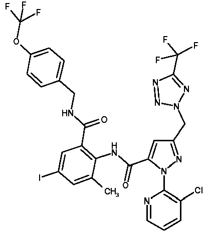 | 4,49 | 806 | (10.24;3.78), (8.82;1.15), (8.81;2.07), (8.80;1.12), (8.47;3.05), (8.47;3.23), (8.46;3.30), (8.45;3.08), (8.14;2.83), (8.14;2.82), (8.12;3.09), (8.12;2.84), (7.76;3.57), (7.65;3.93), (7.60;2.84), (7.59;2.84), (7.58;2.72), (7.57;2.61), (7.34;5.73), (7.33;8.17), (7.32;8.60), (7.21;5.48), (7.19;4.08), (6.50;0.79), (6.29;11.55), (5.75;3.04), (4.34;5.21), (4.33;5.11), (3.65;0.32), (3.61;0.35), (3.54;0.51), (3.51;0.61), (3.47;0.77), (3.32;2361.39), (3.30;19.53), (3.24;0.79), (3.20;0.33), (2.67;1.54), (2.67;2.00), (2.67;1.48), (2.58;0.48), (2.54;3.24), (2.52;8.70), (2.51;118.83), (2.51;221.44), (2.50;288.45), (2.50;198.42), (2.49;93.75), (2.34;0.70), (2.33;1.37), (2.33;1.86), (2.32;1.40), (2.13;16.00), (2.07;4.67), (1.24;0.53), (0.00;3.60) |
| 173 | 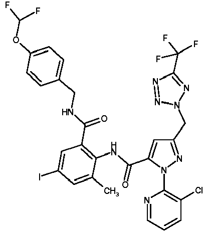 | 4,49 | 788 | (10.23;4.54), (8.79;1.13), (8.77;2.38), (8.76;1.12), (8.48;3.19), (8.47;3.40), (8.46;3.46), (8.46;3.30), (8.14;3.09), (8.14;3.07), (8.12;3.47), (8.12;3.19), (7.76;3.48), (7.76;3.75), (7.63;3.93), (7.63;3.73), (7.60;3.28), (7.59;3.17), (7.58;3.06), (7.57;3.03), (7.33;2.81), (7.32;7.68), (7.28;5.05), (7.26;5.96), (7.15;5.68), (7.05;6.03), (7.02;5.03), (6.96;2.79), (6.29;11.56), (4.31;4.35), (4.29;4.31), (3.32;745.72), (3.30;4.84), (3.27;0.47), (2.67;0.40), (2.67;0.52), (2.67;0.38), (2.54;0.84), (2.52;2.20), (2.51;30.31), (2.51;56.45), (2.50;73.73), (2.50;50.31), (2.49;23.61), (2.33;0.37), (2.33;0.49), (2.32;0.36), (2.12;16.00), (2.07;2.55), (0.00:1.89) |
| 174 | 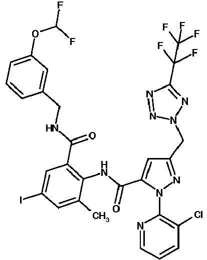 | 4,4 | 838 | (10.24;4.50), (8.82;1.14), (8.81;2.34), (8.79;1.09), (8.47;3.17), (8.47;3.39), (8.46;3.39), (8.46;3.26), (8.13;3.08), (8.13;3.09), (8.11;3.35), (8.11;3.12), (7.77;3.53), (7.76;3.75), (7.65;3.99), (7.64;3.71), (7.59;3.26), (7.58;3.19), (7.57;3.01), (7.56;2.98), (7.35;2.89), (7.34;0.36), (7.30;7.54), (7.29;1.91), (7.28;0.38), (7.27;3.83), (7.25;2.55), (7.17;5.78), (7.12;2.49), (7.10;2.09), (7.07;3.31), (7.02;1.89), (7.02;1.61), (7.00;1.59), (7.00;1.39), (6.98;2.89), (6.32;11.12), (4.34;4.33), (4.32;4.31), (3.43;0.38), (3.32;1061.81), (3.29;10.12), (2.68;0.36), (2.67;0.69), (2.67;0.90), (2.67;0.69), (2.66;0.37), (2.54;1.54), (2.52;4.05), (2.51;52.10), (2.51;96.77), (2.50;126.16), (2.50;86.91), (2.49;41.05), (2.33;0.62), (2.33;0.84), (2.32;0.61), (2.12;16.00), (2.11;0.52), (2.09;0.59), (2.07;1.37), (1.36;0.56), (1.35;0.55), (1.24;1.29), (0.00;4.77) |
| 175 | 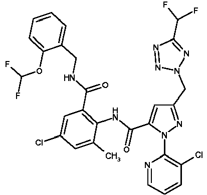 | 3,47 | 678 | (10.25;4.56), (8.75;1.20), (8.73;2.34), (8.72;1.11), (8.48;3.02), (8.48;3.14), (8.47;3.28), (8.47;3.12), (8.13;2.92), (8.13;2.91), (8.11;3.12), (8.11;2.96), (7.65;2.24), (7.60;3.10), (7.59;3.31), (7.58;3.04), (7.57;3.18), (7.55;0.34), (7.53;0.52), (7.53;0.78), (7.52;5.05), (7.48;3.28), (7.48;3.73), (7.45;0.58), (7.44;0.39), (7.40;4.17), (7.40;3.80), (7.39;2.89), (7.37;2.65), (7.37;0.60), (7.35;0.33), (7.35;0.75), (7.33;0.76), (7.32;0.45), (7.31;0.66), (7.29;2.72), (7.28;2.16), (7.28;9.93), (7.27;3.25), (7.25;1.87), (7.24;1.49), (7.23;0.33), (7.22;0.95), (7.20;0.56), (7.19;5.61), (7.15;2.81), (7.13;2.12), (7.08;1.71), (7.06;2.73), (7.06;2.61), (7.05;1.28), (7.04;1.25), (7.04;0.56), (7.00;2.66), (6.87;0.34), (6.24;0.74), (6.21;11.54), (5.75;1.97), (4.49;0.82), (4.47;0.81), (4.35;4.34), (4.34;4.39), (3.46;0.32), (3.32;1518.75), (3.19;0.37), (2.68;0.54), (2.67;1.01), (2.67;1.36), (2.67;1.04), (2.66;0.55), (2.54:2.05), (2.52:5.38), (2.51;76.61), (2.51;144.68), (2.50;190.54), (2.50;134.22), (2.49;65.69), (2.34;0.54), (2.33;1.01), (2.33;1.35), (2.32;1.10), (2.30;0.47), (2.18;0.91), (2.16;16.00), (2.14;0.65), (2.13;0.58), (2.11;0.44), (2.07;1.90), (2.06;0.36), (1.43;0.34), (1.36;4.07), (1.26;0.50), (1.24;1.42), (0.00:5.03) |
| 176 | 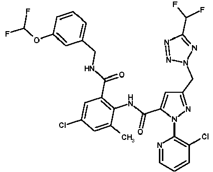 | 3,42 | 678 | |
| 177 | 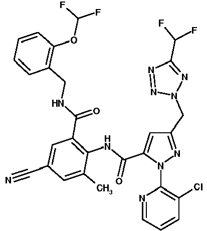 | 3,07 | 669 | (10.50;4.72), (8.85;1.16), (8.83;2.43), (8.82;1.20), (8.49;2.78), (8.48;3.08), (8.47;3.10), (8.47;3.06), (8.14;2.76), (8.13;2.86), (8.12;3.12), (8.11;2.97), (7.89;4.07), (7.84;4.28), (7.84;3.81), (7.65;2.63), (7.61;2.83), (7.59;2.85), (7.59;2.70), (7.57;2.69), (7.52;5.83), (7.39;3.24), (7.39;2.76), (7.34;2.21), (7.32;7.60), (7.30;1.40), (7.30;1.22), (7.28;2.36), (7.26;1.79), (7.26;1.58), (7.20;5.20), (7.16;3.16), (7.15;2.33), (7.11;1.80), (7.09;2.91), (7.07;1.31), (7.01;2.54), (6.23;12.10), (4.36;4.67), (4.34;4.65), (3.31;1047.78), (3.29;9.04), (3.25;0.57), (2.67;0.80), (2.67;1.07), (2.66;0.81), (2.54;1.69), (2.52;4.39), (2.51;60.12), (2.50;113.32), (2.50;149.27), (2.50;103.46), (2.49;49.46), (2.33;0.72), (2.33;1.03), (2.32;0.72), (2.32;0.37), (2.22;16.00), (2.07;5.72), (0.06;0.38), (0.00;2.75) |
| 178 | 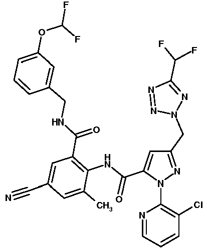 | 3,08 | 669 | (10.49;4.41), (8.93;1.17), (8.91;2.35), (8.90;1.17), (8.48;2.90), (8.48;3.07), (8.47;3.13), (8.47;3.02), (8.14;2.79), (8.13;2.86), (8.12;3.13), (8.11;2.96), (7.88;3.88), (7.82;4.24), (7.81;3.88), (7.65;2.67), (7.60;2.89), (7.58;2.82), (7.58;2.76), (7.56;2.65), (7.52;5.89), (7.39;2.98), (7.37;2.96), (7.32;5.54), (7.31;2.63), (7.29;4.32), (7.27;2.96), (7.19;6.20), (7.16;2.76), (7.14;2.17), (7.10;3.64), (7.04;2.08), (7.04;1.82), (7.02;1.77), (7.02;1.58), (7.00;3.09), (6.49;0.33), (6.23;11.97), (4.35;4.82), (4.34;4.79), (3.43;0.33), (3.40;0.46), (3.31;1110.96), (3.29;9.73), (2.71;1.42), (2.67;0.83), (2.67;1.11), (2.66;0.82), (2.54;1.74), (2.52;4.49), (2.51;64.13), (2.50;120.04), (2.50;156.74), (2.50;108.52), (2.49;51.87), (2.33;0.79), (2.33;1.07), (2.32;0.79), (2.29;0.40), (2.22;16.00), (2.13;0.38), (2.07;3.18), (1.24;0.32), (0.95;0.34), (0.00;2.19) |
| 179 | 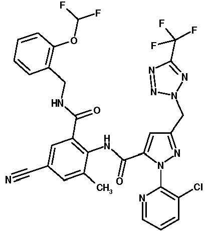 | 3,55 | 687 | (10.51;4.69), (8.84;2.80), (8.48;3.74), (8.47;3.73), (8.14;3.40), (8.12;3.46), (8.00;0.33), (7.89;4.94), (7.85;5.40), (7.61;2.58), (7.59;2.81), (7.59;2.65), (7.58;2.24), (7.38;2.93), (7.34;7.09), (7.32;3.79), (7.29;2.21), (7.29;2.19), (7.27;4.56), (7.25;3.13), (7.19;5.40), (7.16;6.34), (7.14;4.52), (7.10;2.61), (7.08;4.15), (7.06;1.93), (7.01;2.59), (6.49;0.61), (6.30;11.20), (6.27;0.56), (5.75;0.64), (4.36;6.74), (4.35;6.56), (3.67;0.33), (3.65;0.44), (3.58;0.46), (3.47;0.86), (3.37;3.98), (3.31;4509.45), (3.29;37.26), (3.26;4.09), (3.22;1.17), (3.18;0.69), (3.14;0.47), (3.10;0.35), (3.08;0.40), (2.70;0.50), (2.70;0.44), (2.67;2.87), (2.67;3.82), (2.67;2.90), (2.54;5.46), (2.52;15.07), (2.51;230.65), (2.51;435.73), (2.50;573.29), (2.50;398.07), (2.49;191.42), (2.42;1.08), (2.41;0.96), (2.38;0.74), (2.37;0.67), (2.33;3.09), (2.33;3.95), (2.32;2.98), (2.29;0.62), (2.26;0.80), (2.22;16.00), (2.16;0.65), (2.13;0.67), (2.13;0.58), (2.11;0.38), (2.08;0.42), (2.07;13.23), (2.02;0.40), (1.99;0.69), (1.91;0.71), (1.85;0.33), (1.40;0.39), (1.24;1.04), (1.11;0.84), (0.89;0.33), (0.06;0.87), (0.00;5.48) |
| 180 | 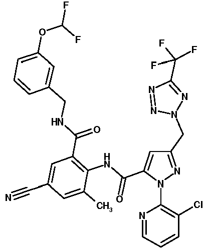 | 3,54 | 687 | (10.51;4.51), (8.94;1.14), (8.92;2.38), (8.91;1.13), (8.48;2.95), (8.48;3.08), (8.47;3.19), (8.47;3.01), (8.14;2.93), (8.14;2.86), (8.12;3.25), (8.12;2.99), (7.89;3.56), (7.89;3.86), (7.82;4.12), (7.82;3.72), (7.60;3.00), (7.59;2.92), (7.58;2.84), (7.57;2.77), (7.37;2.70), (7.35;5.35), (7.30;1.83), (7.28;3.91), (7.27;2.68), (7.18;5.33), (7.15;2.55), (7.13;2.00), (7.10;3.26), (7.04;1.85), (7.03;1.61), (7.02;1.58), (7.01;1.42), (7.00;2.70), (6.31;12.33), (4.35;4.48), (4.34;4.43), (3.32;360.31), (2.67;0.40), (2.54;0.63), (2.52;1.71), (2.51;23.35), (2.51;42.86), (2.50;55.03), (2.50;37.85), (2.49;17.92), (2.33;0.37), (2.22;16.00), (2.07;1.33), (0.00;0.89) |
| 181 | 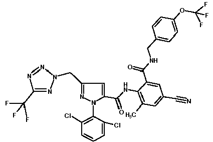 | 4,46 | 738 | (10.55;0.35), (10.53;0.44), (10.51;0.57), (9.00;0.36), (8.92;0.83), (8.57;0.49), (8.03;0.48), (8.01;0.93), (7.93;0.87), (7.92;0.89), (7.91;0.86), (7.90;0.53), (7.87;2.16), (7.83;2.75), (7.64;0.56), (7.60;3.19), (7.58;6.42), (7.56;1.24), (7.52;2.59), (7.50;2.44), (7.50;1.95), (7.48;1.58), (7.46;1.26), (7.44;0.98), (7.42;1.44), (7.39;5.15), (7.37;5.84), (7.35;0.86), (7.33;0.55), (7.30;0.36), (7.24;0.33), (7.22;1.08), (7.20;4.33), (7.18;3.28), (7.15;0.80), (7.13;0.69), (7.08;0.63), (6.87;1.20), (6.61;0.80), (6.32;5.34), (6.28;0.37), (6.28;0.39), (6.11;0.96), (4.82;0.92), (4.40;0.82), (4.38;0.79), (4.35;4.02), (4.33;4.02), (3.98;0.34), (3.93;0.39), (3.77;0.36), (3.74;0.38), (3.67;0.41), (3.67;0.42), (3.63;0.58), (3.62;1.37), (3.60;2.70), (3.60;1.39), (3.59;1.48), (3.57;0.65), (3.56;0.62), (3.53;0.70), (3.45;1.34), (3.31;3653.26), (3.29;33.67), (3.22;0.74), (3.20;0.51), (3.18;0.38), (3.16;0.36), (3.15;0.37), (2.89;0.34), (2.67;2.32), (2.67;3.11), (2.67;2.32), (2.63;0.59), (2.62;0.57), (2.60;0.77), (2.54;4.24), (2.52;14.34), (2.51;183.68), (2.51;338.07), (2.50;437.89), (2.50;302.49), (2.49;144.16), (2.41;0.42), (2.34;1.24), (2.33;2.28), (2.33;3.02), (2.32;2.16), (2.29;2.43), (2.27;0.34), (2.20;8.37), (2.18;3.42), (2.14;3.07), (2.07;4.23), (1.78;1.01), (1.77;1.07), (1.76;2.93), (1.75;1.06), (1.74;0.93), (1.36;16.00), (1.24;0.74), (1.17;0.32), (0.00;4.80) |
| 182 | 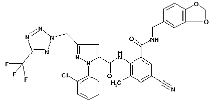 | 3,64 | 664 | (10.41;0.64), (8.80;0.89), (7.86;2.84), (7.79;3.48), (7.78;2.99), (7.56;1.53), (7.54;1.72), (7.54;2.93), (7.53;0.52), (7.52;0.52), (7.49;0.79), (7.48;1.37), (7.48;1.44), (7.47;2.02), (7.46;1.66), (7.45;2.31), (7.44;6.03), (7.43;3.11), (7.39;0.46), (7.39;0.35), (7.30;2.82), (7.24;0.47), (6.82;0.92), (6.81;4.22), (6.75;1.49), (6.74;1.12), (6.74;9.78), (6.73;9.12), (6.28;8.95), (6.07;0.58), (6.06;1.44), (5.98;0.61), (5.97;0.40), (5.96;3.29), (5.95;16.00), (4.23;3.63), (4.22;3.54), (3.32;310.43), (3.30;2.12), (2.52;1.01), (2.51;13.56), (2.51;25.17), (2.50;32.71), (2.50;22.64), (2.49;10.76), (2.20;11.83), (1.40;0.64) |
| 183 | 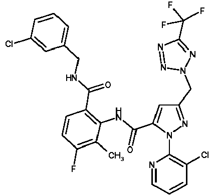 | 3,82 | 648 | (10.44;2.91), (8.81;1.50), (8.80;3.09), (8.78;1.53), (8.48;4.17), (8.48;4.49), (8.47;4.50), (8.47;4.41), (8.17;0.35), (8.16;0.39), (8.14;4.24), (8.14;4.27), (8.12;4.74), (8.12;4.47), (8.10;0.36), (7.60;4.35), (7.59;4.31), (7.58;4.98), (7.57;4.01), (7.47;1.24), (7.46;0.86), (7.46;1.92), (7.46;2.31), (7.45;2.30), (7.43;2.98), (7.43;3.01), (7.41;2.55), (7.37;0.52), (7.33;11.18), (7.32;5.83), (7.32;5.97), (7.30;0.43), (7.29;0.34), (7.27;0.63), (7.26;0.93), (7.25;5.65), (7.25;3.69), (7.24;9.27), (7.24;10.55), (7.23;2.46), (7.21;6.85), (7.21;3.51), (7.19;3.12), (6.30;16.00), (6.28;0.36), (6.08;0.42), (4.35;6.57), (4.34;6.62), (4.05;3.68), (3.31;770.67), (3.29;12.21), (2.89;0.88), (2.67;0.44), (2.67;0.56), (2.67;0.43), (2.54;0.69), (2.51;32.36), (2.51;60.84), (2.50;79.88), (2.50;55.86), (2.49;27.08), (2.33;0.39), (2.33;0.56), (2.32;0.44), (2.07;0.73), (2.05;13.74), (2.05;14.00), (2.02;0.42), (1.36;0.41), (0.00;2.50) |
| 184 | 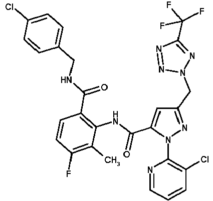 | 3,8 | 648 | (8.80;0.38), (8.49;1.87), (8.48;1.97), (8.48;2.07), (8.47;1.95), (8.15;1.79), (8.15;1.78), (8.13;2.01), (8.13;1.89), (7.61;1.90), (7.59;1.85), (7.59;1.81), (7.57;1.77), (7.44;0.86), (7.43;1.06), (7.42;1.13), (7.40;1.08), (7.39;0.66), (7.38;6.45), (7.37;0.42), (7.32;2.18), (7.28;0.70), (7.28;0.69), (7.28;0.77), (7.26;16.00), (7.24;0.60), (7.22;0.84), (7.19;1.28), (7.17;0.65), (6.30;7.02), (4.33;3.22), (4.32;3.23), (3.79;2.03), (3.31;507.40), (3.29;11.12), (2.87;0.34), (2.67;0.39), (2.67;0.49), (2.66;0.38), (2.54;0.63), (2.51;29.50), (2.50;54.66), (2.50;71.01), (2.50;48.69), (2.49;22.92), (2.33;0.36), (2.33;0.48), (2.32;0.36), (2.07;0.39), (2.04;6.16), (2.04;6.16), (0.00;0.48) |
| 185 | 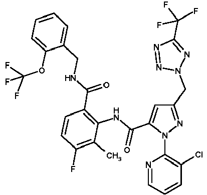 | 4,04 | 698 | (10.43;1.72), (8.77;2.10), (8.76;1.23), (8.50;4.30), (8.49;4.69), (8.48;4.68), (8.48;4.60), (8.15;4.11), (8.15;4.13), (8.13;4.66), (8.13;4.36), (7.62;4.44), (7.61;4.38), (7.60;4.14), (7.59;4.11), (7.49;0.39), (7.48;2.05), (7.46;2.51), (7.46;2.97), (7.44;2.32), (7.38;2.92), (7.37;4.17), (7.34;3.00), (7.34;3.13), (7.32;13.24), (7.31;1.15), (7.30;1.15), (7.27;0.53), (7.25;2.32), (7.23;3.47), (7.23;5.74), (7.22;3.17), (7.21;3.01), (7.20;1.53), (7.19;1.50), (6.32;0.36), (6.30;16.00), (6.08;0.44), (4.42;6.53), (4.40;6.58), (3.96;0.35), (3.60;0.33), (3.54;0.43), (3.51;0.53), (3.49;0.60), (3.48;0.63), (3.48;0.68), (3.45;0.89), (3.33;2381.80), (3.30;34.75), (3.27;1.97), (3.26;1.03), (3.22;0.41), (3.21;0.38), (2.93;0.40), (2.69;0.57), (2.68;0.86), (2.68;1.26), (2.67;0.87), (2.61;0.32), (2.57;0.56), (2.55;1.55), (2.52;67.90), (2.51;127.34), (2.51;166.95), (2.51;115.73), (2.50;55.73), (2.34;0.79), (2.34;1.10), (2.33;0.83), (2.33;0.44), (2.24;0.33), (2.07;13.87), (2.06;14.10), (1.37;0.83), (0.01;0.45) |
| 186 | 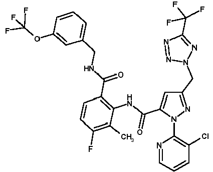 | 4,07 | 698 | (10.43;3.94), (8.85;1.45), (8.84;2.89), (8.82;1.43), (8.49;4.31), (8.48;4.64), (8.47;4.64), (8.47;4.49), (8.15;4.21), (8.14;4.15), (8.13;4.74), (8.12;4.34), (7.65;0.33), (7.60;4.42), (7.59;4.40), (7.58;4.25), (7.57;4.24), (7.56;0.32), (7.50;0.34), (7.49;0.46), (7.48;0.43), (7.45;2.08), (7.43;2.38), (7.43;2.59), (7.41;2.31), (7.37;2.25), (7.35;5.46), (7.33;5.10), (7.32;9.68), (7.28;4.15), (7.27;3.29), (7.25;4.43), (7.24;2.94), (7.22;4.32), (7.20;2.69), (7.19;2.61), (7.17;1.93), (6.30;16.00), (6.07;0.37), (4.53;0.33), (4.40;6.20), (4.38;6.17), (4.08;0.47), (3.54;0.32), (3.53;0.37), (3.49;0.49), (3.45;0.67), (3.32;1851.72), (3.30;24.41), (3.20;0.45), (2.89;0.36), (2.68;0.73), (2.67;0.97), (2.67;0.70), (2.66;0.42), (2.54;1.23), (2.51;55.48), (2.51;103.58), (2.50;135.44), (2.50;93.24), (2.49;44.11), (2.34;0.38), (2.33;0.67), (2.33;0.91), (2.32;0.65), (2.07;1.61), (2.05;13.66), (2.05;13.57), (0.00;5.57) |
| 187 | 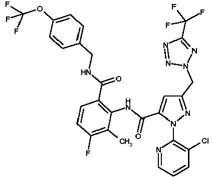 | 4,05 | 698 | (10.44;2.93), (8.81;1.44), (8.80;2.84), (8.78;1.38), (8.49;0.35), (8.48;4.37), (8.48;4.54), (8.47;4.64), (8.46;4.54), (8.15;4.17), (8.15;4.27), (8.13;4.69), (8.13;4.41), (7.61;4.56), (7.59;4.61), (7.59;4.33), (7.57;4.35), (7.45;2.32), (7.43;2.63), (7.43;2.76), (7.41;2.36), (7.36;7.30), (7.35;10.72), (7.34;9.16), (7.31;0.43), (7.29;0.41), (7.23;2.88), (7.21;8.87), (7.19;5.29), (6.30;16.00), (6.08;0.43), (4.37;6.13), (4.36;6.17), (4.06;0.82), (3.48;0.42), (3.43;0.77), (3.32;1624.76), (3.30;21.25), (3.25;0.84), (3.21;0.43), (2.89;0.34), (2.67;0.65), (2.67;0.83), (2.67;0.60), (2.66;0.39), (2.64;0.74), (2.55;1.24), (2.54;1.08), (2.51;46.84), (2.51;87.99), (2.50;115.41), (2.50;79.97), (2.49;38.33), (2.33;0.59), (2.33;0.80), (2.32;0.63), (2.07;1.69), (2.05;13.64), (2.05;13.74), (2.02;0.38), (0.00;0.41) |
| 188 | 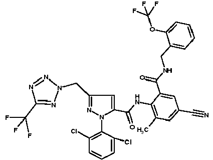 | 4,13 | 738 | (10.51;4.63), (10.49;0.95), (8.91;1.29), (8.90;2.63), (8.88;1.26), (7.89;4.14), (7.89;4.58), (7.85;4.75), (7.84;3.91), (7.59;4.05), (7.59;4.69), (7.57;10.14), (7.55;1.76), (7.52;4.27), (7.51;1.24), (7.51;3.11), (7.50;2.37), (7.49;0.70), (7.48;1.73), (7.47;0.43), (7.45;2.16), (7.45;2.15), (7.43;2.61), (7.41;7.18), (7.40;0.38), (7.39;0.89), (7.38;0.94), (7.37;2.14), (7.36;2.13), (7.35;1.73), (7.35;2.62), (7.35;2.60), (7.34;2.42), (7.33;3.07), (7.33;2.33), (7.32;0.97), (7.31;1.09), (7.26;0.35), (7.26;0.35), (7.24;1.98), (7.24;1.81), (7.22;2.37), (7.20;1.07), (7.20;1.01), (6.33;12.37), (6.12;1.74), (4.38;5.08), (4.36;5.14), (3.31;211.72), (3.28;2.76), (2.54;0.40), (2.51;15.83), (2.50;29.33), (2.50;38.00), (2.50;26.36), (2.49;12.63), (2.29;0.41), (2.22;16.00), (2.14;0.42), (2.07;2.95), (1.99;0.46), (0.01;0.36), (0.00;7.18) |
| 189 | 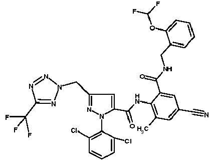 | 3,75 | 721 | (10.52;4.77), (10.50;1.01), (8.85;1.33), (8.83;2.74), (8.82;1.32), (8.73;0.87), (8.00;0.57), (8.00;0.52), (7.98;0.50), (7.98;0.50), (7.88;3.98), (7.88;4.52), (7.84;4.72), (7.84;3.95), (7.60;4.32), (7.60;4.99), (7.58;9.93), (7.56;1.78), (7.54;0.55), (7.53;4.20), (7.51;1.09), (7.51;3.15), (7.50;2.60), (7.50;0.70), (7.49;0.67), (7.49;1.84), (7.47;0.43), (7.47;0.66), (7.46;0.50), (7.45;0.51), (7.44;0.58), (7.44;0.59), (7.41;7.42), (7.39;0.79), (7.39;2.83), (7.36;2.37), (7.36;2.46), (7.35;2.07), (7.34;2.82), (7.34;2.81), (7.32;0.91), (7.31;1.20), (7.30;1.79), (7.29;3.32), (7.27;1.80), (7.27;1.49), (7.26;0.70), (7.25;0.71), (7.24;0.32), (7.23;0.32), (7.22;0.69), (7.21;1.23), (7.20;5.71), (7.17;3.00), (7.15;2.10), (7.11;0.38), (7.10;0.74), (7.09;1.99), (7.09;2.00), (7.07;2.86), (7.07;2.76), (7.05;1.40), (7.05;1.35), (7.02;0.61), (7.02;2.67), (6.33;12.48), (6.11;1.72), (4.83;1.72), (4.34;5.02), (4.33;5.02), (3.31;487.84), (2.67;0.32), (2.67;0.44), (2.67;0.34), (2.54;0.67), (2.51;24.93), (2.50;46.81), (2.50;61.35), (2.50;42.89), (2.49;20.87), (2.33;0.34), (2.33;0.46), (2.32;0.40), (2.29;0.51), (2.21;16.00), (2.14;0.51), (2.07;1.18), (0.01;0.48), (0.00;10.59), (-0.01;0.47) |
| 190 | 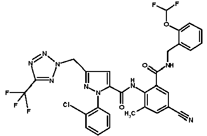 | 3,88 | 686 | (10.44;4.88), (8.86;1.20), (8.85;2.39), (8.83;1.15), (8.73;1.19), (8.00;0.67), (8.00;0.69), (7.98;0.71), (7.98;0.70), (7.89;4.06), (7.88;4.62), (7.85;4.80), (7.84;3.81), (7.58;0.45), (7.57;0.53), (7.57;1.71), (7.56;2.18), (7.55;2.37), (7.55;3.82), (7.53;1.02), (7.53;0.84), (7.50;1.07), (7.49;1.41), (7.49;2.00), (7.48;2.53), (7.47;2.16), (7.47;2.39), (7.46;3.02), (7.45;4.70), (7.44;5.85), (7.43;4.75), (7.43;4.08), (7.42;0.76), (7.42;0.48), (7.41;0.39), (7.39;1.43), (7.39;1.31), (7.39;1.22), (7.38;2.72), (7.37;1.37), (7.36;2.45), (7.36;2.44), (7.34;3.12), (7.31;7.02), (7.30;2.20), (7.29;3.72), (7.27;2.25), (7.27;1.65), (7.26;1.54), (7.26;1.30), (7.25;1.13), (7.24;0.48), (7.23;0.48), (7.22;0.93), (7.21;2.09), (7.20;1.88), (7.19;5.04), (7.17;2.89), (7.15;2.12), (7.10;0.80), (7.09;0.44), (7.07;2.14), (7.06;2.05), (7.05;2.91), (7.05;2.78), (7.03;1.30), (7.03;1.23), (7.02;0.98), (7.01;0.65), (7.01;2.40), (6.28;11.93), (6.06;2.12), (4.83;2.52), (4.35;4.44), (4.34;4.53), (3.31;223.22), (2.67;0.33), (2.54;0.42), (2.51;19.56), (2.50;36.61), (2.50;47.82), (2.50;33.54), (2.49;16.35), (2.33;0.36), (2.22;16.00), (2.07;0.43), (0.01;0.45), (0.00;9.80), (-0.01;0.44) |
| 191 | 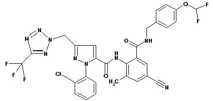 | 3,81 | 686 | (10.43;3.72), (8.91;1.20), (8.90;2.28), (8.88;1.17), (8.50;1.67), (7.87;4.42), (7.86;0.46), (7.85;2.30), (7.84;0.88), (7.83;0.92), (7.83;2.49), (7.82;0.65), (7.81;4.88), (7.80;4.40), (7.56;2.16), (7.55;2.82), (7.55;4.11), (7.53;0.77), (7.53;0.87), (7.50;2.05), (7.49;1.77), (7.48;1.96), (7.47;2.54), (7.47;1.92), (7.45;2.51), (7.44;4.89), (7.44;7.39), (7.43;5.11), (7.42;4.30), (7.41;0.56), (7.40;0.52), (7.39;2.05), (7.39;1.58), (7.37;1.97), (7.37;2.60), (7.36;0.88), (7.36;1.01), (7.35;2.98), (7.33;5.92), (7.32;6.29), (7.31;3.22), (7.31;3.91), (7.30;6.55), (7.28;1.28), (7.26;2.12), (7.24;1.95), (7.19;1.95), (7.17;1.61), (7.16;6.46), (7.14;1.82), (7.13;1.04), (7.05;1.67), (7.03;6.61), (7.03;3.28), (7.01;5.50), (7.00;1.43), (6.99;0.78), (6.98;2.79), (6.29;12.16), (6.07;2.44), (4.76;2.95), (4.31;4.68), (4.30;4.66), (3.31;418.68), (3.28;4.06), (2.67;0.38), (2.54;0.52), (2.51;22.38), (2.50;41.73), (2.50;54.39), (2.50;37.79), (2.49;18.23), (2.33;0.40), (2.22;16.00), (2.07;0.59), (0.01;0.57), (0.00;12.22), (-0.01:0.52) |
| 192 | 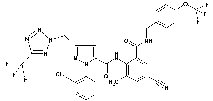 | 3,98 | 703 | (10.44;5.03), (8.94;1.22), (8.93;2.33), (8.92;1.14), (7.88;4.14), (7.88;4.45), (7.83;4.75), (7.83;4.12), (7.56;1.91), (7.56;2.28), (7.54;3.06), (7.54;3.24), (7.53;0.64), (7.53;0.71), (7.49;1.20), (7.49;1.42), (7.48;0.68), (7.48;2.37), (7.47;2.59), (7.46;1.09), (7.46;2.01), (7.45;1.97), (7.45;2.85), (7.44;1.18), (7.43;3.31), (7.43;3.49), (7.42;5.06), (7.41;4.77), (7.40;6.09), (7.38;3.78), (7.38;6.83), (7.34;6.07), (7.28;1.07), (7.21;1.12), (7.19;5.18), (7.17;3.84), (6.29;12.12), (6.07;1.97), (4.35;4.51), (4.34;4.52), (3.31;239.27), (3.29;4.24), (2.54;0.33), (2.51;13.10), (2.50;24.30), (2.50;31.57), (2.50;21.71), (2.49;10.33), (2.22;16.00), (1.99;0.34), (0.00;6.29) |
| 193 | 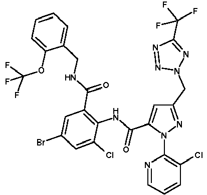 | 4,3 | 778/7 80 | (10.80;0.32), (10.53;0.36), (10.51;0.34), (10.47;0.39), (10.46;0.39), (10.42;0.35), (10.40;0.39), (8.99;0.41), (8.96;0.41), (8.92;0.88), (8.91;0.91), (8.88;0.68), (8.47;4.46), (8.47;4.97), (8.46;4.96), (8.46;4.75), (8.12;4.46), (8.12;4.35), (8.10;4.87), (8.10;4.51), (8.00;0.49), (7.94;3.52), (7.90;0.35), (7.85;0.62), (7.74;0.80), (7.74;0.63), (7.68;7.24), (7.67;7.20), (7.65;0.60), (7.63;0.65), (7.62;0.50), (7.60;4.26), (7.59;4.27), (7.58;4.14), (7.57;4.13), (7.55;0.37), (7.43;1.20), (7.42;1.24), (7.41;1.51), (7.36;6.36), (7.35;5.97), (7.33;4.27), (7.33;4.16), (7.31;8.71), (7.30;1.52), (7.21;2.95), (7.21;2.75), (7.19;3.55), (7.18;1.62), (7.17;1.85), (6.65;0.51), (6.30;16.00), (6.07;0.40), (4.89;0.34), (4.43;0.72), (4.41;0.75), (4.37;8.16), (4.36;7.92), (4.31;0.38), (3.89;2.44), (3.83;0.33), (3.83;0.33), (3.81;0.43), (3.75;0.36), (3.71;0.33), (3.70;0.37), (3.66;0.39), (3.64;0.42), (3.63;0.45), (3.62;0.50), (3.58;0.51), (3.58;0.53), (3.55;0.58), (3.53;0.65), (3.49;0.90), (3.48;0.91), (3.45;1.16), (3.39;2.57), (3.31;3296.79), (3.29;52.61), (3.21;0.82), (3.14;0.34), (2.67;1.87), (2.67;2.37), (2.66;1.92), (2.64;0.44), (2.62;0.43), (2.59;0.68), (2.54;3.60), (2.51;143.03), (2.50;269.20), (2.50;353.73), (2.50;248.45), (2.49;121.24), (2.41;0.38), (2.40;0.38), (2.33;1.91), (2.33;2.43), (2.32;1.70), (2.29;0.39), (2.18;0.43), (2.07;0.93), (1.36;2.39), (1.24;0.65), (0.01;1.80), (0.00;38.39), (-0.01;1.85), (-0.02;0.40) |
| 194 | 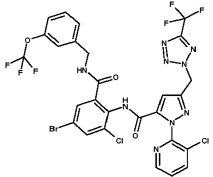 | 4,32 | 778/7 80 | (14.72;0.91), (10.48;6.04), (8.95;1.74), (8.93;3.08), (8.92;2.13), (8.59;1.09), (8.48;3.69), (8.47;4.78), (8.47;4.28), (8.46;4.25), (8.14;1.08), (8.13;4.19), (8.12;4.23), (8.11;4.56), (8.10;4.41), (8.00;0.91), (7.95;6.59), (7.95;6.77), (7.82;1.00), (7.65;7.27), (7.64;7.10), (7.62;1.01), (7.59;4.43), (7.58;4.26), (7.57;4.02), (7.56;3.64), (7.50;1.11), (7.48;1.50), (7.40;1.01), (7.38;6.32), (7.35;2.92), (7.33;5.93), (7.31;4.42), (7.26;5.74), (7.19;2.50), (7.16;2.01), (6.30;16.00), (4.85;1.48), (4.40;0.93), (4.36;5.88), (4.35;5.86), (4.21;0.93), (4.15;1.01), (3.92;1.01), (3.83;0.97), (3.82;0.92), (3.80;0.94), (3.74;1.02), (3.74;1.08), (3.73;1.01), (3.72;0.95), (3.71;1.39), (3.70;1.16), (3.67;1.02), (3.65;1.25), (3.64;1.21), (3.64;1.10), (3.60;1.53), (3.59;1.53), (3.58;1.53), (3.57;1.49), (3.55;1.65), (3.52;1.88), (3.48;2.35), (3.43;3.94), (3.41;4.38), (3.38;7.38), (3.31;8386.29), (3.24;4.62), (3.22;2.59), (3.21;2.32), (3.19;1.28), (3.18;1.31), (3.18;1.03), (3.14;0.96), (2.68;3.74), (2.67;5.98), (2.67;8.51), (2.66;6.27), (2.64;1.34), (2.61;1.59), (2.56;3.88), (2.54;10.89), (2.51;480.04), (2.50;901.42), (2.50;1184.25), (2.50;821.67), (2.49;395.34), (2.44;2.27), (2.42;1.83), (2.33;5.96), (2.33;7.88), (2.32;6.03), (2.07;2.49), (1.92;1.12), (1.36;1.54), (1.24;2.75), (1.19;0.92), (0.15;2.50), (0.01;28.03), (0.00;671.35), (-0.01;30.15), (-0.15;2.60), (-3.53;1.03) |
| 195 | 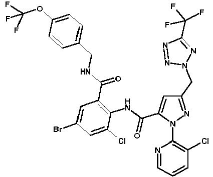 | 4,31 | 778/7 80 | (10.49;4.92), (10.21;0.33), (8.92;1.56), (8.91;3.19), (8.89;1.50), (8.47;4.07), (8.47;4.27), (8.46;4.21), (8.46;4.05), (8.14;4.08), (8.13;4.15), (8.12;4.58), (8.11;4.21), (8.10;0.32), (8.00;0.52), (7.95;6.82), (7.94;6.81), (7.91;0.45), (7.86;0.41), (7.73;0.51), (7.72;0.50), (7.67;0.96), (7.66;7.37), (7.65;7.01), (7.60;4.52), (7.59;3.93), (7.58;4.11), (7.57;3.83), (7.48;0.39), (7.46;1.09), (7.44;0.75), (7.41;6.65), (7.37;0.88), (7.35;7.28), (7.33;8.59), (7.21;6.07), (7.19;4.50), (7.15;0.47), (6.66;0.42), (6.30;16.00), (6.08;0.43), (4.82;0.43), (4.39;0.44), (4.37;0.48), (4.34;5.97), (4.32;6.04), (4.28;0.57), (4.26;0.51), (4.08;0.64), (3.69;0.32), (3.66;0.35), (3.62;0.42), (3.58;0.44), (3.56;0.47), (3.55;0.55), (3.52;0.62), (3.47;0.84), (3.44;1.21), (3.39;2.96), (3.31;2668.97), (3.26;2.16), (2.68;0.82), (2.67;1.30), (2.67;1.79), (2.67;1.29), (2.54;2.37), (2.51;105.94), (2.51;198.43), (2.50;260.02), (2.50;180.91), (2.49;87.30), (2.42;0.39), (2.33;1.24), (2.33;1.72), (2.32;1.27), (2.29;0.35), (2.18;0.39), (2.07;1.34), (1.67;0.34), (1.40;1.03), (1.36;0.55), (1.24;0.40), (0.00;11.81) |
| 196 | 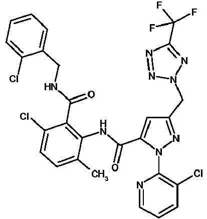 | 3,64 | 664 | (10.35;0.33), (8.79;1.03), (8.78;2.16), (8.77;1.07), (8.48;2.76), (8.47;2.91), (8.46;3.06), (8.46;2.98), (8.15;2.63), (8.15;2.74), (8.13;2.89), (8.13;2.87), (7.60;2.79), (7.59;2.84), (7.58;2.75), (7.57;2.77), (7.54;0.33), (7.40;2.93), (7.37;5.93), (7.36;2.57), (7.36;2.52), (7.35;1.09), (7.34;3.45), (7.34;3.93), (7.34;4.76), (7.32;2.52), (7.32;2.71), (7.30;8.47), (7.26;0.43), (7.25;0.46), (7.14;1.06), (7.13;1.05), (7.12;1.90), (7.10;1.08), (7.10;1.05), (6.97;1.37), (6.96;2.21), (6.96;2.20), (6.94;1.01), (6.30;11.52), (6.07;0.38), (5.75;0.37), (4.57;0.50), (4.55;0.44), (4.39;3.52), (4.38;3.55), (3.79;0.98), (3.67;0.41), (3.60;0.33), (3.57;0.39), (3.56;0.35), (3.53;0.41), (3.31;1454.70), (3.29;20.48), (3.20;0.61), (3.19;0.53), (3.18;0.40), (2.74;2.26), (2.67;1.22), (2.67;1.53), (2.66;1.20), (2.62;0.35), (2.54;2.20), (2.51;84.58), (2.50;156.53), (2.50;203.51), (2.50;140.67), (2.49;67.02), (2.33;1.11), (2.33;1.33), (2.32;1.03), (2.12;16.00), (2.09;0.89), (2.07;1.04), (1.40;0.39), (1.36;0.47), (1.24;0.50), (0.00;6.13) |
| 197 | 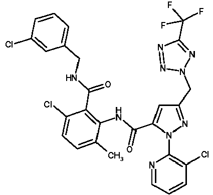 | 3,74 | 664 | (10.31;4.02), (8.78;1.12), (8.76;2.28), (8.75;1.09), (8.47;2.84), (8.47;3.08), (8.46;3.13), (8.46;3.03), (8.15;2.77), (8.15;2.81), (8.13;3.17), (8.13;2.95), (7.59;2.82), (7.58;2.89), (7.57;2.87), (7.56;2.70), (7.46;0.34), (7.37;4.33), (7.37;3.80), (7.35;5.50), (7.32;4.22), (7.30;2.00), (7.28;0.36), (7.26;8.00), (7.20;1.68), (7.18;2.69), (7.17;1.39), (7.16;2.21), (7.15;2.63), (7.13;3.27), (7.11;2.88), (7.09;1.05), (6.30;11.66), (4.33;4.18), (4.31;4.14), (3.40;0.76), (3.31;1082.29), (2.67;0.74), (2.67;0.93), (2.66;0.75), (2.54;1.27), (2.51;52.17), (2.50;97.23), (2.50;127.03), (2.50;88.46), (2.49;42.64), (2.33;0.68), (2.33;0.86), (2.32;0.66), (2.32;0.33), (2.10;16.00), (2.07;0.67), (1.36;1.09), (0.01;0.85), (0.00;18.04), (-0.01;0.70) |
| 198 | 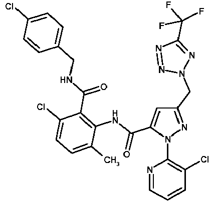 | 3,68 | 664 | (8.76;1.05), (8.75;2.13), (8.73;1.03), (8.48;2.78), (8.48;2.96), (8.47;3.01), (8.47;2.88), (8.15;2.69), (8.15;2.65), (8.13;3.01), (8.13;2.73), (7.60;2.79), (7.59;2.76), (7.58;2.65), (7.57;2.64), (7.39;3.23), (7.37;2.80), (7.35;5.71), (7.32;4.17), (7.29;2.29), (7.28;7.93), (7.25;3.82), (7.23;6.16), (7.21;0.55), (7.18;7.31), (7.17;2.18), (7.16;1.74), (7.15;4.12), (6.29;11.14), (4.31;3.56), (4.29;3.57), (3.81;1.09), (3.31;728.11), (3.29;11.93), (3.26;1.01), (2.67;0.46), (2.67;0.63), (2.66;0.49), (2.54;0.87), (2.51;36.13), (2.50;67.17), (2.50;87.49), (2.50;60.44), (2.49;28.92), (2.33;0.43), (2.33;0.57), (2.32;0.42), (2.10;16.00), (2.07;0.34), (0.00;5.14) |
| 199 | 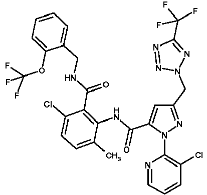 | 3,92 | 714 | (10.34;3.18), (8.81;1.13), (8.79;2.25), (8.78;1.11), (8.47;2.78), (8.47;3.03), (8.46;3.08), (8.46;3.01), (8.15;2.74), (8.14;2.65), (8.13;3.05), (8.12;2.74), (7.60;2.86), (7.59;2.89), (7.58;2.73), (7.57;2.71), (7.39;2.82), (7.38;2.44), (7.37;5.85), (7.36;2.30), (7.34;4.37), (7.31;2.02), (7.28;8.00), (7.27;1.09), (7.27;1.22), (7.25;2.64), (7.23;1.70), (7.23;1.68), (7.21;1.85), (7.20;0.75), (7.03;1.32), (7.03;1.27), (7.01;2.00), (6.99;1.02), (6.32;0.35), (6.30;11.41), (4.38;3.52), (4.37;3.56), (3.51;0.32), (3.48;0.42), (3.45;0.54), (3.40;0.85), (3.39;1.10), (3.31;1347.68), (3.29;18.51), (3.26;0.98), (3.24;0.65), (2.67;0.68), (2.67;0.92), (2.67;0.69), (2.66;0.43), (2.57;0.41), (2.54;1.35), (2.51;52.84), (2.50;98.44), (2.50;128.31), (2.50;89.16), (2.49;42.93), (2.33;0.64), (2.33;0.82), (2.32;0.58), (2.11;16.00), (2.07;0.39), (1.36;0.96), (1.09;0.33), (0.00;4.53) |
| 200 | 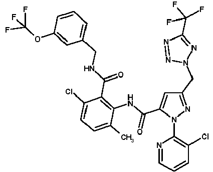 | 3,94 | 714 | (10.32;2.81), (8.83;1.23), (8.81;2.24), (8.80;1.10), (8.59;0.43), (8.48;3.11), (8.47;3.07), (8.47;3.32), (8.46;2.95), (8.16;2.94), (8.15;2.79), (8.13;3.17), (8.13;2.99), (7.81;0.38), (7.65;0.34), (7.64;0.34), (7.62;0.40), (7.60;2.94), (7.59;3.02), (7.58;2.93), (7.56;2.75), (7.54;0.41), (7.50;0.46), (7.48;0.41), (7.44;0.34), (7.43;0.36), (7.42;0.58), (7.40;0.42), (7.38;2.79), (7.36;5.75), (7.32;5.21), (7.31;3.10), (7.30;2.75), (7.27;9.32), (7.25;2.82), (7.23;2.32), (7.21;3.65), (7.19;1.60), (7.10;1.65), (7.09;1.20), (6.30;11.95), (4.85;0.65), (4.54;0.39), (4.37;3.90), (4.36;4.05), (4.29;0.40), (4.03;0.33), (4.00;0.42), (3.99;0.45), (3.90;0.33), (3.81;0.33), (3.76;0.38), (3.73;0.46), (3.69;0.43), (3.68;0.45), (3.64;0.52), (3.63;0.47), (3.59;0.56), (3.55;0.61), (3.54;0.71), (3.49;0.88), (3.46;1.17), (3.45;1.31), (3.43;1.62), (3.31;3807.72), (3.29;53.03), (3.23;1.13), (3.18;0.45), (2.67;1.77), (2.67;2.40), (2.66;1.73), (2.63;0.42), (2.54;3.11), (2.51;143.81), (2.50;268.54), (2.50;350.66), (2.50;242.25), (2.49;115.84), (2.39;0.34), (2.34;0.39), (2.33;1.85), (2.33;2.43), (2.32;1.84), (2.10;16.00), (2.07;1.55), (1.36;0.45), (1.24;0.66), (0.00;26.63), (-0.01;1.17) |
| 201 | 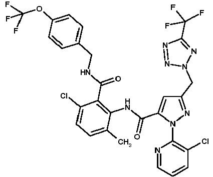 | 3,93 | 714 | (10.36;0.36), (8.79;1.10), (8.78;2.25), (8.76;1.03), (8.47;2.76), (8.47;3.04), (8.46;3.14), (8.45;2.81), (8.15;2.66), (8.15;2.67), (8.13;3.05), (8.13;2.78), (7.59;2.78), (7.58;2.74), (7.57;2.67), (7.56;2.70), (7.49;1.70), (7.47;2.17), (7.38;2.83), (7.35;6.36), (7.34;5.35), (7.34;9.55), (7.32;10.14), (7.30;2.34), (7.12;3.92), (7.10;3.24), (6.31;0.33), (6.28;11.34), (4.82;0.34), (4.36;3.48), (4.34;3.43), (3.81;3.53), (3.62;0.33), (3.62;0.34), (3.61;0.39), (3.59;0.38), (3.59;0.34), (3.58;0.35), (3.55;0.47), (3.31;2349.63), (3.29;38.27), (3.24;1.30), (3.20;0.60), (3.18;0.49), (3.15;0.32), (2.67;1.43), (2.67;1.84), (2.67;1.29), (2.54;2.32), (2.51;106.98), (2.50;199.61), (2.50;260.07), (2.50;179.41), (2.49;85.45), (2.42;0.39), (2.39;0.34), (2.33;1.31), (2.33;1.86), (2.32;1.39), (2.29;0.40), (2.10;16.00), (2.07;1.04), (1.36;0.89), (1.24;0.55), (0.00;15.89) |
| 202 | 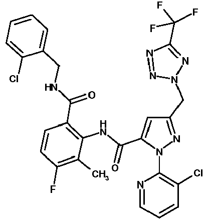 | 3,79 | 648 | (10.43;5.93), (8.78;1.55), (8.76;3.22), (8.75;1.61), (8.49;3.96), (8.49;4.27), (8.48;4.34), (8.48;4.03), (8.16;3.92), (8.15;3.84), (8.14;4.35), (8.13;3.86), (7.61;4.07), (7.60;4.01), (7.59;3.81), (7.58;3.79), (7.50;2.02), (7.48;2.32), (7.48;2.52), (7.46;2.14), (7.41;3.28), (7.41;3.36), (7.39;4.01), (7.39;3.93), (7.32;10.07), (7.31;2.83), (7.29;3.27), (7.25;3.15), (7.24;1.90), (7.23;7.13), (7.20;3.73), (7.18;2.71), (7.17;2.69), (7.16;3.42), (7.16;3.25), (7.14;1.28), (6.30;16.00), (4.40;6.44), (4.39;6.38), (3.57;0.35), (3.54;0.37), (3.52;0.38), (3.50;0.47), (3.48;0.52), (3.31;1987.53), (3.29;17.05), (2.67;1.24), (2.67;0.96), (2.66;0.55), (2.54;1.88), (2.51;75.50), (2.50;137.92), (2.50;176.78), (2.50;122.45), (2.49;58.54), (2.33;0.90), (2.33;1.22), (2.32;0.88), (2.07;4.20), (2.06;13.80), (2.06;13.69), (1.99;0.40), (1.24;0.39), (0.00;11.74) |
| 203 | 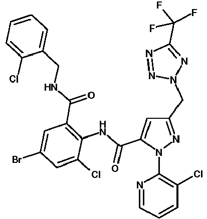 | 4,07 | 728/7 30 | (10.49;6.21), (8.90;1.60), (8.89;3.33), (8.88;1.70), (8.49;3.76), (8.48;4.05), (8.48;4.21), (8.47;3.95), (8.14;3.67), (8.13;3.67), (8.12;4.18), (8.11;3.69), (7.96;5.94), (7.96;5.90), (7.70;6.70), (7.70;6.31), (7.61;3.63), (7.60;3.69), (7.59;3.44), (7.58;3.24), (7.41;3.71), (7.41;3.57), (7.39;4.89), (7.39;5.12), (7.38;7.21), (7.32;0.35), (7.30;2.81), (7.30;3.06), (7.28;3.61), (7.25;1.73), (7.24;1.72), (7.23;3.41), (7.22;3.03), (7.21;2.19), (7.16;2.98), (7.16;2.83), (7.14;3.75), (7.13;1.52), (6.49;0.67), (6.30;16.00), (4.37;6.77), (4.36;6.67), (4.33;0.34), (3.71;0.36), (3.66;0.33), (3.63;0.39), (3.62;0.38), (3.60;0.34), (3.58;0.42), (3.55;0.52), (3.54;0.49), (3.48;0.70), (3.46;0.79), (3.44;0.96), (3.42;1.25), (3.41;1.64), (3.31;2452.94), (3.28;25.59), (3.19;0.78), (3.18;0.79), (3.16;0.54), (3.15;0.50), (3.10;0.36), (3.08;0.33), (3.07;0.36), (2.67;1.60), (2.67;2.11), (2.66;1.56), (2.66;1.05), (2.54;3.54), (2.51;124.99), (2.50;224.09), (2.50;284.42), (2.50;198.04), (2.42;0.54), (2.41;0.46), (2.37;0.33), (2.33;1.54), (2.33;2.02), (2.32;1.51), (2.07;0.65), (1.24;0.49), (1.24;0.68), (0.89;0.35), (0.00;8.33) |
| 204 | 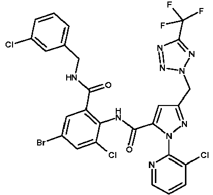 | 4,09 | 728/7 30 | (10.47;5.95), (8.89;1.80), (8.88;3.24), (8.86;1.68), (8.47;3.91), (8.47;4.04), (8.46;4.17), (8.46;3.97), (8.14;0.45), (8.12;3.66), (8.12;3.80), (8.10;4.09), (8.10;3.89), (7.95;6.06), (7.94;6.18), (7.65;6.85), (7.64;6.58), (7.61;0.36), (7.60;0.62), (7.59;3.80), (7.58;3.78), (7.57;3.54), (7.56;3.47), (7.38;6.74), (7.32;5.32), (7.26;1.05), (7.24;7.01), (7.24;6.98), (7.24;4.77), (7.23;4.54), (7.23;6.30), (7.21;1.95), (7.19;2.98), (7.18;1.97), (7.17;1.24), (7.17;1.10), (6.49;1.67), (6.30;16.00), (5.75;0.52), (4.32;6.82), (4.30;6.66), (3.72;0.33), (3.63;0.35), (3.62;0.41), (3.61;0.34), (3.56;0.42), (3.53;0.55), (3.53;0.45), (3.51;0.57), (3.31;2064.96), (3.28;21.66), (3.18;0.42), (3.17;0.41), (2.67;1.43), (2.67;1.93), (2.66;1.45), (2.65;0.34), (2.54;3.17), (2.51;109.62), (2.50;201.24), (2.50;261.02), (2.50;183.82), (2.49;90.40), (2.33;1.34), (2.33;1.70), (2.32;1.32), (2.07;0.52), (1.40;0.54), (1.24;0.69), (0.00;9.76), (-0.01;0.63) |
| 205 | 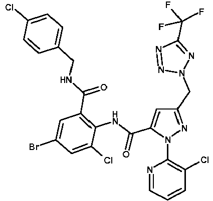 | 4,08 | 728/7 30 | (10.47;4.46), (8.89;1.28), (8.88;2.38), (8.87;1.26), (8.48;2.71), (8.48;2.90), (8.47;3.00), (8.47;2.89), (8.14;2.57), (8.14;2.57), (8.12;2.86), (8.12;2.74), (7.94;4.00), (7.94;3.91), (7.64;4.58), (7.63;4.42), (7.61;2.52), (7.59;2.57), (7.59;2.48), (7.57;2.32), (7.39;4.70), (7.32;0.32), (7.28;2.08), (7.27;1.46), (7.25;16.00), (7.25;13.25), (7.22;1.78), (6.48;1.09), (6.31;11.48), (4.30;5.37), (4.28;5.23), (3.43;0.67), (3.42;0.72), (3.39;1.01), (3.30;1247.03), (3.28;14.69), (2.67;1.08), (2.67;1.36), (2.66;1.03), (2.54;2.29), (2.51;77.62), (2.50;140.91), (2.50;181.14), (2.50;126.83), (2.49;62.20), (2.44;0.56), (2.33;0.93), (2.33;1.25), (2.32;0.85), (2.07;0.53), (1.40;0.57), (1.24;0.40), (0.01;0.41), (0.00;7.73) |
| 206 | 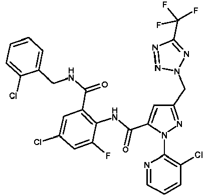 | 3,9 | 668 | (10.42;6.60), (8.98;1.58), (8.96;3.12), (8.95;1.45), (8.49;3.69), (8.49;3.95), (8.48;4.12), (8.48;3.83), (8.15;3.56), (8.15;3.60), (8.13;4.03), (8.13;3.74), (7.69;2.63), (7.69;2.71), (7.67;2.73), (7.66;2.84), (7.62;3.77), (7.61;3.72), (7.60;3.56), (7.59;3.43), (7.50;3.99), (7.49;4.59), (7.42;3.11), (7.42;3.20), (7.40;3.95), (7.40;3.98), (7.38;9.40), (7.33;2.59), (7.32;3.04), (7.31;3.07), (7.27;0.50), (7.26;1.46), (7.24;2.94), (7.24;2.69), (7.23;1.93), (7.22;1.65), (7.19;2.52), (7.18;2.46), (7.17;3.39), (7.16;3.21), (7.15;1.26), (6.30;16.00), (4.40;6.33), (4.39;6.32), (3.60;0.36), (3.56;0.39), (3.55;0.41), (3.54;0.40), (3.50;0.56), (3.45;0.76), (3.31;1840.74), (3.28;17.10), (3.25;1.91), (3.22;0.71), (3.18;0.42), (2.67;1.21), (2.67;1.59), (2.63;0.39), (2.60;0.58), (2.54;2.71), (2.51;93.66), (2.50;171.68), (2.50;222.30), (2.50;157.02), (2.49;77.67), (2.33;1.15), (2.33;1.52), (2.32;1.11), (2.07;0.74), (1.24;0.43), (0.00;5.89) |
| 207 | 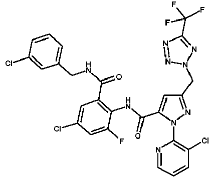 | 3,89 | 668 | (10.41;6.81), (8.98;1.53), (8.97;3.01), (8.95;1.50), (8.48;3.77), (8.48;3.92), (8.47;4.02), (8.47;3.79), (8.14;3.60), (8.14;3.56), (8.12;3.98), (8.12;3.67), (7.68;2.60), (7.68;2.68), (7.66;2.67), (7.65;2.68), (7.60;3.69), (7.59;3.60), (7.58;3.46), (7.57;3.32), (7.46;4.08), (7.46;4.80), (7.38;9.08), (7.34;4.94), (7.27;1.01), (7.26;5.72), (7.26;5.34), (7.25;4.89), (7.25;8.51), (7.24;1.28), (7.23;1.30), (7.22;3.27), (7.22;2.87), (7.21;2.47), (7.20;1.00), (6.30;16.00), (4.35;6.61), (4.34;6.53), (3.31;301.17), (2.51;29.22), (2.50;36.63), (2.50;25.70), (2.08;0.86), (0.00;3.99) |
| 208 | 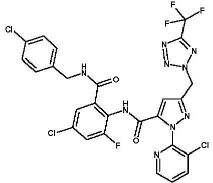 | 3,95 | 668 | (10.40;2.27), (8.98;0.48), (8.96;0.92), (8.95;0.47), (8.49;1.23), (8.48;1.31), (8.48;1.34), (8.47;1.26), (8.15;1.13), (8.15;1.12), (8.13;1.25), (8.13;1.19), (7.68;0.78), (7.67;0.82), (7.65;0.81), (7.65;0.82), (7.62;1.18), (7.60;1.16), (7.60;1.12), (7.58;1.05), (7.44;1.58), (7.39;2.61), (7.29;0.34), (7.27;16.00), (6.31;5.13), (4.33;2.48), (4.31;2.46), (3.31;245.11), (3.28;2.63), (2.51;13.11), (2.50;23.97), (2.50;30.94), (2.50;21.80), (2.49;10.72), (0.00;1.30) |
| 209 | 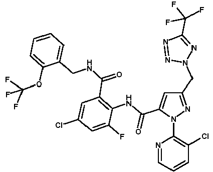 | 4,12 | 718 | (10.42;6.70), (8.99;1.52), (8.97;3.08), (8.96;1.48), (8.48;3.66), (8.48;3.93), (8.47;3.94), (8.47;3.83), (8.14;3.56), (8.14;3.57), (8.12;3.95), (8.12;3.70), (7.69;2.61), (7.69;2.72), (7.67;2.67), (7.66;2.74), (7.62;3.65), (7.60;3.60), (7.59;3.47), (7.58;3.35), (7.47;3.90), (7.47;4.60), (7.47;4.37), (7.40;2.89), (7.38;12.06), (7.35;2.58), (7.35;2.28), (7.34;3.47), (7.33;3.87), (7.33;4.02), (7.31;0.99), (7.31;1.15), (7.24;2.12), (7.24;2.00), (7.22;2.81), (7.20;1.33), (7.20;1.28), (6.31;16.00), (4.41;6.13), (4.39;6.10), (3.31;213.65), (2.54;0.44), (2.51;15.72), (2.50;28.35), (2.50;36.20), (2.50;25.52), (2.08;0.73), (0.00;4.57) |
| 210 | 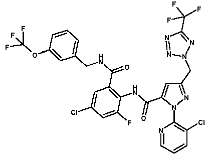 | 4,22 | 718 | (10.41;7.40), (9.03;1.55), (9.02;3.03), (9.00;1.51), (8.48;3.92), (8.48;4.11), (8.47;4.14), (8.47;3.92), (8.14;3.65), (8.14;3.61), (8.12;4.02), (8.12;3.77), (7.69;2.56), (7.68;2.63), (7.66;2.64), (7.66;2.67), (7.65;0.54), (7.63;0.36), (7.61;3.72), (7.59;3.71), (7.59;3.60), (7.57;3.54), (7.48;0.57), (7.45;5.00), (7.38;8.98), (7.35;5.36), (7.33;4.46), (7.29;4.48), (7.27;6.48), (7.20;2.63), (7.18;2.09), (6.30;16.00), (4.54;0.48), (4.53;0.49), (4.39;6.75), (4.38;6.69), (3.40;0.59), (3.31;869.51), (3.28;8.69), (3.22;0.41), (2.67;0.72), (2.67;0.56), (2.54;1.15), (2.51;40.76), (2.50;74.47), (2.50;96.05), (2.50;66.89), (2.49;32.30), (2.33;0.52), (2.33;0.67), (2.32;0.49), (0.00;1.03) |
| 211 | 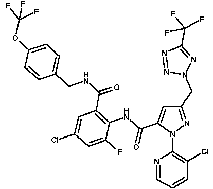 | 4,21 | 718 | (10.42;7.28), (9.00;1.56), (8.99;3.00), (8.97;1.56), (8.48;3.94), (8.48;4.10), (8.47;4.18), (8.46;3.92), (8.15;3.59), (8.15;3.45), (8.13;3.91), (8.13;3.60), (8.03;0.46), (8.01;0.45), (7.68;2.47), (7.68;2.58), (7.66;2.51), (7.65;2.46), (7.61;3.55), (7.60;3.55), (7.59;3.41), (7.58;3.23), (7.49;0.45), (7.46;5.38), (7.43;0.73), (7.40;8.19), (7.38;7.66), (7.36;9.56), (7.33;0.51), (7.22;7.26), (7.20;5.66), (6.30;16.00), (4.51;0.36), (4.50;0.40), (4.37;7.05), (4.35;6.90), (3.31;1142.44), (3.29;12.09), (3.24;0.86), (3.20;0.42), (3.19;0.34), (2.67;0.74), (2.67;0.93), (2.51;55.82), (2.50;101.07), (2.50;129.48), (2.50;91.07), (2.46;0.68), (2.33;0.68), (2.33;0.91), (2.32;0.64), (2.07;0.34), (1.40;0.33), (0.00;5.87), (-0.01;0.33) |
| 212 | 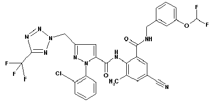 | 3,67 | 686 | (10.46;2.78), (8.98;1.46), (8.97;2.62), (8.96;1.41), (8.54;1.12), (7.90;4.74), (7.82;5.14), (7.66;0.52), (7.65;0.64), (7.59;0.73), (7.56;2.90), (7.55;3.86), (7.54;1.20), (7.52;0.65), (7.48;1.05), (7.48;1.42), (7.47;1.90), (7.47;2.62), (7.46;2.01), (7.46;3.08), (7.45;8.25), (7.44;2.54), (7.43;1.55), (7.43;1.33), (7.41;1.25), (7.40;0.74), (7.39;0.56), (7.38;0.39), (7.36;0.65), (7.32;4.83), (7.31;5.10), (7.31;2.02), (7.29;0.48), (7.29;0.63), (7.27;1.26), (7.26;3.34), (7.26;1.40), (7.25;6.08), (7.24;1.81), (7.24;4.38), (7.22;0.54), (7.20;1.47), (7.19;7.60), (7.18;0.89), (7.15;3.36), (7.14;2.55), (7.12;0.77), (7.09;5.01), (7.08;1.12), (7.07;3.87), (7.04;2.83), (7.03;2.89), (7.02;2.42), (7.02;2.20), (6.29;10.77), (6.07;1.74), (4.81;1.85), (4.33;4.58), (4.32;4.60), (3.39;2.92), (3.37;1474.21), (3.34;14.70), (2.62;0.99), (2.62;1.38), (2.61;0.99), (2.54;0.68), (2.53;2.46), (2.52;3.35), (2.52;4.17), (2.51;76.67), (2.51;161.87), (2.50;220.84), (2.50;159.63), (2.50;74.60), (2.39;0.99), (2.39;1.36), (2.39;0.99), (2.22;16.00), (2.08;1.88), (1.91;0.39), (0.01;1.55), (0.00;27.87), (-0.01;0.96) |
| 213 | 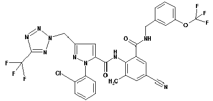 | 4,18 | 704 | (10.46;4.26), (10.45;1.01), (9.01;1.16), (9.00;2.40), (8.99;1.15), (7.90;3.59), (7.90;3.85), (7.90;4.19), (7.83;4.37), (7.83;3.71), (7.56;1.63), (7.56;2.42), (7.56;1.87), (7.55;2.18), (7.55;3.92), (7.54;2.66), (7.54;0.61), (7.53;0.48), (7.53;0.58), (7.48;1.17), (7.48;1.71), (7.47;1.84), (7.47;0.89), (7.47;2.55), (7.46;2.21), (7.46;0.72), (7.45;2.71), (7.45;0.83), (7.45;1.17), (7.44;5.23), (7.44;7.63), (7.43;4.78), (7.43;3.57), (7.43;0.86), (7.43;0.72), (7.39;0.56), (7.38;0.47), (7.37;0.41), (7.37;0.34), (7.35;0.74), (7.34;1.75), (7.32;3.90), (7.32;5.64), (7.31;4.32), (7.31;3.59), (7.29;1.12), (7.27;2.77), (7.21;1.71), (7.20;1.21), (6.29;11.45), (6.07;1.80), (5.76;0.49), (4.37;2.71), (4.36;2.85), (3.34;224.68), (3.32;2.90), (2.62;0.43), (2.61;0.60), (2.61;0.44), (2.52;1.11), (2.52;1.43), (2.52;1.39), (2.51;30.49), (2.51;67.61), (2.50;93.37), (2.50;68.27), (2.50;31.10), (2.39;0.42), (2.39;0.58), (2.38;0.41), (2.22;16.00), (2.21;3.40), (2.08;0.61), (0.00;6.60) |
| 214 | 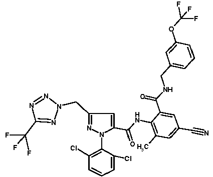 | 4,23 | 738 | (10.54;4.35), (10.52;1.24), (8.99;1.25), (8.98;2.59), (8.97;1.26), (7.90;4.15), (7.90;4.47), (7.83;4.67), (7.83;4.33), (7.61;6.57), (7.60;7.04), (7.59;11.16), (7.59;1.98), (7.59;2.10), (7.58;0.35), (7.57;2.99), (7.52;3.98), (7.51;4.03), (7.50;3.74), (7.49;3.09), (7.48;0.73), (7.42;6.06), (7.37;0.53), (7.36;1.42), (7.36;1.61), (7.35;1.90), (7.34;4.17), (7.32;3.74), (7.32;0.89), (7.31;3.09), (7.30;1.46), (7.28;2.90), (7.23;0.46), (7.21;1.67), (7.21;1.68), (7.20;1.26), (6.34;12.48), (6.12;2.78), (4.36;5.10), (4.35;5.25), (3.35;188.03), (3.33;2.60), (2.51;9.06), (2.51;20.28), (2.50;28.04), (2.50;20.24), (2.50;9.09), (2.22;0.48), (2.21;16.00), (2.20;4.75), (2.08;0.40), (1.18;0.34), (0.00;5.98) |
| 215 | 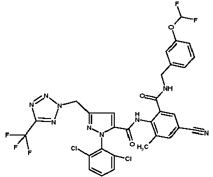 | 3,78 | 720 | (10.53;1.24), (8.96;0.68), (8.95;1.25), (8.94;0.69), (8.55;0.50), (7.89;2.51), (7.82;3.10), (7.82;2.93), (7.61;4.87), (7.61;5.16), (7.59;8.62), (7.59;1.26), (7.57;1.24), (7.54;0.43), (7.52;0.41), (7.52;2.88), (7.51;2.82), (7.50;2.62), (7.49;2.06), (7.43;0.38), (7.42;2.51), (7.41;0.79), (7.40;0.34), (7.37;0.48), (7.36;0.41), (7.34;0.37), (7.34;2.17), (7.31;0.63), (7.28;0.58), (7.28;1.70), (7.27;0.47), (7.27;3.45), (7.25;2.34), (7.24;0.67), (7.22;0.93), (7.21;4.78), (7.15;2.10), (7.14;1.61), (7.12;0.34), (7.10;2.81), (7.10;2.31), (7.09;2.48), (7.04;1.61), (7.03;1.46), (7.02;1.29), (7.02;1.20), (6.34;7.99), (6.12;1.05), (5.76;16.00), (4.81;0.78), (4.32;3.86), (4.31;3.95), (3.35;106.51), (3.33;1.18), (2.52;0.49), (2.52;0.60), (2.52;0.59), (2.51;12.52), (2.51;27.26), (2.50;37.38), (2.50;27.16), (2.50;12.39), (2.21;10.56), (2.20:2.05), (2.08:1.17), (0.00:2.09) |
| 216 | 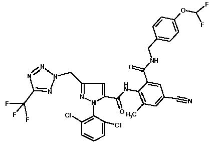 | 4,1 | 720 | (10.53;2.66), (8.92;2.94), (7.89;5.09), (7.85;0.56), (7.83;0.84), (7.81;5.84), (7.68;0.68), (7.66;0.36), (7.61;7.52), (7.60;10.74), (7.58;2.03), (7.53;3.18), (7.51;4.46), (7.50;2.59), (7.45;0.61), (7.43;4.65), (7.39;0.59), (7.37;0.97), (7.32;9.09), (7.30;10.18), (7.30;7.92), (7.26;0.57), (7.25;0.43), (7.21;0.48), (7.19;2.12), (7.18;9.81), (7.16;0.54), (7.15;0.41), (7.06;1.52), (7.06;3.27), (7.05;5.52), (7.05;15.36), (7.03;11.83), (6.34;11.18), (6.29;0.43), (6.25;0.78), (6.13;2.02), (6.10;0.75), (5.76;0.61), (4.76;0.52), (4.30;8.54), (4.29;8.70), (3.60;0.60), (3.39;1.12), (3.38;2.01), (3.35;2738.84), (3.33;50.25), (2.62;2.06), (2.62;2.73), (2.61;1.97), (2.54;1.22), (2.52;4.23), (2.52;5.52), (2.52;6.52), (2.51;161.08), (2.51;328.25), (2.50;436.95), (2.50;315.69), (2.50;147.77), (2.39;2.00), (2.39;2.74), (2.38;1.97), (2.20;16.00), (2.08;4.08), (1.91;0.77), (1.76;0.62), (1.75;0.32), (1.35;0.68), (1.23;0.74), (0.01;1.34), (0.00;33.65), (-0.01;1.12) |
| 217 | 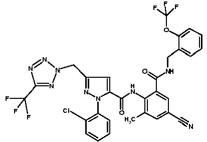 | 4,28 | 704 | (10.46;4.63), (8.96;1.14), (8.95;2.35), (8.94;1.14), (7.91;3.86), (7.85;4.05), (7.85;3.67), (7.56;2.57), (7.54;3.15), (7.49;1.05), (7.48;1.48), (7.48;1.57), (7.48;2.10), (7.47;1.10), (7.47;1.45), (7.46;1.77), (7.46;0.52), (7.45;1.10), (7.44;8.74), (7.44;4.24), (7.43;2.48), (7.38;0.89), (7.38;0.88), (7.37;2.21), (7.35;1.91), (7.35;1.88), (7.33;2.74), (7.32;6.26), (7.20;1.56), (7.20;1.55), (7.19;2.52), (7.18;1.26), (7.17;1.20), (6.29;10.72), (4.37;2.16), (3.37;0.42), (3.34;554.32), (3.32;8.65), (2.62;0.44), (2.62;0.98), (2.61;1.39), (2.61;1.00), (2.61;0.47), (2.54;0.83), (2.53;0.33), (2.52;2.55), (2.52;3.25), (2.52;3.25), (2.51;74.17), (2.51;162.75), (2.50;225.57), (2.50;164.27), (2.50;75.43), (2.39;0.44), (2.39;1.00), (2.39;1.36), (2.38;0.99), (2.38;0.46), (2.23;16.00), (2.08;1.43), (0.00;9.58) |
| 218 | 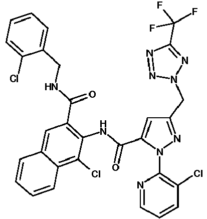 | 4,1 | 700 | (10.70;7.02), (9.00:1.65), (8.99;3.52), (8.97;1.71), (8.50;4.96), (8.49;5.39), (8.49;5.51), (8.48;5.50), (8.25;3.75), (8.22;4.16), (8.18;9.68), (8.14;8.11), (8.14;5.87), (8.12;9.12), (8.12;6.25), (7.80;1.74), (7.80;1.89), (7.79;2.98), (7.78;3.61), (7.78;1.97), (7.76;2.72), (7.76;2.59), (7.73;2.68), (7.73;2.72), (7.71;3.78), (7.69;1.70), (7.69;1.63), (7.60;5.00), (7.59;4.84), (7.58;4.76), (7.57;4.77), (7.45;9.13), (7.43;4.10), (7.43;4.51), (7.41;4.87), (7.41;5.10), (7.38;2.69), (7.38;2.86), (7.37;3.36), (7.36;3.46), (7.26;1.50), (7.26;1.57), (7.24;3.54), (7.24;3.26), (7.22;2.63), (7.22;2.26), (7.20;3.03), (7.20;3.14), (7.19;3.96), (7.18;3.87), (7.17;1.45), (7.16;1.39), (6.33;16.00), (4.43;6.27), (4.41;6.38), (3.35;158.94), (3.35;166.27), (3.34;307.01), (3.32;7.00), (2.68;0.40), (2.67;0.53), (2.67;0.38), (2.54;0.33), (2.52;0.80), (2.51;56.86), (2.50;76.47), (2.33;0.37), (2.33;0.50), (2.32;0.37), (2.07;2.63), (0.00;9.36) |
| 219 | 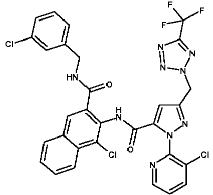 | 4,13 | 700 | (10.67;5.41), (9.00;1.26), (8.98;2.74), (8.97;1.26), (8.48;3.85), (8.48;4.14), (8.47;4.24), (8.47;4.20), (8.23;3.06), (8.21;3.42), (8.13;6.92), (8.12;13.11), (8.11;6.34), (8.10;7.23), (7.79;1.49), (7.79;1.57), (7.77;2.78), (7.75;2.20), (7.75;1.97), (7.72;2.21), (7.70;2.99), (7.68;1.39), (7.58;3.88), (7.57;3.86), (7.56;3.57), (7.55;3.71), (7.46;7.45), (7.37;4.98), (7.29;0.36), (7.27;1.38), (7.26;14.09), (7.26;16.00), (7.24;1.32), (6.33;13.75), (4.38;5.10), (4.36;5.46), (3.32;483.93), (3.30;20.38), (2.67;0.62), (2.67;0.89), (2.67;0.61), (2.54;0.47), (2.52;1.28), (2.51;94.99), (2.50;126.99), (2.33;0.60), (2.33;0.83), (2.32;0.67), (2.07;0.72), (1.23;0.48), (0.01;0.52), (0.00;17.62), (-0.01:0.53) |
| 220 | 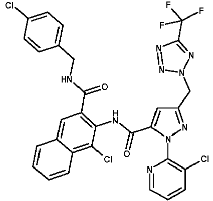 | 4,11 | 700 | (10.68;1.30), (8.99;0.41), (8.98;0.72), (8.96;0.43), (8.49;1.07), (8.48;1.15), (8.48;1.07), (8.23;0.80), (8.21;0.89), (8.13;1.01), (8.11;2.65), (8.09;1.03), (7.79;0.46), (7.77;0.79), (7.75;0.58), (7.72;0.61), (7.70;0.75), (7.68;0.39), (7.59;0.85), (7.58;0.91), (7.57;0.81), (7.56;0.82), (7.46;1.54), (7.29;16.00), (6.33;3.47), (4.36;2.04), (4.35;2.07), (3.34;748.67), (3.31;12.46), (2.68;0.51), (2.67;0.68), (2.67;0.42), (2.54;0.42), (2.52;0.94), (2.52;1.44), (2.51;65.92), (2.50;89.58), (2.50;58.46), (2.33;0.50), (2.33;0.72), (2.32;0.49), (2.07;0.99), (1.24;0.40), (0.00;4.72) |
| 221 | 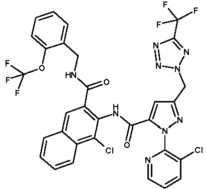 | 4,36 | 750 | (10.70;6.70), (9.00;1.51), (8.99;3.28), (8.98;1.56), (8.49;4.77), (8.48;5.09), (8.47;5.22), (8.47;5.15), (8.24;3.53), (8.22;3.94), (8.15;9.23), (8.12;7.61), (8.12;5.29), (8.10;8.58), (8.10;5.55), (7.80;1.65), (7.80;1.73), (7.78;2.84), (7.78;3.44), (7.78;1.82), (7.76;2.60), (7.76;2.39), (7.73;2.53), (7.73;2.51), (7.71;3.50), (7.69;1.59), (7.69;1.54), (7.60;4.82), (7.58;4.62), (7.58;4.53), (7.56;4.61), (7.46;9.06), (7.45;3.12), (7.43;3.81), (7.37;0.62), (7.35;2.58), (7.35;2.76), (7.33;7.96), (7.32;0.69), (7.32;0.73), (7.25;2.39), (7.25;2.13), (7.24;2.65), (7.23;1.83), (7.22;1.23), (7.21;1.42), (6.33;16.00), (4.43;5.76), (4.42;5.92), (3.33;387.50), (3.31;8.41), (2.67;0.38), (2.52;0.56), (2.51;37.72), (2.50;50.62), (2.50;33.71), (2.33;0.33), (2.07;3.00), (0.00;3.66) |
| 222 | 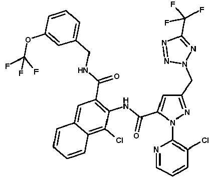 | 4,38 | 750 | (19.00;0.40), (15.68;0.39), (12.89;0.40), (12.51;0.39), (12.33;0.38), (10.68;6.83), (9.05;1.61), (9.03;3.43), (9.02;1.56), (8.80;0.38), (8.49;4.67), (8.48;5.20), (8.48;5.14), (8.47;5.24), (8.24;3.57), (8.22;4.08), (8.13;5.00), (8.13;5.66), (8.12;9.69), (8.11;8.67), (8.11;5.62), (8.09;3.84), (8.08;0.41), (7.80;1.78), (7.79;1.77), (7.78;2.99), (7.78;3.40), (7.76;2.74), (7.76;2.68), (7.72;2.53), (7.72;2.58), (7.70;3.54), (7.69;1.86), (7.68;1.71), (7.59;4.97), (7.57;4.70), (7.57;4.60), (7.55;4.87), (7.46;9.19), (7.43;0.43), (7.38;1.70), (7.36;4.86), (7.34;4.81), (7.33;4.91), (7.31;6.22), (7.20;2.62), (7.19;2.08), (6.33;16.00), (5.31;0.40), (4.42;5.74), (4.41;5.82), (3.40;0.51), (3.37;1.07), (3.33;2450.00), (3.31;46.04), (3.25;0.53), (3.22;0.43), (3.19;0.41), (2.68;1.40), (2.67;1.97), (2.67;1.41), (2.55;0.44), (2.54;1.06), (2.52;2.94), (2.51;212.05), (2.50;288.68), (2.50;188.48), (2.33;1.47), (2.33;1.99), (2.32;1.54), (2.07;2.59), (1.24;1.05), (0.00;10.64), (-0.01;0.40), (-3.31;0.46) |
| 223 | 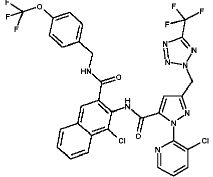 | 4,36 | 750 | (19.01;0.74), (15.97;0.72), (14.49;0.71), (11.82;0.69), (10.70;6.80), (9.02;1.77), (9.01;3.79), (8.99;1.61), (8.48;4.30), (8.48;4.96), (8.47;5.14), (8.46;4.61), (8.24;3.89), (8.23;0.99), (8.22;4.07), (8.14;5.05), (8.13;5.34), (8.13;9.79), (8.12;8.84), (8.11;6.66), (8.10;4.15), (7.79;1.98), (7.78;3.01), (7.77;3.51), (7.76;2.77), (7.75;2.77), (7.72;2.66), (7.70;3.53), (7.68;1.85), (7.59;4.52), (7.58;4.52), (7.57;4.23), (7.56;3.92), (7.48;8.96), (7.47;0.79), (7.45;0.72), (7.40;7.87), (7.38;9.41), (7.24;7.11), (7.22;5.93), (6.33;16.00), (5.93;0.78), (5.29;0.77), (4.40;6.39), (4.39;6.35), (3.33;2682.03), (3.30;82.41), (3.28;1.56), (3.26;0.92), (2.68;2.57), (2.67;2.86), (2.66;1.30), (2.54;2.04), (2.52;5.59), (2.51;369.97), (2.50;487.64), (2.45;0.89), (2.33;2.34), (2.33;3.20), (2.32;2.54), (2.07;5.23), (1.40;4.44), (1.23;1.66), (0.00;32.79) |
| 224 | 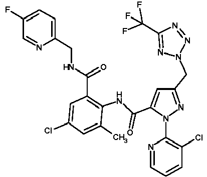 | 3,24 | 649 | (10.27;1.18), (10.22;4.30), (8.87;0.60), (8.73;1.19), (8.71;2.32), (8.70;1.14), (8.49;0.96), (8.48;1.15), (8.47;3.83), (8.47;3.99), (8.46;3.33), (8.46;3.18), (8.40;0.92), (8.40;0.92), (8.28;1.55), (8.28;2.36), (8.28;1.63), (8.27;1.65), (8.27;2.31), (8.15;0.88), (8.15;1.02), (8.14;2.92), (8.13;2.98), (8.13;1.28), (8.13;1.10), (8.12;3.18), (8.11;2.99), (7.67;1.31), (7.66;1.30), (7.65;1.70), (7.64;2.18), (7.64;1.48), (7.62;1.61), (7.62;1.53), (7.60;1.23), (7.60;3.22), (7.59;1.41), (7.59;3.14), (7.58;1.46), (7.58;3.04), (7.57;1.26), (7.57;2.90), (7.50;1.38), (7.49;1.65), (7.48;3.37), (7.48;3.95), (7.45;0.44), (7.44;0.39), (7.43;1.16), (7.42;1.02), (7.40;3.95), (7.40;3.43), (7.36;1.24), (7.35;2.15), (7.34;2.07), (7.33;1.83), (7.32;1.23), (7.31;2.32), (7.29;7.32), (6.29;12.04), (4.52;3.81), (4.51;3.89), (4.41;1.18), (4.40;1.16), (3.62;0.34), (3.60;0.38), (3.57;1.65), (3.56;0.45), (3.50;0.60), (3.32;1569.14), (3.17;0.45), (2.70;0.40), (2.67;1.77), (2.67;2.33), (2.66;1.74), (2.66;0.98), (2.54;4.44), (2.52;12.21), (2.51;130.46), (2.50;236.74), (2.50;305.19), (2.50;212.28), (2.49;101.64), (2.34;0.78), (2.33;1.52), (2.33;2.03), (2.32;1.41), (2.32;0.68), (2.17;4.59), (2.15;16.00), (2.07;4.82), (1.60;0.61), (1.40;2.65), (1.24;0.64), (0.89;0.49), (0.01;1.26), (0.00;23.59), (-0.01;0.92) |
| 225 | 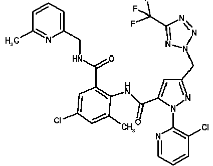 | 2,24 | 645 | (10.26;0.69), (8.83;0.89), (8.48;2.09), (8.47;2.21), (8.47;2.28), (8.46;2.22), (8.14;1.93), (8.14;1.94), (8.12;2.21), (8.12;2.07), (7.60;2.01), (7.59;2.03), (7.58;1.95), (7.57;1.89), (7.48;2.39), (7.47;1.65), (7.45;5.48), (7.43;1.80), (7.29;2.94), (7.04;2.37), (7.03;2.46), (7.02;2.39), (7.01;2.04), (6.28;7.82), (4.38;3.53), (4.36;3.53), (3.31;955.39), (3.29;13.70), (2.67;0.74), (2.67;0.98), (2.67;0.75), (2.57;0.37), (2.54;1.37), (2.52;4.65), (2.51;56.56), (2.50;104.01), (2.50;134.93), (2.50;95.46), (2.49;46.96), (2.44;0.67), (2.41;16.00), (2.37;0.48), (2.33;0.79), (2.33;1.03), (2.32;0.82), (2.31;0.45), (2.16;11.45), (2.13;0.39), (2.07;1.07), (0.00;0.90) |
| 226 | 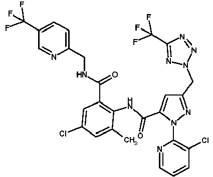 | 3,7 | 699 | (10.30;0.47), (9.00;0.91), (8.99;1.76), (8.98;0.94), (8.94;0.32), (8.82;2.85), (8.82;2.87), (8.47;2.92), (8.46;3.14), (8.45;3.21), (8.45;3.08), (8.14;2.91), (8.14;2.90), (8.12;3.21), (8.12;3.01), (7.99;1.68), (7.99;1.71), (7.97;1.79), (7.97;1.76), (7.60;2.97), (7.59;2.93), (7.58;2.84), (7.57;2.84), (7.51;3.03), (7.51;3.75), (7.48;2.84), (7.46;4.44), (7.46;5.53), (7.33;7.39), (6.27;11.30), (4.51;3.78), (4.50;3.78), (4.11;0.89), (3.32;804.64), (2.68;0.48), (2.67;0.87), (2.67;1.14), (2.67;0.85), (2.66;0.46), (2.54;1.47), (2.52;5.10), (2.51;64.71), (2.51;119.88), (2.50;156.29), (2.50;110.12), (2.49;54.04), (2.34;0.62), (2.33;0.95), (2.33;1.20), (2.32;0.90), (2.18;16.00), (2.13;0.42), (2.07;2.49), (0.00;1.15) |
| 227 | 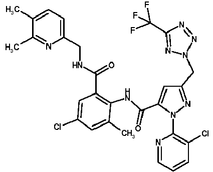 | 2,1 | 659 | (8.85;0.38), (8.48;2.47), (8.48;2.61), (8.47;2.68), (8.47;2.65), (8.15;2.24), (8.14;2.26), (8.13;2.43), (8.13;2.34), (7.60;2.35), (7.59;2.28), (7.59;2.26), (7.58;2.34), (7.49;1.83), (7.44;2.70), (7.44;2.39), (7.31;1.85), (7.30;2.37), (7.29;2.36), (6.97;2.25), (6.96;2.13), (6.30;7.96), (4.34;3.31), (4.33;3.32), (3.35;96.84), (3.32;0.42), (2.52;0.62), (2.52;0.79), (2.52;0.78), (2.51;15.34), (2.51;33.54), (2.50;45.80), (2.50;33.39), (2.50;15.16), (2.41;0.52), (2.39;0.35), (2.37;16.00), (2.23;0.46), (2.19;12.56), (2.16;11.90), (0.00;4.25) |
| 228 | 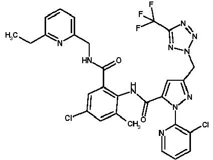 | 2,22 | 659 | (8.90;0.55), (8.48;2.28), (8.48;2.41), (8.48;2.45), (8.47;2.43), (8.15;2.15), (8.15;2.15), (8.14;2.30), (8.14;2.20), (7.60;2.24), (7.59;2.19), (7.59;2.15), (7.58;2.22), (7.50;1.67), (7.50;1.86), (7.48;1.66), (7.47;3.58), (7.46;2.76), (7.46;2.34), (7.45;2.10), (7.31;2.28), (7.06;2.13), (7.04;2.08), (7.04;2.05), (7.02;1.91), (6.30;7.81), (4.39;3.29), (4.38;3.31), (3.35;79.87), (3.32;0.36), (2.72;1.34), (2.70;4.17), (2.69;4.25), (2.68;1.41), (2.52;0.50), (2.52;0.63), (2.52;0.60), (2.51;13.42), (2.51;29.52), (2.50;40.31), (2.50;29.27), (2.50;13.18), (2.17;11.22), (1.23;0.38), (1.22;7.17), (1.21;0.61), (1.20;16.00), (1.20;0.37), (1.19;7.15), (0.00;5.56) |
| 229 | 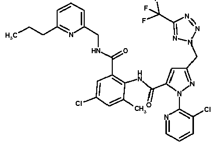 | 2,94 | 673 | (10.30;0.33), (8.89;0.71), (8.48;2.20), (8.48;2.32), (8.47;2.40), (8.47;2.36), (8.15;2.12), (8.15;2.13), (8.14;2.29), (8.13;2.21), (7.60;2.09), (7.59;2.08), (7.59;2.06), (7.58;2.04), (7.50;1.82), (7.47;2.13), (7.46;3.13), (7.46;6.59), (7.45;2.40), (7.31;1.75), (7.04;3.22), (7.04;2.73), (7.03;2.96), (7.02;2.51), (6.30;7.46), (4.38;3.94), (4.37;3.99), (3.35;206.57), (3.32;0.82), (2.66;2.99), (2.65;3.70), (2.64;3.12), (2.62;0.41), (2.61;0.55), (2.61;0.40), (2.52;1.06), (2.52;1.36), (2.52;1.34), (2.51;28.25), (2.51;62.03), (2.50;84.71), (2.50;61.62), (2.50;27.82), (2.42;0.47), (2.39;0.37), (2.39;0.52), (2.38;0.37), (2.16;11.60), (2.08;0.75), (1.69;0.50), (1.68;2.02), (1.67;3.30), (1.66;3.42), (1.64;2.10), (1.63;0.54), (0.92;0.33), (0.92;0.48), (0.91;7.50), (0.91;0.91), (0.90;0.99), (0.90;16.00), (0.89;0.80), (0.89;0.60), (0.89;7.02), (0.01;0.33), (0.00;11.45) |
| 230 | 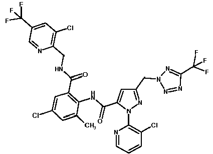 | 4,23 | 733 | (8.95;0.40), (8.82;0.73), (8.75;3.08), (8.75;3.01), (8.46;3.07), (7338.46;3.28), (8.46;3.68), (8.45;3.85), (8.43;3.18), (8.42;3.15), (8.16675;2.95), (8.15;2.96), (8.14;3.27), (8.14;3.14), (7.60;3.13), (7.59;3.07), (7.59;3.16), (7.58;3.15), (7.51;2.48), (7.51;2.89), (7.46;3.50), (7.45;3.10), (7.34;5.99), (6.28;11.40), (4.62;3.63), (4.61;3.67), (4.05;0.80), (3.40;0.40), (3.39;1.14), (3.38;1.16), (3.36;0.75), (3.34;219.93), (3.32;1.09), (2.62;0.52), (2.61;0.72), (2.61;0.51), (2.52;1.36), (2.52;1.76), (2.52;1.66), (2.51;36.55), (2.51;81.24), (2.50;111.56), (2.50;81.09), (2.50;36.67), (2.39;0.49), (2.39;0.71), (2.38;0.50), (2.16;16.00), (2.08;0.50), (1.36;0.48), (1.23;0.41), (1.10;1.09), (1.09;2.30), (1.08;1.11), (0.01;0.67), (0.00;25.09), (-0.01;0.70) |
| 231 | 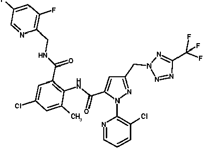 | 3,5 | 667 | (8.78;0.55), (8.77;0.46), (8.48;3.73), (8.48;3.88), (8.47;3.71), (8.47;3.68), (8.33;4.82), (8.33;4.93), (8.15;2.92), (8.15;2.92), (8.14;3.16), (8.14;3.00), (7.87;1.38), (7.87;1.31), (7.86;1.67), (7.86;1.78), (7.86;1.66), (7.85;1.60), (7.84;1.35), (7.84;1.25), (7.60;3.19), (7.59;3.11), (7.59;3.08), (7.58;3.16), (7.48;2.28), (7.37;3.07), (7.37;2.93), (7.30;2.41), (6.31;11.67), (5.76;2.28), (4.48;3.64), (4.48;3.69), (3.86;0.60), (3.61;0.50), (3.61;0.40), (3.60;1.22), (3.60;0.41), (3.59;0.53), (3.34;125.91), (3.32;1.03), (2.62;0.51), (2.61;0.71), (2.61;0.50), (2.52;1.38), (2.52;1.77), (2.52;1.71), (2.51;37.09), (2.51;81.57), (2.50;111.41), (2.50;80.88), (2.50;36.45), (2.39;0.50), (2.39;0.69), (2.38;0.49), (2.14;16.00), (2.13;0.58), (2.08;0.47), (1.77;0.53), (1.77;0.45), (1.76;1.50), (1.75;0.45), (1.75;0.50), (1.23;0.88), (0.01;0.81), (0.00;28.28), (-0.01;0.74) |
| 232 | 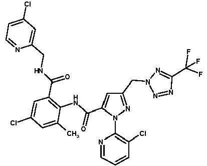 | 3,45 | 665 | (8.48;0.42), (8.47;0.45), (8.45;4.17), (8.45;5.94), (8.44;4.87), (8.44;4.76), (8.43;3.53), (8.11;1.57), (8.11;1.55), (8.10;1.67), (8.10;1.59), (7.60;3.72), (7.60;3.81), (7.59;2.71), (7.59;3.76), (7.59;3.66), (7.57;1.58), (7.56;1.58), (7.55;1.55), (7.55;1.52), (7.48;2.63), (7.38;3.54), (7.37;4.15), (7.37;3.37), (7.36;3.38), (7.36;3.25), (7.36;1.77), (7.35;2.48), (7.35;1.78), (7.28;0.49), (6.28;4.57), (4.44;3.31), (4.43;3.31), (4.25;0.72), (4.24;0.72), (3.81;16.00), (3.34;124.87), (2.62;0.53), (2.62;1.11), (2.61;1.54), (2.61;1.11), (2.61;0.53), (2.52;3.15), (2.52;3.95), (2.52;3.74), (2.51;77.87), (2.51;171.93), (2.50;234.47), (2.50;169.65), (2.50;75.37), (2.39;0.47), (2.39;1.05), (2.39;1.46), (2.38;1.03), (2.38;0.45), (2.18;0.42), (2.16;6.46), (2.08;1.10), (1.35;1.94), (1.23;0.41), (0.01;1.05), (0.00;37.26), (-0.01;1.03) |
| 233 | 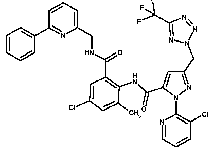 | 4,27 | 707 | (8.95;0.73), (8.48;3.03), (8.48;3.26), (8.47;3.31), (8.47;3.12), (8.14;3.17), (8.13;3.63), (8.13;2.14), (8.12;3.94), (8.11;4.21), (8.09;4.67), (8.09;5.98), (8.07;5.97), (7.89;0.35), (7.88;0.51), (7.86;1.33), (7.84;1.31), (7.83;1.32), (7.81;0.54), (7.78;2.66), (7.76;4.04), (7.70;2.89), (7.68;4.50), (7.67;2.12), (7.59;2.91), (7.58;2.93), (7.57;2.81), (7.56;2.77), (7.50;8.43), (7.49;6.73), (7.49;8.29), (7.47;3.91), (7.47;5.39), (7.45;2.76), (7.45;4.21), (7.45;2.67), (7.44;1.67), (7.43;4.14), (7.43;1.11), (7.41;1.61), (7.39;0.87), (7.32;3.07), (7.22;3.32), (7.21;3.01), (6.87;0.71), (6.28;10.29), (4.51;5.17), (4.50;5.07), (3.96;3.32), (3.87;0.33), (3.84;0.35), (3.80;0.37), (3.74;0.43), (3.72;0.45), (3.68;0.49), (3.66;0.53), (3.60;0.70), (3.32;2760.95), (3.30;39.06), (3.20;0.37), (2.70;0.39), (2.67;2.02), (2.67;2.68), (2.67;1.95), (2.66;1.10), (2.63;0.44), (2.63;0.49), (2.54;4.98), (2.51;149.64), (2.51;268.91), (2.50;343.69), (2.50;238.57), (2.49;113.85), (2.33;1.76), (2.33;2.27), (2.32;1.57), (2.32;0.77), (2.17;16.00), (2.07;1.17), (1.90;0.34), (1.36;8.51), (1.24;0.70), (0.89;0.70), (0.01;0.85), (0.00;14.62), (-0.01;0.53) |
| 234 | 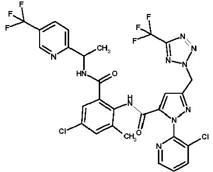 | 4,04 | 713 | (10.23;4.65), (8.81;5.18), (8.79;2.44), (8.47;3.02), (8.47;3.21), (8.46;3.29), (8.46;3.14), (8.15;2.88), (8.14;2.87), (8.13;3.24), (8.12;3.00), (8.03;1.65), (8.02;1.62), (8.01;1.78), (8.00;1.71), (7.60;3.01), (7.59;2.97), (7.58;2.87), (7.57;2.84), (7.55;2.65), (7.53;2.46), (7.50;3.24), (7.49;3.69), (7.43;3.94), (7.42;3.37), (7.32;7.60), (6.27;11.75), (5.05;1.21), (5.03;1.85), (5.01;1.21), (5.00;0.33), (3.38;0.65), (3.31;655.23), (3.29;6.72), (2.68;0.38), (2.67;0.64), (2.67;0.81), (2.67;0.62), (2.54;1.81), (2.51;48.22), (2.51;86.43), (2.50;110.15), (2.50;76.08), (2.49;36.39), (2.33;0.65), (2.33;0.78), (2.32;0.56), (2.17;16.00), (2.07;1.04), (1.37;8.60), (1.35;8.49), (0.00;3.84) |
| 235 | 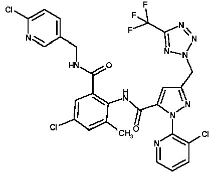 | 3,42 | 665 | (10.23;4.52), (8.86;1.19), (8.84;2.39), (8.83;1.19), (8.49;2.90), (8.48;3.11), (8.47;3.21), (8.47;3.02), (8.32;3.26), (8.31;3.18), (8.14;2.77), (8.14;2.78), (8.12;3.20), (8.12;2.98), (7.69;1.95), (7.68;1.97), (7.67;2.21), (7.66;2.10), (7.60;2.89), (7.59;2.94), (7.58;2.84), (7.57;2.70), (7.48;3.35), (7.48;3.49), (7.38;3.92), (7.38;3.52), (7.33;3.92), (7.31;8.11), (6.49;0.47), (6.32;0.34), (6.30;11.56), (4.34;4.72), (4.32;4.71), (3.74;0.35), (3.71;0.33), (3.66;0.37), (3.65;0.40), (3.63;0.42), (3.59;0.48), (3.55;0.55), (3.52;0.59), (3.48;0.88), (3.31;2204.85), (3.28;22.22), (3.18;0.36), (2.74;0.33), (2.71;0.36), (2.70;0.57), (2.67;2.22), (2.67;2.80), (2.66;2.05), (2.54;6.18), (2.51;157.75), (2.50;286.83), (2.50;369.79), (2.50;258.54), (2.49;126.48), (2.43;0.38), (2.33;1.75), (2.33;2.46), (2.32;1.72), (2.16;16.00), (2.07;3.54), (1.24;0.81), (0.00;16.09), (-0.01;0.80) |
| 236 | 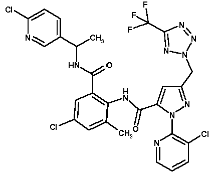 | 3,63 | 679 | (10.19;4.55), (8.74;2.33), (8.73;2.46), (8.49;3.08), (8.48;3.09), (8.47;3.19), (8.47;3.07), (8.37;3.55), (8.36;3.67), (8.14;2.86), (8.13;2.84), (8.12;3.20), (8.11;2.85), (7.75;1.98), (7.75;1.91), (7.73;2.25), (7.72;2.09), (7.60;3.02), (7.59;2.91), (7.58;2.76), (7.57;2.70), (7.47;3.28), (7.47;3.50), (7.37;3.86), (7.35;4.49), (7.34;6.42), (7.30;7.44), (6.49;0.34), (6.30;11.82), (6.28;0.37), (5.01;0.39), (5.00;1.30), (4.98;1.97), (4.96;1.33), (4.94;0.33), (3.75;0.34), (3.69;0.32), (3.67;0.36), (3.65;0.40), (3.63;0.43), (3.60;0.41), (3.57;0.49), (3.56;0.48), (3.55;0.51), (3.54;0.55), (3.52;0.54), (3.50;0.65), (3.50;0.64), (3.47;0.84), (3.44;1.03), (3.43;1.26), (3.41;1.61), (3.31;2246.20), (3.28;21.14), (3.23;0.73), (3.21;0.50), (2.70;0.58), (2.67;2.14), (2.67;2.89), (2.66;2.17), (2.61;0.74), (2.54;6.28), (2.51;162.23), (2.50;293.23), (2.50;375.62), (2.50;259.64), (2.49;124.90), (2.33;1.80), (2.33;2.49), (2.32;1.78), (2.16;16.00), (2.07;3.63), (1.33;8.06), (1.31;8.00), (1.24;0.94), (1.22;0.36), (0.00;18.55), (-0.01;0.77) |
| 237 | 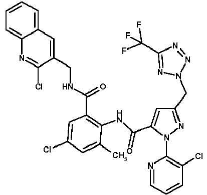 | 3.94 | 715 | (10.32;4.52), (8.96;1.17), (8.95;2.33), (8.94;1.11), (8.46;2.98), (8.46;3.23), (8.45;3.30), (8.45;3.10), (8.25;5.18), (8.14;0.40), (8.09;2.74), (8.09;2.74), (8.07;3.15), (8.07;2.84), (7.95;2.38), (7.93;2.94), (7.86;2.25), (7.84;2.55), (7.80;1.55), (7.79;1.50), (7.78;2.12), (7.77;2.60), (7.76;1.55), (7.75;1.44), (7.62;1.80), (7.62;1.86), (7.60;2.94), (7.58;1.51), (7.58;1.47), (7.56;4.44), (7.55;5.10), (7.54;3.03), (7.53;3.32), (7.52;4.04), (7.52;3.22), (7.27;7.62), (6.49;0.79), (6.22;11.54), (4.52;4.19), (4.51;4.12), (3.31;1733.27), (3.28;21.93), (3.20;0.87), (3.19;0.80), (3.16;0.66), (3.06;0.33), (2.70;0.40), (2.67;1.73), (2.67;2.22), (2.66;1.64), (2.66;0.97), (2.54;4.45), (2.52;10.46), (2.51;131.55), (2.50;242.61), (2.50;315.11), (2.50;220.67), (2.49;108.73), (2.41;0.65), (2.36;0.48), (2.34;1.09), (2.33;1.78), (2.33;2.37), (2.32;1.73), (2.32;0.99), (2.30;0.42), (2.28;0.32), (2.26;0.32), (2.21;16.00), (2.07;3.22), (1.40;0.39), (1.23;0.91), (0.01;0.87), (0.00;17.46), (-0.01;0.81) |

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60 µl Volumen), bestimmt. Als Lösungsmittel wurde d₆-DMSO verwendet, wobei als Referenz Tetramethylsilan (0.00 ppm) eingesetzt wurde. Die Beispiele in obiger Tabelle wurden in d₆-DMSO als Lösungsmittel aufgenommen, mit Ausnahme des Beispiels Nr. 39 (DMF), Nr. 51 (CDCl₃) und Nr. 69, welches in CD₃CN als Lösungsmittel aufgenommen wurde. Die Meßtemperatur beträgt 303K, falls d₆-DMSO als Lösungsmittel verwendet wird, und 298K, falls CD₃CN als Lösungsmittel verwendet wird.

In Einzelfällen wurden die Proben mit einem Bruker Avance II 600 oder III 600 bestimmt.

### Anwendungsbeispiele

### Beispiel 1

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung verwischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis),* die von allen Stadien der Grünen Pfirsichblattlaus (Myzus *persicae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100 % bei einer Aufwandmenge von 500g/ha: 10, 29, 30, 31, 37, 38, 39, 43, 57

Bei diesem Test zeigen z. B. die folgenden Verbindungen der Herstellungsbeispiele Wirkung von 80 % bei einer Aufwandmenge von 100g/ha: 6, 27, 35, 41, 48, 54, 72, 87

Bei diesem Test zeigen z. B. die folgenden Verbindungen der Herstellungsbeispiele Wirkung von 90 % bei einer Aufwandmenge von 100g/ha: 1, 7, 14, 20, 32, 44, 65, 97, 153, 193, 224, 231, 236

Bei diesem Test zeigen z. B. die folgenden Verbindungen der Herstellungsbeispiele Wirkung von 100 % bei einer Aufwandmenge von 100g/ha: 2, 3, 4, 15, 25, 26, 28, 34, 36, 42, 45, 46, 49, 50, 51, 58, 62, 70, 140, 150, 151, 152, 206

### Beispiel 2

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.
Bei diesem Test zeigen z. B. die folgenden Verbindungen der Herstellungsbeispiele Wirkung von 100 % bei einer Aufwandmenge von 500 g/ha: 29, 30, 31, 32, 33, 37, 38, 39, 40

Bei diesem Test zeigen z. B. die folgenden Verbindungen der Herstellungsbeispiele Wirkung von 100 % bei einer Aufwandmenge von 100 g/ha: 1, 2, 3, 4, 5, 6, 7, 8, 9, 10, 11, 12, 13, 14, 15, 20, 24, 25, 26, 27, 28, 34, 35, 36, 41, 42, 43, 44, 45, 46, 47, 48, 49, 50, 51, 52, 54, 56, 57, 58, 61, 62, 63, 64, 65, 66, 67, 68, 69, 70, 71, 72, 73, 74, 75, 76, 77, 81, 82, 83, 84, 85, 86, 87, 88, 89, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 134, 136, 137, 138, 139, 140, 141, 142, 143, 144, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 178, 180, 182, 183, 184, 185, 186, 187, 188, 189, 190, 191, 192, 193, 194, 195, 196, 197, 202, 203, 204, 205, 206, 207, 208, 209, 210, 211, 212, 213, 214, 215, 216, 217, 224, 225, 226, 228, 229, 230, 231, 232, 233, 234, 235, 236

### Beispiel 3

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (Zea *mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 500g/ha: 29, 30, 31, 32, 33, 37, 38, 39, 40

Bei diesem Test zeigen z. B. die folgenden Verbindungen der Herstellungsbeispiele Wirkung von 83 % bei einer Aufwandmenge von 100g/ha: 17, 178, 182

Bei diesem Test zeigen z. B. die folgenden Verbindungen der Herstellungsbeispiele Wirkung von 100 % bei einer Aufwandmenge von 100g/ha: 1, 2, 3, 4, 5, 6, 7, 8, 9, 10, 11, 12, 13, 14, 15, 16, 18, 19, 20, 21, 23, 24, 25, 26, 27, 28, 34, 35, 36, 41, 42, 43, 44, 45, 46, 47, 48, 49, 50, 52, 54, 55, 56, 57, 60, 61, 62, 63, 64, 65, 66, 67, 68, 69, 70, 71, 72, 73, 74, 75, 76, 77, 78, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 134, 136, 137, 138, 139, 140, 141, 142,143, 144, 146, 148, 149, 150, 151, 152, 153, 154, 155, 156, 157, 158, 159, 160, 161, 163, 164, 165, 166, 167, 168, 169, 170, 171, 172, 174, 175, 180, 181, 183, 184, 185, 186, 187, 188, 189, 190, 191, 192, 193, 194, 195, 196, 197, 202, 203, 204, 205, 206, 207, 208, 209, 210, 211, 212, 213, 214, 215, 216, 217, 221, 222, 223, 224, 225, 226,230, 231, 232, 233, 234, 235, 236

### Beispiel 4

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach 7 Tagen wird die Wirkung in % bestimmt. Die Wirkungskontrolle erfolgt auf Ablage fertiler Eier. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg/ Tier: 24, 26, 27, 30, 31, 33, 36, 37, 38, 40, 42, 43, 44, 65, 69

### Beispiel 5

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 48 h wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100 ppm: 24, 26, 27, 30, 31, 33, 36, 37, 38, 40, 42, 43, 44, 65, 69

### Beispiel 6

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica*-Adulten besetzt.

Nach 48 h wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 80% bei einer Aufwandmenge von 100 ppm: 26, 36, 37

Bei diesem Test zeigen z.B. die folgenden Verbindungen der Herstellungsbeispiele eine Wirkung von 90% bei einer Aufwandmenge von 100 ppm : 42

Bei diesem Test zeigen z.B. die folgenden Verbindungen der Herstellungsbeispiele eine Wirkung von 100% bei einer Aufwandmenge von 100 ppm : 38, 44

Bei diesem Test zeigen z.B. die folgenden Verbindungen der Herstellungsbeispiele eine Wirkung von 100% bei einer Aufwandmenge von 20 ppm : 27

Bei diesem Test zeigen z.B. die folgenden Verbindungen der Herstellungsbeispiele eine Wirkung von 80% bei einer Aufwandmenge von 20 ppm : 30, 65, 69

### Beispiel 7

### Phaedon cochleariae -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele überlegene Wirksamkeit gegenüber dem Stand der Technik: siehe Tabelle

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

| Substanz | Struktur | Objekt | Konzentration | % Wirkung |
|---|---|---|---|---|
| Beispiel 1 erfindungsgemäß | 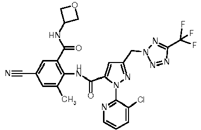 | MYZUPE | 20g/ha | 100 6d |
| Nr. 1 bekannt WO2007/144100 | 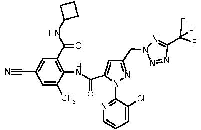 | MYZUPE | 20g/ha | 80 6d |
| Beispiel 29 erfindungsgemäß | 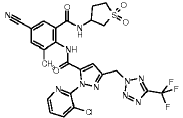 | MYZUPE | 100 g/ha | 100 6d |
| Nr. 2 bekannt WO2007/144100 | 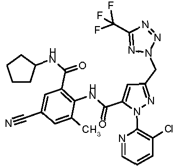 | MYZUPE | 100 g / ha | 70 6d |
| | | | | |
| Beispiel 36 erfindungsgemäß | 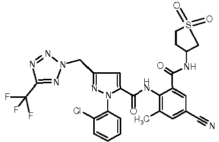 | SPODFR | 100g/ha | 100 7d |
| | | MYZUPE | 100g/ha | 100 6d |
| Nr. 3 bekannt WO2007/144100 | 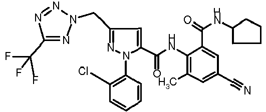 | SPODFR | 100g/ha | 83 7d |
| | | MYZUPE | 100g/ha | 0 6d |
| | | | | |
| Beispiel 2 erfindungsgemäß | 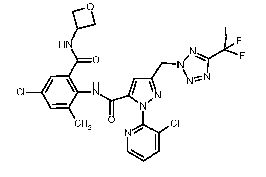 | PHAECO | 20g/ha | 100 7d |
| | | SPODFR | 20g/ha | 100 7d |
| | | MYZUPE | 100g/ha | 100 6d |
| Nr. 4 bekannt WO2007/144100 | 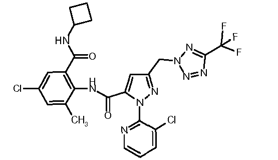 | PHAECO | 20g/ha | 33 7d |
| | | SPODFR | 20g/ha | 83 7d |
| | | MYZUPE | 100g/ha | 0 6d |

## Patentansprüche

1. Anthranilsäurediamid-Derivate der Formel (I) 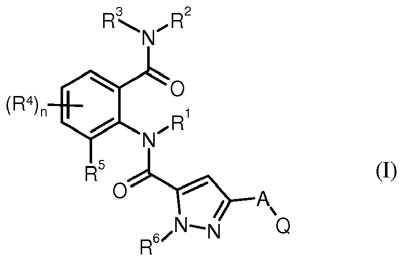 in welcher
R¹ für Wasserstoff steht,
R² für Wasserstoff steht,
R³ für einfach oder mehrfach, gleich oder verschieden substituiertes Methyl, Ethyl, Propyl, iso-Propyl, Cyclopropyl, Butyl, sec-Butyl und iso-Butyl steht, wobei die Substituenten unabhängig voneinander ausgewählt sind aus Aziridin, Oxiran, Thiiran, Azetidin, Oxetan, Thietan, Pyrrolidin, Pyrrolin, Pyrazolidin, Pyrazolin, Imidazolidin, Imidazolin, Tetrahydrofuran, Tetrahydrothiophen, Thiazolidin, Isothiazolidin, Piperidin, Piperazin, Hexahydropyridazin, Hexahydropyrimidin, Tetrahydropyran, Dioxan, Tetrahydrothiopyran, Morpholin, Thiomorpholin, Azepan, Diazepan, Oxepan, Dioxepan, Thiepan, Dithiepan, Pyrrolidon, Pyrrolidinon, Imidazolidon, Imidazolidinon, Triazolinon, Triazolidinon, Tetrazolinon, Tetrazolidinon, Thiazolon, Thiazolidinon, Oxazolon, Oxazolidinon Phenyl oder Naphthalin, welche einfach oder mehrfach, gleich oder verschieden substituiert sind mit Methyl, Ethyl, Propyl, iso-Propyl, Cyclopropyl, Fluor, Chlor, Brom, Cyano, Methoxy, Trifluormethyl, SF₅, Trifluormethoxy, Methylthio, Methylsulfonyl, Methoxycarbonyl, Methylcarbonyl oder Benzyl,
R³ weiterhin für einfach oder mehrfach, gleich oder verschieden substituiertes Methyl, Ethyl, Propyl, iso-Propyl, Butyl, sec-Butyl und iso-Butyl steht, wobei die Substituenten unabhängig voneinander ausgewählt sind aus Furan, Thiophen, Pyrazol, Triazol, Imidazol, Thiazol, Oxazol, Isoxazol, Isothiazol, Thiadiazol, Oxadiazol, Pyrrol, Pyridin, Pyrimidin, Pyridazin oder Pyrazin, welche einfach oder mehrfach, gleich oder verschieden substituiert sind mit Methyl, Ethyl, Propyl, iso-Propyl, Cyclopropyl, Fluor, Chlor, Brom, Cyano, Methoxy, Trifluormethyl, Trifluormethoxy, SF₅, Methylthio, Methylsulfonyl, Methoxycarbonyl, Methylcarbonyl oder Benzyl,
R³ weiterhin für substituiertes Phenyl, Furan, Thiophen, Pyrazol, Triazol, Imidazol, Thiazol, Oxazol, Isoxazol, Isothiazol, Thiadiazol, Oxadiazol, Pyrrol, Pyridin, Pyrimidin, Pyridazin oder Pyrazin steht, wobei die Substituenten unabhängig voneinander ausgewählt sind aus Methyl, Ethyl, Propyl, iso.Propyl, Cyclopropyl, Fluor, Chlor, Brom, Cyano, Methoxy, Trifluormethyl, SF₅, Trifluormethoxy, Methylthio, Methylsulfonyl, Methoxycarbonyl oder Methylcarbonyl,
R³ weiterhin für substituiertes Aziridin, Oxiran, Thiiran, Azetidin, Oxetan, Thietan, Pyrrolidin, Pyrrolin, Pyrazolidin, Pyrazolin, Imidazolidin, Imidazolidinon, Imidazolin, Tetrahydrofuran, Tetrahydrothiophen, Tetrahydrothiophendioxid, Thiazolin, Thiazolidin, Isothiazolidin, Piperidin, Piperazin, Hexahydropyridazin, Hexahydropyrimidin, Tetrahydropyran, Dihydrofuranon, Dioxan, Tetrahydrothiopyran, Morpholin, Thiomorpholin, Thiomorpholindioxid, Azepan, Diazepan, Oxepan, Dioxepan, Thiazolin, Thiepan, Dithiepan, Dihydrothiophenon, Cyclobuten, Cyclopenten, Cyclopentadien, Cyclohexen, Cyclohexadien steht, wobei die Substituenten unabhängig voneinander ausgewählt sind aus Methyl, Ethyl, Propyl, iso-Propyl, Cyclopropyl, Fluor, Chlor, Brom, Cyano, Methoxy, Trifluormethyl, Trifluormethoxy, Methylthio, Methylsulfonyl, Methoxycarbonyl, Methylcarbonyl oder Benzyl,
R⁴ für Wasserstoff, Methyl, Trifluormethyl, Cyano, Fluor, Chlor, Brom, Iod oder Trifluormethoxy steht. Ausserdem stehen zwei benachbarte Reste R⁴ für -(CH₂)₄-, oder-(CH=CH-)₂-,
R⁵ für Methyl, Fluor, Chlor, Brom oder Iod steht,
A für -CH₂-, -CH(CH₃), C(CH₃)₂, -CH₂CH₂-, -CH(CN)-, -CH₂O- oder -C(=O)-CH₂-steht,
n für 0 bis 3 steht
R⁶ für Methyl steht oder für den Rest 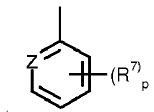 steht,
R⁷ für Fluor, Chlor oder Brom steht,
p für 1 steht,
Z für N, CCl oder CH steht,
Q für einen gegebenenfalls einfach oder mehrfach substituierten 5- oder 6-gliedrigen heteroaromatischen Ring der Reihe Q-36 bis Q-40 ,Q43, Q-58 bis Q-59, Q62, Q63, einen aromatisches 9-gliedriges annelliertes heterobicyclisches Ringsystem Q-54 bis Q-56 sowie für ein 5-gliedrigen heterocyclischen Ring Q-60 bis Q-61 steht, wobei die Substituenten unabhängig voneinander ausgewählt sein können aus C₁-C₃-Alkyl, C₁-C₃-Haloalkyl, C₁-C₂-Alkoxy, Halogen, Cyano, Hydroxy, Nitro oder C₁-C₂-Haloalkoxy,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können,

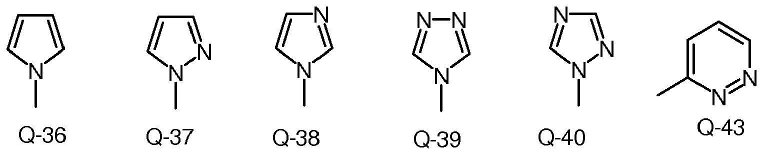

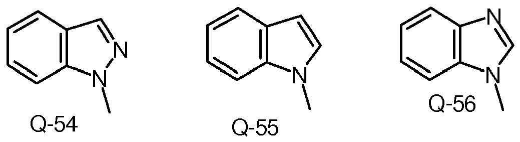

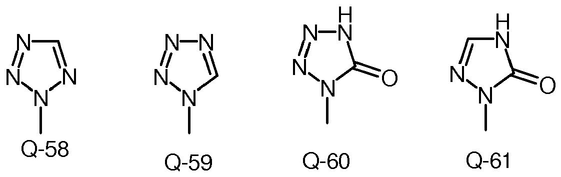

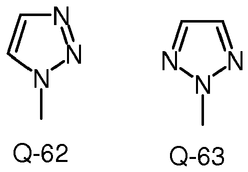

2. Verbindungen der Formel (I) gemäß Anspruch 1, **dadurch gekennzeichnet, dass**
A für CH₂, CH(CH₃), -CH₂O- oder -C(=O)-CH₂- steht,

3. Verbindungen der Formel (I) gemäß einem der Ansprüche 1 bis 2, **dadurch gekennzeichnet, dass**
R⁴ für Chlor oder Brom steht,
R⁴ weiterhin für Iod oder Cyano steht,
zwei benachbarte Reste R⁴ für -(CH=CH-)₂ stehen,
R⁵ für Methyl oder Chlor steht,
R⁷ für Chlor oder Brom steht,
Q für einen gegebenenfalls einfach oder mehrfach substituierten heteroaromatischen Ring der Reihe Q-37, Q-38, Q-39, Q-40, Q43, Q-58, Q-59, Q62 und Q63, sowie für ein 5-gliedrigen heterocyclischen Ring Q-60 steht, wobei die Substituenten unabhängig voneinander ausgewählt sein können aus Chlor, Fluor, Iod, Brom, Cyano, Trifluormethyl und Pentafluorethyl.,
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, C₁-C₄-Alkoxy, C₁-C₄-Haloalkoxy substituiert sein können.

4. Mischungen von Verbindungen der allgemeinen Formel (I) gemäß einem der Ansprüche 1 bis 3, in welchen Q für Q62 und Q63 steht, wobei das Verhältnis einer Verbindung der Formel (I), in welchen Q für Q62 steht, zu einer Verbindung der Formel (I), in welchen Q für Q63 steht, 60:40 bis 99:1 beträgt.

5. Mischungen von Verbindungen der allgemeinen Formel (I) gemäß einem der Ansprüche 1 bis 3, in welchen Q für Q58 und Q59 steht, wobei das Verhältnis einer Verbindung der Formel (I), in welchen Q für Q58 steht, zu einer Verbindung der Formel (I), in welchen Q für Q59 steht, 60:40 bis 99:1 beträgt.

6. Zusammensetzungen enthaltend mindestens eine Verbindung der Formel (I) oder eine Mischung von Verbindungen der Formel (I) gemäß einem der Ansprüche 1 bis 5 sowie mindestens ein Salz der Formel (XI) 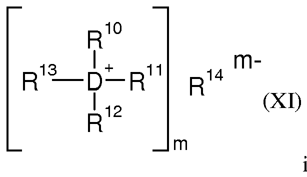 in welcher
D für Stickstoff oder Phosphor steht,
R¹⁰, R¹¹, R¹², and R¹³ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R¹⁴ für ein anorganisches oder organisches Anion steht.

7. Zusammensetzungen enthaltend mindestens eine Verbindung der Formel (I) oder eine Mischung von Verbindungen der Formel (I) gemäß einem der Ansprüche 1 bis 5 sowie mindestens einen Penetrationsförderer der Formel (XII)
R-O-(-AO)_{V}-R' (XII),
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AOfür einen Ethylenoxid-Rest, einen Propylenoxid-Rest, einen Butylenoxid-Rest oder für Gemische aus Ethylenoxid- und Propylenoxid-Resten oder Butylenoxid-Resten steht und
V für Zahlen von 2 bis 30 steht.

8. Agrochemische Zusammensetzungen enthaltend mindestens eine Verbindung der Formel (I) oder eine Mischung von Verbindungen der Formel (I) gemäß einem der Ansprüche 1 bis 5 oder eine Zusammensetzung gemäß Anspruch 6 oder 7, sowie Streckmittel und/oder oberflächenaktive Stoffe.

9. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1 bis 5 oder eine Zusammensetzung gemäß Anspruch 6 oder 7 mit Streckmitteln und/oder oberflächenaktiven Stoffen gemischt wird.

10. Verwendung einer Verbindung der allgemeinen Formel (I) oder einer Mischung von Verbindungen gemäß einem der Ansprüche 1 bis 5 oder einer Zusammensetzung gemäß einem der Ansprüche 6 bis 8 zur Bekämpfung tierischer Schädlinge ausgenommen zur therapeutischen Behandlung des tierischen oder menschlichen Körpers

11. Verfahren zur Bekämpfung tierischer Schädlinge- ausgenommen zur therapeutischen Behandlung des tierischen oder menschlichen Körpers, **dadurch gekennzeichnet, dass** man eine Verbindung der allgemeinen Formel (I) oder eine Mischung von Verbindungen der allgemeinen Formel (I) gemäß einem der Ansprüche 1 bis 5 oder eine Zusammensetzung gemäß einem der Ansprüche 6 bis 8 auf tierische Schädlinge und/oder phytopathogene Pilze und/oder deren Lebensraum und/oder Saatgut einwirken lässt.

## Claims

1. Anthranilic diamide derivatives of the formula (I) 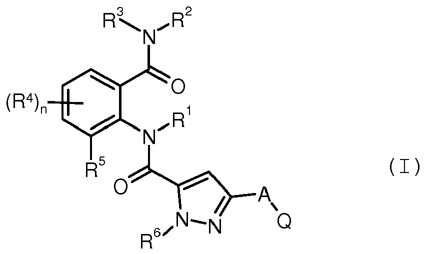 in which
R¹ represents hydrogen,
R² represents hydrogen,
R³ represents mono- or polysubstituted methyl, ethyl, propyl, isopropyl, cyclopropyl, butyl, sec-butyl and isobutyl, where the substituents are identical or different and independently of one another are selected from the group consisting of aziridine, oxirane, thiirane, azetidine, oxetane, thietane, pyrrolidine, pyrroline, pyrazolidine, pyrazoline, imidazolidine, imidazoline, tetrahydrofuran, tetrahydrothiophene, thiazolidine, isothiazolidine, piperidine, piperazine, hexahydropyridazine, hexahydropyrimidine, tetrahydropyran, dioxane, tetrahydrothiopyran, morpholine, thiomorpholine, azepane, diazepane, oxepane, dioxepane, thiepane, dithiepane, pyrrolidone, pyrrolidinone, imidazolidone, imidazolidinone, triazolinone, triazolidinone, tetrazolinone, tetrazolidinone, thiazolone, thiazolidinone, oxazolone, oxazolidinone, phenyl and naphthalene which are mono- or polysubstituted by identical or different substituents from the group consisting of methyl, ethyl, propyl, isopropyl, cyclopropyl, fluorine, chlorine, bromine, cyano, methoxy, trifluoromethyl, SF₅, trifluoromethoxy, methylthio, methylsulphonyl, methoxycarbonyl, methylcarbonyl and benzyl,
R³ furthermore represents mono- or polysubstituted methyl, ethyl, propyl, isopropyl, butyl, sec-butyl and isobutyl, where the substituents are identical or different and independently of one another are selected from the group consisting of furan, thiophene, pyrazole, triazole, imidazole, thiazole, oxazole, isoxazole, isothiazole, thiadiazole, oxadiazole, pyrrole, pyridine, pyrimidine, pyridazine and pyrazine which are mono- or polysubstituted by identical or different substituents from the group consisting of methyl, ethyl, propyl, isopropyl, cyclopropyl, fluorine, chlorine, bromine, cyano, methoxy, trifluoromethyl, trifluoromethoxy, SF₅, methylthio, methylsulphonyl, methoxycarbonyl, methylcarbonyl and benzyl,
R³ furthermore represents substituted phenyl, furan, thiophene, pyrazole, triazole, imidazole, thiazole, oxazole, isoxazole, isothiazole, thiadiazole, oxadiazole, pyrrole, pyridine, pyrimidine, pyridazine or pyrazine, where the substituents independently of one another are selected from the group consisting of methyl, ethyl, propyl, isopropyl, cyclopropyl, fluorine, chlorine, bromine, cyano, methoxy, trifluoromethyl, SF₅, trifluoromethoxy, methylthio, methylsulphonyl, methoxycarbonyl and methylcarbonyl,
R³ furthermore represents substituted aziridine, oxirane, thiirane, azetidine, oxetane, thietane, pyrrolidine, pyrroline, pyrazolidine, pyrazoline, imidazolidine, imidazolidinone, imidazoline, tetrahydrofuran, tetrahydrothiophene, tetrahydrothiophene dioxide, thiazoline, thiazolidine, isothiazolidine, piperidine, piperazine, hexahydropyridazine, hexahydropyrimidine, tetrahydropyran, dihydrofuranone, dioxane, tetrahydrothiopyran, morpholine, thiomorpholine, thiomorpholine dioxide, azepane, diazepane, oxepane, dioxepane, thiazoline, thiepane, dithiepane, dihydrothiophenone, cyclobutene, cyclopentene, cyclopentadiene, cyclohexene, cyclohexadiene, where the substituents independently of one another are selected from the group consisting of methyl, ethyl, propyl, isopropyl, cyclopropyl, fluorine, chlorine, bromine, cyano, methoxy, trifluoromethyl, trifluoromethoxy, methylthio, methylsulphonyl, methoxycarbonyl, methylcarbonyl and benzyl,
R⁴ represents hydrogen, methyl, trifluoromethyl, cyano, fluorine, chlorine, bromine, iodine or trifluoromethoxy. Moreover, two adjacent radicals R⁴ represent -(CH₂)₄- or -(CH=CH-)₂-,
R⁵ represents methyl, fluorine, chlorine, bromine or iodine,
A represents -CH₂-, -CH(CH₃), C(CH₃)₂, -CH₂CH₂-, -CH(CN)-, -CH₂O- or -C(=O)-CH₂-,
n represents 0 to 3,
R⁶ represents methyl or represents the radical 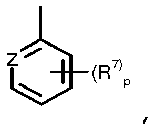
R⁷ represents fluorine, chlorine or bromine,
p represents 1,
Z represents N, CCl or CH,
Q represents an optionally mono- or polysubstituted 5- or 6-membered heteroaromatic ring from the group consisting of Q-36 to Q-40, Q-43, Q-58 to Q-59, Q-62, Q-63, an aromatic 9-membered fused heterobicyclic ring system Q-54 to Q-56 or a 5-membered heterocyclic ring Q-60 to Q-61 where the substituents independently of one another may be selected from the group consisting of C₁-C₃-alkyl, C₁-C₃-haloalkyl, C₁-C₂-alkoxy, halogen, cyano, hydroxyl, nitro and C₁-C₂-haloalkoxy, or where the substituents independently of one another may be selected from the group consisting of phenyl and a 5- or 6-membered
heteroaromatic ring, where phenyl or the ring may optionally be mono- or polysubstituted by identical or different substituents from the group consisting of C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, C₁-C₄-alkoxy, C₁-C₄-haloalkoxy,

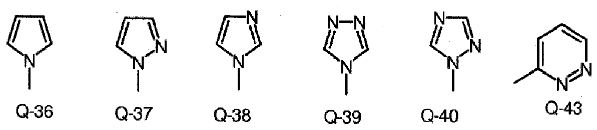

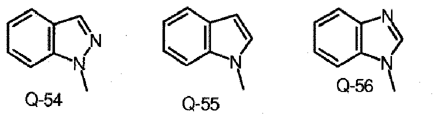

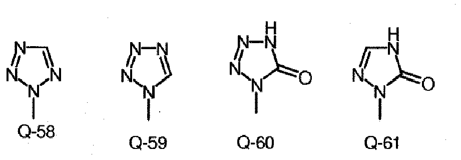

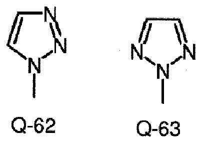

2. Compounds of the formula (I) according to Claim 1, **characterized in that**
A represents CH₂, CH(CH₃), -CH₂O- or -C(=O)-CH₂-.

3. Compounds of the formula (I) according to either Claim 1 or 2, **characterized in that**
R⁴ represents chlorine or bromine,
R⁴ furthermore represents iodine or cyano, two adjacent radicals R⁴ represent -(CH=CH-)₂,
R⁵ represents methyl or chlorine,
R⁷ represents chlorine or bromine,
Q represents an optionally mono- or polysubstituted heteroaromatic ring from the group consisting of Q-37, Q-38, Q-39, Q-40, Q-43, Q-58, Q-59, Q-62 and Q-63 or a 5-membered heterocyclic ring Q-60 where the substituents independently of one another may be selected from the group consisting of chlorine, fluorine, iodine, bromine, cyano, trifluoromethyl and pentafluoroethyl,
or wherein the substituents independently of one another may be selected from the group consisting of phenyl and a 5- or 6-membered heteroaromatic ring, where phenyl or the ring may optionally be mono- or polysubstituted by identical or different substituents from the group consisting of C₁-C₆-alkyl, C₂-C₆-alkenyl, C₂-C₆-alkinyl, C₃-C₆-cycloalkyl, C₁-C₆-haloalkyl, C₂-C₆-haloalkenyl, C₂-C₆-haloalkinyl, C₃-C₆-halocycloalkyl, halogen, CN, NO₂, C₁-C₄-alkoxy, C₁-C₄-haloalkoxy.

4. Mixtures of compounds of the general formula (I) according to any of Claims 1 to 3, in which Q is Q-62 and Q-63, the ratio of a compound of the formula (I) in which Q is Q-62 to a compound of the formula (I) in which Q is Q-63 being 60:40 to 99:1.

5. Mixtures of compounds of the general formula (I) according to any of Claims 1 to 3, in which Q is Q-58 and Q-59, the ratio of a compound of the formula (I) in which Q is Q-58 to a compound of the formula (I) in which Q is Q-59 being 60:40 to 99:1.

6. Compositions comprising at least one compound of the formula (I) or a mixture of compounds of the formula (I) according to any of Claims 1 to 5 and at least one salt of the formula (XI) 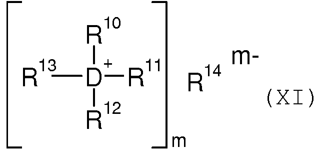 in which
D represents nitrogen or phosphorus,
R¹⁰, R¹¹, R¹² and R¹³ independently of one another represent hydrogen or in each case optionally substituted C₁-C₈-alkyl or mono- or polyunsaturated, optionally substituted C₁-C₈-alkylene where the substituents may be selected from the group consisiting of halogen, nitro and cyano,
m represents 1, 2, 3 or 4,
R¹⁴ represents an inorganic or organic anion.

7. Compositions comprising at least one compound of the formula (I) or a mixture of compounds of the formula (I) according to any of Claims 1 to 5 and at least one penetrant of the formula (XII) R-O-(-AO)_{V}-R' (XII),
in which
R represents straight-chain or branched alkyl having 4 to 20 carbon atoms,
R' represents hydrogen, methyl, ethyl, n-propyl, isopropyl, n-butyl, isobutyl, tert-butyl, n-pentyl or n-hexyl,
AO represents an ethylene oxide radical, a propylene oxide radical, a butylene oxide radical or represents mixtures of ethylene oxide and propylene oxide radicals or butylene oxide radicals and
V represents a number from 2 to 30.

8. Agrochemical compositions comprising at least one compound of the formula (I) or a mixture of compounds of the formula (I) according to any of Claims 1 to 5 or a composition according to Claim 6 or 7, and also extenders and/or surfactants.

9. Process for producing agrochemical compositions, **characterized in that** at least one compound of the general formula (I) or a mixture of compounds of the general formula (I) according to any of Claims 1 to 5 or a composition according to Claim 6 or 7 is mixed with extenders and/or surfactants.

10. Use of a compound of the general formula (I) or a mixture of compounds according to any of Claims 1 to 5 or of a composition according to any of Claims 6 to 8 for controlling animal pests, except for the therapeutic treatment of the animal or human body.

11. Method for controlling animal pests, except for the therapeutic treatment of the animal or human body, **characterized in that** a compound of the general formula (I) or a mixture of compounds of the general formula (I) according to any of Claims 1 to 5 or a composition according to any of Claims 6 to 8 is allowed to act on animal pests and/or phytopathogenic fungi and/or their habitat and/or seed.

## Revendications

1. Dérivés de diamide d'acide anthranilique de formule (I) 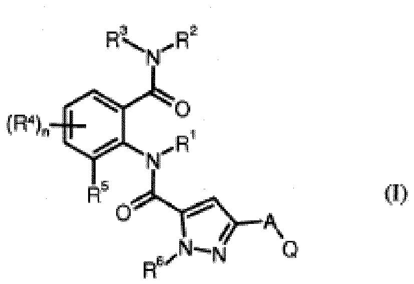 dans laquelle
R¹ représente un atome d'hydrogène,
R² représente un atome d'hydrogène,
R³ représente un groupe méthyle, éthyle, propyle, isopropyle, cyclopropyle, butyle, sec-butyle et isobutyle, portant un ou plusieurs substituants identiques ou différents, les substituants étant choisis indépendamment les uns des autres parmi aziridine, oxirane, thiirane, azétidine, oxétane, thiétane, pyrrolidine, pyrroline, pyrazolidine, pyrazoline, imidazolidine, imidazoline, tétra-hydrofurane, tétrahydrothiophène, thiazolidine, isothiazolidine, pipéridine, pipérazine, hexa-hydropyridazine, hexahydropyrimidine, tétra-hydropyrane, dioxane, tétrahydrothiopyrane, morpholine, thiomorpholine, azépane, diazépane, oxépane, dioxépane, thiépane, dithiépane, pyrrolidone, pyrrolidinone, imidazolidone, imidazolidinone, triazolinone, triazolidinone, tétrazolinone, tétrazolidinone, thiazolone, thiazolidinone, oxazolone, oxazolidinone, phényle ou naphthalène, qui portent un ou plusieurs substituants identiques ou différents méthyle, éthyle, propyle, isopropyle, cyclopropyle, fluoro, chloro, bromo, cyano, méthoxy, trifluorométhyle, SF₅, trifluorométhoxy, méthylthio, méthylsulfonyle, méthoxycarbonyle, méthylcarbonyle ou benzyle,
R³ en outre représente un groupe méthyle, éthyle, propyle, isopropyle, butyle, sec-butyle et isobutyle, portant un ou plusieurs substituants identiques ou différents, les substituants étant choisis indépendamment les uns des autres parmi furane, thiophène, pyrazole, triazole, imidazole, thiazole, oxazole, isoxazole, isothiazole, thiadiazole, oxadiazole, pyrrole, pyridine, pyrimidine, pyridazine ou pyrazine, qui portent un ou plusieurs substituants identiques ou différents méthyle, éthyle, propyle, isopropyle, cyclopropyle, fluoro, chloro, bromo, cyano, méthoxy, trifluorométhyle, trifluorométhoxy, SF₅, méthylthio, méthylsulfonyle, méthoxycarbonyle, méthylcarbonyle ou benzyle,
R³ en outre représente un groupe phényle, furane, thiophène, pyrazole, triazole, imidazole, thiazole, oxazole, isoxazole, isothiazole, thiadiazole, oxadiazole, pyrrole, pyridine, pyrimidine, pyridazine ou pyrazine substitué, les substituants étant choisis indépendamment les uns des autres parmi méthyle, éthyle, propyle, isopropyle, cyclopropyle, fluoro, chloro, bromo, cyano, méthoxy, trifluorométhyle, SF₅, trifluorométhoxy, méthylthio, méthylsulfonyle, méthoxycarbonyle ou méthylcarbonyle,
R³ en outre représente un groupe aziridine, oxirane, thiirane, azétidine, oxétane, thiétane, pyrrolidine, pyrroline, pyrazolidine, pyrazoline, imidazolidine, imidazolidinone, imidazoline, tétrahydrofurane, tétrahydrothiophène, tétrahydro-thiophènedioxyde, thiazoline, thiazolidine, isothiazolidine, pipéridine, pipérazine, hexa-hydropyridazine, hexahydropyrimidine, tétra-hydropyrane, dihydrofuranone, dioxane, tétra-hydrothiopyrane, morpholine, thiomorpholine, thio-morpholinedioxyde, azépane, diazépane, oxépane, dioxépane, thiazoline, thiépane, dithiépane, dihydrothiophénone, cyclobutène, cyclopentène, cyclopentadiène, cyclohexène, cyclohexadiène substitué, les substituants étant choisis indépendamment les uns des autres parmi méthyle, éthyle, propyle, isopropyle, cyclopropyle, fluoro, chloro, bromo, cyano, méthoxy, trifluorométhyle, trifluorométhoxy, méthylthio, méthylsulfonyle, méthoxycarbonyle, méthylcarbonyle ou benzyle,
R⁴ représente un atome d'hydrogène, le groupe méthyle, trifluorométhyle, cyano, un atome de fluor, de chlore, de brome, d'iode ou le groupe trifluorométhoxy ; en outre, deux radicaux R⁴ voisins représentent -(CH₂)₄- ou -(CH=CH-)₂-,
R⁵ représente le groupe méthyle, un atome de fluor, de chlore, de brome ou d'iode,
A représente -CH₂-, -CH(CH₃), C(CH₃)₂, -CH₂CH₂-, -CH(CN)-, -CH₂O- ou -C(=O)-CH₂-,
n représente 0 à 3
R⁶ représente le groupe méthyle ou le radical 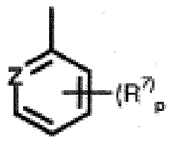
R⁷ représente un atome de fluor, chlore ou brome,
p représente 1,
Z représente N, CCl ou CH,
Q représente un cycle hétéroaromatique à 5 ou 6 chaînons de la série Q-36 à Q-40, Q43, Q-58 et Q-59, Q-62, Q-63, éventuellement une ou plusieurs fois substitué, un système cyclique hétéro-bicyclique condensé aromatique à 9 chaînons Q-54 à Q-56, ainsi qu'un cycle hétérocyclique à 5 chaînons Q-60 et Q-61, les substituants pouvant être choisis indépendamment les uns des autres parmi alkyle en C₁-C₃, halogénoalkyle(C₁-C₃), alcoxy en C₁-C₂, halogéno, cyano, hydroxy, nitro ou halogénoalcoxy(C₁-C₂),
ou les substituants pouvant être choisis indépendamment les uns des autres parmi phényle ou un cycle hétéroaromatique à 5 ou 6 chaînons, le groupe phényle ou le cycle pouvant éventuellement porter un ou plusieurs substituants identiques ou différents alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogéno-cycloalkyle(C₃-C₆), halogéno, CN, NO₂, alcoxy en C₁-C₄, halogénoalcoxy(C₁-C₄),

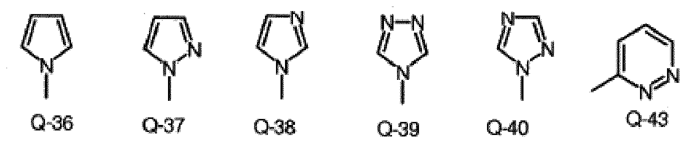

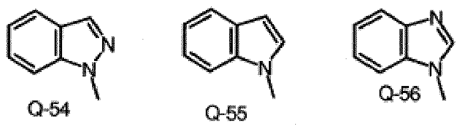

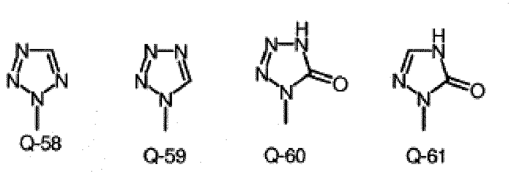

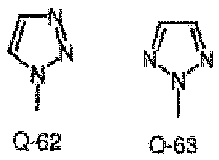

2. Composés de formule (I) selon la revendication 1, **caractérisés en ce que**
A représente CH₂, CH(CH₃), -CH₂O- ou -C(=O)-CH₂-,

3. Composés de formule (I) selon l'une quelconque des revendications 1 et 2, **caractérisés en ce que**
R⁴ représente un atome de chlore ou de brome,
R⁴ en outre représente un atome d'iode ou le groupe cyano,
deux radicaux R⁴ voisins représentent -(CH=CH-)₂,
R⁵ représente le groupe méthyle ou un atome de chlore,
R⁷ représente un atome de chlore ou de brome,
Q représente un cycle hétéroaromatique de la série Q-37, Q-38, Q-39, Q-40, Q-43, Q-58, Q-59, Q-62 et Q-63, éventuellement une ou plusieurs fois substitué, ainsi qu'un cycle hétérocyclique à 5 chaînons Q-60, les substituants pouvant être choisis indépendamment les uns des autres parmi chloro, fluoro, iodo, bromo, cyano, trifluorométhyle et pentafluoroéthyle,
ou les substituants pouvant être choisis indépendamment les uns des autres parmi phényle ou un cycle hétéroaromatique à 5 ou 6 chaînons, le groupe phényle ou le cycle pouvant éventuellement porter un ou plusieurs substituants identiques ou différents alkyle en C₁-C₆, alcényle en C₂-C₆, alcynyle en C₂-C₆, cycloalkyle en C₃-C₆, halogénoalkyle(C₁-C₆), halogéno-alcényle(C₂-C₆), halogénoalcynyle(C₂-C₆), halogéno-cycloalkyle(C₃-C₆), halogéno, CN, NO₂, alcoxy en C₁-C₄, halogénoalcoxy (C₁-C₄).

4. Mélanges de composés de formule générale (I) selon l'une quelconque des revendications 1 à 3, dans lesquels Q représente Q62 et Q63, le rapport d'un composé de formule (I), dans lequel Q représente Q62, à un composé de formule (I), dans lequel Q représente Q63, valant de 60:40 à 99:1.

5. Mélanges de composés de formule générale (I) selon l'une quelconque des revendications 1 à 3, dans lesquels Q représente Q58 et Q59, le rapport d'un composé de formule (I), dans lequel Q représente Q58, à un composé de formule (I), dans lequel Q représente Q59, valant de 60:40 à 99:1.

6. Compositions contenant au moins un composé de formule (I) ou un mélange de composés de formule (I) selon l'une quelconque des revendications 1 à 5, ainsi qu'au moins un sel de formule (XI) 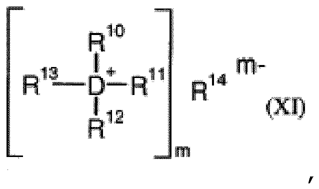 dans laquelle
D représente un atome d'azote ou de phosphore,
R¹⁰, R¹¹, R¹² et R¹³ représentent indépendamment les uns des autres un atome d'hydrogène ou un groupe alkyle en C₁-C₈ chaque fois éventuellement substitué ou alkylène en C₁-C₈ une ou plusieurs fois insaturé, éventuellement substitué, les substituants pouvant être choisis parmi halogéno, nitro et cyano,
m représente 1, 2, 3 ou 4,
R¹⁴ représente un anion organique ou inorganique.

7. Compositions contenant au moins un composé de formule (I) ou un mélange de composés de formule (I) selon l'une quelconque des revendications 1 à 5, ainsi qu'au moins un agent favorisant la pénétration, de formule (XII)
R-O-(-AO)_{V}-R' (XII),
dans laquelle
R représente un groupe alkyle à chaîne droite ou ramifié ayant de 4 à 20 atomes de carbone,
R' représente un atome d'hydrogène, le groupe méthyle, éthyle, n-propyle, isopropyle, n-butyle, isobutyle, tert-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène et
v représente des nombres de 2 à 30.

8. Compositions agrochimiques contenant au moins un composé de formule (I) ou un mélange de composés de formule (I) selon l'une quelconque des revendications 1 à 5 ou une composition selon la revendication 6 ou 7, ainsi que des excipients et/ou des substances tensioactives.

9. Procédé pour la préparation de compositions agrochimiques, **caractérisées en ce qu'**on mélange au moins un composé de formule générale (I) ou un mélange de composés de formule générale (I) selon l'une quelconque des revendications 1 à 5 ou une composition selon la revendication 6 ou 7 avec des excipients et/ou des substances tensioactives.

10. Utilisation d'un composé de formule générale (I) ou d'un mélange de composés selon l'une quelconque des revendications 1 à 5 ou d'une composition selon l'une quelconque des revendications 6 à 8, pour la lutte contre des ravageurs animaux, à l'exclusion du traitement thérapeutique de l'organisme humain ou animal.

11. Procédé pour la lutte contre des ravageurs animaux - à l'exclusion du traitement thérapeutique de l'organisme humain ou animal, **caractérisé en ce qu'**on fait agir sur des ravageurs animaux et/ou des champignons phytopathogènes et/ou leur habitat et/ou des semences un composé de formule générale (I) ou un mélange de composés de formule générale (I) selon l'une quelconque des revendications 1 à 5 ou une composition selon l'une quelconque des revendications 6 à 8.
